# EUROPÄISCHE PATENTSCHRIFT

(11) **EP 2 556 073 B1**
(45) Veröffentlichungstag und Bekanntmachung des Hinweises auf die Patenterteilung: **23.07.2014**
(21) Anmeldenummer: 11715187.8
(22) Anmeldetag: 04.04.2011
(51) Int. Cl.: C07D 471/04, C07D 487/04, C07D 498/04, C07D 513/04, A01N 43/56

(54) **BICYCLISCHE PYRIDINYLPYRAZOLE**
BICYCLIC PYRIDINYL PYRAZOLES
PYRIDINYLPYRAZOLE BICYCLIQUE

(30) Priorität: 07.04.2010 US 321704 P; 07.04.2010 EP 10159210
(43) Veröffentlichungstag der Anmeldung: 13.02.2013
(73) Patentinhaber: Bayer Intellectual Property GmbH, 40789 Monheim (DE)
(72) Erfinder: MATTES, Amos, 40674 Langenfeld (DE); HELMKE, Hendrik, 65835 Liederbach (DE); HILLEBRAND, Stefan, 41462 Neuss (DE); PERIS, Gorka, 50733 Köln (DE); SUDAU, Alexander, 42799 Leichlingen (DE); RODEFELD, Lars, 51375 Leverkusen (DE); GAUGER, Stefan, 51377 Leverkusen (DE); BENTING, Jürgen, 42799 Leichlingen (DE); DAHMEN, Peter, 41470 Neuss (DE); MEISSNER, Ruth, 51375 Leverkusen (DE); WACHENDORFF-NEUMANN, Ulrike, 56566 Neuwied (DE); HADANO, Hiroyuki, Shimotsuke-shi Tochigi 329-0434 (JP)
(74) Vertreter: BIP Patents
(86) Internationale Anmeldenummer: PCT/EP2011/055153
(87) Internationale Veröffentlichungsnummer: WO 2011/124539

(56) Entgegenhaltungen:
- WO-A1-02/094833
- WO-A2-2008/024978
- WO-A2-2009/076440

## Beschreibung

Die vorliegende Erfindung betrifft neue bicyclische Pyridinylpyrazole, mehrere Verfahren zu deren Herstellung und deren Verwendung zur Bekämpfung unerwünschter Mikroorganismen im Pflanzenschutz und Materialschutz sowie zur Reduktion von Mykotoxinen in Pflanzen und Pflanzenteilen. Die vorliegende Erfindung betrifft weiterhin ein Verfahren zur Bekämpfung phytopathogener Pilze sowie zur Reduktion von Mykotoxinen in Pflanzen und Pflanzenteilen im Pflanzenschutz sowie Pflanzenschutzmittel enthaltend bicyclische Pyridinylpyrazole.

Es ist bereits bekannt, dass bestimmte Arylpyrazole fungizide Eigenschaften besitzen (vgl. z.B. WO 2009/076440, WO 2003/049542 und WO 2001/03015). Die Wirksamkeit der dort beschriebenen Stoffe ist gut, lässt aber in manchen Fällen zu wünschen übrig.

In WO 2002/094833 werden bestimmte Heteroaryl substituierte Pyrazole beschrieben, die medizinisch genutzt werden können, hier als TGF-beta Signaltransduktionsinhibitoren. Ähnliche Verbindungen sind auch beschrieben in WO 1998/052937, WO 2002/094833, EP-A 1 553 096, WO 2004/029043, WO 1998/052940, WO 2000/031063, WO 1995/031451, WO 2002/057265 und WO 2000/039116. Eine Wirkung auf pilzliche Pathogene wird jedoch nicht beschrieben.

In WO 2007/105058 werden bestimmte Heteroaryl substituierte Pyrazole beschrieben, die als Modulatoren oder Inhibitoren des humanen Raf-Enzyms verwendet werden können. Die Wirkung auf pilzliche Pathogene wird jedoch nicht beschrieben.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die erfmdungsgemäßen bicyclischen Pyridinylpyrazole die genannten Aufgaben zumindest in Teilaspekten lösen und sich als Pflanzenschutzmittel, insbesondere als Fungizide eignen.

Gegenstand der Erfindung sind Verbindungen der Formel (**I**), 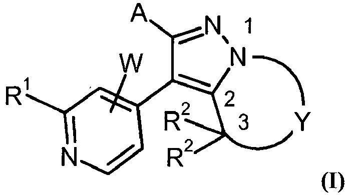 in welcher die Symbole folgende Bedeutungen haben:
- Y: bildet zusammen mit dem angrenzenden Stickstoffatom "1" und den beiden Kohlenstoffatomen "2" und "3" einen 5- bis 7-gliedrigen nicht aromatischen heterocyclischen Ring, dessen weitere Ringglieder aus der Gruppe C(R²)₂, O, S, NR³, C(R²)=C(R²), C(R²)=N, N=N, C(=O), C(=S), C(=NR⁴), S(=O)ₚ(=NR⁴)_{q} und SiR^{5a}R^{5b} ausgewählt sind;
- R²: steht jeweils unabhängig voneinander für H, Halogen, Cyano, Hydroxy, -CHO, -NHCHO, -N₃, - N=C=O, -N=C=S, -SH, -C(=O)NH₂, -C(=O)NHCN, -C(=O)OR⁶, -C(=O)NHOR^{6a}, C₁-C₅-Alkyl, C₂-C₅-Alkenyl, C₂-C₅-Alkynyl, C₃-C₆-Cycloalkyl, C₃-C₆-Halocycloalkyl, C₃-C₆-Cycloalkenyl, C₁-C₅-Haloalkyl, C₁-C₅-Alkoxy, C₁-C₅-Haloalkoxy, C₃-C₆-Cycloalkoxy, C₂-C₅-Alkenyloxy, C₃-C₅-Haloalkenyloxy, C₂-C₅-Alkynyloxy, C₂-C₅-Alkylcarbonyl, C₂-C₅-Alkylcarbonyloxy, C₂-C₅-Haloalkylcarbonyloxy, C₃-C₅-Alkoxycarbonylalkoxy, C₁-C₅-Alkylthio, C₁-C₅-Haloalkylthio, C₃-C₆-Cycloalkylthio, C₂-C₅-Alkyl(thiocarbonyl), C₂-C₅-Alkylthio(thiocarbonyl), C₁-C₅-Alkylsulfinyl, C₁-C₅-Haloalkylsulfinyl, C₃-C₆-Cycloalkylsulfinyl, C₁-C₅-Alkylsulfonyl, C₁-C₅-Haloalkylsulfonyl, C₃-C₆-Cycloalkylsulfonyl, C₃-C₅-Trialkylsilyl, C₃-C₅-Halotrialkylsilyl, C₁-C₅-Alkylamino, C₂-C₅-Haloalkylamino, C₃-C₆-Cycloalkylamino, C₂-C₅-Dialkylamino oder C₃-C₅-Halodialkylamino;
- R³: steht für H, -CN, -C(=O)NH₂, -C(=O)NHCN, -CHO, -NHCHO, -C(=O)OR⁶, -C(=O)NHOR^{6a}, Hydroxy, C₁-C₅-Alkyl, C₂-C₅-Alkenyl, C₂-C₅-Alkynyl, C₃-C₆-Cycloalkyl, C₃-C₆-Halocycloalkyl, C₃-C₆-Cycloalkenyl, C₄-C₁₀-Cycloalkylalkyl, C₄-C₇-Alkylcycloalkyl, C₅-C₇-Alkylcycloalkylalkyl, C₂-C₅-Haloalkyl, C₁-C₄-Alkylcarbonyl, C₁-C₄-Haloalkylcarbonyl, C₃-C₇-Cycloalkylcarbonyl, C₁-C₆-Alkoxycarbonyl, C₁-C₄-Haloalkoxy-carbonyl, C₃-C₆-Cycloalkoxycarbonyl, C₂-C₆-Alkoxyalkylcarbonyl, C₂-C₆-Alkoxyalkoxycarbonyl, C₁-C₆-(Alkylthio)carbonyl, C₁-C₆-Alkoxy(thio-carbonyl), C₁-C₆-Alkyl(thiocarbonyl), C₁-C₆-Alkylthio(thiocarbonyl), C₁-C₆-Alkylaminocarbonyl, C₃-C₆-Cycloalkylaminocarbonyl, C₂-C₆-Dialkyl-aminocarbonyl, C₁-C₆-Alkylamino(thiocarbonyl), C₂-C₆-Dialkylamino-(thiocarbonyl), C₂-C₆-Alkoxy(alkyl)aminocarbonyl, C₁-C₅-Alkoxy, C₁-C₅-Haloalkoxy, C₁-C₅-Alkylthio, C₁-C₅-Haloalkylthio, C₃-C₆-Cycloalkylthio, C₁-C₅-Alkylaminosulfonyl, C₃-C₅-Trialkylsilyl oder C₃-C₅-Halotrialkyl-silyl;
- R⁴: steht jeweils für H, Cyano, Amino, Hydroxy, C₁-C₆-Alkyl, C₃-C₆-Cycloalkyl, C₁-C₄-Alkylcarbonyl, C₁-C₄-Haloalkylcarbonyl, C₁-C₄-Alkoxy, Phenyl oder Benzoyl;
- R^{5a},R^{5b}: stehen unabhängig voneinander für C₁-C₄-Alkyl, C₂-C₅-Alkenyl, C₂-C₅-Alkynyl, C₃-C₅-Cycloalkyl, C₃-C₆-Halocycloalkyl, C₄-C₇-Cycloalkylalkyl, C₄-C₇-Alkylcycloalkyl, C₅-C₇-Alkylcycloalkylalkyl, C₁-C₅-Haloalkyl, C₁-C₅-Alkoxy oder C₁-C₅-Haloalkoxy;
- R⁶: steht jeweils für H, C₁-C₆-Allcyl, C₁-C₆-Haloalkyl, C₃-C₆-Cycloallcyl, C₄-C₇-Cycloalkylalkyl, C₄-C₇-Alkylcycloalkyl oder Benzyl;
- R^{6a}: steht jeweils für C₁-C₆-Alkyl, C₁-C₆-Haloalkyl, C₃-C₆-Cycloalkyl, C₄-C₇-Cycloalkylalkyl oder C₄-C₇-Alkylcycloalkyl;
- A: steht für einen Phenylring, der gegebenenfalls einfach oder mehrfach mit R⁷ substituiert sein kann oder für einen Thiophenylring der gegebenenfalls einfach oder mehrfach mit R⁸ substituiert sein kann;
- R⁷: steht unabhängig voneinander für Halogen, C₁-C₆-Alkyl, C₂-C₆-Alkenyl, C₂-C₆-Alkynyl, C₃-C₆-Cycloalkyl, C₃-C₆-Halocycloalkyl, C₄-C₇-Alkylcycloalkyl, C₁-C₆-Haloalkyl, C₂-C₆-Haloalkenyl, Cyano, Nitro, C₁-C₆-Alkoxy, C₁-C₆-Haloalkoxy, C₁-C₆-Alkylthio, C₁-C₆-Alkylsulfinyl, C₁-C₆-Alkylsulfonyl, C₁-C₆-Haloalkylthio, C₁-C₆-Haloalkylsulfinyl, C₁-C₆-Haloalkylsulfonyl, C₁-C₆-Alkylamino, C₂-C₆-Dialkylamino, C₁-C₆- Alkylcarbonyl, C₁-C₆-Alkoxycarbonyl, C₁-C₆-Alkylaminocarbonyl, C₂-C₆-Dialkylaminocarbonyl oder C₃-C₆-Trialkylsilyl;
- R⁸: steht unabhängig voneinander für Halogen, Cyano, C₁-C₃-Alkyl; C₁-C₃-Alkoxy, C₁-C₃-Haloalkyl, C₁-C₃-Haloalkoxy,
- R¹: steht für H, Halogen, Cyano, Hydroxy, C₁-C₄-Alkyl, CONR^{9a}R^{9b}, COOH, COOR¹², -NR^{9a}R^{9b}, - N(R^{9b})COR^{9a}, -N(R^{9b})CSR^{9a}, -N(R^{9b})COOR¹², -N(R^{9b})SO₂R¹², -NR¹⁰-NR^{11a}R^{11b}, -S(O)ₘR¹², -OR¹², - N=CR^{13a}R^{13b} oder -NR¹⁰N=CR^{14a}R^{14b};
- R^{9a} und R^{11a}: stehen unabhängig voneinander jeweils für H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₂-C₆-Alkoxyalkyl, C₃-C₆-Alkoxyalkoxyalkyl, C₃-C₆-Alkoxyalkenyl, C₃-C₆-Alkoxyalkynyl, C₃-C₆-Dialkoxyalkyl, C₄-C₁₀-Trialkoxyalkyl, C₂-C₆-Haloalkoxyalkyl, C₂-C₆-Alkoxyhaloalkyl, C₂-C₆-Haloalkoxyhaloalkyl, C₁-C₆-Hydroxyalkyl, C₁-C₁₀-Cyanoalkyl, C₂-C₆-Alkylthioalkyl, C₂-C₆-Alkylsulfinylalkyl, C₃-C₆-Alkylaminoalkyl, C₃-C₆-Haloalkylaminoalkyl, C₅-C₁₀-Cycloalkylaminoalkyl, C₄-C₁₀-Dialkylaminoalkyl, C₄-C₁₀-Halodialkylaminoalkyl, C₅-C₁₀-Cycloalkyl(alkyl)aminoalkyl, oder - (CR^{15a}R^{15b})ₘR¹⁶;
- R^{9b} und R^{11b}: stehen unabhängig voneinander jeweils für H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₂-C₆-Alkoxyalkyl, C₃-C₆-Alkoxyalkoxyalkyl, C₃-C₆-Alkoxyalkenyl, C₃-C₆-Alkoxyalkynyl, C₃-C₆-Dialkoxyalkyl, C₄-C₁₀-Trialkoxyalkyl, C₂-C₆-Haloalkoxyalkyl, C₂-C₆-Alkoxyhaloalkyl, C₂-C₆-Haloalkoxyhaloalkyl, C₁-C₆-Hydroxyalkyl, C₁-C₁₀-Cyanoalkyl, C₂-C₆-Alkylthioalkyl, C₂-C₆-Alkylsulfinylalkyl, C₃-C₆₋Alkylaminoalkyl, C₃-C₆-Haloalkylaminoalkyl, C₅-C₁₀-Cycloalkylaminoalkyl, C₄-C₁₀-Dialkylaminoalkyl, C₄-C₁₀-Halodialkylaminoalkyl, C₅-C₁₀-Cycloalkyl(alkyl)aminoalkyl, oder - (CR^{15a}R^{15b})ₘR¹⁶;
oder
- R^{9a} und R^{9b}: bzw. R^{11a} und R^{11b} bilden jeweils mit dem Stickstoffatom oder der (NCO) Einheit oder der (NCS) Einheit, mit dem/ mit der sie verknüpft sind, einen 3 bis 6-gliedrigen Ring, der gegebenenfalls zusätzlich Ringglieder ausgewählt aus der Gruppe O, NR³, C(=O), C(=S), C(=NR⁴), SiR^{5a}R^{5b} und S(=O)ₚ(=NR⁴)_{q}, enthalten kann und der gegebenenfalls an den Ringkohlenstoffatomen mit 1 bis 4 Substituenten ausgewählt aus der Gruppe bestehend aus Halogen, -CN, C₁-C₂-Alkyl und C₁-C₂-Alkoxy substituiert sein kann;
- R¹²: steht jeweils für C₁-C₁₀-Alkyl, C₂-C₁₀-Alkenyl, C₂-C₁₀-Alkynyl, C₁-C₁₀-Haloalkyl, C₂-C₁₀-Haloalkenyl, C₂-C₁₀-Haloalkynyl, C₂-C₁₀-Alkoxyalkyl, C₃-C₁₀-Alkoxyalkoxyalkyl, C₃-C₁₀-Alkoxyalkenyl, C₃-C₁₀-Alkoxyalkynyl, C₃-C₁₀-Dialkoxyalkyl, C₄-C₁₀-Trialkoxyalkyl, C₂-C₁₀-Haloalkoxyalkyl, C₂-C₁₀-Alkoxyhaloalkyl, C₂-C₁₀-Haloalkoxyhaloalkyl, C₂-C₁₀-Hydroxyalkyl, C₂-C₁₀-Cyanoalkyl, C₂-C₁₀-Alkylthioalkyl, C₂-C₁₀-Alkylsulfinylalkyl, C₃-C₁₀-Alkylaminoalkyl, C₃-C₁₀-Haloalkylaminoalkyl, C₅-C₁₀-Cycloalkylaminoalkyl, C₄-C₁₀-Dialkylaminoalkyl, C₄-C₁₀-Halodialkylaminoalkyl, C₆-C₁₀-Cycloalkyl(alkyl)-aminoalkyl, oder -(CR^{15a}R^{15b})ₘR¹⁶;
- R^{15a}, R^{15b}: stehen unabhängig voneinander für H, Halogen, C₁-C₅-Alkyl, C₁-C₅-Haloalkyl oder C₁-C₅-Alkoxy;
oder
- ein geminales Paar von R^{15a} und R^{15b}: bildet zusammen mit dem Kohlenstoffatom, an das sie gebunden sind, C(=O) oder einen C₃-C₆-Cycloalkylring oder einen C₃-C₆-Halocycloalkyl ring;
- R¹⁶: steht für Phenyl, C₃-C₈-Cycloalkyl, C₃-C₈-Cycloalkenyl, einen 5- oder 6-gliedrigen heteroaromatischen Ring oder Naphthalenyl oder ein 8-, 9- oder 10-gliedriges heteroaromatisches bicyclisches Ringsystem; oder einen 5- oder 6-gliedrigen heterocyclischen nicht aromatischen Ring, der gegebenenfalls Ringglieder ausgewählt aus der Gruppe C(=O), C(=S), C(=NR⁴), SiR^{5a}R^{5b} und S(=O)ₚ(=NR⁴)_{q} enthält; jeder Ring oder jedes Ringsystem kann an den Ringkohlenstoffatomen gegebenenfalls substituiert sein mit bis zu 5 Substituenten unabhängig voneinander ausgewählt aus R¹⁷;
- R¹⁷: steht jeweils unabhängig voneinander für Halogen, C₁-C₆-Alkyl, C₂-C₆-Alkenyl, C₂-C₆-Alkynyl, C₃-C₆-Cycloalkyl, C₄-C₁₀-Alkylcycloalkyl, C₃-C₆-Halocycloalkyl, C₁-C₆-Haloalkyl, C₂-C₆-Haloalkenyl, Cyano, Nitro, C₁-C₆-Alkoxy, C₃-C₈-Cycloalkoxy, C₃-C₈-Halocycloalkoxy, C₁-C₆-Haloalkoxy, C₁-C₆-Alkylthio, C₁-C₆-Alkylsulfinyl, C₁-C₆-Alkylsulfonyl, C₁-C₆-Haloalkylthio, C₁-C₆-Haloalkylsulfinyl, C₁-C₆-Haloalkylsulfonyl, C₁-C₆-Alkylamino, C₁-C₆-Dialkylamino, C₁-C₆-Alkylcarbonyl, C₁-C₆-Alkoxycarbonyl, C₁-C₆-Alkylaminocarbonyl, C₃-C₆-Dialkylaminocarbonyl, C₃-C₆-Trialkylsilyl, Phenyl, Naphthalenyl oder einen 5- oder 6-gliedrigen heteroaromatischen Ring;
- m: steht für 0, 1 oder 2;
- R¹⁰: steht jeweils für H, C₁-C₅-Alkyl, C₂-C₅-Alkenyl, C₂-C₅-Alkynyl, C₃-C₆-Cycloalkyl, C₃-C₆-Halocycloalkyl, C₁-C₅-Haloalkyl, C₂-C₅-Haloalkenyl, C₂-C₅-Haloalkynyl, C₂-C₅-Alkoxyalkyl, C₂-C₅-Alkylcarbonyl oder C₁-C₅-Alkoxy;
- R^{13a}, R^{13b}: stehen unabhängig voneinander für H, -CN, -C(=O)OR¹⁸, C₁-C₆-Alkyl, C₂-C₆-Alkenyl, C₂-C₆-Alkynyl, C₃-C₈-Cycloalkyl, C₃-C₈-Halocycloalkyl, C₃-C₈-Cycloalkenyl, C₄-C₁₀-Cycloalkylalkyl, C₄-C₁₀-Alkylcycloalkyl, C₅-C₁₀-Alkylcycloalkylalkyl, C₁-C₆-Alkylamino, C₂-C₆-Dialkylamino, C₂-C₆-Alkylaminoalkyl, C₂-C₆-Haloalkylaminoalkyl, C₄-C₆-Cycloalkylaminoalkyl, C₃-C₆-Dialkylaminoalkyl, C₃-C₆-Halodialkylaminoalkyl, C₅-C₁₀-Cycloalkyl(alkyl)aminoalkyl, C₁-C₆-Alkoxy, C₁-C₆-Haloalkoxy, C₃-C₁₀-Cycloalkoxy, C₁-C₁₀-Alkylthio, C₁-C₁₀-Haloalkylthio, C₃-C₁₀-Cycloalkylthio, C₃-C₁₀-Trialkylsilyl oder C₃-C₁₀-Halotrialkylsilyl, oder Phenyl oder einen 5- oder 6-gliedrigen heteroaromatischen Ring, ein 8-, 9- oder 10-gliedrigen heteroaromatisches bicyclisches Ringsystem, oder einen 5- oder 6-gliedrigen heterocyclischen nicht aromatischen Ring, der gegebenenfalls Ringglieder ausgewählt aus der Gruppe NR³, C(=O), C(=S), C(=NR⁴), SiR^{5a}R^{5b} und S(=O)ₚ(=NR⁴)_{q} enthält; jeder Ring oder jedes Ringsystem kann an den Ringkohlenstoffatomen gegebenenfalls substituiert sein mit 1 bis 5 Substituenten ausgewählt aus der Gruppe bestehend aus C₁-C₃-Alkyl, Halogen, -CN and C₁-C₃-Alkoxy; oder
- R^{13a} und R^{13b}: bilden zusammen mit dem Kohlenstoffatom, an das sie gebunden sind, einen 3- bis 6-gliedrigen Ring, besagter Ring kann gegebenenfalls Ringglieder enthalten ausgewählt aus der Gruppe NR³, C(=O), C(=S), C(=NR⁴), SiR^{5a}R^{5b} oder S(=O)ₚ(=NR⁴)_{q} und kann gegebenenfalls an den Ringkohlenstoffatomen mit 1 bis 4 Susbstituenten ausgewählt aus der Gruppe C₁-C₂-Alkyl, Halogen, -CN und C₁-C₂-Alkoxy substituiert sein;
- R^{14a}, R^{14b}: stehen unabhängig voneinander für H, -CN, -C(=O)OR¹⁸, C₁-C₆-Alkyl, C₂-C₆-Alkenyl, C₂-C₆-Alkynyl, C₃-C₈-Cycloalkyl, C₃-C₈-Halocycloalkyl, C₃-C₈-Cycloalkenyl, C₄-C₁₀-Cycloalkylalkyl, C₄-C₁₀-Alkylcycloalkyl, C₅-C₁₀-Alkylcycloalkylalkyl, C₁-C₆-Alkylamino, C₂-C₆-Dialkylamino, C₂-C₆-Alkylaminoalkyl, C₂-C₆-Haloalkylaminoalkyl, C₄-C₆-Cycloalkylaminoalkyl, C₃-C₆-Dialkylaminoalkyl, C₃-C₆-Halodialkylaminoalkyl, C₅-C₁₀-Cycloalkyl(alkyl)aminoalkyl, C₁-C₆-Alkoxy, C₁-C₆-Haloalkoxy, C₃-C₁-Cycloalkoxy, C₁-C₁₀-Alkylthio, C₁-C₁₀-Haloalkylthio, C₃-C₁₀-Cycloalkylthio, C₃-C₁₀-Trialkylsilyl or C₃-C₁₀-Halotrialkylsilyl; oder Phenyl oder einen 5- oder 6-gliedrigen heteroaromatischen Ring, ein 8-, 9- oder 10-gliedrigen heteroaromatisches bicyclisches Ringsystem, oder einen 5- oder 6-gliedrigen heterocyclischen nicht aromatischen Ring, der gegebenenfalls Ringglieder ausgewählt aus der Gruppe NR³, C(=O), C(=S), C(=NR⁴), SiR^{5a}R^{1b} und S(=O)ₚ(=NR⁴)_{q} enthält; jeder Ring oder jedes Ringsystem kann an den Ringkohlenstoffatomen gegebenenfalls substituiert sein mit 1 bis 5 Substituenten ausgewählt aus der Gruppe bestehend aus C₁-C₃-Alkyl, Halogen, -CN and C₁-C₃-Alkoxy;
oder
- R^{14a}, R^{14b}: bilden zusammen mit dem Kohlenstoffatom, an das sie gebunden sind, einen 3- bis 6-gliedrigen Ring, besagter Ring kann gegebenenfalls Ringkomponenten enthalten ausgewählt aus der Gruppe NR³, C(=O), C(=S), C(=NR⁴), SiR^{5a}R^{5b} oder S(=O)p(=NR⁴)_{q} und kann gegebenenfalls an den Ringkohlenstoffatomen mit 1 bis 4 Susbstituenten ausgewählt aus der Gruppe C₁-C₂-Alkyl, Halogen, -CN und C₁-C₂-Alkoxy substituiert sein;
- p, q: stehen unabhängig voneinander für 0, 1 oder 2 vorausgesetzt, dass die Summe von p und q 1 oder 2 ist,
- R¹⁸: steht für C₁-C₆-Alkyl, C₁-C₆-Haloalkyl, C₃-C₆-Cycloalkyl, C₄-C₇-Cycloalkylalkyl oder C₄-C₇-Alkylcycloalky;
- W: steht für H oder
- W: steht für Halogen, CN, C₁-C₄-Haloalkyl, C₁-C₄-Alkyl, C₁-C₄-Alkoxy;
sowie agrochemisch wirksame Salze davon.

Verbindungen der Formel (**I**) eignen sich sehr gut zur Bekämpfung von unerwünschten Mikroorganismen. Sie zeigen vor allem eine starke fungizide Wirksamkeit und lassen sich sowohl im Pflanzenschutz als auch im Materialschutz sowie zur Reduktion von Mykotoxinen in Pflanzen und Pflanzenteilen verwenden.

Die Verbindungen der Formel (**I**) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen Verbindungen sind durch die Formel (**I**) allgemein definiert.

Bevorzugt werden Verbindungen der Formel (**I**), in denen ein oder mehrere der Symbole eine der folgenden Bedeutungen haben:
- Y: bildet zusammen mit dem angrenzenden Stickstoffatom "1" und den beiden Kohlenstoffatomen "2" und "3" einen 5- bis 7-gliedrigen nicht aromatischen heterocyclischen Ring, dessen weitere Ringglieder aus der Gruppe C(R²)₂, O, S, SO₂, NR³, -C(R²)=C(R²)-, C(=O) und C(=S) ausgewählt sind;
- R²: steht jeweils unabhängig voneinander für H, Halogen, Cyano, Hydroxy, -CHO, -C(=O)OR⁶, - C(=O)NHOR^{6a}, C₁-C₅-Alkyl, C₂-C₅-Akenyl, C₂-C₅-Alkynyl, C₃-C₆-Cycloalkyl, C₃-C₆-Halocycloalkyl, C₃-C₆-Cycloalkenyl, C₁-C₅-Haloalkyl, C₁-C₅-Alkoxy, C₁-C₅-Haloalkoxy, C₃-C₆-Cycloalkoxy, C₂-C₅-Alkenyloxy, C₃-C₅-Haloalkenyloxy, C₂-C₅-Alkynyloxy, C₂-C₅-Alkylcarbonyl, C₁-C₅-Alkylthio, C₁-C₅-Haloalkylthio oder C₃- C₆-Cycloalkylthio;
- R³: steht für H, -CN, -C(=O)NH₂, -C(=O)NHCN, -CHO, -C(=O)OR⁶, -C(=O)NHOR^{6a}, C₁-C₅-Alkyl, C₂-C₅-Haloalkyl, C₁-C₄-Alkylcarbonyl, C₁-C₄-Haloalkylcarbonyl, C₃-C₆-Cycloalkylcarbonyl, C₁-C₄-Alkoxycarbonyl, C₁-C₄-Haloalkoxycarbonyl, C₃-C₆-Cycloalkoxycarbonyl, C₃-C₆-Alkoxyalkyl-carbonyl, C₃-C₆-Alkoxyalkoxycarbonyl, C₁-C₄-(Alkylthio)carbonyl, C₁-C₄-Alkoxy(thiocarbonyl), C₁-C₄-Alkyl(thiocarbonyl), C₁-C₄-Alkylthio(thio-carbonyl), C₁-C₄-Alkylaminocarbonyl, C₃-C₆-Cycloalkylaminocarbonyl, C₂-C₆-Dialkylaminocarbonyl, C₂-C₆-Alkylamino(thiocarbonyl), C₂-C₆-Dialkyl-amino(thiocarbonyl) oder C₃-C₆-Alkoxy(alkyl)aminocarbonyl;
- R⁶: steht jeweils für H or C₁-C₄-Alkyl;
- R^{6a}: steht jeweils für C₁-C₄-Alkyl;
- A: steht für einen Phenylring der gegebenenfalls einfach oder mehrfach mit R⁷ substituiert sein kann oder für einen Thiophenylring der gegebenenfalls einfach oder mehrfach mit R⁸ substituiert sein kann;
- R⁷: steht unabhängig voneinander für Halogen, C₁-C₄-Alkyl, C₁-C₄-Haloalkyl, Cyano, C₁-C₄-Alkoxy, C₁-C₄-Haloalkoxy, C₁-C₄-Alkylthio, C₁-C₄-Alkylsulfmyl, C₁-C₄-Alkylsulfonyl oder C₁-C₄-Haloalkylthio;
- R⁸: steht unabhängig voneinander für Halogen, Cyano, C₁-C₃-Alkyl, C₁-C₃-Alkoxy, C₁-C₃-Haloalkyl;
- R¹: steht für H, Halogen, C₁-C₃ Alkyl, Cyano, -NR^{9a}R^{9b}, -N(R^{9b})COR^{9a}, -N(R^{9b})CSR^{9a}, - N(R^{9b})COOR¹², -OR¹², -S(O)mR^{6a}, COOR¹² oder -CONR^{9a}R⁹⁶;
- R^{9a}: steht jeweilsfür H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₂-C₆-Alkoxyalkyl, C₃-C₆-Alkoxyalkoxyalkyl, C₃-C₆-Alkoxyalkenyl, C₃-C₆-Alkoxyalkynyl, C₃-C₆-Dialkoxyalkyl, C₂-C₆-Haloalkoxyalkyl, C₂-C₆-Alkoxyhaloalkyl, C₂-C₆-Haloalkoxyhaloalkyl, C₁-C₆-Hydroxyalkyl, C₁-C₆-Cyanoalkyl, C₂-C₆-Alkylthioalkyl, C₂-C₆-Alkylsulfinylalkyl, C₃-C₆-Alkylaminoalkyl, C₃-C₆-Haloalkylaminoalkyl, C₅-C₁₀-Cycloalkylaminoalkyl C₄-C₁₀-Dialkylaminoalkyl, C₄-C₁₀- Halodialkylaminoalkyl oder - (CR^{15a}R^{15b})ₘR¹⁶;
- R^{9b}: steht jeweils für H, C₁-C₆-Alkyl, C₂-C₆-Alkenyl, C₂-C₆-Alkynyl, C₁-C₆-Haloalkyl or - (CR^{15a}R^{15b})ₘR¹⁶; oder
- R^{9a},R⁹⁶: bilden jeweils zusammen mit dem Stickstoff oder der (NCO) oder der (NCS) Einheit, mit dem/ mit der sie verknüpft sind, einen 3 bis 6-gliedrigen Ring, der gegebenenfalls zusätzlich auch Ringglieder enthalten kann ausgewählt aus der Gruppe NR³, C(=O), C(=S), O und gegebenenfalls an den Ringkohlenstoffatomen substituiuert ist mit 1 bis 4 Substituenten ausgewählt aus der Gruppe bestehend aus Halogen, -CN, C₁-C₂-Alkyl und C₁-C₂-Alkoxy;
- R¹²: steht jeweils für C₁-C₆-Alkyl, C₂-C₆-Alkenyl, C₂-C₆-Alkynyl, C₁-C₆-Haloalkyl, C₂-C₆-Haloalkenyl oder - (CR^{15a}R^{15b})ₘR¹⁶;
- R^{15a}, R¹⁵: steht unabhängig voneinander für H, Halogen oder C₁-C₄-Alkyl;
- R¹⁶: steht für Phenyl, C₃-C₈-Cycloalkyl, C₃-C₈-Cycloalkenyl, einen 5- oder 6-gliedrigen heteroaromatischen Ring oder Naphthalenyl oder ein 8-, 9- oder 10-gliedriges heteroaromatisches bicyclisches Ringsystem; oder einen 5- oder 6-gliedrigen heterocyclischen nicht aromatischen Ring, der gegebenenfalls Ringglieder ausgewählt aus der Gruppe C(=O), C(=S), C(=NR⁴) enthält; jeder Ring oder jedes Ringsystem kann an den Ringkohlenstoffatomen gegebenenfalls substituiert sein mit bis zu 3 Substituenten unabhängig voneinander ausgewählt von R¹⁷;
- R¹⁷: steht jeweils unabhängig voneinander für Halogen, Cyano, C₁-C₆-Alkyl, C₂-C₆-Alkenyl, C₂-C₆-Alkynyl, C₃-C₆-Cycloalkyl, C₁-C₆-Haloalkyl, C₁-C₆-Alkoxy;
- m: steht für 0, 1 oder 2,
- W: steht für H oder
- W: steht für Fluor, Chlor, CN, CF₃, Methyl, Ethyl, Methoxy.

Besonders bevorzugt werden Verbindungen der Formel (I), in denen ein oder mehrere der Symbole eine der folgenden Bedeutungen haben:
- Y: bildet zusammen mit dem angrenzenden Stickstoffatom "1" und den beiden Kohlenstoffatomen "2" und "3" einen 5- bis 7-gliedrigen nicht aromatischen heterocyclischen Ring, ausgewählt aus der Gruppe: H-1, H-2, H-3, H-4, H-5, H-6, H-7, H-8, H-9 und H-10 dargestellt in Schema 1, wobei s eine Zahl zwischen 0 und 4 ist.

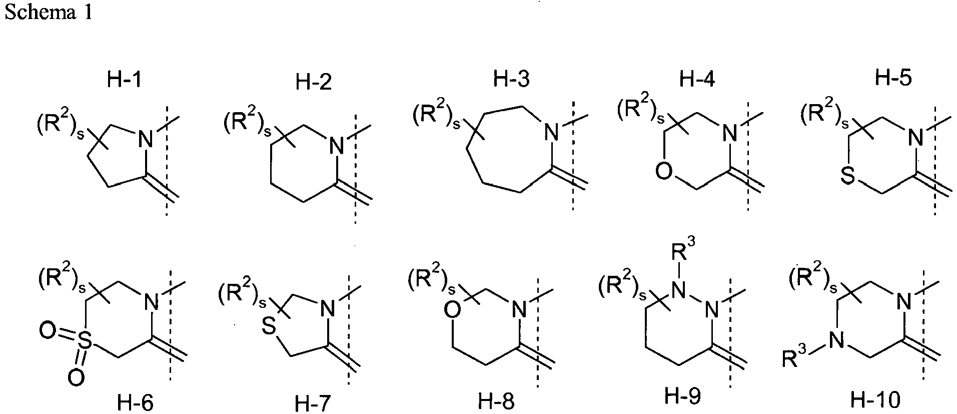

- R²: steht jeweils unabhängig voneinander für H, F, Cl, Br, I, Cyano, Hydroxy, -CHO, -C(=O)OR⁶, Methyl, Ethyl, iso-Propyl, n-Propyl, Trifluormethyl, Difluormethyl, Dichlormethyl, Pentafluorethyl, Methoxy, Ethoxy, n-Propoxy, iso-Propoxy, Trifluormethoxy, Difluormethoxy, oder Acetyl, Propionyl, Isobutyryl, 2,2-Dimethylpropanoyl;
- R³: steht für H, -CHO, Methyl, Ethyl, iso-Propyl, n-Propyl, Acetyl, Propionyl, Isobutyryl, 2,2-Dimethylpropanoyl, Trifluoracetyl, Difluoracetyl, CH₃OC(O), CH₃CH₂C(O), (CH₃)₂CHC(O) oder CF₃OC(O), CF₂HOC(O);
- R⁶: steht jeweilsfür H, Methyl, Ethyl, iso-Propyl, n-Propyl;
- A: steht für einen Phenyl -oder Thiophenring, der gegebenenfalls mit Resten ausgewählt aus F, Cl, Br, I, Cyano, Methyl, Ethyl, iso-Propyl, n-Propyl, Trifluormethyl, Difluormethyl, Dichlormethyl, Pentafluorethyl, oder Methoxy, Ethoxy, n-Propoxy, iso-Propoxy substituiert ist;
- R¹: steht für H, F, Cl, Br, I, CH₃, S(O)ₘMe, -NR^{9a}R^{9b}, N(R^{9b})COR^{9a}, N(R^{9b})COOR¹²;
- R^{9a}: steht jeweils für H, Methyl, Ethyl, iso-Propyl, n-Propyl, n-Butyl, sec-Butyl, tert-Butyl, iso-Butyl, n-Pentyl, n-Hexyl" -CH=CH₂, -CH₂CH=CH₂, -CH=CHCH₃, -CH₂C≡CH, -C≡CH" Trifluormethyl, Difluormethyl, Dichlormethyl, Pentafluorethyl, Methoxymethyl, Ethoxymethyl, Methoxyethyl, tert-Butoxy-methyl, Hydroxymethyl, 1-Hydroxyethyl, 2-Hydroxypropan-2-yl oder -(CH₂)ₘR¹⁶;
- R^{9b}: steht jeweils für H, Methyl, Ethyl, iso-Propyl, n-Propyl, n-Butyl, sec-Butyl, tert-Butyl, iso-Butyl, n-Pentyl, n-Hexyl, -CH₂CH=CH₂, -CH=CHCH₃, -CH₂C≡CH , -C≡CH;
- R^{9a},R^{9b}: bilden jeweils zusammen mit dem Stickstoff, mit dem sie verknüpft sind, einen 5 bis 6-gliedrigen Ring welcher gegebenenfalls an den Ringkohlenstoffatomen substituiert ist mit 1 bis 2 Substituenten ausgewählt aus der Gruppe bestehend aus F, Cl, Br, I, -CN and Methyl, Ethyl;
- R¹²: steht für Methyl, Ethyl, iso-Propyl, n-Propyl, n-Butyl, sec-Butyl, tert-Butyl, iso-Butyl, - CH₂CH=CH₂, -CH=CHCH₃, CH₂C≡CH, -C≡CH, Trifluormethyl, Difluormethyl, Dichlormethyl, oder -(CH₂)ₘR¹⁶;
- R¹⁶: ist steht für cyclopropyl, cyclobutyl, cyclopentyl, cyclohexyl, cyclopentenyl, cyclohexenyl, Phenyl oder Thienyl, von denen ein jeder gegebenenfalls mit bis zu 2 Resten ausgewählt aus der Gruppe R¹⁷ substituiert sein kann;
- R¹⁷: steht jeweils unabhängig voneinander für F, Cl, Br, I , Methyl, Ethyl, iso-Propyl, n-Propyl, n-Butyl, sec-Butyl, tert-Butyl, iso-Butyl, Trifluormethyl, Difluormethyl, Dichlormethyl, Methoxy, Ethoxy oder Cyano;
- m: ist 0, 1 und 2,
- W: steht für H oder
- W: steht für Fluor, Chlor, CN, CF₃, Methyl, Ethyl.

Ganz besonders bevorzugt werden Verbindungen der Formel (I), in denen ein oder mehrere der Symbole eine der folgenden Bedeutungen haben:
- Y: bildet zusammen mit dem angrenzenden Stickstoffatom "1" und den beiden Kohlenstoffatomen "2" und "3" einen 5- bis 7-gliedrigen nicht aromatischen heterocyclischen Ring, ausgewählt aus der Gruppe: H-1, H-2, H-3, H-4, H-5 and H-8, dargestellt in Schema 2, wobei s eine Zahl zwischen 0 und 4 ist;

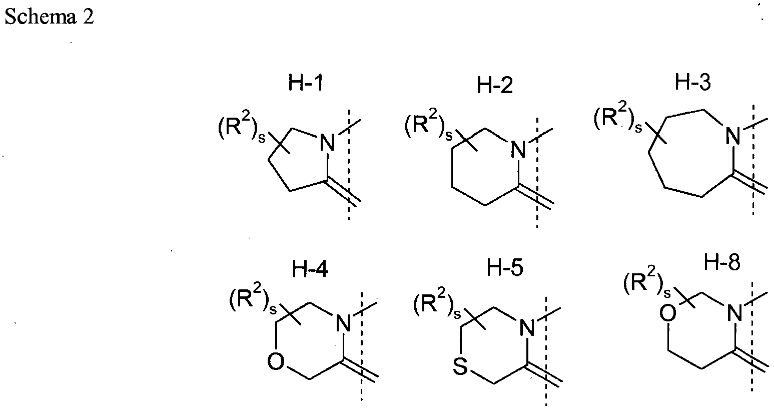

- R²: steht jeweils unabhängig voneinander für H, Fluor, Chlor, Cyano, CF₃, Methyl, oder Methoxy;
- A: steht für einen Phenyl -oder Thiophenring, der gegebenenfalls mit Resten ausgewählt aus F, Cl, Cyano, CH₃, CF₃ substituiert ist;
- R¹: steht für H, Fluor, Chlor, S(O)ₘMe, NR^{9a}R^{9b}, N(R^{9b})COR^{9a}, N(R^{9b})COOR¹²;
- R^{9a}: steht jeweils für H, Methyl, Ethyl, n-Propyl, iso-Propyl, n-Butyl, iso-Butyl, sec-Butyl, tert-Butyl, n-Pentyl, n-Hexyl, Methoxymethyl, Ethoxymethyl, Ethoxyethyl, Methoxyethyl, Methoxypropyl, Hydroxymethyl, 1-Hydroxyethyl, 2-Hydroxyethyl , Hydroxypropyl oder - (CH₂)ₘR¹⁶;
- R^{9b}: steht jeweils für H, Methyl, Ethyl, n-Propyl, iso-Propyl, n-Butyl, Allyl oder Propargyl;
- R¹²: steht für Methyl, Ethyl, n-Propyl, iso-Propyl, t-Butyl, Allyl, Propargyl oder -(CH₂)ₘR¹⁶;
- R¹⁶: steht für Cyclopropyl, Cyclopentyl, Cyclobutyl, Cyclohexyl, Thienyl oder Phenyl, von denen ein jeder gegebenenfalls mit einem Rest ausgewählt aus der Gruppe R¹⁷ substituiert sein kann;
- m: steht für 0, 1 oder 2;
- R¹⁷: steht für Methyl, Ethyl, Fluor, Chlor, CF₃, OMe, Cyano
- W: steht für H oder
- W: steht für Fluor, Chlor, Cyano
sowie agrochemisch wirksamen Salzen davon als Fungizide.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

*Bevorzugt* sind solche Verbindungen der Formel (**I**), in welcher alle Reste jeweils die oben genannten bevorzugten Bedeutungen haben.

*Besonders bevorzugt* sind solche Verbindungen der Formel (**I**), in welcher alle Reste jeweils die oben genannten besonders bevorzugten Bedeutungen haben.

*Ganz besonders bevorzugt* sind solche Verbindungen der Formel (**I**), in welcher alle Reste jeweils die oben genannten ganz besonders bevorzugten Bedeutungen haben.

Weiterhin ganz besonders bevorzugt werden Verbindungen der Formel (**I**), in denen
- R¹: für Wasserstoff steht,
wobei die übrigen Substituenten eine oder mehrere der oben genannten Bedeutungen haben sowie die agrochemisch wirksamen Salzen davon.

Weiterhin ganz besonders bevorzugt werden Verbindungen der Formel (**I**), in denen
- R¹: für NR^{9a}R^{9b} steht,
wobei die übrigen Substituenten eine oder mehrere der oben genannten Bedeutungen haben sowie die agrochemisch wirksamen Salzen davon. Weiterhin ganz besonders bevorzugt werden Verbindungen der Formel (**I**), in denen
- R¹: für N(R^{9b})COR^{9a} steht,
wobei die übrigen Substituenten eine oder mehrere der oben genannten Bedeutungen haben sowie die agrochemisch wirksamen Salzen davon.

Weiterhin ganz besonders bevorzugt werden Verbindungen der Formel (**I**), in denen
- W: für Wassserstoff steht,
wobei die übrigen Substituenten eine oder mehrere der oben genannten Bedeutungen haben sowie die agrochemisch wirksamen Salzen davon.

Weiterhin ganz besonders bevorzugt werden Verbindungen der Formel (**I**), in denen
- A: für einen Phenyl -oder Thiophenring steht, der gegebenenfalls mit Resten ausgewählt aus F, Cl, Cyano, CH₃, CF₃ substituiert ist,
wobei die übrigen Substituenten eine oder mehrere der oben genannten Bedeutungen haben sowie die agrochemisch wirksamen Salzen davon

Weiterhin ganz besonders bevorzugt werden Verbindungen der Formel (**I**), in denen
- Y: zusammen mit dem angrenzenden Stickstoffatom "1" und den beiden Kohlenstoffatomen "2" und "3" einen 5- bis 7-gliedrigen nicht aromatischen heterocyclischen Ring bildet, ausgewählt aus der Gruppe H-1, H-2, H-3, H-4, H-5 and H-8 (siehe oben in Schema 2), in dem s eine ganze Zahl von 0 bis 4 ist und die Substituenten R² unabhängig voneinander für H, Fluor, Chlor, Cyano, CF₃, Methyl oder Methoxy stehen,
wobei die übrigen Substituenten eine oder mehrere der oben genannten Bedeutungen haben sowie die agrochemisch wirksamen Salzen davon.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl;
**Thioalkyl:** gesättigte, geradkettige oder verzweigte Alkylthioreste mit 1 bis 6 Kohlenstoffatomen, wie beispielsweise (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methyl-butylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethyl-butylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Thiohaloalkyl:** geradkettige oder verzweigte Alkylthiogruppen mit 1 bis 6 Kohlenstoffatomen (wie vorstehend genannt), wobei in diesen Gruppen teilweise oder vollständig die Wasserstoffatome durch Halogenatome wie vorstehend genannt ersetzt sein können, wie beispielsweise (aber nicht beschränkt auf) C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Alkenyl:** ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1Butenyl, 2Butenyl, 3Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1Butenyl, 2-Methyl-1Butenyl, 3-Methyl-1Butenyl, 1-Methyl-2Butenyl, 2-Methyl-2Butenyl, 3-Methyl-2Butenyl, 1-Methyl-3Butenyl, 2-Methyl-3Butenyl, 3-Methyl-3Butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2Butenyl, 1,1,-Dimethyl-3Butenyl, 1,2-Dimethyl-1Butenyl, 1,2-Dimethyl-2Butenyl, 1,2-Dimethyl-3Butenyl, 1,3-Dimethyl-1Butenyl, 1,3-Dimethyl-2Butenyl, 1,3-Dimethyl-3Butenyl, 2,2-Dimethyl-3Butenyl, 2,3-Dimethyl-1Butenyl, 2,3-Dimethyl-2Butenyl, 2,3-Dimethyl-3Butenyl, 3,3-Dimethyl-1Butenyl, 3,3-Dimethyl-2Butenyl, 1-Ethyl-1Butenyl, 1-Ethyl-2Butenyl, 1-Ethyl-3Butenyl, 2-Ethyl-1Butenyl, 2-Ethyl-2Butenyl, 2-Ethyl-3Butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl:** geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1Butinyl, 2Butinyl, 3Butinyl, 1-Methyl-2-propinyl, 1-Pentinyl, 2-Pentinyl, 3-Pentinyl, 4-Pentinyl, 1-Methyl-2Butinyl, 1-Methyl-3Butinyl, 2-Methyl-3Butinyl, 3-Methyl-1Butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2Butinyl, 1,1-Dimethyl-3Butinyl, 1,2-Dimethyl-3Butinyl, 2,2-Dimethyl-3Butinyl, 3,3-Dimethyl-1Butinyl, 1-Ethyl-2Butinyl, 1-Ethyl-3Butinyl, 2-Ethyl-3Butinyl und 1-Ethyl-1-methyl-2-propinyl;
**Cycloalkyl:** monocyclische, gesättigte Kohlenwasserstoffgruppen mit 3 bis 8 Kohlenstoffringgliedern, wie Cyclopropyl, Cyclobutyl, Cyclopentyl und Cyclohexyl;
**Cylcoalkenyl:** monocyclische, nicht aromatische Kohlenwasserstoffgruppen mit 3 bis 8 Kohlenstoffringgliedern mit mindestens einer Doppelbindung, wie Cyclopenten-1-yl, Cyclohexen-1-yl, Cyclohepta-1,3-dien-1-yl;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Halocycloalkyl:** monocyclische, gesättigte Kohlenwasserstoffgruppen mit 3 bis 8 Kohlenstoffringgliedern (wie vorstehend genannt), wobei in diesen Gruppen teilweise oder vollständig die Wasserstoffatome durch Halogenatome wie vorstehend genannt ersetzt sein können, z.B. (aber nicht beschränkt auf) 2-Fluorcyclopropyl, 2,2-Difluorcyclopropyl, 3,3-Difluorcyclobutyl, 2-Fluorcyclopentyl, 3-Fluorcyclopentyl;
**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5 5 -yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**Aryl:** 6- bis 14-gliedriges, vollständig ungesättigtes carbocyclisches Ringsystem, z.B. (aber nicht beschränkt auf) Phenyl, 1-Naphthyl, 2-Naphthyl, 2-Anthryl, 1-Anthryl;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**Alkoxy:** einen geradkettigen oder verzweigten Alkoxyrest, wie z.B. (aber nicht beschränkt auf) Methoxy, Ethoxy, n-Propoxy, 1-Methylethoxy, n-Butoxy, 1-Methylpropoxy, 2-Methylpropoxy oder 1,1-Dimethylethoxy, insbesondere für Methoxy oder Ethoxy;
**Alkylthio:** steht für geradkettiges oder verzweigtes Alkylthio z.B. (aber nicht beschränkt auf) Methylthio, Ethylthio, n- und i-Propylthio, n-, i-, sek.- und tert.-Butylthio, n-Pentylthio und seine Isomeren wie 1-, 2-und 3-Methyl-butylthio. Die Alkylthiogruppen können durch 1 bis 3 Halogenatome (vorzugsweise Chlor und/oder Fluor) substituiert sein, z.B. (aber nicht beschränkt auf) sind Di- und Trifluormethylthio sowie Difluorchlormethylthio.
**Halogenalkoxy:** steht für einen geradkettigen oder verzweigten Alkoxyrest in dem ein oder mehrere Wasserstoffatome durch Fluor, Chlor oder Brom ersetzt wurden, z.B. (aber nicht beschränkt auf) -OCF₃, -OCHF₂.
**Acyloxy:** steht für einen über das Sauerstoffatom verbundenen geradkettigen, verzweigten, cyclischen, gesättigten oder ungesättigten Acyloxy rest, z.B. (aber nicht beschränkt auf) Acetyloxy, Propionyloxy, Isobutyryloxy.

Ein weiterer Gegenstand der vorliegenden Erfindung betrifft ein Verfahren zum Herstellen der erfindungsgemäßen bicyclischen Pyridinylpyrazole der Formeln (I) umfassend wenigstens einen der folgenden Schritte (a) bis (d):
a) Umsetzung von Pyridinylalkinen der allgemeinen Formel (II) mit Bicyclen der allgemeinen Formel (III) zu Bicyclischen Pyrazolen der allgemeinen Formel (Ia) gegebenenfalls in Gegenwart eines Lösungsmittels gemäß dem nachfolgenden Reaktionsschema (Schema 3). 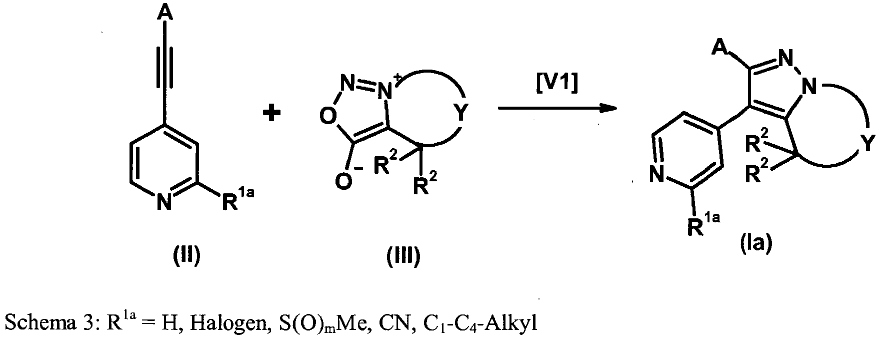
b) Umsetzung von 4-Halogenpyridinen der allgemeinen Formel (IV) mit (Het)Arylalkinen der allgemeinen Formel (V) zu Pyridinylalkinen der allgemeinen Formel (II) gegebenenfalls in Gegenwart eines geeigneten Katalysators und in Gegenwart eines Lösungsmittels gemäß dem nachfolgenden Reaktionsschema (Schema 4): 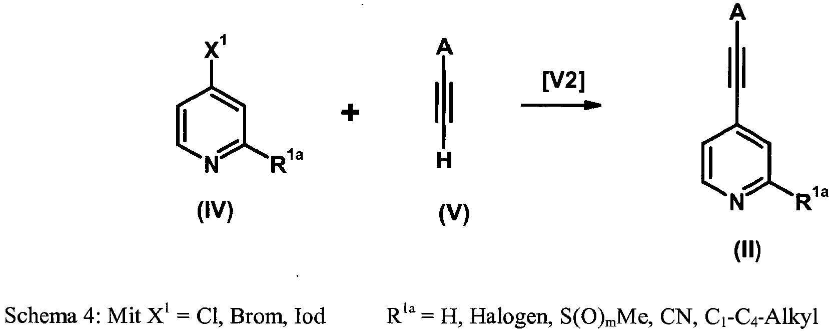
c) Umsetzung von Aminosäuren der allgemeinen Formel (VI) zu Verbindungen der allgemeinen Formel (III) gemäß dem nachfolgenden Reaktionschema (Schema 5): 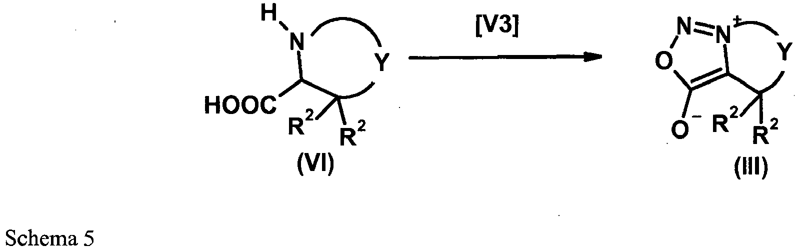
d) Umsetzung von Verbindungen der allgemeinen Formel (Ib) zu Verbindungen der allgemeinen Formel (Ic) gegebenenfalls in Gegenwart einer Base, gegebenenfalls in Gegenwart eines Lösungsmittels gemäß dem nachfolgenden Schema (Schema 6):

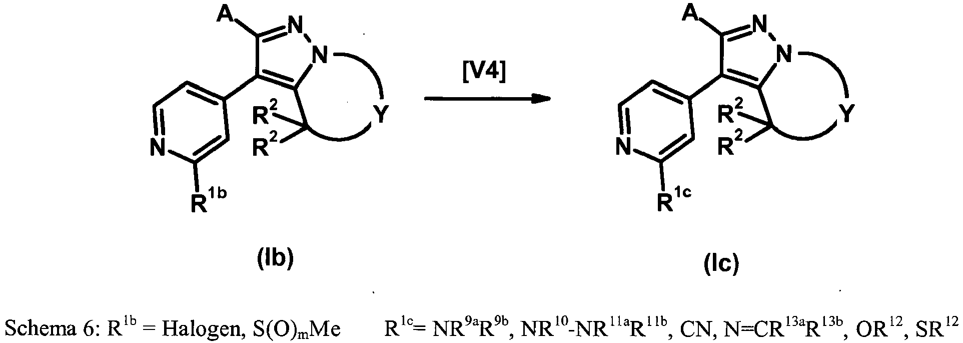

Die erfindungsgemäßen bicyclischen Pyridinylpyrazole der Formel (I) lassen sich auf unterschiedliche Weise herstellen. Im Folgenden sind mögliche Verfahren zunächst schematisch dargestellt. Wenn nicht anders angegeben, haben die angegebenen Reste die oben angegebenen Bedeutungen. Eine generelle Synthesewegsübersicht findet sich in Schema 7:

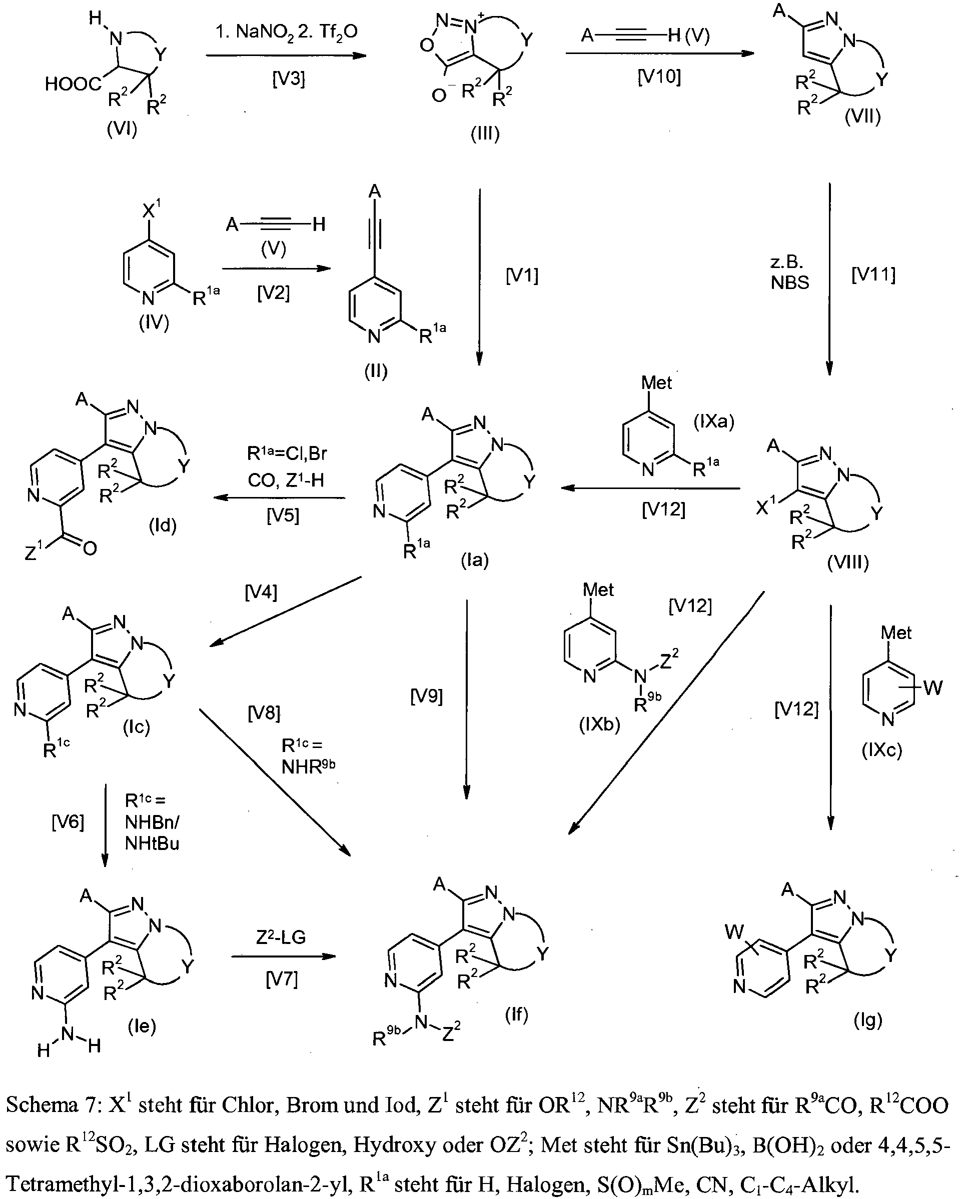

### Verfahrenschritt [VI]:

Die Umsetzung von Pyridinylalkinen der allgemeinen Formel (II) mit Bicyclen der allgemeinen Formel (III) gegebenenfalls in Gegenwart eines Lösungsmittels liefert die Bicyclischen Pyrazole der allgemeinen Formel (Ia).

Als geeignete Lösungsmittel zur Durchführung des erfindungsgemäßen Verfahrensschrittes dienen alle unter den Reaktionsbedingungen inerten Lösungsmittel sowie -gemische daraus. Bevorzugt sind aromatische Kohlenwasserstoffe (z.B. Benzol, Toluol, Xylol oder Mesitylen).

Die Reaktionstemperatur zur Durchführung des erfindungsgemäßen Verfahrensschrittes [VI] liegt zwischen 80 °C und 250 °C, vorzugsweise zwischen 120 °C und dem Siedepunkt des verwendeten Lösungsmittels. Die Reaktionszeit zur Durchführung des erfindungsgemäßen Verfahrensschrittes [VI] beträgt in Abhängigkeit vom Maßstab der Reaktion zwischen 12 und 96 h, bevorzugt zwischen 16 h und 24 h.

### Verfahrenschritt [V2]:

Die Umsetzung von 4-Halogenpyridinen der allgemeinen Formel (IV) (X¹ = Chlor, Brom, Iod) mit (Het)Arylalkinen der allgemeinen Formel (V) liefert in einer Sonogashira Reaktion Pyridinylalkine der allgemeinen Formel (II). Geeignte Katalysatoren und Reaktionsbedingungen der Sonogashira-Reaktion finden sich in A. de Meijere/F. Diederich, (eds.) Metal-Catalyzed Cross-Coupling Reactions, Wiley-VCh, Weinheim, 2004. Die hierfür benötigten 4-Halopyridine (IV) sind entweder kommerziell verfügbar oder lassen sich beispielsweise nach literaturbekannten Methoden aus den entsprechenden N-Oxiden (*Bioorg. Med. Chem. Lett.* **2009**, *19*, 2244-2248), 4-Nitropyridinen (*Chem. Pharm. Bull.* **1990**, *38*, 2446-58) oder 4-Aminopyridinen (*J. Prak. Chem.,* **1959**, *9*, 164-72) gewinnen. (Het-)Arylalkine (V) sind entweder kommerziell verfügbar oder lassen sich aus den entsprechenden (Het-)Aryl-Bromiden oder -Iodiden durch Umsetzung mit z.B. Trimethylsilylacetylen über eine Sonogashira-Reaktion, wie beispielhaft in *Org. Lett.* **2003**, *42*, 1842 beschrieben darstellen.

### Verfahrenschritt [V3]:

Aminosäuren der allgemeinen Formel (VI) lassen sich mit Natriumnitrit in verdünnter wäßrig saurem Medium (z.B. Salzsäure oder Essigsäure) nitrosieren und anschließend mit einen wasserentziehenden Mittel zu Verbindungen der allgemeinen Formel (III) umsetzen, wie beispielhaft in *Heterocycles* **1990,** *31*, 481 oder *J. Med. Chem.* **2006,** *49*, 4623 beschrieben. Geeignete wasserentziehende Mittel sind Chlorameisensäureester (z.B. ClCOOMe) oder Anhydride (z.B. Tf₂O). Die cyclischen Aminosäuren (VI) sind entweder kommerziell verfügbar oder lassen sich durch literaturbekannte Methoden darstellen (z.B. *Tetrahedron* **2006**, *62*, 8687 oder *J*. *Med. Chem.* **2006**, *49*, 4623).

### Verfahrenschritt [V4]:

Eine Möglichkeit zur Darstellung von Verbindungen der allgemeinen Formel (Ic, mit R^{1c}= NR^{9a}R^{9b}, NR¹⁰-NR^{11a}R^{11b}, CN, N=CR^{13a}R^{13b}, OR¹², SR¹²) ist durch Verfahrensschritt [V4] beschrieben. Hierfür werden die geeigneten Austrittsgruppen in (Ia) z.B. R^{1a} = Halogen, SMe, SOMe oder SO₂Me durch entsprechende Amine HNR^{9a}R^{9b}, Hydrazine HNR¹⁰-NR^{11a}R^{11b}, Imine HN=CR^{13a}R^{13b}, Alkohole HOR¹⁰ oder Cyanid gegebenenfalls in Gegenwart einer Base und gegebenenfalls in Gegenwart eines Lösungsmittels substituiert.

Als Lösungsmittel zur Durchführung des erfindungsgemäßen Verfahrensschrittes [V4] aus Schema 7 können die Amine HNR^{9a}R^{9b} oder Alkohole OR¹⁰ selbst oder alle üblichen, unter den Reaktionsbedingungen inerten Lösungsmittel, wie zyklische und azyklische Ether (Dimethoxymethan, Tetrahydrofuran, Dioxan), aromatische Kohlenwasserstoffe (z.B. Toluol), Nitrile (z.B. Acetonitril, Propionitril,) und Amide (z.B. Dimethylformamid, Dimethylacetamid, N-Methylpyrrolidon) verwendet werden oder die Reaktion kann in Mischungen von zwei oder mehreren dieser Lösungsmittel ausgeführt werden. Die bevorzugten Lösungsmittel sind Dimethylformamid oder Acetonitril.

Geeignete Basen sind Alkalimetallhydroxide, Alkalimetallhydride, Alkalimetallcarbonate, Alkalimetallacetate, Alkalimetallalkoholate, sowie tertiäre Amine. Bevorzugte Basen sind Natriumhydrid, Natriumcarbonat, Kaliumcarbonat oder Cäsiumcarbonat.

Die Reaktionstemperatur zur Durchführung des erfindungsgemäßen Verfahrensschrittes [V4] liegt zwischen 0 °C und 180 °C, vorzugsweise zwischen Raumtemperatur und 100 °C. Die Reaktion kann unter Normaldruck oder unter Druck durchgeführt werden. Die Reaktionszeit beträgt in Abhängigkeit vom Maßstab der Reaktion zwischen 5 min und 24 h, bevorzugt zwischen 30 min und 6 h.

### Verfahrenschritt [V5]:

Eine Möglichkeit zur Überführung von Halogenpyridinen der allgemeinen Formel (Ia) (R^{1a} = Cl, Br) in die entsprechenden Ester und Amide ist durch Verfahrensschritt [V5] gegeben. Pd-katalysierte Carbonylierungen von (Ia) liefern in Gegenwart von Alkoholen die entsprechenden Ester der allgemeinen Formel (Id), mit Z¹=OR¹², vgl. J. Med. Chem. 2006, 49, 3563-3580, während in Gegenwart von Aminen die entsprechenden Amide (Id), mit Z¹=NR^{9a}R^{9b} wie z.B. in Chemistry Euro. J. 2004, 10, 746-757 beschrieben gebildet werden. Als CO-Quelle können Kohlenmonoxid selbst oder Metallcarbonyle (z.B. Mo(CO)₆) dienen.

Als Lösungsmittel zur Durchführung des erfindungsgemäßen Verfahrensschritt [V5] aus Schema 7 können die Alkohole bzw. die Amine selbst und darüber hinaus alle üblichen, unter den Reaktionsbedingungen inerten Lösungsmittel, wie zyklische und azyklische Ether (z.B. Dioxan), aromatische Kohlenwasserstoffe (z.B. Toluol), Sulfoxide (z.B. DMSO) und Amide (z.B. Dimethylformamid, Dimethylacetamid, N-Methylpyrrolidon) verwendet werden oder die Reaktion kann in Mischungen von zwei oder mehreren dieser Lösungsmittel ausgeführt werden. Die bevorzugten Lösungsmittel sind die Alkohole bzw. die Amine selbst sowie Dimethylformamid.

Geeignete Basen zur Durchführung des erfindungsgemäßen Verfahrensschritt [V5] aus Schema 7 sind Alkalimetallcarbonate (z.B. Kaliumcarbonat), zyklische Amidine (z.B. DBU) sowie tertiäre Amine (z.B. Triethylamin).

Bevorzugt zur Durchführung des erfindungsgemäßen Verfahrensschritt [V5] sind Palladiumkatalysatoren, in denen das Palladium in der Oxidationsstufe (0) oder (II) vorliegt, wie z.B. Tetrakis(triphenylphosphin)-palladium, Bis(triphenylphosphin)-palladiumdichlorid Bis(diphenylphosphino)ferrocenpalladium-dichlorid sowie Palladium(II)acetat. Der Katalysator kann phosphorhaltige Liganden enthalten oder es können dem Reaktionsgemisch phosphorhaltige Liganden separat zugesetzt werden. Als phosphorhaltige Liganden eignen sich bevorzugt Tri-n-alkylphosphane, Triarylphosphane, Dialkylarylphosphane, Alkyldiarylphosphane wobei die drei Substituenten am Phosphor gleich oder verschieden sein können und wobei einer oder mehrere Substituenten die Phosphorgruppen mehrerer Phosphane verknüpfen können, wobei ein Teil dieser Verknüpfung auch ein Metallatom sein kann. Besonders bevorzugt sind Phosphane wie Triphenylphosphan und 1,4-Bis(diphenylphosphino)propan und 1,1'-Bis(diphenylphosphino)ferrocene.

Die Reaktion zur Durchführung des erfindungsgemäßen Verfahrensschritt [V5] wird in einem Temperaturbereich von 25° bis 150 °C, besonders bevorzugt bei 80° bis120 °C durchgeführt. Die Reaktion kann unter Normaldruck oder unter Druck durchgeführt werden. Die Reaktionszeit variiert in Abhängigkeit vom Maßstab der Reaktion und der Reaktionstemperatur, aber liegt im Allgemeinen zwischen einigen Minuten und 20 Stunden.

### Verfahrenschritt [V6]:

Eine Möglichkeit zur Darstellung von Verbindungen der allgemeinen Formel (Ie) ist durch Verfahrensschritt [V6] beschrieben. Geeignete säurelabile Amine (z.B. R^{1c}=NHBn (vgl. WO 2008/132434) oder NHtBu (vgl. Tetrahedron Lett. 2005, 46, 3883-3887)) lassen sich im sauren Medium zu (2-Aminopyridin-4-yl)pyrazole (Ie) spalten. Geeignete Säuren hierfür sind Mineralsäuren (z.B. H₂SO₄, HCl), Lewissäuren (z.B. BBr₃, AlCl₃) oder organische Säuren (z.B. F₃COOH, CF₃SO₃H). Als Lösungsmittel dienen die Säuren selbst oder alle üblichen, unter den Reaktionsbedingungen inerten Lösungsmittel, wie halogenierte Kohlenwasserstoffe (z.B. Dichlormethan) oder aromatische Kohlenwasserstoffe (z.B. Toluol). Die Reaktionstemperatur liegt zwischen 0 °C und 100 °C. Die Reaktionszeit beträgt in Abhängigkeit vom Maßstab der Reaktion zwischen 5 min und 24 h, bevorzugt zwischen 30 min und 12 h.

### Verfahrenschritt [V7] und [V8]:

Über Verfahrensschritt [V7] und [V8] sind ausgehend von den Aminen (Ie) und (Ic für R^{1c} = primäres Amin) mittels Carbonsäure-Chloriden oder -Anhydriden die entsprechenden Amide (If, mit Z²=R^{9a}CO), mit Chlorameisensäureestern die entsprechenden Carbamate (If, mit Z²=R¹²COO) und mit Sulfonsäure-Chloriden oder -Anhydriden die entsprechenden Sulfonamide (If, mit Z²=R¹²SO₂) zugänglich. Die Verbindungen der allgemeinen Formel Z²-LG sind entweder kommerziell verfügbar oder anhand literaturbekannter Methoden der organischen Synthese darstellbar (R. C. Larock, Comprehensive Organic Transformations, 2. Ausgabe, 1999, Wiley-VCH, Seite 1929 ff. und darin zitierte Literatur).

Als Lösungsmittel zur Durchführung des erfindungsgemäßen Verfahrensschritt [V7] und [V8] können alle üblichen, unter den Reaktionsbedingungen inerten Lösungsmittel, wie z.B. zyklische und azyklische Ether (z.B. Tetrahydrofuran, Dioxane), aromatische Kohlenwasserstoffe (z.B. Toluol), halogenierte Kohlenwasserstoffe (z.B. Dichlormethan), Ketone (z. B. Aceton), Amide (z.B. Dimethylformaid) und Nitrile (z.B. Acetonitril) verwendet werden oder die Reaktion kann in Mischungen von zwei oder mehreren dieser Lösungsmittel ausgeführt werden. Die bevorzugten Lösungsmittel sind Tetrahydrofuran, Dichlormethan und Acetonitril.

Als Säurefänger zur Durchführung der Verfahrensschritte [V7] und [V8] können geeignete Basen verwendet werden. Bevorzugt sind tertiäre Amine (z.B. Triethylamin, Ethyldiisopropylamin), Alkalicarbonate (z.B. Natriumcarbonat) oder Alkalimetallhydroxide (Natriumhydroxid).

Die Reaktion zur Durchführung der Verfahrensschritte [V7] und [V8] wird normalerweise bei Temperaturen von 0 °C - 100 °C durchgeführt und vorzugsweise bei Raumtemperatur, aber sie kann auch bis zur Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Alternativ können Carboxamide und Sulfonamide der allgemeinen Formel (If) mit Z²=R^{9a}CO, R¹²SO₂ auch aus den entsprechenden Säuren Z²-OH in Gegenwart eines Kupplungsreagenzes synthetisiert werden analog zu in der Literatur beschriebenen Vorschriften (z.B. Tetrahedron 2005, 61, 10827-10852, und darin zitierte Referenzen).

Geeignete Kupplungsreagenzien zur Durchführung der Verfahrensschritte [V7] und [V8] sind beispielsweise Carbodiimide (z.B. N- (3-Dimethylaminopropyl)-N'-ethyl-carbodiimid gegebenenfalls mit 4-Dimethylamino-pyridin oder 1-Hydroxy-benzotriazol) Phosphoniumionen (z.B. Brom-tripyrrolidinophosphonium-hexafluorophosphat) oder Uroniumionen (z.B. O-(7-Azabenzotriazol-1-yl)-N,N,N',N'-tetramethyl-uroniumhexa-fluorophosphat).

Gegebenenfalls kann eine Base zur Durchführung der Verfahrensschritte [V7] und [V8], wie z.B. Triethylamin oder Ethyldiisopropylamin in der Reaktion verwendet werden. Als Lösungsmittel zur Durchführung der Verfahrensschritte [V7] und [V8] können alle üblichen, unter den Reaktionsbedingungen inerten Lösungsmittel, wie für die Umsetzung mit Säurechloriden beschrieben, verwendet werden.

### Verfahrenschritt [V9]:

Wie z.B. in DE-A 1 037 399 beschrieben gelingt die Einführung eines Amid-Restes auch Pd-katalysiert direkt ausgehend von den 2-Halopyridinen (Ia, mit R^{1a} = Chlor, Brom Iod).

### Verfahrenschritt [V10]:

Analog zu den in Verfahrensschritt [V1] beschriebenen Reaktionsbedingungen lassen sich Bicyclen der allgemeinen Formel (III) auch mit Het-Arylalkinen der allgemeinen Fomel (V) zu bicyclischen Pyrazole der allgemeinen Formel (VII) umsetzen.

### Verfahrenschritt [V11]:

4-H-Pyrazole der allgemeinen Formel (VII) lassen sich mit geeigneten Halogenierungsreagenzien nach literaturbekanntem Verfahren (z.B. Bioorg. Med. Chem. Lett. 2008, 18, 509-512) in die entsprechenden 4-Halogen-Pyrazole (X¹ = Cl, Br, I) der allgemeinen Formel (VIII) überführen. Als Halogenierungsmittel können z.B. elementares Chlor, Brom, Iod oder N-Halo-Succinimide (NCS, NBS, NIS) oder auch Sulfurylchlorid und Pyridiniumtribromid verwendet werden.

Die Halogenierung zur Durchführung des Verfahrensschritts [V11] kann in Gegenwart eines unter den Reaktionsbedingungen inerten Lösungsmittels durchgeführt werden. Bevorzugt sind Alkohole (z.B. Methanol, Ethanol), zyklische und azyklische Ether (z.B. Diethylether, Tetrahydrofuran, Dioxan), Amide (z.B. Dimethylformamid, Dimethylacetamid), Sulfoxide (z.B. Dimethylsulfoxid), aromatische Kohlenwasserstoffe (z.B. Benzol, Toluol), halogenierte Kohlenwasserstoffe (z.B. Dichlormethan, Chloroform) sowie Carbonsäuren (z.B. Essigsäure).

Die Reaktionstemperatur liegt zwischen 0 °C und dem Siedepunkt des Lösungsmittels, vorzugsweise zwischen Raumtemperatur und 80 °C. Die beträgt in Abhängigkeit vom Maßstab der Reaktion zwischen 5 min und 24 h, bevorzugt zwischen 30 min und 6 h.

### Verfahrenschritt [V12]:

Eine weitere Möglichkeit zur Synthese der erfindungsgemäßen Pyridinylpyrazole (Ia), (If) oder (Ig) ist durch Verfahrensschritt [V12] gegeben. In einer Suzuki-Reaktion (z.B. analog. *Organic Lett.* **2005**, *7*, 4753-4756) werden die aus Verfahrensschritt [V11] gewonnenen 4-Halogen-Pyrazole (VIII) mit Pyridinen der allgemeinen Formel (IXa/b/c) zur Reaktion gebracht. Die für die Suzuki-Reaktion eingesetzten 4-Pyridinylboronsäuren und -ester (IXa/b/c, Met=B(OH)₂ bzw. 4,4,5,5-Tetramethyl-1,3,2-dioxaborolan-2-yl) sind kommerziell verfügbar oder aus den entsprechenden 4-Brompyridinen mittels Pd-katalysierter Umsetzung mit Bispinacolatodiboran (z. B. Bioorg. Med. Chem. Lett. 2006, 16, 1277-1281) oder durch Metallierung/Borierung (z. B. Synthesis, 2003, 469-483) herstellbar.

Als Lösungsmittel für die Suzuki-Reaktion können alle üblichen, unter den Reaktionsbedingungen inerten Lösungsmittel, wie Alkohole (z.B. Ethanol, Ethylenglykol), zyklische und azyklische Ether (Dimethoxymethan, Tetrahydrofuran, Dioxan), aromatische Kohlenwasserstoffe (z.B. Toluol), Ketone (z.B. Aceton, Ethylmethylketon), Nitrile (z.B. Acetonitril, Propionitril,) und Amide (z.B. Dimethylformamid, Dimethylacetamid, N-Methylpyrrolidon) und Wasser verwendet werden oder die Reaktion kann in Mischungen von zwei oder mehreren dieser Lösungsmittel ausgeführt werden. Die bevorzugten Lösungsmittel sind Dioxan oder Tetrahydrofuran.

Geeignete Basen sind Alkalimetallhydroxide, Alkalimetallcarbonate, Alkalimetallhydrogencarbonate, Alkalimetallacetate, Alkalimetallalkoholate, sowie tertiäre Amine. Bevorzugte Basen sind Cäsiumcarbonat, Natriumcarbonat, Kaliumcarbonat oder Kaliumacetat.

Bevorzugt zur Durchführung des Verfahrensschritt [V12] sind Palladiumkatalysatoren, in denen das Palladium in der Oxidationsstufe (0) oder (II) vorliegt, wie z.B. Tetrakis(triphenylphosphin)-palladium, Bis(triphenylphosphin)-palladiumdichlorid und Bis(diphenylphosphino)ferrocenpalladium-dichlorid oder auch Palladium(II)acetat und Palladium(II)chlorid.

Der Katalysator kann phosphorhaltige Liganden enthalten oder es können dem Reaktionsgemisch phosphorhaltige Liganden separat zugesetzt werden. Als phosphorhaltige Liganden eignen sich bevorzugt Tri-n-alkylphosphane, Triarylphosphane, Dialkylarylphosphane, Alkyldiarylphosphane und/oder Heteroarylphosphane, wie Tripyridylphosphan und Trifurylphosphan, wobei die drei Substituenten am Phosphor gleich oder verschieden sein können und wobei einer oder mehrere Substituenten die Phosphorgruppen mehrerer Phosphane verknüpfen können, wobei ein Teil dieser Verknüpfung auch ein Metallatom sein kann. Besonders bevorzugt sind Phosphane wie Triphenylphosphan, Tri-tert-butylphosphan, Tricyclohexylphosphan.

Die Suzuki Kupplung wird in einem Temperaturbereich von 25 ° bis 200 °C, besonders bevorzugt bei 80 ° bis 150 °C durchgeführt. Die Reaktionszeit variiert in Abhängigkeit vom Maßstab der Reaktion und der Reaktionstemperatur, aber liegt im Allgemeinen zwischen einigen Minuten und 48 Stunden.

Als alternative C-C-Kupplungsmethode für Verfahrensschritt [V12] können die 4-Halogenpyrazole (VIII) auch in einer Stille-Reaktion (z.B. analog. Med. Chem. Lett. 2006, 16, 3550) mit Pyridinylstannanen (IXa/b/c, Met = Sn(Alkyl)₃) zu den Pyridinylpyrazolen (Ia), (If) oder (Ig) umgesetzt werden. Die Darstellung von 4-Pyridinylstannanen (IX) ist ebenfalls literaturbekannt (z.B. J. Med. Chem. 2003, 46, 284-302; Tetrahedron 2004, 60, 6113-6120). Für die Stille-Kupplung kann die Auswahl eines Katalysators, gegebenenfalls eines anorganischen oder organischen Halogenid-Salzes, gegebenenfalls eines Liganden und eines geeigneten Lösungsmittels bei geeigneten Temperaturen je nach eingesetztem Zinnalkyl-Substrat variieren.

Als Lösungsmittel für die Stille-Kupplung können alle üblichen, unter den Reaktionsbedingungen inerten Lösungsmittel, wie zyklische und azyklische Ether (z. B. Dimethoxymethan, Tetrahydrofuran, Dioxan), aromatische Kohlenwasserstoffe (z.B. Toluol), Amide (z.B. Dimethylformamid, Dimethylacetamid, N-Methylpyrrolidon) und Sulfoxide (z.B. Dimethylsufoxid) verwendet werden oder die Reaktion kann in Mischungen von zwei oder mehreren dieser Lösungsmittel ausgeführt werden.

Halogenidsalze, die vorzugsweise Verwendung finden, sind z.B. Kupferhalogenide (z.B. CuBr oder CuI), Cäsiumhalogenide (z. B. CsF) und Tetraalkylammoniumhalogenide (z. B. TBAF).

Bevorzugt sind Palladiumkatalysatoren, in denen das Palladium in der Oxidationsstufe (0) oder (II) vorliegt, wie z.B. Tetrakis(triphenylphosphin)-palladium, Bis(triphenylphosphin)-palladiumdichlorid und Bis(diphenylphosphino)ferrocenpalladium-dichlorid oder auch Palladium(II)acetat und Palladium(II)chlorid.

Der Katalysator kann phosphorhaltige Liganden enthalten oder es können dem Reaktionsgemisch phosphorhaltige Liganden separat zugesetzt werden. Als phosphorhaltige Liganden eignen sich bevorzugt Tri-n-alkylphosphane, Triarylphosphane, Dialkylarylphosphane, Alkyldiarylphosphane und/oder Heteroarylphosphane, wie Tripyridylphosphan und Trifurylphosphan, wobei die drei Substituenten am Phosphor gleich oder verschieden sein können und wobei einer oder mehrere Substituenten die Phosphorgruppen mehrerer Phosphane verknüpfen können, wobei ein Teil dieser Verknüpfung auch ein Metallatom sein kann. Besonders bevorzugt sind Phosphane wie Triphenylphosphan, Tri-tert-butylphosphan, Tricyclohexylphosphan.

Die Stille-Kupplung wird in einem Temperaturbereich von 25 ° - 200 °C, besonders bevorzugt bei 60°-150 °C durchgeführt. Die Reaktionszeit variiert in Abhängigkeit vom Maßstab der Reaktion und der Reaktionstemperatur, aber liegt im Allgemeinen zwischen einigen Minuten und 48 Stunden.

Alternativ zu den in Schema 7 beschriebenen Verfahren lassen sich die intermediären Bicyclischen Pyrazole (VII) und (VIII) auch nach folgendem allgemeinen Schema aus den mit geeigneten Substituenten Y¹ und Y² versehenen monocyclischen Pyrazolen (X) herstellen (Schema 8):

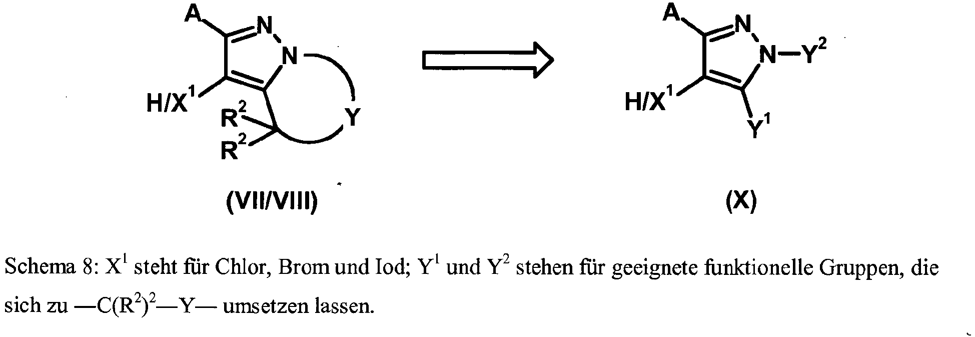

Exemplarisch für die in Schema 8 beschriebene Retrosynthese seien hier die folgenden Verfahren aufgeführt (Schema 9 und Schema 10):

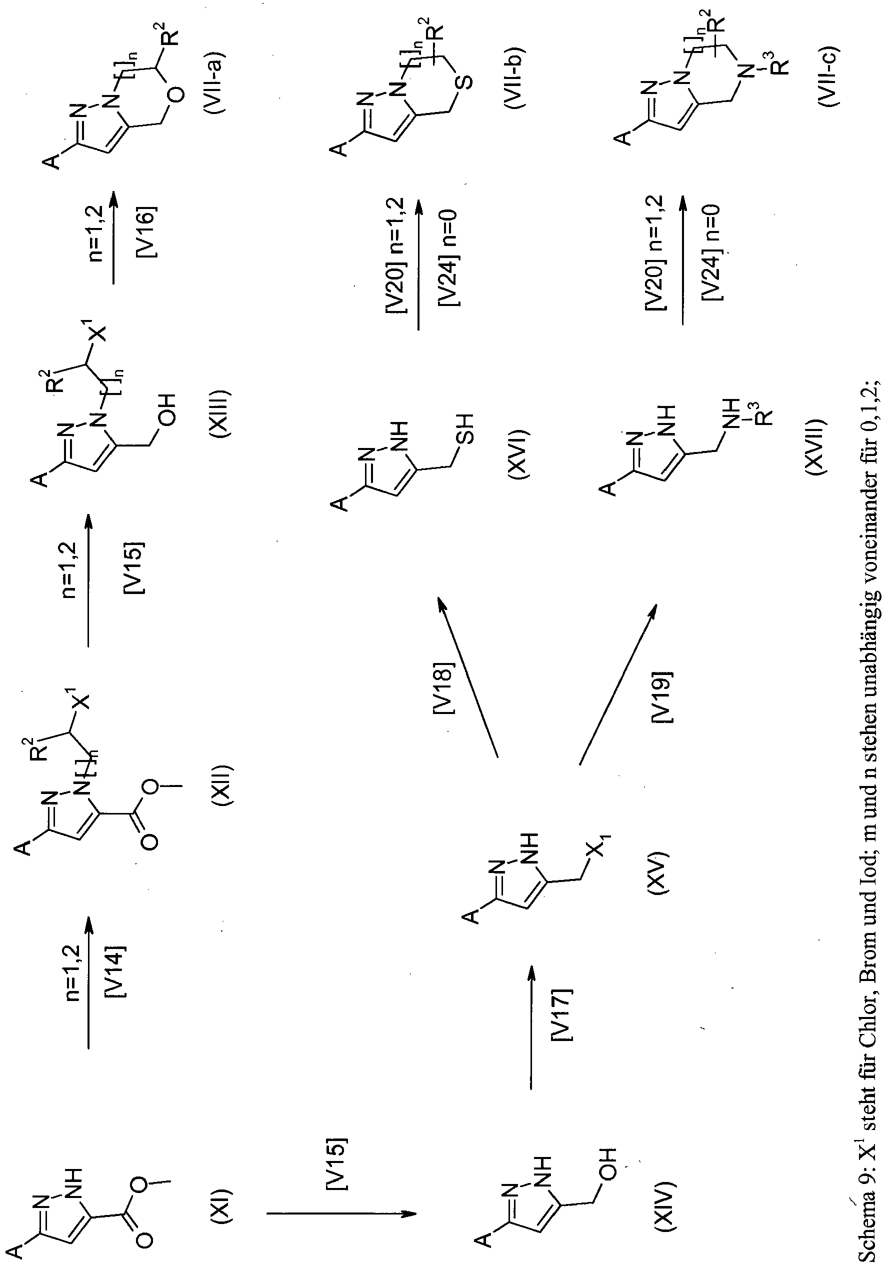

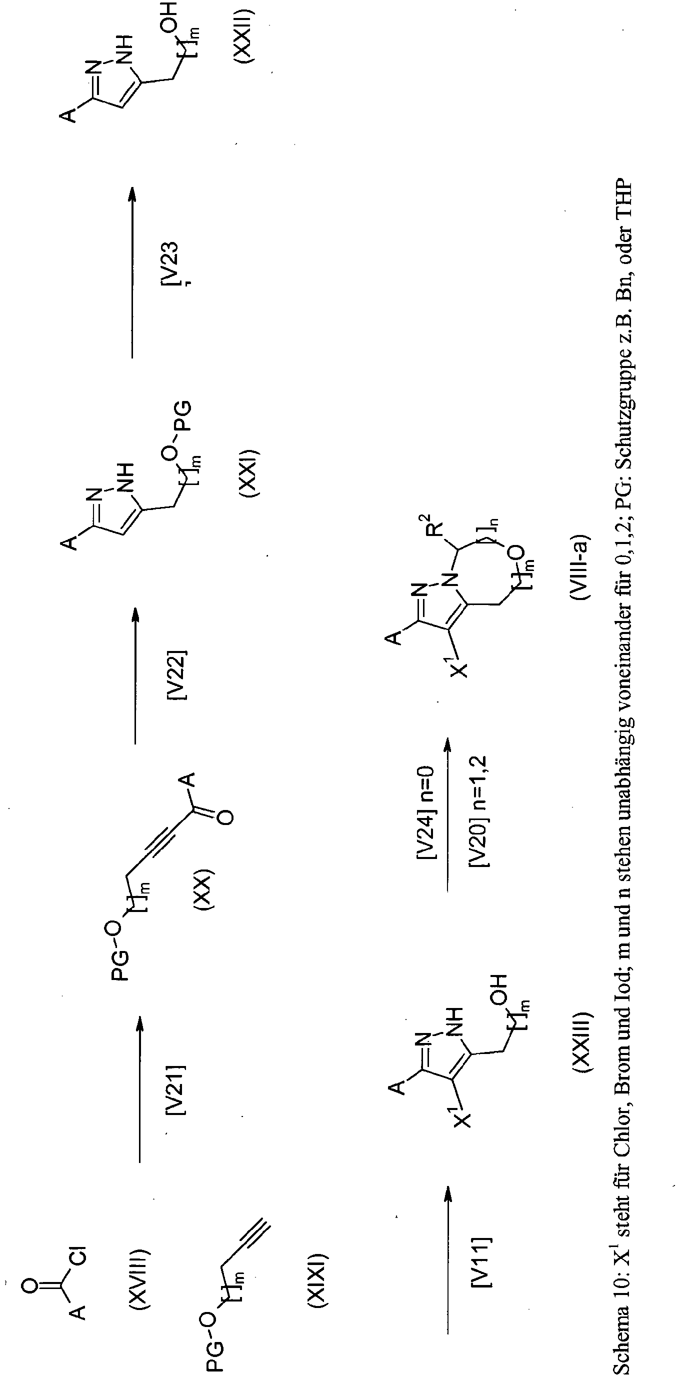

### Verfahrenschritt [V14]:

Pyrazolester der allgemeinen Formel (XI) lassen sich mit geeigneten Bisalkylidenhalogeniden (z.B. 1-Brom-2-chlorethan) gegebenenfalls in Gegenwart einer Base und gegebenenfalls in Gegenwart eines Lösungsmittels nach literaturbekanntem Verfahren (z.B. Bioorg. Med. Chem. 2008, 16, 10165-10171) in die entsprechenden N-Haloalkylpyrazolester (X¹ = Cl, Br, I) der allgemeinen Formel (XII) überführen. Die Pyrazolester (XI) sind entweder kommerziell verfügbar oder lassen sich nach literaturbekannten Verfahren, z.B. Zhejiang Daxue Xuebao, Lixueban (2008), 35(6), 641-643, aus wohlfeilen Acetophenonen herstellen.

Geeignete Basen sind Alkalimetallhydroxide, Alkalimetallhydride, Alkalimetallcarbonate, sowie tertiäre Amine. Bevorzugte Basen sind Natriumhydrid, Natriumcarbonat, Kaliumcarbonat oder Cäsiumcarbonat. Als Lösungsmittel können alle üblichen, unter den Reaktionsbedingungen inerten Lösungsmittel, wie zyklische und azyklische Ether (Dimethoxymethan, Tetrahydrofuran, Dioxan), aromatische Kohlenwasserstoffe (z.B. Toluol), Nitrile (z.B. Acetonitril, Propionitril,), Ketone (z.B. Aceton) und Amide (z.B. Dimethylformamid, Dimethylacetamid, N-Methylpyrrolidon) verwendet werden oder die Reaktion kann in Mischungen von zwei oder mehreren dieser Lösungsmittel ausgeführt werden. Die bevorzugten Lösungsmittel sind Dimethylformamid, Aceton oder Acetonitril.

Die Reaktionstemperatur liegt zwischen 0 °C und 180 °C, vorzugsweise zwischen Raumtemperatur und 100 °C.

### Verfahrenschritt [V15]:

Pyrazolester der allgemeinen Formel (XI) oder (XII) lassen sich mit geeigneten Reduktionsmitteln nach literaturbekanntem Verfahren (z.B. WO 2007/018314) in die entsprechenden Alkohole der allgemeinen Formel (XIII) bzw. (XIV) überführen.

Als geeignte Reduktionsmittel dienen hierfür Alanate oder Boronate (z.B. LiAlH₄, DIBAL-H, LiBH₄, für R² = H) oder Grignard-Verbindungen (z.B. MeMgCl für R² = Me).

Als Lösungsmittel können alle üblichen, unter den Reaktionsbedingungen inerten Lösungsmittel, wie zyklische und azyklische Ether (Dimethoxymethan, Tetrahydrofuran, Dioxan) oder Kohlenwasserstoffe (z.B. Toluol, Hexan) oder Gemische eingesetzt werden.

### Verfahrenschritt [V16]:

Hydroxymethylpyrazole der allgemeinen Formel (XIII) lassen sich gegebenenfalls in Gegenwart einer Base und gegebenenfalls in Gegenwart eines Lösungsmittels in die entsprechenden Bicyclischen Pyrazole der allgemeinen Formel (VIIa) cyclisieren. Geeignete Basen sind Alkalimetallhydroxide, Alkalimetallhydride, Alkalimetallcarbonate, Alkalimetallacetate, Alkalimetallalkoholate, sowie tertiäre Amine. Bevorzugte Basen sind Natriumhydrid, Natriumcarbonat, Kaliumcarbonat oder Cäsiumcarbonat.

Als Lösungsmittel können alle üblichen, unter den Reaktionsbedingungen inerten Lösungsmittel, wie zyklische und azyklische Ether (Dimethoxymethan, Tetrahydrofuran, Dioxan), aromatische Kohlenwasserstoffe (z.B. Toluol), Nitrile (z.B. Acetonitril, Propionitril,), Ketone (z.B. Aceton) und Amide (z.B. Dimethylformamid, Dimethylacetamid, N-Methylpyrrolidon) verwendet werden oder die Reaktion kann in Mischungen von zwei oder mehreren dieser Lösungsmittel ausgeführt werden. Die bevorzugten Lösungsmittel sind Dimethylformamid, Aceton oder Acetonitril.

Die Reaktionstemperatur liegt zwischen 0 °C und 180 °C, vorzugsweise zwischen Raumtemperatur und 100 °C.

### Verfahrenschritt [V17]:

Hydroxymethylprazole der allgemeinen Formel (XIV) lassen sich mit geeigneten Halogenierungsreagenzien nach literaturbekanntem Verfahren (z.B. Bioorg. Med. Chem. Lett. 2007, 17, 5199-5203) in die entsprechenden Halomethyl-Pyrazole (X¹ = Cl, Br, I) der allgemeinen Formel (XV) überführen. Als Halogenierungsmittel können z.B. Thionylchlorid, Pyridiniumtribromid, Br₂/PPh₃, oder I₂/PPh₃ verwendet werden. Die Halogenierung kann gegebenenfalls in Gegenwart eines unter den Reaktionsbedingungen inerten Lösungsmittels durchgeführt werden. Bevorzugt sind zyklische und azyklische Ether (z.B. Diethylether, Tetrahydrofuran, Dimethoxyethan), Amide (z.B. Dimethylformamid, Dimethylacetamid), Sulfoxide (z.B. Dimethylsulfoxid), aromatische Kohlenwasserstoffe (z.B. Toluol), halogenierte Kohlenwasserstoffe (z.B. Dichlormethan, Chloroform). Die Reaktionstemperatur liegt zwischen 0 °C und dem Siedepunkt des Lösungsmittels, vorzugsweise zwischen Raumtemperatur und 80 °C.

### Verfahrenschritt [V18]:

Halomethylprazole der allgemeinen Formel (XV) lassen sich in die entsprechenden Sulfanylmethyl-Pyrazole der allgemeinen Formel (XVI) überführen. Als Schwefelungsreagenz können z.B. Schwefelwasserstoff, Natriumsulfid, Thioharnstoff oder Kaliumthioacetat verwendet werden.

Als geeignete Lösungsmittel dienen alle unter den Reaktionsbedingungen inerten Lösungsmittel sowie -gemische daraus. Bevorzugt sind Alkohole (z.B. Methanol, Ethanol), Wasser, zyklische und azyklische Ether (z.B. Dioxan, Dimethoxyethan), Amide (z.B. Dimethylformamid, Dimethylacetamid), Ketone (z.B. Aceton) und Nitrile (z.B. Acetonitril). Gegebenenfalls können Hilfsbasen, wie z.B. Alkalicarbonte (Natrium- Kaliumcarbonat, Natriumhydrogencarbonat), Alkalihydroxide (z.B. Natrium-, Kaliumhydroxid), Aromatische Amine (z.B. Pyridin) oder Tertiäre Amine (Triethylamin, Ethyldiisopropylamin) dem Reaktionsgemisch zugesetzt werden

Die Reaktionstemperatur liegt zwischen 0 °C und dem Siedepunkt des Lösungsmittels, vorzugsweise zwischen Raumtemperatur und 80 °C.

### Verfahrenschritt [V19]:

Halomethylprazole der allgemeinen Formel (XV) lassen sich nach literaturbekanntem Verfahren (z.B. Journal of Medicinal Chemistry 2010, 53, 1473-1482) mittels entsprechender Amine H₂NR³ in Aminomethyl-Pyrazole der allgemeinen Formel (XVII) überführen.

Als geeignete Lösungsmittel dienen alle unter den Reaktionsbedingungen inerten Lösungsmittel sowie -gemische daraus. Bevorzugt sind Alkohole (z.B. Methanol, Ethanol), Wasser, zyklische und azyklische Ether (z.B. Dioxan, Tetrahydrofuran), Amide (z.B. Dimethylformamid, Dimethylacetamid) und Nitrile (z.B. Acetonitril). Gegebenenfalls können Hilfsbasen, wie Alkalicarbonte (z.B. Natrium- Kaliumcarbonat, Natriumhydrogencarbonat), Aromatische Amine (z.B. Pyridin) oder Tertiäre Amine (Triethylamin, Ethyldiisopropylamin) dem Reaktionsgemisch zugesetzt werden

Die Reaktionstemperatur liegt zwischen 0 °C und dem Siedepunkt des Lösungsmittels, vorzugsweise zwischen Raumtemperatur und 80 °C.

### Verfahrenschritt [V20]:

5-Sulfanylmethyl- oder 5-Aminomethylpyrazole der allgemeinen Formel (XVI) und (XVII) lassen sich mit geeigneten Bisalkylidenhalogeniden (z.B. 1,2-Dibromethan) oder Bisalkylidenmesylaten und tosylaten (z.B. Ethylendimesylat) zu den bicyclischen Pyrazolen (VIIb) und (VIIc) zyklisieren. Als Lösungsmittel können alle üblichen, unter den Reaktionsbedingungen inerten Lösungsmittel, wie zyklische und azyklische Ether (Dimethoxymethan, Tetrahydrofuran, Dioxan), aromatische Kohlenwasserstoffe (z.B. Toluol), Nitrile (z.B. Acetonitril, Propionitril,), Ketone (z.B. Aceton) und Amide (z.B. Dimethylformamid, Dimethylacetamid, N-Methylpyrrolidon) verwendet werden oder die Reaktion kann in Mischungen von zwei oder mehreren dieser Lösungsmittel ausgeführt werden. Die bevorzugten Lösungsmittel sind Dimethylformamid, Aceton oder Acetonitril.

Gegebenenfalls können dem Reaktionsgemisch auch geeignete Basen wie Alkalimetallcarbonate oder tertiäre Amine zugesetzt werden. Bevorzugte Basen sind Natrium-, oder Kaliumcarbonat sowie Triethylamin. Die Reaktionstemperatur liegt zwischen 0 °C und 180 °C, vorzugsweise zwischen Raumtemperatur und 100 °C.

### Verfahrenschritt [V21]:

Ausgehend von kommerziell verfügbaren Benzosäurechloriden und terminalen Alkinen der allgemeinen Formeln (XVIII und XIX) lassen sich nach literaturbekannten Verfahren (z.B. Organic Letters 2008, 10, 2629-2632) in einer Sonogashira Reaktion analog zu Verfahrenschritt [V2] oder unter basischen Bedingungen (z.B. Organic Letters 2010, 12, 1952-1955) Alkinylarylketone der allgemeinen Formel (XX) herstellen. Als geeignte Basen dienen hierzu bevorzugt Butyllithium und Grignard-Verbindungen. Als Lösungsmittel können alle üblichen, unter den Reaktionsbedingungen inerten Lösungsmittel, wie zyklische und azyklische Ether (Dimethoxymethan, Tetrahydrofuran, Diethylether), oder Kohlenwasserstoffe (z.B. Toluol, Hexan), verwendet werden oder die Reaktion kann in Mischungen von zwei oder mehreren dieser Lösungsmittel ausgeführt werden. Die Reaktionstemperatur liegt zwischen -78 °C und dem Siedepunkt des Lösungsmittels, vorzugsweise zwischen - 70 °C und Raumtemperatur.

### Verfahrenschritt [V22]:

Alkinylarylketone der allgemeinen Formel (XX) lassen sich nach literaturbekanntem Verfahren (z. B. Tetrahedron Letters 1989, 30, 2049-52) mit Hydrazin in die ensprechenden Pyrazole (XXI) überführen. Die bevorzugten Lösungsmittel für die Umsetzung sind Alkohole, wie Methanol und Ethanol, Wasser und Essigsäure sowie deren Gemische. Die Reaktionstemperatur liegt zwischen 0 °C und dem Siedepunkt des Lösungsmittels vorzugsweise zwischen Raumtemperatur und 80 °C.

### Verfahrenschritt [V23]:

Die mit einer geeigneten Schutzgruppe blockierten Pyrazol-Alkohole können in Verfahrensschritt [V23] nach literaturbekannten Verfahren (vgl. Greene/Wuts Protective Groups in Organic Synthesis, Wiley, 1999 oder Kocienski, Protecting Groups, Thieme, 2005) in die freien Pyrazol-Alkohole überführt werden. So läßt sich beispielsweise eine Benzyl-Schutzgruppe hydrogenolytisch oder mittels FeCl₃ abspalten, eine säurelabile THP-Schutzgruppe mittels Toluolsulfonsäure.

### Verfahrenschritt [V24]:

5-Sulfanylalkyl-, 5-Aminoalkyl- und 5-Hydroxyalkyl-pyrazole der allgemeinen Formel (XVI), (XVII) und (XIII) lassen sich nach literaturbekanntem Verfahren (vgl. Synthesis 1979, 440-1) zu den bicyclischen Pyrazolen der allgemeinen Formel (VIIb), (VIIc) und (VIIIa) cyclisieren. Hierfür dienen entsprechende Aldehyde (z.B. Formaldehyde, acetaldehyd) oder Ketone (z.B. Aceton) als Reaktionspartner. Gegebenenfalls kann dem Reaktionsgemsich eine Säure, wie z.B. p-Toluolsulfonsäure, als Katalysator zugesetzt werden. Als Lösungsmittel kann der Reaktionspartner (z.B. Aceton) selbst oder alle üblichen, unter den Reaktionsbedingungen inerten Lösungsmittel, wie zyklische und azyklische Ether (Dimethoxymethan, Tetrahydrofuran, Dioxan), aromatische Kohlenwasserstoffe (z.B. Toluol), Nitrile (z.B. Acetonitril, Propionitril,), Amide (z.B. Dimethylformamid, Dimethylacetamid, N-Methylpyrrolidon) verwendet werden. Die Reaktionstemperatur liegt zwischen 0 °C und dem Siedepunkt des Lösungsmittels vorzugsweise zwischen Raumtemperatur und 100 °C.

Eine alternative Syntheseweg zum Verbindungen VII und VIII findet sich in Schema 11:

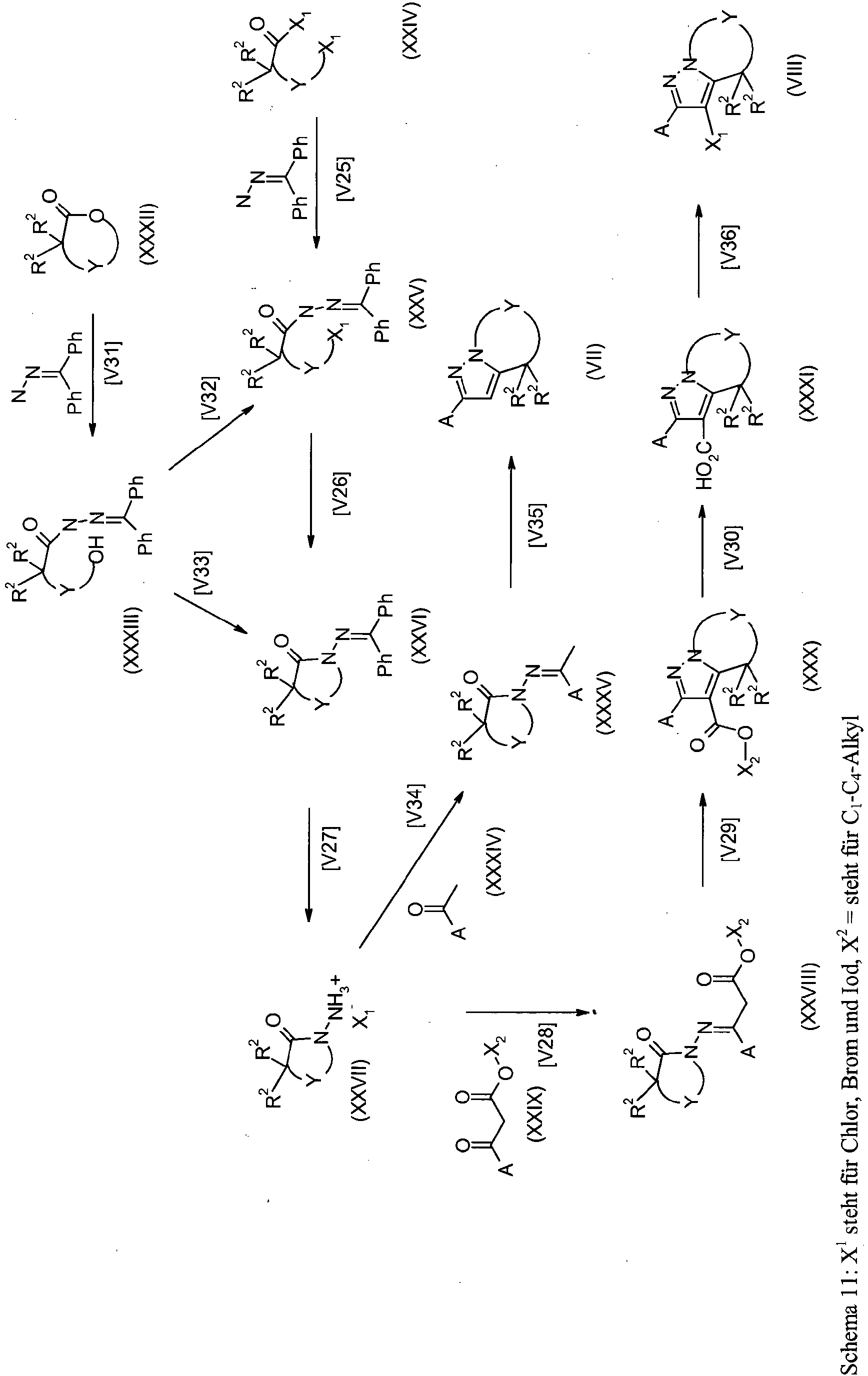

### Verfahrenschritt [V25]:

Die Umsetzung von Verbindungen der allgemeinen Formel (XXIV) mit Benzophenonhydrazon gegebenenfalls in Gegenwart eines Lösungsmittels und einer Base liefert nach literaturbekannten Methoden (z.B. J. Am. Chem. Soc. 1981, 103, 7743-7752) Verbindungen der allgemeinen Formel (XXV). Die hierfür benötigten Verbindungen (XXIV) sind entweder kommerziell verfügbar oder lassen sich beispielsweise nach literaturbekannten Methoden aus den Laktonen (Synthesis 2008, 20, 3229-3236) gewinnen.

Als geeignete Lösungsmittel zur Durchführung des Verfahrensschrittes [V25] dienen alle unter den Reaktionsbedingungen inerten Lösungsmittel sowie Gemische daraus. Bevorzugt ist Dichloromethan. Geeignete Basen sind Pyridin sowie tertiäre Amine. Bevorzugt ist Pyridin. Die Reaktionstemperatur liegt zwischen 0 °C und 100 °C. Die Reaktionszeit zur Durchführung beträgt in Abhängigkeit vom Maßstab der Reaktion zwischen 12 und 96 h, bevorzugt zwischen 16 h und 24 h.

### Verfahrenschritt [V26]:

Die Umsetzung von Verbindungen der allgemeinen Formel (XXV) gegebenenfalls in Gegenwart eines Lösungsmittels und einer Base liefert nach literaturbekannten Methoden (z.B. J. Am. Chem. Soc. 1981, 103, 7743-7752) Verbindungen der allgemeinen Formel (XXVI).

Als geeignete Lösungsmittel dienen alle unter den Reaktionsbedingungen inerten Lösungsmittel sowie Gemische daraus. Bevorzugt ist Tetrahydrofuran. Geeignete Basen sind Alkalimetallhydride. Bevorzugt ist Natriumhydrid. Die Reaktionstemperatur liegt zwischen 0 °C und 50 °C. Die Reaktionszeit beträgt in Abhängigkeit vom Maßstab der Reaktion zwischen 12 und 96 h, bevorzugt zwischen 16 h und 24 h.

### Verfahrenschritt [V27]:

Die Umsetzung von Verbindungen der allgemeinen Formel (XXVI) gegebenenfalls in Gegenwart eines Lösungsmittels und einer Säure liefert nach literaturbekannten Methoden (z.B. WO2007018818) N-Amino-Lactame der allgemeinen Formel (XXVII).

Als geeignete Lösungsmittel zur Durchführung des Verfahrensschrittes [V27] dienen alle unter den Reaktionsbedingungen inerten Lösungsmittel sowie Gemische daraus. Bevorzugt ist Tetrahydrofuran. Geeignete Säuren sind anorganische Säure, sowie organische Säure. Bevorzugt ist Salzsäure. Die Reaktionstemperatur zur Durchführung des Verfahrensschrittes [V27] liegt zwischen 0 °C und 50 °C. Die Reaktionszeit beträgt in Abhängigkeit vom Maßstab der Reaktion zwischen 5 min und 2h, bevorzugt 30 min.

### Verfahrenschritt [V28]:

Die Umsetzung von N-Amino-Lactamen der allgemeinen Formel (XXVII) mit Ketoestern (XXIX) liefert nach literaturbekannten Methoden (z.B. Tetrahedron 2007, 63, 11763-11770) Verbindungen der allgemeinen Formel (XXIX) Die hierfür benötigten Ketoester (XXVIII) sind entweder kommerziell verfügbar oder lassen sich beispielsweise nach literaturbekannten Methoden *(*Helv. Chim. Acta 2010, 93, 1261-1273) gewinnen.

Als geeignete Lösungsmittel dienen alle unter den Reaktionsbedingungen inerten Lösungsmittel sowie Gemische daraus. Bevorzugt ist Pyridin. Die Reaktionstemperatur liegt zwischen 0 °C und 50 °C. Die Reaktionszeit zur Durchführung des Verfahrensschrittes [V28] beträgt in Abhängigkeit vom Maßstab der Reaktion zwischen 24 h und 72 h, bevorzugt 48 h.

### Verfahrenschritt [V29]:

Wie z.B. in z.B. Eur. J. Med. Chem 1984, 19, 215-218 beschrieben lassen sich Verbindungen der allgemeinen Formel (XXIX) unter basischen Bedingungen zu Pyrazolestern der allgemeinen Formel (XXX) cyclisieren.

Als geeignete Lösungsmittel dienen alle unter den Reaktionsbedingungen inerten Lösungsmittel sowie Gemische daraus. Bevorzugt ist Dimethylformamid. Geeignete Basen sind Alkalimetallhydride und Alkalicarbonate. Bevorzugt ist Cäsiumcarbonat. Die Reaktionstemperatur liegt zwischen 0 °C und 150 °C. Die Reaktionszeit beträgt in Abhängigkeit vom Maßstab der Reaktion zwischen 2 h und 8 h, bevorzugt zwischen 4 h und 6 h.

### Verfahrenschritt [V30]:

Die Hydrolyse von Pyrazolestern der allgemeinen Formel (XXX) liefert Pyrazolcarbonsäuren der allgemeinen Formel (XXXI) nach literaturbekannten Methoden (z.B. Org. Lett. 2005, 7, 4753-4756).

Als geeignete Lösungsmittel dienen alle unter den Reaktionsbedingungen inerten Lösungsmittel sowie Gemische daraus. Bevorzugt ist Methanol. Geeignete Basen sind Alkalihydroxide. Bevorzugt ist Natriumhydroxid. Die Reaktionstemperatur liegt zwischen 0 °C und 100 °C. Die Reaktionszeit beträgt in Abhängigkeit vom Maßstab der Reaktion zwischen 1 h und 8 h, bevorzugt zwischen 3 h und 6 h.

### Verfahrenschritt [V31]:

Die Umsetzung von Lactonen der allgemeinen Formel (XXXII) mit Benzophenonhydrazon in Gegenwart einer Lewis-Säure liefert die Verbindungen der allgemeinen Formel (XXXIII).

Als geeignete Lösungsmittel dienen alle unter den Reaktionsbedingungen inerten Lösungsmittel sowie Gemische daraus. Bevorzugt ist Dichloromethan. Geeignete Lewis Säuren sind Trialkylaluminium-Derivate. Bevorzugt ist Trimethylaluminium. Die Reaktionstemperatur liegt zwischen 0 °C und 100 °C. Die Reaktionszeit beträgt in Abhängigkeit vom Maßstab der Reaktion zwischen 1 h und 8 h, bevorzugt zwischen 3 h und 6 h.

### Verfahrenschritt [V32]:

Die Umsetzung von Alkoholen der allgemeinen Formel (XXXIII) mit einem geeigneten Halogenierungsreagenz liefert Verbindungen der allgemeinen Formel (XXV) nach literaturbekannten Methoden (z.B. Artikel "Triphenylphosphine-Carbon Tetrabromide" im Encyclopeadia of Reagents for Organic Synthesis, Wiley, 1995).

Als geeignete Lösungsmittel dienen alle unter den Reaktionsbedingungen inerten Lösungsmittel sowie Gemische daraus. Bevorzugt ist Dichloromethan. Geeignete Halogenierungsreagenzien sind z.B. Triphenylphosphan/Halogenkohlenwasserstoff Kombinationen, und Halogenschwefel Verbindungen (z.B. Thionylchlorid). Bevorzugt ist die Kombination Triphenylphosphan/Tetrabrommethan. Die Reaktionstemperatur liegt zwischen 0 °C und 100 °C. Die Reaktionszeit beträgt in Abhängigkeit vom Maßstab der Reaktion zwischen 30 min und 8 h, bevorzugt 1 h.

### Verfahrenschritt [V33]:

Die Umsetzung von Verbindungen der allgemeinen Formel (XXXIII) mit einer Halogensulfonsäure und gegebenenfalls einer Base liefert die Verbindungen der allgemeinen Formel (XXVI).

Als geeignete Lösungsmittel dienen alle unter den Reaktionsbedingungen inerten Lösungsmittel sowie Gemische daraus. Bevorzugt sind Dichloromethan und Tetrahydrofuran. Geeignete Halogensulfonsäuren sind z.B. Methylsulfonylchlorid und Tolylsulfonylchlorid. Bevorzugt ist Methylsulfonylchlorid. Geeignete Basen sind Pyridin-Derivate und Alkalimetallhydride. Bevorzugt sind Pyridin und Natriumhydrid. Die Reaktionstemperatur liegt zwischen 0 °C und 100 °C. Die Reaktionszeit beträgt in Abhängigkeit vom Maßstab der Reaktion zwischen 6 h und 48 h, bevorzugt zwischen 12 h und 24 h.

### Verfahrenschritt [V34]:

Die Umsetzung von N-Amino-Lactamen der allgemeinen Formel (XXVII) mit Acetophenonen der allgemeinen Formel (XXXIV) gegebenenfalls in Gegenwart eines Lösungsmittels liefert die Verbindungen der allgemeinen Formel (XXXV) nach literaturbekannten Methoden (z.B. Eur. J. Med. Chem 2010, 45, 3384-3388). Die hierfür benötigten Acetophenone (XXXIV) sind kommerziell verfügbar.

Als geeignete Lösungsmittel dienen alle unter den Reaktionsbedingungen inerten Lösungsmittel sowie Gemische daraus. Bevorzugt ist Ethanol. Die Reaktionstemperatur liegt zwischen 0 °C und 100 °C. Die Reaktionszeit beträgt in Abhängigkeit vom Maßstab der Reaktion zwischen 6 h und 12 h, bevorzugt 12 h.

### Verfahrenschritt [V35]:

Die Umsetzung von Verbindungen der allgemeinen Formel (XXXV) mit Essigsäureahydrid gegebenenfalls in Gegenwart eines Lösungsmittels liefert Pyrazole der allgemeinen Formel (VII) nach literaturbekannten Methoden (z.B. Zeitschrift für Naturforschung-B 2003, 58, 678-685).

Als geeignete Lösungsmittel dienen alle unter den Reaktionsbedingungen inerten Lösungsmittel sowie Gemische daraus. Bevorzugt ist Essigsäureanhydrid. Die Reaktionstemperatur liegt zwischen 0 °C und 150 °C. Die Reaktionszeit beträgt in Abhängigkeit vom Maßstab der Reaktion zwischen 2 h und 8 h, bevorzugt zwischen 4 h und 6 h.

### Verfahrenschritt [V36]:

Die Umsetzung von Pyrazolcarbonsäuren der allgemeinen Formel (XXXI) mit einer Base und eines Halogenierungreagenzes liefert nach literaturbekannten Methoden (z.B. Org. Lett. 2005, 7, 4753-4756) 5-Halopyrazole der allgemeinen Formel (VIII).

Als geeignete Lösungsmittel dienen alle unter den Reaktionsbedingungen inerten Lösungsmittel sowie Gemische daraus. Bevorzugt ist Dimethylformamid. Geeignete Halogenierungreagenzien sind Halogensuccinimid Reagenzien. Bevorzugt ist N-Jodsuccinimid. Geeignete Basen sind Alkalicarbonate. Bevorzugt ist Natriumhydrogencarbonat. Die Reaktionstemperatur liegt zwischen 0 °C und 100 °C. Die Reaktionszeit beträgt in Abhängigkeit vom Maßstab der Reaktion zwischen 1 h und 48 h, bevorzugt 12 h.

Neu und ebenfalls Teil der Erfmdung sind Verbindungen der allgemeinen Formel (VIIIb) 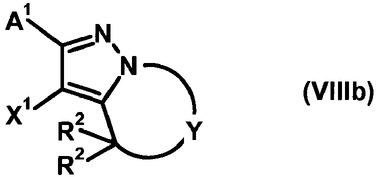 in welcher die Symbole die folgenden Bedeutungen haben:
- A¹ steht für einen Phenylring der gegebenenfalls einfach oder mehrfach an der 3-, 4- oder 5-Position mit R⁷ subtituiert sein kann oder für einen Thiophenylring der gegebenenfalls einfach oder mehrfach mit R⁸ substituiert sein kann,
- R², R⁷, R⁸ und Y haben die oben angegebenen allgemeinen, bevorzugten, besonders bevorzugten oder ganz besonders bevorzugten Bedeutungen und
- X¹ steht für Chlor, Brom Jod;
mit der Maßgabe, dass
3-Brom-5,6-dihydro-2-phenyl-4H-Pyrrolo[1,2-b]pyrazole, 3-Iod-2-phenyl-Pyrazolo[1,5-c]pyrimidine-7(6H)-thion und 3-Brom-2-phenyl-Pyrazolo[1,5-c]pyrimidine-7(6H)-thion ausgenommen sind.

Neu und ebenfalls Teil der Erfindung sind Verbindungen der allgemeinen Formel (VIIx) 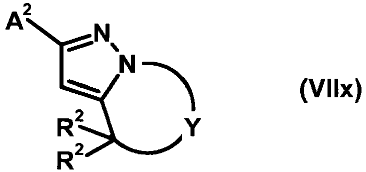 in welcher die Symbole die folgenden Bedeutungen haben:
- A² steht für einen gegebenenfalls einfach oder mehrfach substituierten Thiophenylring oder für einen gegebenenfalls einfach oder mehrfach an der 3-, 4- oder 5-Position substituierten Phenylring, wobei die Substituenten ausgewählt sind aus der Gruppe F, Cl, Br, I, Cyano, Methyl, Ethyl, iso-Propyl, n-Propyl,Trifluormethyl, Difluormethyl, Dichlormethyl, Pentafluorethyl, Methoxy, Ethoxy, n-Propoxy, iso-Propoxy;
- Y bildet zusammen mit dem angrenzenden Stickstoffatom "1" und den beiden Kohlenstoffatomen "2" und "3" einen 5- bis 7-gliedrigen 5 nicht aromatischen heterocyclischen Ring, ausgewählt aus der Gruppe: H-2, H-3, H-4, H-5, H-6, H-7, H-8 und H-9 dargestellt in Schema 1, wobei s eine Zahl zwischen 0 und 4 ist.
- R² hat die oben angegebenen besonders bevorzugten oder ganz besonders bevorzugten Bedeutungen;

Die vorliegende Erfindung betrifft weiterhin ein Pflanzenschutzmittel zum Bekämpfen unerwünschter Pilze sowie zur Reduktion von Mykotoxinen in Pflanzen und Pflanzenteilen, umfassend wenigstens eines der bicyclischen Pyridinylpyrazol-Derivate der Formel (I). Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein nicht therapeutisches Verfahren zum Bekämpfen unerwünschter Mikroorganismen sowie zur Reduktion von Mykotoxinen in Pflanzen und Pflanzenteilen, dadurch gekennzeichnet, dass man erfindungsgemäß bicyclische Pyridinylpyrazol-Derivate der Formel (I) auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfat, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl-dispergierbare Pulver, Ölmischbare fließfähige Konzentrate, Öl-mischbare Flüssigkeiten, Schäume, Pasten, Pestizid-ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Pilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfmdungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfmdungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfmdungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke fungizide Wirkung auf und können zur Bekämpfung von unerwünschten Pilzen im Pflanzenschutz und im Materialschutz eingesetzt werden.

Die erfindungsgemäßen bicyclischen Pyridinylpyrazol-Derivate lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht werden.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfmdungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und - plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Poaceae sp.* (z.B. Zuckerrohr), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom defmiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Intemodienzahl und - abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Eltemlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfmdungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylhamstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP 1 A und VIP2A besteht.
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Emteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekomgröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor pilzlicher Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha;
Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora.

Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Altemaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southem Blight (Sclerotinia rolfsii), Thielaviopsis Root Rot (Thielaviopsis basicola).

Als Organismen, die einen Abbau oder eine Veränderung der technischen Materialien bewirken können, seien Pilze genannt. Vorzugsweise wirken die erfindungsgemäßen Wirkstoffe gegen Pilze, insbesondere Schimmelpilze, Holz verfärbende und Holz zerstörende Pilze (Basidiomyceten). Es seien beispielsweise Pilze der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride.

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfmdungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die aufgeführten Pflanzen können besonders vorteilhaft erfindungsgemäß mit den bicyclischen Pyridinylpyrazol-Derivaten der Formel (I) oder den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Herstellung und die Verwendung der erfindungsgemäßen Wirkstoffe der Formeln (I) geht aus den folgenden Beispielen hervor. Die Erfindung ist jedoch nicht auf diese Beispiele beschränkt.

### Beispiel für Verfahrensschritt [V1] aus Schema 3 & 7:

### Beispiel 2: 2-(4-Fluorphenyl)-3-(pyridin-4-yl)-4,5,6,7-tetrahydropyrazolo[1,5-a]pyridin

Ein Gemisch aus 1.64 g (11.1 mmol) 4,5,6,7-Tetrahydro[1,2,3]oxadiazolo[3,4-a]pyridin-8-ium-3-olat und 2.19 g (11.1 mmol) 4-{[4-Fluorphenyl]ethinyl}pyridin in 40 ml Mesitylen wird für 16 h bei 165°C unter Argon gerührt. Nach dem Abkühlen engt man das Reaktionsgemisch unter vermindertem Druck ein. Nach säulenchromatographischer Reinigung über Kieselgel (Cyclohexan/Ethylacetet) erhält man 888 mg (27%) des gewünschten Produktes; logP(HCOOH): 1.03; ¹H-NMR(CDCl₃-d*1*): 8.52 (d, 2H), 7.38 (dd, 2H), 7.08 (d, 2H), 7.00 (dd, 2H), 4.25 (dd, 2H), 2.85 (dd, 2H), 2.12 (m, 2H), 1.92 (m, 2H).

### Beispiel für Verfahrensschritt [V2] aus Schema 4 & 7:

### 4-{[4-(Trifluormethyl)phenyl]ethinyl}pyridin (II-1)

Ein Gemisch aus 1,20 g (6,17 mmol) 4-Brompyridin Hydrochlorid, 1,57 g (9,26 mmol) 1-Ethinyl-4-(trifluormethyl)benzol, 88 mg (0,46 mmol) Kupfer(I)iodid und 251 mg (0.31 mmol) Pd(dppf)Cl₂ in 10 ml Triethylamin werden unter Argon 3 h auf 90 °C erhitzt. Nach dem Einengen nimmt man das Reaktionsgemisch in 100 ml Ethylacetat auf und wäscht mit 2x 100ml 1M HCl. Die wässrige Phase wird mit Natronlauge auf pH10 eingestellt und mit 3x 100 ml Chloroform extrahiert. Die vereinigten organischen Phasen werden mit gesättigter wässriger NaCl-Lösung gewaschen, über Na₂SO₄ getrocknet und unter vermindertem Druck vom Lösungsmittel befreit. Man erhält 380 mg (24%) des gewünschten Produktes; logP(HCOOH): 2.86; ¹H-NMR(DMSO-d*6*) δ: 8.66 (d, 2H), 7.84 (s, 4H), 7.58 (d, 2H)

Analog lassen sich herstellen:
- **4-{[3-(Trifluormethyl)phenyl]ethinyl}pyridin (II-2);** logP(pH7): 3.50 mit MS (ESI): 248.1 ([M+H]⁺).
- **4-{[4-(Methoxy)phenyl]ethinyl}pyridin (II-3);** logP(pH7): 2,68; ¹H-NMR(DMSO-d6) δ: 8.60 (d, 2H), 7.55 (d, 2H), 7.48 (d, 2H), 7.02 (d, 2H).
- **4-{[4-Fluorphenyl]ethinyl}pyridin (II-4);** logP(pH7): 2,81; ¹H-NMR(DMSO-d6) δ: 8.63 (d, 2H), 7.68 (dd, 2H), 7.52 (d, 2H), 7.32 (dd, 2H).
- **4-{[4-Chlorphenyl]ethinyl}pyridin (II-5);** logP(HCOOH): 2,43; mit MS (ESI): 214.0/216.0 ([M+H]⁺).
- **2-Chlor-4-[(4-fluorphenyl)ethinyl]pyridin (II-6);** logP(HCOOH): 3,80 mit MS (ESI): 232.1/234.1 ([M+H]⁺).

### Beispiel für Verfahrensschritt [V3] aus Schema 5 & 7:

### 5,6,7,8-Tetrahydro-4H-[1,2,3]oxadiazolo[3,4-a]azepin-9-ium-3-olat (III-1)

Eine Lösung aus 7,80 g (43,4 mmol) Azepan-2-carbonsäure und 4,10 g (59,5 mmol) Natriumnitrit in 50ml Wasser wird bei 0°C mit HCl (konz.) auf pH3 angesäuert und anschließend 1 h gerührt. Das Reaktionsgemisch wird mit 50 ml Ethylacetat versetzt, die organische Phase abgetrennt und die wäßrige Phase mit 2x 50 ml Ethylacetat extrahiert. Die vereinigten organischen Phasen werden über MgSO4 getrocknet und unter vermindertem Druck vom Lösungsmittel befreit. Die erhaltene 1-Nitrosoazepan-2-carbonsäure (6,80 g, 67%) wird ohne weitere Aufreinigung in 15 ml Acetonitril aufgenommen und bei 0 °C tropfenweise mit 12,4 g (59,2 mmol) Trifluoressigsäureanhydrid versetzt. Nach 2 h Rühren bei Raumtemperatur setzt man 8,19 g (59,2 mmol) Kaliumcarbonat zu und lässt weitere 20 min nachrühren. Das Reaktionsgemisch wird mit 40 ml Ethylacetat und 20 ml Wasser versetzt und die wässrige Phase mit 3x 40 ml Ethylacetat extrahiert. Die vereinigten organischen Phasen werden über MgSO₄ getrocknet und unter vermindertem Druck vom Lösungsmittel befreit. Nach säulenchromatographischer Reinigung über Kieselgel (Cyclohexan/Ethylacetet) erhält man 5,50 g (90%) des gewünschten Produktes; logP(HCOOH): 0,6; ¹H-NMR(DMSO-d*6*): 4.45 (m, 2H), 2.56 (m, 2H), 1.83 (m, 4H), 1.57 (m, 2H)

Analog lassen sich herstellen:
- **4,5,6,7-Tetrahydro[1,2,3]oxadiazolo[3,4-a]pyridin-8-ium-3-olat (III-2);** logP(HCOOH): 0,59; ¹H-NMR(MeCN-d*3*) δ: 4.22 (dd, 2H), 2.50 (dd, 2H), 2.02 (m, 2H), 1.87 (m, 2H).
- **5,6-Dihydro-4H-pyrrolo[1,2-c][1,2,3]oxadiazol-7-ium-3-olat (III-3);** ¹H-NMR(DMSO-d*6*) δ: 4,47 (dd, 2H), 2.73 (m, 2H), 2.67 (m, 2H).
- **5-tert-Butoxy-5,6-dihydro-4H-pyrrolo[1,2-c][1,2,3]oxadiazol-7-ium-3-olat (III-4);** logP(HCOOH): 1.34; ¹H-NMR(DMSO-d*6*) δ: 5.03 (m, 1H), 4.74 (dd, 1H), 4.27 (dd, 1H), 3.11 (dd, 1H), 1.18 (s, 9H).

### Beispiel für Verfahrensschritt [V4] aus Schema 7:

### Beispiel 70: 4-[2-(Phenyl)-5,6-dihydro-4H-pyrrolo[1,2-b]pyrazol-3-yl]-N-isopropylpyridin-2-amin (Ic)

Ein Gemisch aus 1.10 g (0.39 mmol) 3-(2-Fluorpyridin-4-yl)-2-phenyl-5,6-dihydro-4H-pyrrolo[1,2-b]pyrazol und 1.5 ml Isopropylamin werden in einem Autoklaven auf 140 °C erhitzt. Nach 16 h engt man das Reaktionsgemisch unter vermindertem Druck ein. Nach säulenchromatographischer Reinigung an Kieselgel (Cyclohexan/Ethylacetet) erhält man 50 mg (35%) des gewünschten Produktes; logP(HCOOH): 1.21; ¹H-NMR(DMSO-d*6*): 7.81 (d, 1H), 7.36 (m, 5H), 6.28 (s, 1H), 6.21 (m, 2H), 4.15 (dd, 2H), 3.84 (m, 1H), 2.98 (dd, 2H), 2.59 (m, 2H), 1.08 (d, 6H).

### Beispiel für Verfahrensschritt [V6] aus Schema 7:

### Beispiel 3: 4-[2-(4-Fluorphenyl)-4,5,6,7-tetrahydropyrazolo[1,5-a]pyridin-3-yl]pyridin-2-amin (Ie)

Unter Eiskühlung werden 1.29 g (1.29 mmol) N-Benzyl-4-[2-(4-fluorphenyl)-4,5,6,7-tetrahydropyrazolo[1,5-a]pyridin-3-yl]pyridin-2-amin mit 10 ml konz. Schwefelsäure versetzt und 1 h bei Raumtemperatur nachgerührt. Das Reaktionsgemisch wird in Eiswasser eingerührt, mittels konz. NaOH auf pH10 eingestellt und mit 3x 50 ml Dichlormethan extrahiert. Die vereinigten organischen Phasen werden mit Wasser gewaschen, über MgSO₄ getrocknet und unter vermindertem Druck vom Lösungsmittel befreit. Man erhält 239 mg (35%) des gewünschten Produktes; logP(HCOOH): 1.21; ¹H-NMR(DMSO-d*6*): 7.81 (d, 1H), 7.416 (dd, 2H), 7.15 (dd, 2H), 6.28 (s, 1H), 6.24 (d, 1H), 5.83 (br.s, 2H), 4.15 (dd, 2H), 2.73 (dd, 2H), 2.02 (m, 2H), 1.80 (m, 2H).

### Beispiel für Verfahrensschritt [V7] aus Schema 7:

### Beispiel 26: Methyl-{4-[2-(4-fluorphenyl)-4,5,6,7-tetrahydropyrazolo[1,5-a]pyridin-3-yl]pyridin-2-yl}carbamat (If)

Zu einer Lösung von 200 mg (0.51 mmol) 4-[2-(4-Fluorphenyl)-4,5,6,7-tetrahydropyrazolo[1,5-a]pyridin-3-yl]pyridin-2-amin und 167 mg (1.29 mmol) Diisopropylethylamin in 20 ml THF werden 125 mg (1.29 mmol) Chlorameisensäuremethylester zugetropft. Nach 16 h versetzt man das Reaktionsgemisch mit 10 ml Wasser und extrahiert mit 3x 50 ml Ethyacetat. Die vereinigten organischen Phasen werden mit Wasser gewaschen, über MgSO₄ getrocknet und unter vermindertem Druck vom Lösungsmittel befreit. Der Rückstand wird in 10 ml 6N methanolische Ammoniak-Lösung wiederaufgenommen und 12h nachgerührt. Das Reaktionsgemisch wird eingeengt und der erhaltene Rückstand säulenchromatographisch über Kieselgel (Cyclohexan/Ethylacetat) aufgereinigt. Man erhält 167 mg (87%) des gewünschten Produktes; logP(HCOOH): 2.11; ¹H-NMR(MeCN-d*3*): 8.13 (br.s, 1H), 8.10 (d, 1H), 7.76 (s, 1H), 7.42 (dd, 2H), 7.05 (dd, 2H), 6.76 (d, 1H), 4.16 (dd, 2H), 3.69 (s, 3H), 2.82 (dd, 2H), 2.09 (m, 2H), 1.88 (m, 2H).

### Beispiel für Verfahrensschritt [V 10] aus Schema 7:

### 2-(4-Fluorphenyl)-4,5,6,7-tetrahydropyrazolo[1,5-a]pyridin (VII-1)

Ein Gemisch aus 2.44 g (16.5 mmol) 4,5,6,7-Tetrahydro[1,2,3]oxadiazolo[3,4-a]pyridin-8-ium-3-olat und 2.15 g (16.5 mmol) 4-Fluorphenylacetylen in 80 ml Mesitylen wird für 16 h bei 165°C unter Argon gerührt. Nach dem Abkühlen engt man das Reaktionsgemisch unter vermindertem Druck ein. Nach säulenchromatographischer Reinigung über Kieselgel (Cyclohexan/Ethylacetet) erhält man 888 mg (27%) des gewünschten Produktes; (logP(HCOOH): 2.76; ¹H-NMR(MeCN-d*3*): 7.76 (dd, 2H), 7.11 (dd, 2H), 6.29 (s, 1H), 4.09 (dd, 2H), 2.79 (dd, 2H), 2.02 (m, 2H), 1.84 (m, 2H).

Analog lassen sich herstellen:
- **2-(Phenyl)-5,6-dihydro-4H-pyrrolo[1,2-b]pyrazol (VII-2);** logP(HCOOH): 2.19; ¹H-NMR(DMSO-d6): 7.75 (d, 2H), 7.38 (dd, 2H), 7.25 (dd, 1H), 6.43 (s, 1H), 4.09 (dd, 2H), 2.86 (dd, 2H), 2.55 (m, 2H).
- **2-(4-Fluorphenyl)-5,6-dihydro-4H-pyrrolo[1,2-b]pyrazol (VII-3);** logP(HCOOH): 2.42; ¹H-NMR(DMSO-d*6*): 7.78 (dd, 2H), 7.19 (dd, 2H), 6.42 (s, 1H), 4.07 (dd, 2H), 2.85 (dd, 2H), 2.54 (m, 2H).
- **2-(4-Fluorphenyl)-5,6,7,8-tetrahydro-4H-pyrazolo[1,5-a]azepin (VII-3);** logP(HCOOH): 3.27; ¹H-NMR(DMSO-d*6*): 7.74 (dd, 2H), 7.19 (dd, 2H), 6.45 (s, 1H), 4.24 (m, 2H), 2.76 (m, 2H), 1.79 (m, 2H), 1.68 (m, 2H), 1.58 (m, 2H).
- **2-(4-Chlorphenyl)-5,6-dihydro-4H-pyrrolo[1,2-b]pyrazol (VII-4);** logP(HCOOH): 2.86; ¹H-NMR(DMSO-d*6*): 7.76 (d, 2H), 7.42 (d, 2H), 6.46 (s, 1H), 4.09 (dd, 2H), 2.86 (dd, 2H), 2.54 (m, 2H).
- **2-(Phenyl)-4,5,6,7-tetrahydropyrazolo[1,5-a]pyridin (VII-5);** (logP(HCOOH): 2.57; ¹H-NMR(MeCN-d*3*): 7.75 (dd, 2H), 7.36 (dd, 2H), 7.27 (dd, 1H), 6.33 (s, 1H), 4.10 (dd, 2H), 2.80 (dd, 2H), 2.05 (m, 2H), 1.84 (m, 2H).
- **2-[3-(Trifluormethyl)phenyl]-5,6-dihydro-4H-pyrrolo[1,2-b]pyrazol (VII-6);** logP(HCOOH): 3.25; ¹H-NMR(DMSO-d*6*): 8.05 (m, 2H), 7.62 (m, 2H), 6.60 (s, 1H), 4.12 (dd, 2H), 2.88 (dd, 2H), 2.56 (m, 2H).
- **2-[4-(Methoxy)phenyl]-5,6-dihydro-4H-pyrrolo[1,2-b]pyrazol (VII-7);** logP(HCOOH): 2.17; ¹H-NMR(DMSO-d*6*): 7.66 (d, 2H), 6.93 (d, 2H), 6.34 (s, 1H), 4.06 (dd, 2H), 3.76 (s, 3H), 2.84 (dd, 2H), 2.54 (m, 2H).
- **2-[4-Methylphenyl]-5,6-dihydro-4H-pyrrolo[1,2-b]pyrazol (VII-8);** logP(HCOOH): 2.64; ¹H-NMR(DMSO-d*6*): 7.63 (d, 2H), 7.17 (d, 2H), 6.37 (s, 1H), 4.08 (dd, 2H), 2.85 (dd, 2H), 2.54 (m, 2H), 2.30 (s, 3H).
- **2-[4-(Trifluormethyl)phenyl]-5,6-dihydro-4H-pyrrolo[1,2-b]pyrazol (VII-9);** logP(HCOOH): 3.20; ¹H-NMR(DMSO-d*6*): 7.66 (d, 2H), 7.71 (d, 2H), 6.57 (s, 1H), 4.13 (dd, 2H), 2.89 (dd, 2H), 2.57 (m, 2H).
- **2-[3-Fluorphenyl]-5,6-dihydro-4H-pyrrolo[1,2-b]pyrazol (VII-10);** logP(HCOOH): 2.46; ¹H-NMR(DMSO-d*6*): 7.60 (d, 1H), 7.52 (d, 1H), 7.42 (dd, 1H), 7.09 (dd, 1H), 6.51 (s, 1H), 4.10 (dd, 2H), 2.86 (dd, 2H), 2.55 (m, 2H).
- **2-(2-Thienyl)-4,5,6,7-tetrahydropyrazolo[1,5-a]pyridin (VII-11);** logP(HCOOH): 2.42; ¹H-NMR(MeCN-d*3*): 7.26 (m, 2H), 7.03 (dd, 1H), 6.23 (s, 1H), 4.06 (dd, 2H), 2.78 (dd, 2H), 2.02 (m, 2H), 1.86 (m 2H).
- **2-(3-Thienyl)-4,5,6,7-tetrahydropyrazolo[1,5-a]pyridin (VII-12);** logP(HCOOH): 2.25; ¹H-NMR(MeCN-d3): 7.53 (s, 1H), 7.42 (m, 2H), 6.22 (s, 1H), 4.07 (dd, 2H), 2.78 (dd, 2H), 2.02 (m, 2H), 1.86 (m 2H).
- **2-[3-Thienyl]-5,6-dihydro-4H-pyrrolo[1,2-b]pyrazol (VII-13);** logP(HCOOH): 2.01; ¹H-NMR(DMSO-d*6*): 7.66 (d, 1H), 7.53 (m, 1H), 7.42 (d, 1H), 6.30 (s, 1H), 4.05 (dd, 2H), 2.84 (dd, 2H), 2.55 (m, 2H).

### Beispiel für Verfahrensschritt [V11] aus Schema 7:

### 2-(4-Fluorphenyl)-3-iod-4,5,6,7-tetrahydropyrazolo[1,5-a]pyridin (VIII-1)

Zu einer Lösung von 2.66 g (12.3 mmol) 2-(4-Fluorphenyl)-4,5,6,7-tetrahydropyrazolo[1,5-a]pyridin in 50 ml Dichlormethan tropft man eine Lösung von 2.20 g (13.5 mmol) Iodmonochlorid in 5 ml Dichlormethan zu und lässt 12 h nachrühren. Das Reaktionsgemisch wird mit 100 ml Wasser versetzt und mit 3x 50 ml Dichlormethan extrahiert. Die vereinigten organischen Phasen werden über MgSO₄ getrocknet und unter vermindertem Druck vom Lösungsmittel befreit. Man erhält 4.40 g (98%) des gewünschten Produktes; (logP(HCOOH): 3.79; ¹H-NMR(MeCN-d*3*): 7.82 (dd, 2H), 7.18 (dd, 2H), 4.11 (dd, 2H), 2.67 (dd, 2H), 2.06 (m, 2H), 1.94 (m, 2H).

Analog lassen sich herstellen:
- **2-(4-Fluorphenyl)-3-iod-5,6,7,8-tetrahydro-4H-pyrazolo[1,5-a]azepin (VIII-2);** logP(HCOOH): 4.35 ¹H-NMR(DMSO-d*6*): 7.77 (d, 2H), 7.28 (dd, 2H), 7.25 (dd, 1H), 4.33 (m, 2H), 2.84 (m, 2H), 1.82 (m, 2H), 1.72 (m, 2H), 1.61 (m, 2H).
- **2-[3-(Trifluormethyl)phenyl]-3-iod-5,6-dihydro-4H-pyrrolo[1,2-b]pyrazol (VIII-3);** logP(HCOOH): 4.11 ¹H-NMR(DMSO-d*6*): 8.10 (m, 2H), 7.72 (m, 2H), 4.24 (dd, 2H), 2.88 (dd, 2H), 2.56 (m, 2H).
- **2-(4-Chlorphenyl)-3-iod-5,6-dihydro-4H-pyrrolo[1,2-b]pyrazol (VIII-4);** logP(HCOOH): 3.88 ¹H-NMR(DMSO-d*6*): 7.80 (d, 2H), 7.50 (d, 2H), 4.21 (dd, 2H), 2.83 (dd, 2H), 2.58 (m, 2H).
- **2-(Phenyl)-3-iod-5,6-dihydro-4H-pyrrolo[1,2-b]pyrazol (VIII-5);** logP(HCOOH): 3.09 ¹H-NMR(DMSO-d*6*): 7.77 (d, 2H), 7.45 (dd, 2H), 7.34 (dd, 1H), 4.21 (dd, 2H), 2.82 (dd, 2H), 2.58 (m, 2H).
- **2-(4-Fluorphenyl)-3-iod-5,6-dihydro-4H-pyrrolo[1,2-b]pyrazol (VIII-6);** logP(HCOOH): 3.30 ¹H-NMR(DMSO-d*6*): 7.78 (dd, 2H), 7.28 (dd, 2H), 4.20 (dd, 2H), 2.85 (dd, 2H), 2.52 (m, 2H).
- **2-[4-Methylphenyl]-3-iod-5,6-dihydro-4H-pyrrolo[1,2-b]pyrazol (VIII-7);** logP(HCOOH): 3.54 ¹H-NMR(DMSO-d*6*): 7.64 (d, 2H), 7.23 (d, 2H), 4.20 (dd, 2H), 2.83 (dd, 2H), 2.55 (m, 2H), 2.33 (s, 3H).
- **2-[3-Thienyl]-3-iod-5,6-dihydro-4H-pyrrolo[1,2-b]pyrazol (VIII-8);** logP(HCOOH): 2.97 ¹H-NMR(DMSO-d*6*): 7.93 (d, 1H), 7.59 (dd, 1H), 7.53 (d, 1H), 4.19 (dd, 2H), 2.81 (dd, 2H), 2.55 (m, 2H).
- **2-[4-(Methoxy)phenyl]-3-iod-5,6-dihydro-4H-pyrrolo[1,2-b]pyrazol (VIII-9);** logP(HCOOH): 3.01 ¹H-NMR(DMSO-d*6*): 7.68 (d, 2H), 6.99 (d, 2H), 4.19 (dd, 2H), 3.79 (s, 3H), 2.82 (dd, 2H), 2.54 (m, 2H).
- **2-[4-(Trifluormethyl)phenyl]-3-iod-5,6-dihydro-4H-pyrrolo[1,2-b]pyrazol (VIII-10);** logP(HCOOH): 4.13 ¹H-NMR(DMSO-d*6*): 8.01 (d, 2H), 7.81 (d, 2H), 4.25 (dd, 2H), 2.88 (dd, 2H), 2.57 (m, 2H).
- **2-[3-Fluorphenyl]-3-iod-5,6-dihydro-4H-pyrrolo[1,2-b]pyrazol (VIII-11);** logP(HCOOH): 3.32 ¹H-NMR(DMSO-d*6*): 7.65 (d, 1H), 7.52 (m, 2H), 7.20 (dd, 1H), 4.22 (dd, 2H), 2.81 (dd, 2H), 2.58 (m, 2H).
- **2-(Phenyl)-3-iod-4,5,6,7-tetrahydropyrazolo[1,5-a]pyridin (VIII-12);** (logP(HCOOH): 3.55; ¹H-NMR(MeCN-d*3*): 7.80 (d, 2H), 7.39 (m, 3H), 4.12 (dd, 2H), 2.68 (dd, 2H), 2.05 (m, 2H), 1.84 (m, 2H).
- **2-(2-Thienyl)-3-iod-4,5,6,7-tetrahydropyrazolo[1,5-a]pyridin (VIII-13);** logP(HCOOH): 3.45 ¹H-NMR(MeCN-d*3*): 7.74 (d, 1H), 7.37 (d, 1H), 7.11 (dd, 1H), 4.09 (dd, 2H), 2.66 (dd, 2H), 2.02 (m, 2H), 1.86 (m 2H).
- **2-(3-Thienyl)-3-iod-4,5,6,7-tetrahydropyrazolo[1,5-a]pyridin (VIII-14);** logP(HCOOH): 3.40 ¹H-NMR(MeCN-d*3*): 7.95 (s, 1H), 7.57 (d, 1H), 7.44 (dd, 1H), 4.08 (dd, 2H), 2.66 (dd, 2H), 2.11 (m, 2H), 1.91 (m 2H).
- **3-Iod-2-phenyl-6,7-dihydro-4H-pyrazolo[5,1-c][1,4]oxazin (VIII-15);** logP(HCOOH): 2.82 ¹H-NMR(DMSO-d*6*): 7.77 (d, 2H), 7.47 (dd, 2H), 7.39 (dd, 1H), 4.70 (s, 2H), 4.16 (m, 2H), 4.09 (m, 2H).
- **2-(4-Fluorphenyl)-3-iod-7,7-dimethyl-4,5-dihydropyrazolo[1,5-c][1,3]oxazin (VIII-16);** logP(HCOOH): 4.28 ¹H-NMR(DMSO-d*6*): 7.81 (dd, 2H), 7.29 (dd, 2H), 4.09 (t, 2H), 2.76 (t, 2H), 1.68 (s, 6H).
- **3-Brom-2-(4-fluorphenyl)-6,7-dihydro-4H-pyrazolo[5,1-c][1,4]oxazin (VIII-17);** analog mit 2 eq. NBS logP(HCOOH): 2.90 ¹H-NMR(MeCN-d*3*): δ = 7.90-7.86 (m, 2H), 7.21-7.17 (m, 2H), 4.75 (s, 2H), 4.14-4.09 (m, 4H)
- **3-Brom-2-phenyl-6,7-dihydro-4H-pyrazolo[5,1-c] [1,4]thiazin (VIII-18);** analog mit 2 eq. NBS logP(HCOOH): 3.42 mit MS (ESI): 296/298 [M+H]⁺.

### Beispiel für Verfahrensschritt [V12] aus Schema 7:

### Beispiel 61: 3-(2-Fluorpyridin-4-yl)-2-phenyl-5,6-dihydro-4H-pyrrolo[1,2-b]pyrazol (Ia)

Ein Gemisch aus 1.00 g (3.22 mmol) 2-(Phenyl)-3-iod-5,6-dihydro-4H-pyrrolo[1,2-b]pyrazol, 1.80 g (8.06 mmol) 2-Fluor-4-(4,4,5,5-tetramethyl-1,3,2-dioxaborolan-2-yl)pyridin, 2.63 g (8.06 mmol) Cs₂CO₃ und 526 mg (0.65 mmol) Pd(dppf)Cl₂ in 8 ml THF erhitzt man unter Argon für 16 h auf 60 °C. Das Reaktionsgemisch wird mit 20 ml Wasser versetzt und mit 3x 50 ml Ethylacetat extrahiert. Die vereinigten organischen Phasen werden über MgSO₄ getrocknet und unter vermindertem Druck vom Lösungsmittel befreit. Nach säulenchromatographischer Reinigung an Kieselgel (Cyclohexan/Ethylacetet) erhält man 507 mg (57%) des gewünschten Produktes; logP(HCOOH): 2.53; ¹H-NMR(DMSO-d*6*): 8.10 (d, 1H), 7.40 (m, 5H), 7.07 (d, 1H), 6.85 (s, 1H), 4.19 (dd, 2H), 3.11 (dd, 2H), 2.62 (m, 2H).

### Beispiel 5: N-{4-[2-(4-Fluorphenyl)-4,5,6,7-tetrahydropyrazolo[1,5-a]pyridin-3-yl]pyridin-2-yl}propanamid (If)

Unter Argon tropft man zu einer Lösung von 200 mg (0.55 mmol) 2-(4-Fluorphenyl)-3-iod-4,5,6,7-tetrahydropyrazolo[1,5-a]pyridin, 168 g (0.61 mmol) N-[4-(4,4,5,5-Tetramethyl-1,3,2-dioxaborolan-2-yl)pyridin-2-yl]propanamid in 2ml Dioxan 1.39 ml einer 2M wässrigen Na₂CO₃-Lösung und gibt 32 mg (0.04 mmol) Pd(PCy₃)₂Cl₂ zu. Das Reaktionsgemisch wird in einem Microwellenreaktor 15 min auf 120 °C erhitzt. Nach dem Erkalten wird das Reaktionsgemisch mit 50 ml Ethylacetet versetzt über Kieselgur filtriert, das Filtrat mit Ethylacetat extrahiert. Die vereinigten organischen Phasen werden mit Wasser gewaschen, über MgSO₄ getrocknet und unter vermindertem Druck vom Lösungsmittel befreit. Nach säulenchromatographischer Reinigung an Kieselgel (Cyclohexan/Ethylacetet) erhält man 159 mg (78%) des gewünschten Produktes; logP(HCOOH): 1.97; ¹H-NMR(MeCN-d*3*): 8.54 (br.s, 1H), 8.11 (d, 1H), 8.02 (s, 1H), 7.42 (dd, 2H), 7.04 (dd, 2H), 6.77 (d, 1H), 4.16 (dd, 2H), 2.81 (dd, 2H), 2.40 (q, 2H), 1.77 (m, 2H), 1.11 (t, 3H).

### Beispiel für Verfahrensschritt [V14] aus Schema 9:

### Methyl-1-(2-chlorethyl)-3-(4-fluorphenyl)-1H-pyrazol-5-carboxylat (XI-1)

Zu einer Lösung von 2.20g (10mmol) Methyl-3-(4-fluorphenyl)-1H-pyrazol-5-carboxylat (beschrieben in Zhejiang Daxue Xuebao, Lixueban 2008, 35, 641-643) in 30mL Aceton werden 4.15g (30mmol) Kaliumcarbonat gegeben. Zu dieser Suspension werden innerhalb von 5min 7.17g (50mmol) 1-Brom-2-chlorethan zugegeben. Anschliessend wird die Reaktionsmischung 19h bei 70°C gerührt. Danach werden die unlöslichen Bestandteile abfiltriert und das Lösungsmittel eingeengt. Das erhaltene Rohöl wird säulenchromatografisch an Kieselgel (Eluent Cyclohexan/Essigester) gereinigt. Man erhält 2.5g (80%) des gewünschten Produktes (logP (HCOOH): 3.58; 1H-NMR (MeCN-d3): δ = 7.86-7.84 (m, 2H), 7.20 (s, 1H), 7.19-7.16 (m, 2H), 6.53 (s, 1H), 4.89 (t, 2H), 4.00 (t, 2H), 3.88 (s, 3H).

### Beispiel für Verfahrensschritt [V15] aus Schema 9:

### [1-(2-Chlorethyl)-3-(4-fluorphenyl)-1H-pyrazol-5-yl]methanol (XIII-1)

Zu einer Lösung von 1.1g (4,00 mmol) Methyl-1-(2-chlorethyl)-3-(4-fluorphenyl)-1H-pyrazol-5-carboxylat in 20mL Tetrahydrofuran werden unter Argon bei 0°C 4mL Lithiumaluminiumhydrid-Lösung (1M in Diethylether, 4,00 mmol) gegeben. Anschliessend wird die Mischung 2h bei 0°C gerührt. Danach wird die Reaktionsmischung bei 0°C vorsichtig tropfenweise mit 5M wässriger NaOH Lösung versetzt, bis keine Wasserstoffentwicklung mehr beobachtbar ist. Die enstandene Suspension wird abdekantiert und das Lösungsmittel entfernt. Die Reinigung des erhaltenen Rohprodukts erfolgt säulenchromatografisch an Kieselgel (Cyclohexan/Essigester). Es werden 900mg (86%) des gewünschten Produktes erhalten; logP (HCOOH): 1.84; ¹H-NMR (MeCN-d3): δ = 7.81-7.77 (m, 2H), 7.16-7.12 (m, 2H), 6.53 (s, 1H), 4.64 (d, 2H), 4.45 (t, 2H), 4.00 (t, 2H).

Analog lässt sich herstellen:
- **(3-Phenyl-1H-pyrazol-5-yl)methanol (XIV-1);** logP(HCOOH): 1,14; LC-MS: m/z = 175 [M+H]⁺.

### Beispiel für Verfahrensschritt [V16] aus Schema 9:

### 2-(4-Fluorphenyl)-6,7-dihydro-4H-pyrazolo[5,1-c][1,4]oxazin (VIIa-1)

In einem 100mL Rundkolben werden 780mg (3,00 mmol) [1-(2-Chlorethyl)-3-(4-fluorphenyl)-1H-pyrazol-5-yl]methanol in 20mL N-N-Dimethylformamid gelöst. Dazu werden unter Argon bei 0°C 120mg Natriumhydrid (3,00 mmol) als 60%ige Suspension in Öl gegeben. Anschliessend wird die Mischung 18h bei 25°C gerührt. Danach wird die Reaktionsmischung vorsichtig in Wasser gegeben und mehrmals mit Essigester extrahiert. Anschliessend werden die vereinten organischen Phasen über Na₂SO₄ getrocknet und eingeengt. Die Reinigung erfolgt mittels Kieselgelchromatographie (Cyclohexan/Essigester). Man erhält 480mg (49%) des gewünschten Produktes logP (HCOOH): 2.01; 1H-NMR(MeCN-d3): δ = 7.81-7.77 (m, 2H), 7.15-7.11 (m, 2H), 6.36 (s, 1H), 4.81 (s, 2H), 4.14-4.12 (m, 2H), 4.10-4.08 (m, 2H).

### Beispiel für Verfahrensschritt [V17] aus Schema 9:

### 5-(Chlormethyl)-3-phenyl-1H-pyrazol (XV-1)

Zu einer Lösung von 7,3 g (42,0 mmol) (3-Phenyl-1H-pyrazol-5-yl)methanol in 220mL Dichlormethan werden bei 20°C 6,1 mL Thionylchlorid und 10 Tropfen Dimethylformamid gegeben. Anschliessend wird die Mischung 12 h unter Rückfluss gekocht. Danach wird das Lösungsmittel aus der Reaktionsmischung entfernt. Den Rückstand nimmt man mit 100 mL Dichlormethan auf und versetzt die Mischung so lange mit einer gesättigten Natriumhydrogencarbonat-Lösung bis sich ein pH von 6 bis 7 eingestellt hat. Dann wird die Mischung unter vermindertem Druck vom Lösungsmittel befreit und getrocknet. Man erhält 5,65 g des gewünschten Produktes, das ohne weitere Reinigung weiter umgesetzt wird; logP (HCOOH): 2,05 mit MS (ESI): 193.1/195.1 ([M+H]⁺).

### Beispiel für Verfahrensschritte [V18] und [V20] aus Schema 9:

### 2-Phenyl-6,7-dihydro-4H-pyrazolo[5,1-c] [1,4]thiazin (VIIb-1)

Zu einer Lösung von 5,59 g (29 mmol) 5-(Chlormethyl)-3-phenyl-1H-pyrazol in 200 mL Ethanol werden 4,42 g (58 mmol) Thioharnstoff gegeben. Die Mischung wird 2 Tage bei 20 °C gerührt. Anschließend wird die Mischung weitgehend vom Lösungsmittel befreit und mit 10-20 mL Dichlormethan versetzt. Nach kurzem Rühren wird der Niederschlag abgesaugt und getrocknet. Man erhält 6,3 g eines Produktgemisches, das ohne weitere Aufreinigung weiter verwendet wird. 4,19 g dieses Gemisches werden in 140 mL N,N-Dimethylformamid wiederaufgenommen. Zu dieser Mischung gibt man 4,55 g getrocknetes Kaliumcarbonat und 4,74 mL 1,2-Dibromethan. Anschliessend wird die Mischung 18h bei 60 °C gerührt. Danach wird die Reaktionsmischung vorsichtig in Wasser eingerührt. Anschließend wird die wässrige Phase abdekantiert und der ölige Rückstand wird mit Essigester versetzt. Die organische Phase wird über Na₂SO₄ getrocknet und eingeengt. Die Reinigung erfolgt mittels Kieselgelchromatographie (Cyclohexan/Essigester). Man erhält 620 mg des gewünschten Produktes; logP (HCOOH): 2.46; ¹H-NMR (ppm): δ (DMSO-d6) = 3,20 (t, 2H), 3,94 (s, 2H), 4,32 (t, 2H), 6,54 (s, 1H), 7,31 (t, 1H), 7,40 (t, 2H), 7,65 (d, 2H).

### Beispiel für Verfahrensschritt [V21] aus Schema 10:

### 5-(Benzyloxy)-1-(4-fluorphenyl)pent-2-in-1-on (XX-1)

Zu einer Lösung von 505 mg (3.15 mmol) Benzyl-but-3-in-1-ylether in 10 ml THF tropft man bei -70 °C 2.56 ml einer 1.6 M Butyllithium Lösung zu und lässt 30 min nachrühren. Zu dieser Lösung tropft man 500 mg (5.15 mmol) 4-Fluorbenzoylchlorid zu und lässt die Temperatur auf 0 °C ansteigen. Nach 1 h versetzt man das Reaktionsgemisch mit 10 ml einer gesättigten Ammoniumchlorid-Lösung und extrahiert mit 3 x 50 ml Ethylacetat. Die vereinigten organischen Phasen werden mit Wasser gewaschen, über MgSO₄ getrocknet und unter vermindertem Druck vom Lösungsmittel befreit. Nach säulenchromatographischer Aufreinigung über Kieselgel (Cyclohexan/Ethylacetat) erhält man 430 mg (45%) des gewünschten Produktes; (logP(HCOOH): 3.89; ¹H-NMR(DMSO-d*6*): 8.15 (dd, 2H), 7.34 (m, 7H), 4.57 (s, 2H), 3.70 (t, 2H), 2.90 (t, 2H).

### Beispiel für Verfahrensschritt [V22] aus Schema 10:

### 5-[2-(Benzyloxy)ethyl]-3-(4-fluorphenyl)-1H-pyrazol (XXI)

Zu einer Lösung von 500 mg (1.77 mmol) 5-(Benzyloxy)-1-(4-fluorphenyl)pent-2-in-1-on in 5 ml Ethanol tropft man bei Raumtemperatur 0.17 ml (3.54 mmmol) Hydrazinhydrat zu und rührt 2 h unter Rückfluss nach. Nach dem Erkalten engt man das Reaktionsgemisch unter vermindertem Druck ein und reinigt den Rückstand säulenchromatographisch über Kieselgel (Cyclohexan/Ethylacetat) auf. Man erhält 470 mg (87%) des gewünschten Produktes; (logP(HCOOH): 3.09; ¹H-NMR(DMSO-d6): 7.77 (dd, 2H), 7.34 (m, 8H), 6.49 (s, 1H), 4.51 (s, 2H), 3.70 (t, 2H), 2.89 (t, 2H).

### Beispiel für Verfahrensschritt [V23] aus Schema 10:

### 2-[3-(4-Fluorphenyl)-1H-pyrazol-5-yl]ethanol (XXII)

Man legt 39.4 g (242 mmol) Eisen(III)chlorid in 200 ml Dichlormethan vor. Bei Raumtemperatur tropft man eine Lösung von 7.20 g (24.2 mmol) 5-[2-(Benzyloxy)ethyl]-3-(4-fluorphenyl)-1H-pyrazol in 90 ml Dichlormethan zu. Nach 20 min wäscht man das Reaktionsgemisch mit 2x 100 ml Wasser. Die vereinigten wässrigen Phasen werden mit Natriumhydrogencarbonat auf pH 5-6 eingestellt und mit 2x 100 ml Ethylacetat extrahiert Die vereinigten organischen Phasen werden mit Wasser gewaschen, über MgSO₄ getrocknet und unter vermindertem Druck vom Lösungsmittel befreit. Nach säulenchromatographischer Aufreinigung über Kieselgel (Cyclohexan/Ethylacetat) erhält man 3.40g (67%) des gewünschten Produktes; (logP(HCOOH): 1.39; ¹H-NMR(DMSO-d6): 7.79 (dd, 2H), 7.19 (dd, 2H), 6.47 (s, 1H), 4.79 (t, 1H), 3.65 (m, 2H), 2.76 (t, 2H).

### Beispiel für Verfahrensschritt [V 11] aus Schema 10:

### 2-[3-(4-Fluorphenyl)-4-iod-1H-pyrazol-5-yl]ethanol (XXII)

Analog zu Verfahrenschritt [11] lässt sich 2-[3-(4-Fluorphenyl)-1H-pyrazol-5-yl]ethanol mit 1.1 eq NIS in DMF umsetzen. Man erhält in 89%iger Ausbeute das gewünschte Produkt als Isomerengemisch: (logP(HCOOH): 1.99; ¹H-NMR(DMSO-d6): 13.29 &13.19 (s, 1H) 7.78 & 7.69 (dd, 2H), 7.38 & 7.28 (dd, 2H), 4.92 & 4.72 (t, 1H), 3.62 (m, 2H), 2.79 & 2.73 (t, 2H).

### Beispiel für Verfahrensschritt [V24] aus Schema 10:

### 2-(4-Fluorphenyl)-3-iod-4,5-dihydropyrazolo[1,5-c] [1,3]oxazin (VIIIa-1)

Ein Gemisch aus 200 mg (0.60 mmol) 2-[3-(4-Fluorphenyl)-4-iod-1H-pyrazol-5-yl]ethanol, 36 mg (1.20 mmol) Paraformaldehyd und 10 mg (0.06 mmol) 4-Toluolsulfonsäure in 1 ml Dioxan werden in einem Mikrowellenreaktor 1 h bei 130 °C erhitzt. Nach dem Erkalten engt man das Reaktionsgemisch unter vermindertem Druck ein und reinigt den Rückstand säulenchromatographisch über Kieselgel (Cyclohexan/Ethylacetat) auf. Man erhält 120 mg (57%) des gewünschten Produktes; (logP(HCOOH): 3.23; ¹H-NMR(DMSO-d*6*): 7.81 (dd, 2H), 7.31 (dd, 2H), 5.54 (s, 2H), 4.12 (dd, 2H), 2.80 (dd, 2H).

### Beispiel für Verfahrensschritt [V25] aus Schema 11:

### 4-Chlor-N'-(diphenylmethylen)pentanhydrazid (XXV-1)

Zu einer Lösung von 0,20 g (1,0 mmol) Diphenylmethanonhydrazon und 0.08 mL Pyridin in 15 mL Dichlormethan wird eine Lösung aus 0,16 g (1,0 mmol) 4-Chlorpentanoylchlorid in 15 ml Dichlormethan bei 0°C versetzt. Das Reaktionsgemisch wird 16 h bei Raumtemperatur gerührt. Das Reaktionsgemisch wird mit Ethylacetat/gesättigter wässriger NH₄Cl-Lösung versetzt, die organische Phase abgetrennt und die wäßrige Phase mit 3 x 100 ml Ethylacetat extrahiert. Die vereinigten organischen Phasen werden über Na₂SO₄ getrocknet und unter vermindertem Druck vom Lösungsmittel befreit. Nach säulenchromatographischer Reinigung über Kieselgel (Cyclohexan/Ethylacetet) erhält man 0,32 g (100%) des gewünschten Produktes; logP(HCOOH): 3,76); ¹H-NMR (ppm): δ (DMSO-d*6*) = 1.45 (d, 0.9 H), 1.52 (d, 2.1H), 1.81-2.12 (m, 1H), 2.30-2.42 (m, 1H), 2.88-3.00 (m, 2H), 4.13-4.20 (m, 0.3H), 4.28-4.34 (m, 0.7H), 7.26-7.61 (m, 10H), 9.19 (s, 1H); LC-MS: m/z = 315 [M+H]⁺

### Beispiel für Verfahrensschritt [V26] aus Schema 11:

### 1-[(Diphenylmethylen)amino]-5-methylpyrrolidin-2-on (XXVI-1)

Eine Lösung von 33,0 g (68,0 mmol) 4-Brom-N'-(diphenylmethylen)pentanhydrazid in 200 mL Tetrahydrofuran wird bei 0°C portionsweise mit 2,7 g (68,0 mmol) Natriumhydrid versetzt. Das Reaktionsgemisch wird 16 h bei Raumtemperatur gerührt und im Anschluß mit Ethylacetat/gesättigter wässriger NH₄Cl-Lösung versetzt. Die organische Phase wird abgetrennt und die wäßrige Phase mit 3x 250 ml Ethylacetat extrahiert. Die vereinigten organischen Phasen werden über Na₂SO₄ getrocknet und unter vermindertem Druck vom Lösungsmittel befreit. Nach säulenchromatographischer Reinigung über Kieselgel (Cyclohexan/Ethylacetet) erhält man 17,8 g (89%) des gewünschten Produktes; logP(HCOOH): 2,73; ¹H-NMR (ppm): δ (DMSO-d*6*) = 1.22 (d, 3 H), 2.05-2.15 (m, 4H), 3.86 (m, 1H), 7.24-7.26 (m, 2H), 7.40-7.43 (m, 4H), 7.48-7.53 (m, 4H); LC-MS: m/z = 279 [M+H]⁺

Analog lassen sich herstellen:
- **1-[(Diphenylmethylen)amino]pyrrolidin-2-on (XXVI-2);** logP(HCOOH): 2,23; ¹H-NMR (ppm): δ (DMSO-d6) = 1.83 (m, 2H), 2.15 (t, 2H), 3.31 (t, 2H), 7.25-7.28 (m, 2H), 7.37-7.50 (m, 8H); LC-MS: m/z = 265 [M+H]⁺
- **1-[(Diphenylmethylen)amino]-3-methylpyrrolidin-2-on (XXVI-3);** logP(HCOOH): 2,62; ¹H-NMR (ppm): δ (DMSO-d*6*) = 0.91 (d, 3 H), 1.36-1.46 (m, 1H), 2.07-2.14 (m, 1H), 2.24-2.32 (m, 1H), 3.21-3.30 (m, 2H), 7.24-7.29 (m, 2H), 7.40-7.51 (m, 8H); LC-MS: m/z = 279 [M+H]⁺.

### Beispiel für Verfahrensschritt [V27] aus Schema 11:

### 2-Methyl-5-oxopyrrolidin-1-aminiumchlorid (XXVII-1)

Eine Lösung aus 17,8 g (63,9 mmol) 1-[(Diphenylmethylen)amino]-5-methylpyrrolidin-2-on in 100 mL Tetrahydrofuran wird bei Raumtemperatur mit 50 mL 37%iger Salzsäure versetzt und 1 h nachgerührt Im Anschluß versetzt man das Reaktionsgemisch mit Ethylacetat und trennt die organische Phase ab. Die salzsaure Phase versetzt man wiederholt mit Ethanol/Toluol (1:1) und engt unter vermindertem Druck ein. Das erhaltene Rohprodukt wird ohne weitere Aufreinigung weiter umgesetzt. ¹H-NMR (ppm): δ(DMSO-d*6*) = 1.24 (d, 3 H), 1.60-1.65 (m, 1H), 2.20-2.42 (m, 3H), 3.88 (m, 1H).

Analog lassen sich herstellen:
- **3-Methyl-2-oxopyrrolidin-1-aminiumchlorid (XXVH-2);** ¹H-NMR (ppm): δ (DMSO-d*6*) = 1.08 (d, 3 H), 1.64-1.70 (m, 1H), 2.27-2.34 (m, 1H), 2.46-2.51 (m, 1H), 3.48 (dd, 1H), 3.48 (dd, 1H).
- **2-Oxopyrrolidin-1-aminiumchlorid (XXVII-2);** ¹H-NMR (ppm): δ (DMSO-d*6*) = 1.97-2.07 (m, 2H), 2.22-2.36 (m, 2H), 3.59 (t, 2H).

### Beispiel für Verfahrensschritt [V28] aus Schema 11:

### Methyl-3-(4-fluorphenyl)-3-[(2-methyl-5-oxopyrrolidin-1-yl)imino]propanoat (XXVIII-1)

Ein Gemisch aus 7.50 g (38,2 mmol) Methyl-3-(4-fluorphenyl)-3-oxopropanoat und 11.5 g (76,5 mmol) 2-Methyl-5-oxopyrrolidin-1-aminiumchlorid in 50 ml Pyridin wird für 72 h bei Raumtemperatur gerührt. Das Reaktionsgemisch engt man unter vermindertem Druck ein. Nach säulenchromatographischer Reinigung über Kieselgel (Cyclohexan/Ethylacetet) erhält man 10,4 g (93%) des gewünschten Produktes als Isomerengemisch; (logP(HCOOH): 2,13, und 2,45; ¹H-NMR (ppm): δ (DMSO-d*6*) = 1.16-1.19 (m, 3 H), 1.58-1.62 (m, 2H), 2.20-2.22 (m, 1H), 2.36-2.41 (m, 1H), 3.54 (s, 2.25H), 3.64 (s, 0.75H), 3.80 (d, 1H), 3.90 (d, 1H), 3.80 (q, 1H), 7.24 (t, 0.6H), 7.29 (t, 1.4H), 7.44-7.47 (m, 0.6H), 7.89-7.92 (m, 1.4H); LC-MS: m/z = 293 [M+H]⁺.

Analog lassen sich herstellen:
- **Methyl-3-(4-fluorphenyl)-3-[(2-methyl-5-oxopyrrolidin-1-yl)imino]propanoat (XXVIII-2);** logP(HCOOH): 2,09, und 2,47; ¹H-NMR (ppm): δ (DMSO-d*6*) = 1.12 (d, 3 H), 1.58-1.64 (m, 1H), 1.91-2.00 (m, 1H), 2.10-2.30 (m, 2H), 3.15 (td, 0.17H), 3.29 (td, 0.17H), 3.49 (td, 0.33H), 3.57 (s, 2H), 3.62 (td, 0.33H), 3.64 (s, 1H), 3.80 (d, 1H), 3.89 (d, 1H), 7.21-7.31 (m, 2H), 7.41-7.44 (m, 0.66H), 7.89-7.92 (m, 1.34H); LC-MS: m/z = 293 [M+H]⁺.
- **Ethyl-3-(2-fluorphenyl)-3-[(2-methyl-5-oxopyrrolidin-1-yl)imino]propanoat (XXVIII-3);** logP(HCOOH): 2,34, und 2,74; ¹H-NMR (ppm): δ (DMSO-d*6*) = 0.99-1.06 (m, 3 H), 1.15-1.24 (m, 3H), 1.58-1.64 (m, 1H), 1.81-2.45 (m, 3H), 3.64 (dd, 0.5H), 3.78-4.14 (m, 4.5H), 7.15-7.38 (m, 2H), 7.46-7.55 (m, 1H), 7.64-7.69 (m, 1H); LC-MS: m/z = 307 [M+H]⁺.
- **Ethyl-3-(2-fluorphenyl)-3-[(2-oxopyrrolidin-1-yl)imino]propanoat (XXVIII-4);** logP(HCOOH): 1,97, und 2,39; ¹H-NMR (ppm): δ (DMSO-d*6*) = 0.99-1.22 (m, 3 H), 1.59-1.66 (m, 1H), 1.95-2.09 (m, 2H), 2.30-2.41 (m, 1H), 3.39-3.49 (m, 2H), 3.62-4.01 (m, 4H), 7.19-7.79 (m, 4H); LC-MS: m/z = 293 [M+H]⁺.

### Beispiel für Verfahrensschritt [V29] aus Schema 11:

### Methyl-2-(4-fluorphenyl)-6-methyl-5,6-dihydro-4H-pyrrolo[1,2-b]pyrazol-3-carboxylat (XXX-1)

Eine Lösung aus 10,4 g (35,6 mmol) Methyl-3-(4-fluorphenyl)-3-[(2-methyl-5-oxopyrrolidin-1-yl)imino]propanoat in 100 ml Dimethylformamid wird mit 23,2 g (71,2 mmol) Cäsiumcarbonat versetzt. Das Reaktionsgemisch wird 30 min bei Raumtemperatur und 4 h bei 100 °C gerührt. Im Anschluß versetzt man mit Ethylacetat/Wasser, trennt die organische Phase ab und extrahiert die wäßrige Phase mit 3x 100 ml Ethylacetat. Die vereinigten organischen Phasen werden über Na₂SO₄ getrocknet und unter vermindertem Druck vom Lösungsmittel befreit. Nach säulenchromatographischer Reinigung über Kieselgel (Cyclohexan/Ethylacetet) erhält man 6,6 g (64%) des gewünschten Produktes; logP(HCOOH): 2,84; ¹H-NMR (ppm): δ (DMSO-d6) = 1.44 (d, 3 H), 2.14-2.17 (m, 1H), 2.76-2.78 (m, 1H), 2.98-3.08 (m, 2H), 3.68 (s, 3H), 4.46 (m, 1H), 7.21-7.24 (m, 2H), 7.73-7.76 (m, 2H); LC-MS: m/z = 275 [M+H]⁺.

Analog lassen sich herstellen:
- **Methyl-2-(4-fluorphenyl)-4-methyl-5,6-dihydro-4H-pyrrolo[1,2-b]pyrazol-3-carboxylat (XXX-2);** logP(HCOOH): 2,71; ¹H-NMR (ppm): δ (DMSO-d6) = 1.34 (d, 3 H), 2.16-2.21 (m, 1H), 2.81-2.86 (m, 1H), 3.45-3.52 (m, 1H), 3.70 (s, 3H), 4.11-4.29 (m, 2H), 7.20-7.25 (m, 2H), 7.69-7.72 (m, 2H); LC-MS: m/z = 275 [M+H]⁺.
- **Ethyl-2-(2-fluorphenyl)-5,6-dihydro-4H-pyrrolo[1,2-b]pyrazol-3-carboxylat (XXX-3);** logP(HCOOH): 2,49; ¹H-NMR (ppm): δ (DMSO-d6) = 1.12 (t, 3 H), 2.60 (quintet, 2H), 3.06 (ABq, 2H), 4.06 (q, 2H), 4.19 (ABq, 2H), 7.22-7.25 (m, 2H), 7.41-7.49 (m, 2H); LC-MS: m/z = 275 [M+H]⁺.

### Beispiel für Verfahrensschritt [V30] aus Schema 11:

### 2-(4-Fluorphenyl)-6-methyl-5,6-dihydro-4H-pyrrolo[1,2-b]pyrazol-3-carbonsäure (XXXI-1)

Eine Lösung aus 7,0 g (25,5 mmol) Methyl-2-(4-fluorphenyl)-6-methyl-5,6-dihydro-4H-pyrrolo[1,2-b]pyrazol-3-carboxylat in 50 ml Methanol wird mit 36,8 mL (73,6 mmol) einer 2 M wässrigen NaOH-Lösung versetzt und 3 h nachgerührt. Das Reaktionsgemisch wird unter vermindertem Druck eingeengt, mit Ethylacetat/1 M Salzsäure versetzt, die organische Phase abgetrennt und die wäßrige Phase mit 3 x 100ml Ethylacetat extrahiert. Die vereinigten organischen Phasen werden über Na₂SO₄ getrocknet und unter vermindertem Druck vom Lösungsmittel befreit. Die erhaltene 2-(4-Fluorphenyl)-6-methyl-5,6-dihydro-4H-pyrrolo[1,2-b]pyrazol-3-carbonsäure(5,9 g) wird ohne weitere Aufreinigung weiter umgesetzt.

Analog lassen sich herstellen:
- **2-(4-Fluorphenyl)-4-methyl-5,6-dihydro-4H-pyrrolo[1,2-b]pyrazol-3-carbonsäure (XXXI-2);** logP(HCOOH): 1,84; ¹H-NMR (ppm): δ (DMSO-d*6*) = 1.33 (d, 3 H), 2.12-2.19 (m, 1H), 2.76-2.86 (m, 1H), 3.40-3.49 (m, 1H), 4.08-4.26 (m, 2H), 7.17-7.23 (m, 2H), 7.69-7.74 (m, 2H); LC-MS: m/z = 261 [M+H]⁺.
- **2-(2-Fluorphenyl)-6-methyl-5,6-dihydro-4H-pyrrolo[1,2-b]pyrazol-3-carbonsäure (XXXI-3);** logP(HCOOH): 1,64; m/z = 261 [M+H]⁺.
- **2-(2-Fluorphenyl)-5,6-dihydro-4H-pyrrolo[1,2-b]pyrazol-3-carbonsäure (XXXI-4);** logP(HCOOH): 1,35; ¹H-NMR (ppm): δ (DMSO-d*6*) = 2.58 (quintet, 2H), 3.04 (ABq, 2H), 4.16 (ABq, 2H), 7.20-7.23 (m, 2H), 7.40-7.45 (m, 2H); LC-MS: m/z = 247 [M+H]+.

### Beispiel für Verfahrensschritt [V31] aus Schema 11:

### N'-(diphenylmethylene)-4-hydroxypentanehydrazide (XXXIII-1)

Zu einer Lösung aus 0,78 g (4,0 mmol) Benzophenonhydrazon in 10 ml Dichlormethan tropft man bei Raumtemperatur 6,0 mL (12,0 mmol) einer 2,0 M Trimethylaluminum-Lösung in Heptan und läßt 30 min nachrühren. Dem Reaktiongemisch wird dann eine Lösung aus 0,40 g (4,0 mmol) γ-Valerolakton in 5 mL Dichlormethan zugetropt. Das Reaktionsgemisch wird 2 h bei Rückfluss gerührt. Weitere 0,12 g (1,20 mmol) γ-Valerolakton werden nachgesetzt und das Reaktionsgemisch 1 h bei Rückfluss nachgerührt. Nach dem Erkalten versetzt man das Reaktionsgemisch mit Dichloromethan/Wasser. Die organische Phase wird abgetrennt und die wäßrige Phase mit Dichloromethan extrahiert. Die vereinigten organischen Phasen werden über Na₂SO₄ getrocknet und unter vermindertem Druck vom Lösungsmittel befreit. Nach säulenchromatographischer Reinigung über Kieselgel (Cyclohexan/Ethylacetet) erhält man 0,9 g (76%) des gewünschten Produktes; (logP(HCOOH): 2,25); ¹H-NMR (ppm): δ (DMSO-d*6*) = 1.01 (d, 1H), 1.10 (d, 2H), 1.45-1.72 (m, 2H), 2.16-2.26 (m, 1H), 2.75-2.86 (m, 1H), 3.51-3.57 (m, 0.3H), 3.65-3.71 (m, 0.7H), 4.43 (d, 0.3H), 4.52 (d, 0.7H), 7.25-7.62 (m, 10H), 8.99 (s, 1H); LC-MS: m/z = 297 [M+H]⁺

Analog lassen sich herstellen:
- **N'-(diphenylmethylene)-4-hydroxy-4-phenylbutanehydrazide (XXXIII-2);** logP(HCOOH): 3,07; ¹H-NMR (ppm): δ (DMSO-D₆) = 1.79-1.86 (m, 1H), 1.90-1.98 (m, 1H), 2.19-2.23 (m, 1H), 2.76-2.80 (m, 1H), 4.48-4.52 (m, 0.3H), 4.63-4.67 (m, 0.7H), 5.23 (d, 0.3H), 5.31 (d, 0.7H), 7.19-7.60 (m, 15H), 9.00 (s, 1H); LC-MS: m/z = 359 [M+H]⁺.
- **N'-(diphenylmethylene)-4-hydroxy-2-methylbutanehydrazide (XXXIII-3);** logP(HCOOH): 2.24; ¹H-NMR (ppm): δ (DMSO-d*6*)= 0.98 (d, 1.5H), 1.14 (d, 1.5H), 1.39-1.46 (m, 0.5H), 1.49-1.57 (m, 0.5H), 1.65-1.71 (m, 0.5H), 1.85-1.91 (m, 0.5H), 2.50-2.54 (m, 1H), 3.29-3.39 (m, 1H), 3.41-3.51 (m, 1H), 4.41 (dd, 0.5H), 4.51 (dd, 0.5H), 7.27-7.63 (m, 10H), 8.94 (s, 1H); LC-MS: m/z = 297 [M+H]⁺.

### Beispiel für Verfahrensschritt [V32] aus Schema 11:

### 4-bromo-N'-(diphenylmethylene)-2-methylbutanehydrazide (XXV-2)

Zu einer Lösung aus 6,0 g (20,2 mmol) N'-(diphenylmethylene)-4-hydroxy-2-methylbutanehydrazide, und 6.3 g (24.3 mmol) Triphenylphosphan in 150 ml Dichlormethan wird 8,1 g (24,3 mmol) Carbontetrabromid bei 0 °C versetzt. Das Reaktionsgemisch wird 1 h bei 0 °C gerührt, im Anschluß eingeengt und säulenchromatographisch über Kieselgel gereinigt (Cyclohexan/Ethylacetet). Man erhält 2,7 g (37%) des gewünschten Produktes; (logP(HCOOH): 3,76); ¹H-NMR (ppm (DMSO-d*6*) = 1.12 (d, 3H), 1.79-1.86 (m, 1H), 2.33-2.38 (m, 1H), 2.62-2.67 (m, 1H), 4.10-4.14 (m, 1H), 4.25-4.29 (m, 1H), 7.29-7.65 (m, 10H); LC-MS: m/z = 359 [M+H]⁺.

### Beispiel für Verfahrensschritt [V33] aus Schema 11:

### 1-[(Diphenylmethylen)amino]-5-methylpyrrolidin-2-on (XXVI-4)

Zu einer Lösung von 0,64 g (2,2 mmol) N'-(diphenylmethylene)-4-hydroxypentanehydrazide, 0,19 mL (2,4 mmol) Pyridin und 0.01 mg *N*-*N*-Dimethylaminopyridin in 5 mL Dichlormethan wird bei 0 °C 0,25 mL (3,2 mmol) Methansulfonylsäurechlorid zugetropft. Das Reaktionsgemisch wird 2 h bei Raumtemperatur gerührt und im Anschluß mit Ethylacetat/Wasser versetzt. Die organische Phase wird abgetrennt und die wäßrige Phase mit 3 x 100 ml Ethylacetat extrahiert. Die vereinigten organischen Phasen werden über Na₂SO₄ getrocknet und unter vermindertem Druck vom Lösungsmittel befreit. Nach säulenchromatographischer Reinigung über Kieselgel (Cyclohexan/Ethylacetet) erhält man 0,38 g (44% Reinheit) 5-[2-(diphenylmethylene)hydrazino]-5-oxopentan-2-ylmethanesulfonate; (logP(HCOOH): 2,82 mit LC-MS: m/z = 375 [M+H]⁺). Das Produkt wird in 2 ml Tetrahydrofuran wiederaufgenommen und bei 0 °C mit 0,04 g (1,0 mmol) Natriumhydrid versetzt. Nach 16 h Rühren bei Raumtemperatur wird mit Ethylacetat/gesättigter wässriger NH₄Cl-Lösung versetzt, die organische Phase abgetrennt und die wäßrige Phase mit 3x 100 ml Ethylacetat extrahiert. Die vereinigten organischen Phasen werden über Na₂SO₄ getrocknet und unter vermindertem Druck vom Lösungsmittel befreit. Man erhält 0,21 g (61%) des gewünschten Produktes; (logP(HCOOH): 2,73); ¹H-NMR (ppm (DMSO-d*6*) = 1.22 (d, 3 H), 2.05-2.15 (m, 4H), 3.86 (sextet, 1H), 7.24-7.26 (m, 2H), 7.40-7.43 (m, 4H), 7.48-7.53 (m, 4H); LC-MS: m/z = 279 [M+H]⁺.

### Beispiel für Verfahrensschritt [V36] aus Schema 11:

### 2-(4-fluorophenyl)-3-iodo-6-methyl-5,6-dihydro-4H-pyrrolo[1,2-b]pyrazole (VIII-19)

Eine Lösung von 3,2 g (12,3 mmol) 2-(4-Fluorphenyl)-6-methyl-5,6-dihydro-4H-pyrrolo[1,2-b]pyrazol-3-carbonsäure in 25 ml Dimethylformamid wird mit 3,1 g (36,9 mmol) NaHCO₃, und 2,8 g (12,3 mmol) N-Jodsuccinimid versetzt. Das Reaktionsgemisch wird bei Raumtemperatur 16 h gerührt, mit Wasser versetzt, der ausgefallene Feststoff filtriert und getrocknet. Man erhält 3,30g des gewünschten Produktes in einer Reinheit von 77%, das ohne weitere Aufreinigung weiter umgesetzt wird: (logP(HCOOH): 3,82); ¹H-NMR (ppm): δ (DMSO-d6) = 1.42 (d, 3 H), 2.11-2.18 (m, 1H), 2.72-2.82 (m, 3H), 4.49 (m, 1H), 7.28 (dd, 2H), 7.79 (dd, 2H); LC-MS: m/z = 343 [M+H]⁺.

Analog lassen sich herstellen:
- **2-(4-fluorophenyl)-3-bromo-6-methyl-5,6-dihydro-4H-pyrrolo[1,2-b]pyrazole (VIII-20);** analog mit *N*-Bromsuccinimid; logP(HCOOH): 3,77; ¹H-NMR (ppm): δ (DMSO-d*6*) = 1.43 (d, 3 H), 2.12-2.18 (m, 1H), 2.73-2.89 (m, 3H), 4.47 (m, 1H), 7.29 (dd, 2H), 7.82 (dd, 2H); LC-MS: m/z = 295 [M+H]⁺
- **2-(4-fluorophenyl)-3-iodo-4-methyl-5,6-dihydro-4H-pyrrolo[1,2-b]pyrazole (VIII-21);** logP(HCOOH): 3,67; ¹H-NMR (ppm): δ (DMSO-d*6*) = 1.40 (d, 3 H), 2.09-2.19 (m, 1H), 2.74-2.83 (m, 1H), 3.24-3.32 (m, 1H), 4.07-4.13 (m, 1H), 4.20-4.26 (m, 1H), 7.28 (dd, 2H), 7.77 (dd, 2H); LC-MS: m/z = 343 [M+H]⁺.
- **3-bromo-2-(2-fluorophenyl)-5,6-dihydro-4H-pyrrolo[1,2-b]pyrazole (VIII-22);** analog mit *N-*Bromsuccinimid; logP(HCOOH): 2,81; ¹H-NMR (ppm): δ (DMSO-d*6*) = 2.58 (quintet, 2H), 2.87 (ABq, 2H), 4.19 (ABq, 2H), 7.28-7.32 (m, 2H), 7.46-7.51 (m, 2H); LC-MS: m/z = 281 [M+H]⁺.

Analog zu den zuvor beschriebenen Methoden lassen sich die in der nachstehenden Tabelle 1 aufgeführten Verbindungen der allgemeinen Formel (**Ik**) darstellen.

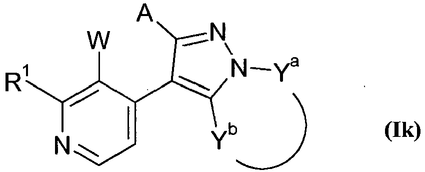

R¹ bedeutet in nachstehender Tabelle beispielsweise R^{1a}, NH-R^{1a} oder NHCOR^{1a}.

**Tabelle 1**

| **Bsp.** | **R¹** | **R^{1a}** | **W** | **A** | **-Y^{a}-Y^{b}-** | **Log P** |
|---|---|---|---|---|---|---|
| 1 | R^{1a} | Chlor | H | 4-Fluorphenyl | -CH₂CH₂CH₂CH₂- | 3,26^{[a]} |
| 2 | R^{1a} | H | H | 4-Fluorphenyl | -CH₂CH₂CH₂CH₂- | 1,03^{[a]} 2,43^{[b]} |
| 3 | NH-R^{1a} | H | H | 4-Fluorphenyl | -CH₂CH₂CH₂CH₂- | 1,21^{[a]}; 2,05^{[b]} |
| 4 | NH-R^{1a} | Benzyl | H | 4-Fluorphenyl | -CH₂CH₂CH₂CH₂- | 1,79^{[a]}; |
| 5 | NH-CO-R^{1a} | Ethyl | H | 4-Fluorphenyl | -CH₂CH₂CH₂CH₂- | 1,97^{[a]}; 2,58^{[b]} |
| 6 | R^{1a} | CH₃ | H | 4-Fluorphenyl | -CH₂CH₂CH₂CH₂- | 1,22^{[a]}; 2,67^{[b]} |
| 7 | R^{1a} | H | H | 4-Fluorphenyl | -CH₂CH₂CH₂- | 2,08^{[b]} |
| 8 | NH-CO-R^{1a} | Ethyl | H | Phenyl | -CH₂CH₂CH₂CH₂- | 1,77^{[a]}; 2,46^{[b]} |
| 9 | R^{1a} | H | H | Phenyl | -CH₂CH₂CH₂CH₂- | 1,06^{[a]}; 2,32^{[b]} |
| 10 | R^{1a} | H | H | Phenyl | -CH₂CH₂CH₂- | 1,96^{[b]} |
| 11 | R^{1a} | H | H | 4-Chlorphenyl | -CH₂CH₂CH₂- | 2,46^{[b]} |
| 12 | R^{1a} | H | H | 4-Methoxyphenyl | -CH₂CH₂CH₂- | 0,72^{[a]} |
| 13 | R^{1a} | Fluor | H | 4-Chlorphenyl | -CH₂CH₂CH₂- | 3,12^{[b]} |
| 14 | NH-R^{1a} | H | H | Phenyl | -CH₂CH₂CH₂CH₂- | 1,12^{[a]}; 1,89^{[b]} |
| 15 | NH-CO-R^{1a} | CH₃ | H | 4-Fluorphenyl | -CH₂CH₂CH₂CH₂- | 1,66^{[a]}; 2,14^{[b]} |
| 16 | NH-CO-R^{1a} | tert-Butoxy | H | 4-Fluorphenyl | -CH₂CH₂CH₂CH₂- | 1,73^{[a]}; 3,04^{[b]} |
| 17 | R^{1a} | H | H | 4-(Trifluormethyl)phenyl | -CH₂CH₂CH₂- | 2,75^{[b]} |
| 18 | NH-CO-R^{1a} | tert-Butoxy | H | 4-Fluorphenyl | -CH₂CH₂CH₂- | 2,5^{[a]} |
| 19 | NH-CO-R^{1a} | CH₃ | H | Phenyl | -CH₂CH₂CH₂CH₂- | 1,57^{[a]}; 2,03^{[b]} |
| 20 | NH-CO-R^{1a} | Cyclopropyl | H | 4-Fluorphenyl | -CH₂CH₂CH₂CH₂- | 2,04^{[a]}; 2,67^{[b]} |
| 21 | NH-CO-R^{1a} | Cyclopropyl | H | Phenyl | -CH₂CH₂CH₂CH₂- | 1,85^{[a]}; 2,52^{[b]} |
| 22 | NH-CO-R^{1a} | Methoxymethyl | H | 4-Fluorphenyl | -CH₂CH₂CH₂CH₂- | 2,3^{[a]}; 2,54^{[b]} |
| 23 | NH-CO-R^{1a} | Methoxymethyl | H | Phenyl | -CH₂CH₂CH₂CH₂- | 2,12^{[a]}; 2,39^{[b]} |
| 24 | NH-CO-R^{1a} | (1S)-1-Hydroxyethyl | H | 4-Fluorphenyl | -CH₂CH₂CH₂CH₂- | 1,91^{[a]}; 2,14^{[b]} |
| 25 | NH-CO-R^{1a} | (1S)-1-Hydroxyethyl | H | Phenyl | -CH₂CH₂CH₂CH₂- | 1,74^{[a]}; 2,01^{[b]} |
| 26 | NH-CO-R^{1a} | Methoxy | H | 4-Fluorphenyl | -CH₂CH₂CH₂CH₂- | 2,11^{[a]}; 2,56^{[b]} |
| 27 | NH-CO-R^{1a} | Methoxy | H | Phenyl | -CH₂CH₂CH₂CH₂- | 1,92^{[a]}; 2,4^{[b]} |
| 28 | NH-CO-R^{1a} | Propan-2-yl | H | 4-Fluorphenyl | -CH₂CH₂CH₂CH₂- | 2,33^{[a]}; 2,87^{[b]} |
| 29 | NH-CO-R^{1a} | Propan-2-yl | H | Phenyl | -CH₂CH₂CH₂CH₂- | 2,13^{[a]}; 2,72^{[b]} |
| 30 | NH-CO-R^{1a} | 2-Methylpropyl | H | 4-Fluorphenyl | -CH₂CH₂CH₂CH₂- | 2,68^{[a]}; 3,18^{[b]} |
| 31 | NH-CO-R^{1a} | 2-Methylpropyl | H | Phenyl | -CH₂CH₂CH₂CH₂- | 2,47^{[a]}; 3,04^{[b]} |
| 32 | NH-CO-R^{1a} | Phenyl | H | 4-Fluorphenyl | -CH₂CH₂CH₂CH₂- | 3^{[a]}; 3,34^{[b]} |
| 33 | NH-CO-R^{1a} | Phenyl | H | Phenyl | -CH₂CH₂CH₂CH₂- | 2,78^{[a]}; 3,2^{[b]} |
| 34 | NH-CO-R^{1a} | Benzyl | H | 4-Fluorphenyl | -CH₂CH₂CH₂CH₂- | 2,9^{[a]}; 3,26^{[b]} |
| 35 | NH-CO-R^{1a} | Benzyl | H | Phenyl | -CH₂CH₂CH₂CH₂- | 2,7^{[a]}; 3,12^{[b]} |
| 36 | NH-CO-R^{1a} | H | H | 4-Fluorphenyl | -CH₂CH₂CH₂CH₂- | 1,28^{[a]}; 2,12^{[b]} |
| 37 | NH-CO-R^{1a} | H | H | Phenyl | -CH₂CH₂CH₂CH₂- | 1,87^{[a]}; 1,98^{[b]} |
| 38 | R^{1a} | [(Dimethylamino)methyliden]a mino | H | Phenyl | -CH₂CH₂CH₂CH₂- | 1,28^{[a]}; 2,28^{[b]} |
| 39 | R^{1a} | [(Dimethylamino)methyliden]a mino | H | 4-Fluorphenyl | -CH₂CH₂CH₂CH₂- | 1,44^{[a]}; 2,47^{[b]} |
| 40 | NH-CO-R^{1a} | Ethyl | H | 4-Fluorphenyl | -CH₂CH₂CH₂- | 1,64^{[a]} |
| 41 | R^{1a} | H | H | 4-Methylphenyl | -CH₂CH₂CH₂- | 2,34^{[b]} |
| 42 | R^{1a} | H | H | 3-(Trifluormethyl)phenyl | -CH₂CH₂CH₂- | 1,42^{[a]} |
| 43 | R^{1a} | H | H | 4-Fluorphenyl | -CH₂CH₂CH₂CH₂CH₂- | 1,4^{[a]} |
| 44 | R^{1a} | H | H | 3-Fluorphenyl | -CH₂CH₂CH₂- | 2,12^{[b]} |
| 45 | R^{1a} | Fluor | H | 4-Fluorphenyl | -CH₂CH₂CH₂- | 2,69^{[a]} |
| 46 | R^{1a} | H | H | Thiophen-3-yl | -CH₂CH₂CH₂- | 0,72^{[a]} |
| 47 | NH-CO-R^{1a} | Thiophen-3-yl | H | 4-Fluorphenyl | -CH₂CH₂CH₂CH₂- | 2,75^{[a]}; 3,24^{[b]} |
| 48 | NH-R^{1a} | Propan-2-yl | H | 4-Chlorphenyl | -CH₂CH₂CH₂- | 3,25^{[b]} |
| 49 | NH-CO-R^{1a}, | tert-Butoxy | H | Thiophen-3-yl | -CH₂CH₂CH₂CH₂- | 2,76^{[a]} |
| 50 | NH-CO-R^{1a} | 2-Hydroxypropan-2-yl | H | Phenyl | -CH₂CH₂CH₂CH₂- | 2^{[a]}; 2,29^{[b]} |
| 51 | NH-CO-R^{1a} | Thiophen-3-yl | H | Phenyl | -CH₂CH₂CH₂CH₂- | 2,49^{[a]}; 3,09^{[b]} |
| 52 | NH-R^{1a} | Propan-2-yl | H | 4-Fluorphenyl | -CH₂CH₂CH₂- | 1,4^{[a]} |
| 53 | NH-R^{1a} | H | H | Thiophen-3-yl | -CH₂CH₂CH₂CH₂- | 0,82^{[a]}; 1,8^{[b]} |
| 54 | R^{1a} | Fluor | H | 3-Fluorphenyl | -CH₂CH₂CH₂- | 2,66^{[b]} |
| 55 | NH-CO-R^{1a} | 2-Hydroxypropan-2-yl | H | 4-Fluorphenyl | -CH₂CH₂CH₂CH₂- | 2,16^{[a]}; 2,41^{[b]} |
| 56 | NH-R^{1a} | Propan-2-yl | H | 3-Fluorphenyl | -CH₂CH₂CH₂- | 2,86^{[b]} |
| 57 | R^{1a} | H | H | 5-Chlorthiophen-2-yl | -CH₂CH₂C(Cl₂)CH(Cl)- | 3,23^{[a]} |
| 58 | NH-CO-R^{1a} | CH₃ | H | Thiophen-3-yl | -CH₂CH₂CH₂CH₂- | 1,47^{[a]}; 1,92^{[b]} |
| 59 | NH-CO-R^{1a} | Ethyl | H | Thiophen-3-yl | -CH₂CH₂CH₂CH₂- | 1,73^{[a]}; 2,25^{[b]} |
| 60 | NH-CO-R^{1a} | Methoxymethyl | H | Thiophen-3-yl | -CH₂CH₂CH₂CH₂- | 2,06^{[a]}; 2,27^{[b]} |
| 61 | R^{1a} | Fluor | H | Phenyl | -CH₂CH₂CH₂- | 2,53^{[bJ} |
| 62 | R^{1a} | H | H | Thiophen-2-yl | -CH₂CH₂CH₂CH₂- | 1,05^{[a]}; 2,2^{[b]} |
| 63 | NH-CO-R^{1a} | Propan-2-yl | H | Thiophen-3-yl | -CH₂CH₂CH₂CH₂- | 2,03^{[a]}; 2,6^{[b]} |
| 64 | NH-CO-R^{1a} | 2-Methylpropyl | H | Thiophen-3-yl | -CH₂CH₂CH₂CH₂- | 2,37^{[a]}; 2,91^{[b]} "' |
| 65 | NH-CO-R^{1a} | Cyclopropyl | H | Thiophen-3-yl | -CH₂CH₂CH₂CH₂- | 1,8^{[a]}; 2,44^{[b]} |
| 66 | NH-CO-R^{1a} | (1R)-1-Hydroxyethyl | H | Thiophen-3-yl | -CH₂CH₂CH₂CH₂- | 1,73^{[a]}; 1,95^{[b]} |
| 67 | NH-CO-R^{1a} | Methoxy | H | Thiophen-3-yl | -CH₂CH₂CH₂CH₂- | 1,92^{[a]}; 2,32^{[b]} |
| 68 | NH-CO-R^{1a} | 2-Hydroxypropan-2-yl | H | Thiophen-3-yl | -CH₂CH₂CH₂CH₂- | 1,89^{[a]}; 2,16^{[b]} |
| 69 | NH-R^{1a} | H | H | 4-Fluorphenyl | -CH₂CH₂CH₂- | 1,8^{[b]} |
| 70 | NH-R^{1a} | Propan-2-yl | H | Phenyl | -CH₂CH₂CH₂- | 2,69^{[b]} |
| 71 | NH-R^{1a} | 2-Methylpropyl | H | 4-Fluorphenyl | -CH₂CH₂CH₂- | 3,18^{[b]} |
| 72 | NH-R^{1a} | 2-Methylpropyl | H | Phenyl | -CH₂CH₂CH₂- | 3,06^{[b]} |
| 73 | NH-R^{1a} | 2-Methylpropyl | H | 3-Fluorphenyl | -CH₂CH₂CH₂- | 1,54^{[a]} |
| 74 | R^{1a} | H | H | 4-Fluorphenyl | -CH(CH₃)CH₂CH₂- | 1,13^{[a]} |
| 75 | NH-CO-R^{1a} | Ethyl | H | Thiophen-2-yl | -CH₂CH₂CH₂CH₂- | 1,89^{[a]}; 2,35^{[b]} |
| 76 | NH-CO-R^{1a} | Ethyl | H | 4-Fluorphenyl | -CH(CH₃)CH₂CH₂- | 1,92^{[a]} |
| 77 | NH-CO-R^{1a} | Propan-2-yl | H | 4-Fluorphenyl | -CH(CH₃)CH₂CH₂- | 2,3^{[a]} |
| 78 | NH-CO-R^{1a} | Cyclopropyl | H | 4-Fluorphenyl | -CH(CH₃)CH₂CH₂- | 2,07^{[a]} |
| 79 | NH-CO-R^{1a} | Cyclopropylmethyl | H | 4-Fluorphenyl | -C(CH₃)₂OCH₂CH₂- | 2,97^{[a]} |
| 80 | R^{1a} | H | H | 5-Chlorthiophen-2-yl | -CH₂CH₂CH₂CH(Cl)- | 2,21^{[a]} |
| 81 | NH-R^{1a} | H | H | Thiophen-2-yl | -CH₂CH₂CH₂CH₂- | 1,04^{[a]}; 1,89^{[b]} |
| 82 | NH-CO-R^{1a} | Methoxymethyl | H | 4-Fluorphenyl | -C(CH₃)₂OCH₂CH₂- | 2,92^{[a]} |
| 83 | NH-CO-R^{1a} | Propan-2-yl | H | 4-Fluorphenyl | -C(CH₃)₂OCH₂CH₂- | 2,81^{[a]} |
| 84 | NH-CO-R^{1a} | Cyclopropyl | H | 4-Fluorphenyl | -C(CH₃)₂OCH₂CH₂- | 2,67^{[a]} |
| 85 | R^{1a} | H | H | 4-Fluorphenyl | -CH₂CH₂CH₂CH(OCH₃)- | 1,23^{[a]}; 2,27^{[b]} |
| 86 | NH-CO-R^{1a} | Ethyl | H | 4-Fluorphenyl | -C(CH₃)₂OCH₂CH₂- | 2,44^{[a]} |
| 87 | NH-CO-R^{1a} | CH₃ | H | Thiophen-2-yl | -CH₂CH₂CH₂CH₂- | 1,54^{[a]}; 2^{[b]} |
| 88 | NH-CO-R^{1a} | Propan-2-yl | H | Thiophene-2-yl | -CH₂CH₂CH₂CH₂- | 2,12^{[a]}; 2,7^{[b]} |
| 89 | NH-CO-R^{1a} | Cyclopropyl | H | Thiophen-2-yl | -CH₂CH₂CH₂CH₂- | 1,9^{[a]}; 2,51^{[b]} |
| 90 | NH-CO-R^{1a} | CH₃ | H | 4-Fluorphenyl | -C(CH₃)₂OCH₂CH₂- | 2,17^{[a]} |
| 91 | NH-CO-R^{1a} | Propan-2-yl | H | 4-Fluorphenyl | -CH₂OCH₂CH₂- | 2,21^{[a]} |
| 92 | NH-CO-R^{1a} | Propan-2-yl | H | 4-Fluorphenyl | -CH₂CH₂OCH₂- | 2^{[a]} |
| 93 | NH-CO-R^{1a} | Cyclopropyl | H | 4-Fluorphenyl | -CH₂CH₂OCH₂- | 1,8^{[a]} |
| 94 | NH-CO-R^{1a} | Methoxymethyl | H | 4-Fluorphenyl | -CH₂OCH₂CH₂- | 2,14^{[a]} |
| 95 | NH-CO-R^{1a} | Ethyl | H | 4-Fluorphenyl | -CH₂OCH₂CH₂- | 1,87^{[a]} |
| 96 | NH-CO-R^{1a} | Ethyl | H | 4-Fluorphenyl | -CH₂CH₂CH₂CH₂CH₂- | 2,44^{[a]} |
| 97 | NH-CO-R^{1a} | Propan-2-yl | H | 4-Fluorphenyl | -CH₂CH₂CH₂CH₂CH₂- | 2,91^{[a]} |
| 98 | -NH-CO-R^{1a} | Benzyl | H | 4-Fluorphenyl | -CH₂CH₂CH₂CH₂CH₂- | 3,52^{[a]} |
| 99 | NH-CO-R^{1a} | CH₃ | H | 4-Fluorphenyl | -CH₂CH₂CH₂CH₂CH₂- | 2,2^{[a]} |
| 100 | NH-CO-R^{1a} | Ethyl | H | Phenyl | -CH₂CH₂OCH₂- | 1,57^{[a]} |
| 101 | NH-CO-R^{1a} | 2-Methylcyclopropyl | H | 4-Fluorphenyl | -C(CH₃)₂OCH₂CH₂- | 2,83^{[a]} |
| 102 | NH-CO-R^{1a} | Cyclobutyl | H | 4-Fluorphenyl | -C(CH₃)₂OCH₂CH₂- | 2,93^{[a]} |
| 103 | NH-R^{1a} | H | H | 4-Fluorphenyl | -CH₂OCH₂CH₂- | 0,99^{[a]} |
| 104 | NH-CO-R^{1a} | Ethyl | H | 4-Fluorphenyl | -CH₂CH₂OCH₂- | 1,71^{[a]} |
| 105 | NH-CO-R^{1a} | 2-Methylpropyl | H | 4-Fluorphenyl | -C(CH₃)₂OCH₂CH₂- | 3,26^{[a]} |
| 106 | NH-CO-R^{1a} | CH₃ | H | 4-Fluorphenyl | -CH₂OCH₂CH₂- | 1,6^{[a]} |
| 107 | NH-CO-R^{1a} | Cyclopropyl | H | 4-Fluorphenyl | -CH₂OCH₂CH₂- | 1,97^{[a]} |
| 108 | NH-CO-R^{1a} | 2-Methylcyclopropyl | H | 4-Fluorphenyl | -CH₂OCH₂CH₂- | 2,19^{[a]} |
| 109 | NH-CO-R^{1a} | Cyclobutyl | H | 4-Fluorphenyl | -CH₂OCH₂CH₂- | 2,26^{[a]} |
| 110 | NH-CO-R^{1a} | Ethyl | H | 4-Fluorphenyl | -CH₂CH₂CH(CH₃)- | 2,02^{[a]} |
| 111 | NH-CO-R^{1a} | Propan-2-yl | H | 4-Fluorphenyl | -CH₂CH₂CH(CH₃)- | 2,34^{[a]} |
| 112 | NH-CO-R^{1a} | 2-Methylpropyl | H | 4-Fluorphenyl | -CH₂OCH₂CH₂- | 2,51^{[a]} |
| 113 | NH-CO-R^{1a} | Cyclopropylmethyl | H | 4-Fluorphenyl | -CH₂CH₂OCH₂- | 2,22^{[a]} |
| 114 | R^{1a} | H | H | 5-Chlorthiophen-2-yl | -CH₂CH₂CH₂CH(OCH₃)- | 1,59^{[a]}; 2,88^{[b]} |
| 115 | NH-CO-R^{1a} | Ethyl | H | Phenyl | -CH₂CH₂SCH₂- | 1,98^{[a]} |
| 116 | NH-CO-R^{1a} | Propan-2-yl | H | 4-Fluorphenyl | -CH(CH₃)OCH₂CH₂- | 2,59^{[a]} |
| 117 | R^{1a} | H | F | 4-Fluorphenyl | -CH(CH₃)CH₂CH₂- | 2,66^{[a]} |
| 118 | R^{1a} | H | F | 4-Fluorphenyl | -CH₂CH₂CH₂CH₂CH₂- | 3,08^{[a]} |

Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.
[c] Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

Die ¹H-NMR-Daten ausgewählter Beispiele sind in Form von ¹H-NMR-Peaklisten notiert. Zu jedem Signalpeak wird erst der δ-Wer in ppm und dann die Signalintensität durch ein Leerzeichen getrennt aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ Intensität₁;δ₂ Intensität₂;........;δᵢ Intensitätᵢ;......; δₙ Intensitätₙ

Das Lösungsmittel, in welchem das NMR-Spektrum aufgenommen wurde, wird in eckigen Klammern hinter der Nummer des Beispieles und vor der Peakliste aufgeführt.

| |
|---|
| Beispiel Nr. 2 [CD ₃CN] 11,99 0,40;8,47 12,97;8,47 7,86;8,46 7,66;8,45 13,47;8,22 0,39;7,67 0,81;7,66 1,21;7,66 0,75;7,65 1,49;7,64 1,55;7,64 1,17;7,62 0,61;7,62 1,58;7,61 1,85;7,61 1,01;7,60 0,94;7,59 0,87;7,59 0,98;7,58 0,43;7,54 1,08;7,54 0,85;7,53 1,20;7,52 1,57;7,51 1,43;7,49 0,70;7,41 0,73;7,41 7,03;7,40 2,74;7,39 7,75;7,38 8,88;7,38 2,97;7,37 8,40;7,36 1,09;7,36 0,52;7,12 15,59;7,12 9,55;7,11 9,27;7,11 15,62;7,08 0,87;7,07 8,56;7,07 2,55;7,06 2,53;7,05 16,00;7,04 2,83;7,03 2,53;7,03 7,69;7,02 0,70;5,73 0,39;5,45 0,48;4,18 5,93;4,16 10,77;4,15 6,32;3,55 0,40;3,27 0,46;3,14 0,37;2,83 5,42;2,81 10,85;2,79 6,16;2,76 0,40;2,62 0,44;2,18 0,54;2,15 551,68;2,13 4,84;2,12 1,12;2,11 1,33;2,11 2,84;2,10 2,13;2,09 3,50;2,08 3,26;2,08 5,42;2,07 3,21;2,06 4,10;2,05 1,23;2,05 2,24;2,00 0,44;1,98 3,51;1,96 557,98;1,96 13,95;1,95 92,46;1,95 172,38;1,94 247,36;1,93 168,83;1,93 85,79;1,90 0,61;1,89 2,35;1,89 1,48;1,88 5,48;1,87 2,91;1,86 4,97;1,86 2,75;1,85 4,23;1,84 1,23;1,83 1,44;1,79 3,22;1,78 0,53;1,77 1,31;1,77 1,53;1,76 0,96;1,76 0,59;1,38 0,40;1,27 0,68;1,20 0,78;1,14 12,44;1,11 1,17;1,10 0,55;0,97 0,41;0,00 6,71;-0,56 0,38;-0,91 0,38 |
| Beispiel Nr. 3 [CD ₃CN] 7,87 7,80;7,86 6,71;7,78 1,44;7,77 1,50;7,76 1,16;7,75 1,04;7,44 9,44;7,44 4,07;7,43 9,65;7,43 8,10;7,42 2,95;7,42 7,08;7,13 1,74;7,11 2,77;7,10 1,52;7,07 9,74;7,06 3,13;7,06 4,53;7,05 16,00;7,05 2,20;7,04 2,43;7,04 6,56;6,37 7,59;6,36 7,23;6,36 6,66;6,35 6,10;6,32 10,23;6,32 10,42;6,30 1,41;4,82 5,76;4,16 8,39;4,15 14,05;4,14 7,58;4,10 1,14;4,09 2,01;4,08 1,12;4,07 0,54;4,05 0,56;3,68 0,43;3,60 2,65;3,53 0,62;3,52 0,49;2,81 1,58;2,80 2,56;2,79 8,07;2,78 14,32;2,77 7,81;2,28 2,05;2,19 1484,50;2,08 2,72;2,07 5,29;2,07 4,98;2,06 8,04;2,06 5,25;2,05 6,72;2,05 4,22;2,04 3,86;2,03 1,81;2,02 1,52;1,97 5,85;1,97 111,79;1,96 16,95;1,95 18,95;1,95 136,97;1,95 254,23;1,94 364,32;1,94 249,63;1,93 135,01;1,87 3,21;1,87 2,52;1,86 7,43;1,86 5,06;1,85 8,47;1,85 5,29;1,84 7,14;1,84 3,12;1,83 4,76;1,83 3,15;1,82 2,23;1,45 5,08;1,27 4,10;1,22 1,13;1,20 2,13;1,20 2,53;1,19 1,09;1,13 7,78;1,11 0,95;0,91 1,33;0,88 0,75;0,00 3,34 |
| Beispiel Nr. 5 [CD ₃CN] 8,56 0,33;8,53 1,20;8,12 3,16;8,12 3,17;8,11 3,14;8,11 3,19;8,02 3,46;7,44 0,52;7,43 3,23;7,43 1,41;7,42 3,61;7,41 3,85;7,40 1,60;7,40 3,57;7,07 0,61;7,07 3,81;7,06 1,27;7,05 1,73;7,04 6,89;7,04 1,51;7,03 1,20;7,02 3,20;6,78 3,10;6,78 3,06;6,77 2,97;6,77 2,94;5,45 3,87;4,18 3,04;4,16 5,56;4,14 3,17;4,07 0,41;4,05 0,45;3,60 0,62;2,83 2,87;2,82 5,71;2,80 3,12;2,58 0,32;2,47 0,39;2,42 2,28;2,40 7,10;2,38 7,25;2,36 2,60;2,32 0,39;2,30 0,47;2,16 3440,79;2,13 13,90;2,12 7,86;2,11 7,71;2,11 7,72;2,10 6,49;2,09 5,91;2,09 4,54;2,08 5,30;2,07 3,92;2,07 4,12;2,05 2,75;1,96 348,62;1,96 48,76;1,95 229,54;1,95 416,81;1,94 574,60;1,93 393,69;1,93 202,54;1,90 4,69;1,88 4,85;1,88 3,70;1,87 4,13;1,86 3,41;1,84 1,76;1,79 2,58;1,78 1,98;1,77 2,97;1,77 3,77;1,76 2,92;1,76 1,71;1,73 0,70;1,71 0,63;1,70 0,58;1,65 0,50;1,62 0,44;1,60 0,41;1,59 0,44;1,58 0,48;1,55 0,42;1,50 0,39;1,46 0,34;1,44 0,38;1,41 0,35;1,40 0,34;1,39 0,38;1,33 0,34;1,33 0,33;1,27 1,81;1,25 0,38;1,23 0,40;1,22 0,85;1,20 1,36;1,19 0,75;1,14 1,26;1,13 7,97;1,11 16,00;1,09 7,52;0,91 1,43;0,90 0,33;0,15 1,39;0,05 0,43;0,01 17,04;0,00 336,21;-0,01 14,03;-0,15 1,41 |
| Beispiel Nr. 6 [CD ₃CN] 8,34 1,99;8,33 2,00;7,40 2,57;7,40 0,94;7,39 2,72;7,39 2,84;7,38 1,00;7,38 2,70;7,06 2,91;7,06 0,85;7,05 0,93;7,05 5,48;7,04 0,92;7,04 0,83;7,03 2,62;7,01 2,33;6,91 1,32;6,90 1,27;6,90 1,29;6,90 1,23;5,45 1,02;4,17 2,21;4,16 3,92;4,15 2,28;2,81 2,08;2,80 4,07;2,79 2,26;2,42 16,00;2,16 311,44;2,09 0,67;2,09 0,47;2,08 1,29;2,08 1,40;2,07 2,04;2,07 1,13;2,06 1,50;2,06 0,96;2,05 1,58;2,05 0,73;2,04 0,36;1,97 0,69;1,97 86,92;1,96 2,30;1,95 3,09;1,95 63,52;1,95 122,98;1,94 177,45;1,94 117,58;1,93 60,40;1,93 1,80;1,92 0,85;1,88 0,77;1,87 0,45;1,87 1,85;1,86 1,06;1,86 1,70;1,85 1,03;1,85 0,96;1,85 1,64;1,84 0,43;1,84 0,61;1,84 0,43;1,83 0,71;1,83 1,04;1,82 0,71;1,82 0,36;1,27 0,37;1,13 2,93;0,91 0,69;0,01 0,95;0,00 35,06;-0,01 1,01 |
| Beispiel Nr. 7 [DMSO-D₆] 8,45 14,21;8,45 9,47;8,44 9,73;8,44 14,23;7,95 1,10;7,44 1,27;7,43 7,47;7,42 3,71;7,41 8,66;7,41 9,90;7,40 4,10;7,39 8,79;7,39 1,33;7,24 1,58;7,24 9,24;7,23 3,34;7,22 4,25;7,21 15,98;7,21 3,93;7,20 3,19;7,19 7,37;7,18 1,16;7,15 16,00;7,14 10,41;7,14 10,38;7,13 15,31;4,19 6,64;4,17 10,29;4,15 6,85;4,10 0,38;3,91 0,56;3,48 0,35;3,47 0,35;3,32 762,33;3,08 6,00;3,06 9,64;3,04 6,98;2,89 8,51;2,73 6,85;2,68 0,65;2,67 0,83;2,67 0,74;2,66 0,58;2,65 2,17;2,63 5,71;2,61 7,80;2,59 4,97;2,58 1,94;2,56 0,69;2,54 1,36;2,51 39,75;2,51 71,47;2,50 91,33;2,50 64,50;2,49 32,07;2,33 0,46;2,33 0,60;2,32 0,50;2,07 1,53;1,99 1,14;1,19 0,33;1,180,65;1,161,37;1,073,83;0,010,66;0,00 11,20;-0,01 0,56 |
| Beispiel Nr. 8 [CD ₃CN] 8,53 1,12;8,11 3,09;8,11 3,15;8,10 3,15;8,10 3,18;8,03 3,46;7,42 2,54;7,41 1,89;7,41 1,96;7,41 3,74;7,40 2,68;7,40 3,90;7,39 4,04;7,33 0,32;7,33 0,34;7,31 2,13;7,31 5,95;7,30 3,89;7,30 6,48;7,29 6,44;7,28 0,74;6,78 3,01;6,77 2,95;6,77 2,96;6,76 2,81;5,45 0,50;4,18 3,17;4,17 5,71;4,15 3,27;2,83 3,01;2,82 5,88;2,80 3,25;2,42 2,29;2,40 7,11;2,38 7,35;2,36 2,55;2,15 675,49;2,12 3,68;2,11 3,82;2,11 3,64;2,10 3,87;2,09 2,99;2,08 3,90;2,08 2,67;2,07 2,97;2,06 1,44;2,05 1,66;2,03 0,64;1,97 4,46;1,96 34,85;1,96 16,18;1,95 78,40;1,95 145,50;1,94 202,52;1,93 139,85;1,93 71,95;1,90 2,62;1,90 2,01;1,89 3,55;1,88 2,55;1,87 3,23;1,87 2,22;1,86 2,62;1,84 1,11;1,81 0,33;1,79 0,43;1,78 0,62;1,77 1,02;1,77 1,30;1,76 0,96;1,76 0,59;1,27 0,63;1,22 0,44;1,20 0,80;1,19 0,44;1,14 0,77;1,13 7,90;1,11 1,86;1,11 16,00;1,10 1,03;1,09 7,46;0,91 0,59;0,01 4,13;0,00 79,89;-0,01 3,62 |
| Beispiel Nr. 9 [CD ₃CN] 8,46 12,86;8,46 7,80;8,45 7,80;8,45 13,17;7,39 0,50;7,39 0,79;7,39 5,54;7,38 5,31;7,38 4,64;7,38 5,42;7,38 6,38;7,37 4,19;7,37 8,36;7,36 1,28;7,32 2,03;7,32 5,24;7,32 16,00;7,31 7,73;7,31 14,42;7,31 7,23;7,30 13,01;7,30 2,39;7,30 0,96;7,12 15,56;7,12 9,27;7,11 9,12;7,11 15,39;5,45 0,33;4,18 6,91;4,17 12,35;4,16 7,20;4,05 0,33;2,83 6,58;2,82 12,77;2,81 7,06;2,17 1619,57;2,10 2,17;2,10 1,46;2,09 4,14;2,09 3,59;2,08 6,62;2,08 5,09;2,07 4,82;2,07 1,47;2,06 2,53;2,06 1,93;2,05 2,74;2,05 1,86;2,04 0,98;1,97 2,98;1,97 279,75;1,96 6,42;1,95 8,89;1,95 167,54;1,95 322,73;1,94 461,38;1,94 316,75;1,93 161,66;1,93 5,13;1,92 2,45;1,89 2,50;1,88 1,49;1,88 6,01;1,87 3,41;1,87 5,52;1,86 3,27;1,86 5,30;1,85 1,40;1,85 2,11;1,85 2,01;1,84 1,01;1,83 1,90;1,83 2,76;1,82 1,89;1,82 0,96;1,28 0,43;1,27 1,33;1,22 0,44;1,20 0,85;1,20 1,84;1,19 0,43;1,13 2,71;1,13 0,55;1,11 1,07;1,10 0,56;0,91 3,98;0,89 0,34;0,88 0,34;0,00 3,57 |
| Beispiel Nr. 10 [DMSO-D₆] 8,44 5,14;8,43 3,87;8,42 4,95;8,13 1,10;7,39 4,34;7,38 16,00;7,37 3,75;7,36 3,33;7,35 1,23;7,35 1,19;7,15 6,72;7,14 4,32;7,14 4,38;7,13 6,19;6,49 5,26;5,75 0,40;4,20 3,14;4,18 4,79;4,16 3,14;4,02 0,35;3,71 0,42;3,69 0,44;3,67 0,43;3,65 0,41;3,62 0,47;3,60 0,47;3,56 0,53;3,53 0,59;3,51 0,72;3,49 0,78;3,48 0,80;3,47 0,90;3,31 912,25;3,08 2,62;3,07 4,22;3,05 3,01;2,69 0,46;2,67 1,30;2,67 1,62;2,67 1,29;2,65 1,31;2,63 2,95;2,62 3,96;2,60 2,80;2,58 1,47;2,54 6,96;2,51 88,21;2,50 153,79;2,50 192,19;2,50 132,85;2,33 0,89;2,33 1,19;2,32 0,89;2,30 0,89;2,07 1,54;1,24 0,76;0,00 20,61 |
| Beispiel Nr. 11 [DMSO-D₆] 8,47 10,09;8,46 6,48;8,46 6,68;8,45 10,02;7,45 5,95;7,45 2,75;7,44 4,42;7,43 16,00;7,43 3,64;7,41 4,20;7,41 15,79;7,40 3,94;7,39 2,73;7,39 5,59;7,16 10,84;7,16 6,93;7,15 6,97;7,15 10,30;4,20 4,19;4,18 6,43;4,16 4,24;4,06 0,59;4,04 1,68;4,02 1,69;4,00 0,58;3,90 0,40;3,31 291,79;3,28 4,09;3,07 3,76;3,06 6,02;3,04 4,32;2,67 0,50;2,67 0,63;2,66 0,53;2,66 0,41;2,65 1,38;2,63 3,57;2,61 4,91;2,59 3,09;2,58 1,21;2,54 1,07;2,51 32,86;2,51 58,60;2,50 74,75;2,50 52,07;2,49 25,07;2,33 0,37;2,33 0,47;2,32 0,34;2,07 0,67;1,99 7,32;1,47 0,42;1,31 0,45;1,19 2,10;1,18 4,13;1,16 2,08;1,07 2,72;0,01 0,60;0,00 10,19;-0,01 0,41 |
| Beispiel Nr. 12 [DMSO-D₆] 8,73 0,63;8,73 0,44;8,72 0,45;8,72 0,67;8,44 3,45;8,43 2,24;8,42 2,28;8,42 3,53;7,83 0,76;7,83 0,50;7,82 0,49;7,82 0,73;7,32 0,50;7,31 3,47;7,31 1,27;7,30 1,39;7,29 3,92;7,28 0,52;7,15 3,71;7,15 2,38;7,14 2,37;7,14 3,56;6,96 0,58;6,95 3,90;6,95 1,36;6,93 1,29;6,93 3,42;6,92 0,47;4,17 1,42;4,15 2,23;4,14 1,48;3,78 16,00;3,31 295,82;3,07 1,33;3,05 2,10;3,03 1,54;2,67 0,40;2,67 0,52;2,66 0,40;2,64 0,49;2,62 1,27;2,60 1,75;2,58 1,16;2,57 0,54;2,54 1,05;2,51 29,97;2,50 54,32;2,50 69,80;2,50 49,02;2,49 24,18;2,33 0,40;2,33 0,49;2,32 0,37;2,07 0,44;1,99 0,83;1,17 0,46;0,00 3,60 |
| Beispiel Nr. 13 [DMSO-D₆] 8,13 0,67;8,12 0,68;7,47 0,74;7,47 0,34;7,46 0,52;7,45 1,81;7,45 0,41;7,43 0,45;7,42 1,82;7,42 0,50;7,41 0,35;7,40 0,73;7,08 0,40;7,07 0,32;7,07 0,39;6,89 0,87;4,21 0,50;4,19 0,78;4,17 0,51;3,91 2,62;3,32 17,61;3,11 0,45;3,09 0,74;3,08 0,52;2,64 0,43;2,62 0,59;2,60 0,37;2,51 1,32;2,51 2,34;2,50 2,98;2,50 2,11;1,99 0,80;1,18 0,44;1,07 16,00 |
| Beispiel Nr. 14 [CD ₃CN] 7,78 0,55;7,77 0,57;7,44 0,48;7,43 0,37;7,43 0,65;7,42 0,80;7,41 0,74;7,40 0,38;7,34 0,33;7,33 0,91;7,33 0,96;7,32 1,37;7,31 1,18;6,44 0,86;6,42 0,63;6,42 0,46;6,40 0,55;6,40 0,46;4,18 0,60;4,16 1,06;4,15 0,62;3,77 0,88;3,67 1,75;2,82 0,57;2,80 1,10;2,79 0,62;2,35 1,03;2,17 15,08;2,11 1,47;2,11 1,47;2,10 1,17;2,09 1,08;2,08 0,95;2,08 1,09;2,07 0,80;2,06 0,84;2,05 0,53;1,96 18,43;1,96 4,21;1,95 20,77;1,95 37,93;1,94 52,45;1,93 36,36;1,93 18,80;1,90 0,50;1,89 0,40;1,88 0,69;1,88 0,51;1,87 0,63;1,86 0,45;1,85 0,53;1,77 0,37;1,20 1,20;1,14 16,00;0,01 0,40;0,00 7,38;-0,01 0,33 |
| Beispiel Nr. 15 [CD ₃CN] 8,64 1,88;8,13 7,15;8,13 6,86;8,12 7,11;8,12 7,02;7,99 3,30;7,97 0,38;7,43 0,74;7,42 7,82;7,42 2,89;7,41 8,21;7,41 8,52;7,40 3,04;7,40 8,16;7,39 0,86;7,07 0,87;7,06 8,62;7,06 2,57;7,05 2,79;7,05 16,00;7,04 2,85;7,03 2,47;7,03 7,75;7,03 0,78;6,80 6,93;6,80 6,87;6,79 6,72;6,79 6,75;5,45 1,03;4,17 6,62;4,16 11,98;4,15 6,87;4,08 0,41;4,07 1,17;4,05 1,19;4,04 0,40;3,55 0,33;3,54 0,35;3,53 0,35;3,53 0,34;2,82 6,30;2,81 12,42;2,80 6,75;2,54 0,36;2,20 3758,35;2,10 2,22;2,09 67,52;2,08 4,51;2,08 6,46;2,07 3,66;2,07 4,64;2,06 2,54;2,06 3,55;2,05 3,65;2,05 2,41;2,05 1,23;1,98 0,64;1,97 6,08;1,97 102,59;1,96 15,98;1,95 19,90;1,95 220,52;1,95 409,99;1,94 602,47;1,94 409,64;1,93 205,63;1,93 6,76;1,93 3,04;1,89 2,11;1,88 1,35;1,87 5,38;1,87 3,28;1,86 5,16;1,86 3,13;1,86 4,74;1,85 1,26;1,84 1,73;1,84 1,28;1,83 2,32;1,83 3,36;1,82 2,31;1,82 1,21;1,27 1,52;1,22 1,53;1,20 3,03;1,19 1,51;1,13 1,41;1,11 2,77;1,10 1,45;0,91 2,34;0,00 5,81 |
| Beispiel Nr. 16 [CD ₃CN] 8,09 0,54;8,09 0,59;7,72 0,69;7,43 0,64;7,42 0,73;7,41 0,79;7,41 0,35;7,40 0,76;7,06 0,69;7,05 1,37;7,03 0,70;6,75 0,50;6,74 0,53;6,74 0,54;6,74 0,56;4,17 0,66;4,16 1,21;4,15 0,69;2,82 0,68;2,81 1,17;2,80 0,63;2,25 0,43;2,24 0,60;2,21 281,02;2,09 0,60;2,08 0,67;2,08 0,44;2,07 0,51;2,05 0,35;1,97 3,93;1,96 1,61;1,96 2,05;1,95 19,68;1,95 36,83;1,94 52,56;1,94 34,71;1,93 17,69;1,88 0,55;1,88 0,40;1,87 0,58;1,86 0,48;1,83 0,32;1,45 16,00;1,31 0,38;1,27 1,83;0,93 0,34;0,88 0,37 |
| Beispiel Nr. 17 [DMSO-D₆] 8,49 7,10;8,49 4,64;8,48 4,71;8,47 7,19;7,83 0,32;7,75 4,48;7,73 6,06;7,63 5,84;7,61 4,30;7,19 7,71;7,18 5,00;7,18 4,93;7,17 7,46;5,75 0,36;4,23 2,98;4,21 4,57;4,19 3,07;4,06 1,23;4,04 3,65;4,02 3,68;4,00 1,25;3,78 0,61;3,31 319,54;3,29 2,38;3,08 2,68;3,06 4,34;3,05 3,16;2,67 0,37;2,67 0,54;2,66 1,06;2,64 2,53;2,63 3,49;2,61 2,21;2,59 0,83;2,54 0,88;2,52 1,90;2,51 21,87;2,51 39,93;2,50 51,66;2,50 36,22;2,49 17,70;2,33 0,36;1,99 16,00;1,91 1,60;1,19 4,47;1,18 8,89;1,16 4,40;1,07 1,32;0,00 0,54 |
| Beispiel Nr. 18 [DMSO-D₆] 9,57 1,03;8,09 0,83;8,08 0,89;7,69 1,06;7,43 0,76;7,43 0,38;7,42 0,90;7,41 0,98;7,40 0,44;7,40 0,86;7,22 0,91;7,21 0,34;7,19 1,56;7,18 0,34;7,17 0,74;6,74 0,72;6,73 0,69;6,72 0,70;6,72 0,67;4,19 0,60;4,17 0,93;4,15 0,61;3,30 299,11;3,28 3,24;3,05 0,53;3,03 0,86;3,01 0,59;2,67 0,38;2,67 0,50;2,66 0,42;2,66 0,38;2,64 0,58;2,62 0,75;2,60 0,55;2,59 0,33;2,54 1,28;2,51 26,66;2,50 47,67;2,50 60,62;2,50 41,83;2,49 20,06;2,33 0,38;2,07 1,33;1,47 1,00;1,42 16,00;1,31 0,45;1,21 0,44;1,20 0,45;1,18 1,06;1,16 1,02;0,00 3,28 |
| Beispiel Nr. 19 [CD ₃CN] 8,63 1,93;8,12 7,24;8,12 7,35;8,11 7,63;8,11 7,35;8,00 3,39;7,42 0,50;7,41 0,52;7,41 2,30;7,41 6,15;7,41 3,47;7,40 3,65;7,40 8,80;7,40 5,33;7,40 8,74;7,39 9,03;7,39 0,97;7,39 0,95;7,39 1,00;7,38 0,52;7,32 0,44;7,32 0,37;7,31 1,39;7,31 3,34;7,31 2,63;7,30 11,84;7,30 9,56;7,30 6,66;7,30 14,41;7,29 16,00;7,29 1,52;6,79 6,84;6,79 6,87;6,78 7,01;6,78 6,88;4,18 6,83;4,17 12,43;4,16 7,13;4,08 0,97;4,06 2,83;4,05 2,84;4,04 0,95;2,82 6,50;2,81 12,83;2,80 7,00;2,29 0,40;2,28 0,49;2,27 0,45;2,26 0,47;2,21 1,58;2,19 3,13;2,16 5,28;2,11 2,10;2,10 2,67;2,09 2,30;2,08 69,67;2,08 8,12;2,07 4,29;2,07 5,19;2,06 1,89;2,06 2,63;2,06 1,23;2,05 1,23;2,05 0,90;2,04 0,60;2,01 0,43;1,99 0,44;1,99 0,40;1,98 0,35;1,98 0,68;1,97 12,82;1,97 8,69;1,96 8,56;1,96 4,73;1,95 5,10;1,95 52,78;1,95 101,61;1,94 147,51;1,94 96,93;1,93 48,66;1,92 0,88;1,89 2,29;1,88 1,51;1,88 5,68;1,87 3,50;1,87 5,52;1,86 3,36;1,86 4,94;1,85 1,40;1,85 1,80;1,83 0,40;1,83 0,65;1,83 0,94;1,82 0,68;1,82 0,38;1,27 1,25;1,22 3,81;1,20 7,66;1,19 3,62;1,16 0,44;1,15 0,51;1,13 1,08;1,11 0,56;1,10 0,36;1,10 0,34;1,06 0,50;0,92 0,32;0,88 0,36;0,01 1,35;0,00 47,40;-0,01 1,39 |
| Beispiel Nr. 20 [CD ₃CN] 8,86 2,00;8,13 6,39;8,13 6,65;8,12 6,76;8,12 6,49;8,00 5,69;7,43 0,71;7,42 7,59;7,42 2,70;7,41 7,94;7,41 8,15;7,40 2,80;7,40 7,87;7,39 0,80;7,06 0,87;7,05 8,49;7,05 2,45;7,04 2,65;7,04 16,00;7,04 2,63;7,03 2,38;7,02 7,62;7,02 0,74;6,77 6,95;6,77 6,87;6,77 6,64;6,76 6,59;5,45 1,47;4,16 5,76;4,15 10,28;4,14 5,91;2,80 5,43;2,79 10,67;2,78 5,81;2,16 685,93;2,09 1,70;2,09 1,24;2,08 3,34;2,08 3,58;2,08 3,08;2,07 5,24;2,06 3,12;2,06 4,74;2,06 2,59;2,05 3,17;2,05 2,05;2,05 2,21;2,04 1,00;1,97 2,65;1,97 353,50;1,96 13,17;1,95 15,55;1,95 169,29;1,95 311,80;1,94 474,83;1,94 324,65;1,93 157,51;1,93 4,26;1,92 1,93;1,92 0,44;1,88 1,84;1,87 1,18;1,87 4,60;1,86 2,85;1,86 4,35;1,85 2,83;1,85 3,48;1,85 4,10;1,84 1,10;1,84 1,49;1,84 1,32;1,83 1,87;1,83 2,56;1,82 1,81;1,82 0,95;1,80 0,92;1,79 1,94;1,78 2,07;1,78 3,38;1,77 1,37;1,77 2,17;1,76 2,12;1,76 1,05;1,45 0,33;1,28 0,42;1,27 1,43;1,13 1,16;1,11 2,38;1,10 1,20;0,91 1,52;0,88 0,36;0,88 2,12;0,87 5,56;0,86 8,14;0,86 5,13;0,86 4,88;0,86 7,78;0,85 3,11;0,85 1,58;0,84 4,26;0,83 7,98;0,83 4,80;0,82 3,83;0,82 8,93;0,82 4,13;0,81 1,41;0,81 1,57;0,01 1,27;0,00 46,54;-0,01 1,28 |
| Beispiel Nr. 21 [CD ₃CN] 8,87 2,15;8,12 6,63;8,12 6,93;8,11 6,97;8,11 6,79;8,01 6,02;7,41 0,53;7,41 0,60;7,41 2,20;7,40 5,95;7,40 3,38;7,40 3,53;7,40 8,34;7,40 5,18;7,39 8,48;7,39 8,64;7,39 0,96;7,38 1,02;7,38 0,49;7,32 0,46;7,32 0,41;7,31 1,52;7,31 3,04;7,31 2,73;7,30 10,96;7,30 9,17;7,30 6,52;7,30 13,60;7,29 16,00;7,28 1,55;6,77 6,75;6,77 6,72;6,76 6,84;6,76 6,62;5,45 2,85;4,17 6,19;4,16 11,15;4,15 6,46;3,54 0,41;3,53 0,41;2,81 5,81;2,80 11,47;2,79 6,30;2,17 121,73;2,09 2,36;2,09 1,92;2,08 4,14;2,08 3,88;2,08 3,85;2,07 6,16;2,07 3,86;2,06 4,57;2,06 2,02;2,06 1,84;2,06 2,37;2,05 2,49;2,05 2,80;2,05 1,73;2,04 0,95;1,97 3,34;1,97 257,13;1,96 11,13;1,95 15,26;1,95 129,36;1,95 246,94;1,94 355,17;1,94 233,11;1,93 114,36;1,93 4,21;1,92 2,10;1,88 2,11;1,88 1,49;1,87 5,18;1,87 3,30;1,86 4,96;1,86 3,17;1,85 5,85;1,85 1,34;1,84 1,70;1,84 0,95;1,83 1,50;1,83 2,13;1,82 1,49;1,82 0,80;1,80 1,09;1,79 2,23;1,78 2,36;1,78 3,76;1,77 1,70;1,77 2,45;1,76 2,37;1,75 1,22;1,51 0,57;1,50 1,09;1,49 1,16;1,49 0,68;1,49 1,86;1,48 0,73;1,48 1,19;1,47 1,17;1,47 0,61;1,27 1,12;1,22 0,47;1,20 0,90;1,19 0,45;1,13 0,60;1,13 1,11;1,11 2,15;1,10 1,04;0,91 1,01;0,88 0,34;0,87 2,22;0,87 6,00;0,86 8,75;0,86 5,69;0,86 5,37;0,85 8,51;0,85 3,90;0,84 1,78;0,84 1,22;0,84 1,23;0,84 4,36;0,83 8,44;0,83 5,12;0,82 4,24;0,82 9,09;0,81 4,27;0,81 1,54;0,80 1,65;0,75 0,81;0,74 0,85;0,74 2,83;0,74 4,26;0,73 2,38;0,73 2,14;0,73 3,86;0,72 1,08;0,72 1,51;0,71 0,64;0,71 0,84;0,70 1,65;0,70 4,33;0,69 2,37;0,69 1,80;0,69 1,23;0,68 4,04;0,68 2,05;0,68 1,01;0,67 0,82;0,01 0,72;0,00 21,79;-0,01 0,67 |
| Beispiel Nr. 22 [CD ₃CN] 8,73 0,33;8,17 1,22;8,16 1,30;8,16 1,32;8,16 1,32;8,02 1,10;8,02 1,12;7,43 1,48;7,42 0,51;7,42 1,57;7,42 0,60;7,41 0,60;7,41 1,56;7,41 0,52;7,40 1,52;7,06 1,55;7,06 0,46;7,05 0,50;7,05 3,02;7,04 0,49;7,04 0,46;7,03 1,50;6,85 1,40;6,85 1,36;6,84 1,30;6,84 1,35;5,45 4,31;4,17 1,04;4,16 1,88;4,15 1,09;3,97 7,87;3,45 16,00;2,84 0,99;2,82 1,95;2,81 1,07;2,17 25,36;2,09 0,59;2,08 0,54;2,08 0,95;2,07 0,55;2,07 0,69;2,06 0,36;1,97 1,99;1,96 0,47;1,95 0,57;1,95 6,22;1,95 11,65;1,94 18,33;1,94 12,52;1,93 5,86;1,89 0,33;1,88 0,85;1,87 0,52;1,87 0,79;1,86 0,50;1,86 0,75;0,00 0,88 |
| Beispiel Nr. 23 [CD ₃CN] 8,72 0,43;8,15 1,44;8,15 1,40;8,15 1,45;8,14 1,45;8,03 1,48;7,41 1,13;7,41 0,66;7,41 0,68;7,41 1,69;7,40 1,06;7,40 1,65;7,40 1,70;7,31 0,62;7,31 2,30;7,31 1,84;7,31 1,28;7,30 2,79;7,30 3,09;6,84 1,33;6,84 1,34;6,84 1,32;6,83 1,31;4,18 1,34;4,17 2,44;4,16 1,40;3,97 8,57;3,45 16,00;3,36 0,42;2,84 1,27;2,83 2,53;2,82 1,37;2,16 56,22;2,10 0,38;2,09 0,79;2,09 0,71;2,08 1,27;2,08 0,75;2,07 0,93;2,06 0,45;1,97 0,63;1,97 10,22;1,96 1,18;1,95 1,42;1,95 14,78;1,95 28,14;1,94 41,34;1,94 27,46;1,93 13,53;1,89 0,43;1,88 1,10;1,88 0,68;1,87 1,07;1,87 0,65;1,86 0,97;1,85 0,35;0,00 3,68 |
| Beispiel Nr. 24 [CD ₃CN] 9,00 0,94;8,16 3,12;8,16 3,14;8,15 3,18;8,15 3,20;8,04 2,84;7,43 0,37;7,43 3,55;7,43 1,35;7,42 3,77;7,41 3,86;7,41 1,38;7,41 3,68;7,40 0,40;7,07 0,40;7,06 3,90;7,06 1,15;7,05 1,26;7,05 7,37;7,05 1,26;7,04 1,12;7,03 3,56;7,03 0,36;6,83 3,22;6,83 3,18;6,82 3,13;6,82 3,14;4,25 0,42;4,24 1,26;4,23 1,29;4,22 0,44;4,17 2,68;4,16 4,74;4,15 2,84;4,06 0,61;4,05 0,63;4,05 0,63;4,00 0,39;3,78 1,62;3,49 1,12;3,36 2,74;2,84 2,14;2,83 4,25;2,82 2,45;2,17 153,67;2,10 0,87;2,10 0,63;2,09 1,70;2,09 1,70;2,08 2,87;2,08 1,66;2,07 1,97;2,07 0,66;2,06 1,06;2,06 0,71;2,05 0,93;2,05 0,66;2,04 0,34;1,97 2,84;1,97 0,47;1,97 53,39;1,96 4,42;1,95 4,98;1,95 56,63;1,95 105,31;1,94 154,76;1,94 105,89;1,93 52,70;1,93 1,80;1,92 0,82;1,92 0,35;1,89 0,90;1,89 0,60;1,88 2,23;1,88 1,39;1,87 2,22;1,87 1,38;1,86 1,73;1,86 0,61;1,85 0,89;1,84 0,37;1,83 0,64;1,83 0,92;1,82 0,63;1,82 0,34;1,37 16,00;1,36 15,93;1,27 0,47;1,22 0,86;1,20 1,58;1,19 0,80;1,13 0,53;1,11 1,07;1,10 0,55;0,91 0,55;0,01 0,36;0,00 14,03;-0,01 0,42 |
| Beispiel Nr. 25 [CD ₃CN] 9,00 1,02;8,15 2,86;8,14 2,91;8,06 3,11;7,42 0,97;7,41 2,70;7,41 1,61;7,41 1,66;7,41 3,85;7,41 2,52;7,40 4,01;7,40 4,04;7,39 0,54;7,32 0,65;7,32 1,39;7,31 1,22;7,31 5,11;7,31 4,58;7,31 3,15;7,30 6,65;7,30 7,97;7,29 0,81;6,82 3,13;6,82 3,20;6,82 3,16;6,81 3,14;5,45 1,47;4,25 0,42;4,24 1,27;4,23 1,29;4,22 0,45;4,18 2,92;4,17 5,19;4,16 3,06;4,00 0,54;2,84 2,47;2,83 4,88;2,82 2,77;2,16 349,12;2,11 0,94;2,10 0,72;2,10 1,86;2,09 1,75;2,09 3,12;2,08 1,80;2,08 2,52;2,07 0,75;2,07 1,05;2,06 0,48;2,06 0,75;2,05 1,10;2,05 0,76;2,04 0,39;1,97 1,32;1,97 135,15;1,96 5,99;1,95 6,98;1,95 67,53;1,95 129,20;1,94 187,18;1,94 124,02;1,93 62,30;1,92 1,31;1,90 1,06;1,89 0,73;1,89 2,51;1,88 1,57;1,88 2,50;1,87 1,53;1,87 1,97;1,85 0,68;1,85 0,91;1,84 0,43;1,83 0,75;1,83 1,09;1,82 0,78;1,82 0,41;1,37 15,89;1,36 16,00;1,27 0,40;1,13 0,47;1,11 0,90;1,10 0,43;0,91 0,61;0,01 0,56;0,00 17,88;-0,01 0,57 |
| Beispiel Nr. 26 [CD ₃CN] 8,13 0,49;8,11 1,39;8,11 1,42;8,10 1,39;8,10 1,37;7,76 1,50;7,43 1,49;7,43 0,59;7,42 1,60;7,42 1,66;7,41 0,60;7,41 1,58;7,07 1,71;7,06 0,56;7,05 0,55;7,05 3,17;7,05 0,59;7,04 0,46;7,04 1,51;6,77 1,33;6,77 1,33;6,76 1,34;6,76 1,33;5,45 0,72;4,17 1,26;4,16 2,30;4,15 1,32;3,69 16,00;2,83 1,22;2,82 2,38;2,81 1,28;2,16 220,97;2,10 0,39;2,09 0,76;2,09 0,71;2,08 1,21;2,08 0,72;2,07 0,88;2,06 0,49;2,06 0,41;2,05 0,59;2,05 0,40;1,97 0,39;1,97 12,90;1,96 2,70;1,95 3,15;1,95 36,26;1,95 69,14;1,94 99,80;1,94 65,90;1,93 33,48;1,93 1,12;1,92 0,51;1,89 0,43;1,88 1,06;1,88 0,68;1,87 1,03;1,87 0,64;1,86 0,93;1,85 0,38;1,83 0,40;1,83 0,59;1,82 0,40;1,27 0,37;1,13 0,33;1,11 0,62;0,00 8,16 |
| Beispiel Nr. 27 [CD ₃CN] 8,10 1,34;8,09 1,28;8,09 1,34;8,09 1,32;8,06 0,39;7,78 1,16;7,77 1,33;7,77 1,32;7,42 1,30;7,41 0,75;7,41 0,73;7,41 1,36;7,41 1,00;7,40 1,68;7,40 1,69;7,32 0,45;7,32 0,77;7,32 0,66;7,31 1,97;7,31 1,93;7,31 1,30;7,30 2,71;7,30 3,57;7,30 0,40;6,76 1,32;6,76 1,30;6,75 1,33;6,75 1,31;5,45 0,51;4,18 1,20;4,17 2,12;4,16 1,24;3,77 0,32;3,72 0,68;3,68 16,00;3,68 0,42;3,55 0,77;2,83 1,16;2,82 2,19;2,81 1,22;2,19 0,59;2,16 494,63;2,13 0,37;2,11 0,43;2,10 0,32;2,10 0,75;2,09 0,71;2,09 1,19;2,08 0,71;2,08 2,88;2,07 0,34;2,07 0,44;2,06 0,64;2,06 1,10;2,05 1,56;2,05 1,11;2,04 0,55;1,97 2,91;1,97 395,06;1,96 8,76;1,95 9,24;1,95 102,32;1,95 189,07;1,94 284,13;1,94 194,04;1,93 96,18;1,93 3,13;1,92 1,46;1,92 0,61;1,92 0,40;1,92 0,35;1,89 0,51;1,89 0,39;1,88 1,07;1,88 0,71;1,87 1,04;1,87 0,69;1,86 0,95;1,86 0,34;1,85 0,41;1,85 2,24;1,84 0,40;1,84 0,62;1,83 1,13;1,83 1,60;1,82 1,12;1,82 0,60;1,29 0,34;1,27 1,09;1,13 0,73;1,11 1,45;1,10 0,74;0,91 1,06;0,01 0,68;0,00 27,09;-0,01 0,83 |
| Beispiel Nr. 28 [CD ₃CN] 8,53 0,63;8,13 1,51;8,12 1,58;8,11 1,62;8,11 1,70;8,04 1,90;7,43 1,65;7,43 0,78;7,42 1,88;7,41 2,03;7,40 0,86;7,40 1,89;7,07 1,88;7,06 0,69;7,05 0,85;7,04 3,43;7,04 0,81;7,03 0,66;7,02 1,63;6,78 1,47;6,77 1,51;6,77 1,48;6,76 1,43;4,17 1,63;4,16 2,91;4,14 1,65;2,83 1,54;2,82 3,05;2,80 1,72;2,66 0,38;2,64 0,96;2,63 1,31;2,61 1,01;2,59 0,42;2,15 400,60;2,12 1,80;2,11 1,81;2,11 1,68;2,10 1,58;2,09 1,71;2,09 1,53;2,08 2,02;2,07 1,43;2,07 1,54;2,06 0,76;2,05 0,86;2,03 0,35;1,97 2,43;1,96 24,07;1,96 7,56;1,95 38,73;1,95 71,16;1,94 98,81;1,93 68,31;1,93 35,30;1,90 1,33;1,90 1,06;1,89 1,84;1,88 1,36;1,87 1,73;1,86 1,22;1,86 1,37;1,84 0,60;1,78 0,34;1,77 0,52;1,77 0,68;1,76 0,49;1,45 1,04;1,27 0,65;1,20 0,45;1,15 16,00;1,13 15,73;1,11 0,60;1,07 1,04;1,05 1,01;0,01 2,02;0,00 37,39;-0,01 1,74 |
| Beispiel Nr. 29 [CD ₃CN] 8,53 0,63;8,11 1,70;8,10 1,67;8,10 1,70;8,05 2,05;7,42 1,35;7,41 1,00;7,41 1,06;7,41 2,01;7,40 1,44;7,40 2,05;7,39 2,11;7,39 0,47;7,31 1,12;7,31 3,10;7,30 2,08;7,30 3,46;7,29 3,34;7,28 0,41;6,77 1,55;6,77 1,54;6,76 1,54;6,75 1,47;4,18 1,72;4,17 3,08;4,15 1,75;2,84 1,66;2,82 3,20;2,80 1,76;2,66 0,43;2,64 1,02;2,63 1,37;2,61 1,07;2,59 0,46;2,15 356,74;2,12 1,56;2,11 1,60;2,11 1,60;2,11 1,61;2,10 1,93;2,09 1,50;2,09 1,99;2,08 1,34;2,07 1,54;2,06 0,70;2,06 0,85;1,97 2,60;1,96 18,27;1,96 7,76;1,95 37,89;1,95 68,94;1,94 95,23;1,93 65,84;1,93 33,89;1,91 1,17;1,90 0,86;1,89 1,71;1,88 1,20;1,88 1,57;1,87 1,04;1,86 1,23;1,85 0,46;1,85 0,46;1,77 0,39;1,77 0,54;1,76 0,39;1,27 0,37;1,21 1,01;1,19 0,93;1,15 16,00;1,13 15,71;1,12 1,88;1,11 0,55;1,11 0,95;0,01 2,16;0,00 36,54;-0,01 1,73 |
| Beispiel Nr. 30 [CD ₃CN] 8,54 0,65;8,13 1,64;8,12 1,66;8,01 1,64;7,43 1,85;7,42 1,94;7,41 2,01;7,40 1,02;7,39 1,86;7,06 1,85;7,04 3,52;7,02 1,57;6,80 1,34;6,80 1,59;6,79 1,44;6,79 1,11;6,04 0,33;6,03 0,38;6,02 0,35;6,01 0,35;5,99 0,40;5,52 0,37;5,48 0,40;4,17 1,85;4,16 3,14;4,14 1,63;4,07 0,65;4,05 0,57;3,57 0,38;3,55 0,61;3,54 0,75;3,54 0,66;3,52 0,60;3,50 0,33;3,27 0,33;2,96 0,48;2,84 1,86;2,82 3,29;2,81 1,88;2,71 0,36;2,66 0,35;2,61 0,37;2,57 0,36;2,49 0,59;2,46 1,44;2,39 0,87;2,37 0,88;2,35 0,93;2,34 1,04;2,32 1,12;2,30 1,25;2,28 1,49;2,24 5,60;2,22 7,93;2,15 3170,97;2,12 12,27;2,11 11,19;2,11 10,36;2,10 7,82;2,09 6,11;2,09 4,76;2,08 4,49;2,07 3,79;2,05 2,81;2,00 8,49;1,99 11,64;1,99 10,01;1,96 131,70;1,96 60,56;1,95 308,30;1,95 561,87;1,94 776,89;1,93 536,23;1,93 275,81;1,89 3,51;1,88 2,84;1,87 2,84;1,86 2,03;1,80 0,73;1,79 1,05;1,78 1,92;1,77 3,39;1,77 4,67;1,76 3,38;1,76 1,63;1,65 0,33;1,45 1,22;1,44 0,42;1,27 2,35;1,22 0,70;1,20 1,46;1,19 0,66;1,13 1,58;1,11 2,80;1,10 1,35;1,07 5,03;1,05 4,94;0,96 15,92;0,94 16,00;0,93 15,83;0,92 7,02;0,92 6,89;0,91 15,68;0,89 0,92;0,15 1,21;0,01 15,64;0,00 294,13;-0,01 14,71;-0,15 1,27 |
| Beispiel Nr. 31 [CD ₃CN] 8,54 0,64;8,12 1,65;8,10 1,68;8,03 1,86;7,41 1,29;7,41 0,98;7,41 1,02;7,41 1,94;7,40 1,45;7,40 2,10;7,39 2,11;7,30 2,92;7,30 2,65;7,30 2,07;7,29 3,54;7,29 3,52;6,79 1,51;6,79 1,53;6,78 1,50;6,77 1,47;4,18 1,68;4,17 3,05;4,15 1,72;2,84 1,62;2,82 3,12;2,81 1,70;2,24 3,77;2,23 5,35;2,15 417,05;2,11 2,57;2,11 2,84;2,10 2,55;2,09 2,32;2,09 2,60;2,07 2,30;2,05 1,29;2,04 0,53;2,02 0,48;1,97 2,43;1,96 8,32;1,96 8,85;1,95 47,32;1,95 86,75;1,94 120,02;1,93 82,95;1,93 42,90;1,90 1,60;1,89 2,01;1,88 1,48;1,87 1,82;1,87 1,30;1,86 1,44;1,84 0,67;1,78 0,42;1,77 0,63;1,77 0,82;1,76 0,61;1,76 0,36;1,27 0,46;1,20 0,55;1,11 0,47;1,00 0,76;0,99 0,81;0,96 16,00;0,95 3,04;0,94 15,57;0,93 1,49;0,91 0,52;0,01 2,62;0,00 45,26;-0,01 2,08 |
| Beispiel Nr. 32 [CD ₃CN] 9,03 3,06;8,21 7,74;8,20 7,84;8,18 9,33;7,94 8,49;7,94 10,32;7,93 2,84;7,93 11,48;7,93 9,55;7,83 1,14;7,83 8,01;7,82 10,18;7,82 2,89;7,81 11,51;7,81 9,74;7,62 1,27;7,62 2,47;7,62 1,54;7,61 1,72;7,61 6,32;7,59 2,50;7,59 4,51;7,59 2,56;7,56 1,25;7,56 2,48;7,55 1,51;7,55 1,71;7,54 6,27;7,54 2,37;7,53 9,89;7,53 8,22;7,53 3,32;7,52 12,14;7,51 2,13;7,51 5,56;7,48 8,32;7,47 3,78;7,46 13,24;7,46 3,71;7,45 10,30;7,45 9,09;7,44 3,17;7,44 8,17;7,43 0,92;7,08 0,86;7,08 8,35;7,08 2,69;7,07 2,92;7,06 16,00;7,06 3,21;7,05 2,49;7,05 7,88;7,04 0,98;6,87 6,69;6,86 6,94;6,86 6,62;6,86 6,82;6,74 0,50;5,98 0,45;4,19 6,53;4,18 11,96;4,17 6,82;4,08 0,72;4,06 2,14;4,05 2,13;4,04 0,71;3,61 0,36;3,54 0,44;3,53 0,44;2,88 6,17;2,87 12,30;2,86 6,61;2,17 354,13;2,12 3,04;2,11 2,50;2,11 4,76;2,10 4,39;2,10 6,77;2,09 4,24;2,09 5,06;2,08 1,99;2,08 2,60;2,06 1,38;2,06 2,22;2,05 3,14;2,05 2,17;2,04 1,24;1,97 9,88;1,97 12,68;1,96 14,29;1,95 15,61;1,95 183,09;1,95 345,53;1,94 504,89;1,94 352,39;1,93 178,21;1,92 2,73;1,91 2,30;1,91 1,62;1,90 5,34;1,90 3,49;1,89 5,41;1,89 3,41;1,88 4,67;1,87 1,70;1,84 1,05;1,83 1,97;1,83 2,89;1,82 1,96;1,82 1,01;1,45 2,51;1,44 0,42;1,27 1,82;1,22 2,61;1,20 5,23;1,19 2,51;1,13 0,35;1,13 1,99;1,11 3,88;1,10 1,90;0,91 1,88;0,88 0,33;0,10 0,60;0,01 4,27;0,00 149,10;-0,01 5,07;-0,10 0,60 |
| Beispiel Nr. 33 [CD ₃CN] 9,03 2,43;8,74 0,34;8,19 16,00;8,19 7,83;8,19 6,92;8,02 0,41;8,01 0,96;8,00 0,71;8,00 0,64;7,94 8,46;7,93 2,60;7,93 9,41;7,93 7,71;7,83 4,74;7,82 5,99;7,82 1,85;7,81 6,90;7,81 5,69;7,62 1,32;7,62 2,14;7,61 1,33;7,60 5,26;7,60 1,96;7,59 2,31;7,59 3,80;7,59 2,12;7,57 0,36;7,56 0,86;7,55 1,68;7,55 1,32;7,55 1,11;7,54 3,72;7,54 1,57;7,53 8,21;7,53 4,41;7,52 9,79;7,51 1,87;7,51 4,48;7,50 0,77;7,49 0,55;7,48 0,92;7,48 1,21;7,48 4,93;7,47 2,29;7,46 6,93;7,45 1,53;7,45 3,60;7,45 3,15;7,45 2,13;7,45 6,32;7,44 4,79;7,44 3,57;7,44 3,59;7,44 3,41;7,43 8,45;7,43 7,43;7,42 0,96;7,42 0,35;7,34 1,02;7,33 0,80;7,33 2,39;7,33 2,06;7,32 7,53;7,32 4,97;7,32 5,02;7,31 12,81;7,31 13,01;7,31 2,75;7,30 1,48;7,29 0,44;7,29 0,42;6,86 5,49;6,86 4,25;6,85 4,34;6,85 5,58;4,20 5,27;4,19 9,71;4,18 5,53;4,06 0,65;4,05 0,63;3,54 0,81;3,53 0,81;2,89 5,00;2,88 9,93;2,87 5,40;2,17 62,37;2,12 3,51;2,12 2,94;2,11 4,68;2,11 4,26;2,10 6,14;2,10 4,02;2,09 4,61;2,09 2,06;2,08 2,39;2,06 1,00;2,06 1,49;2,05 1,92;2,05 1,41;2,04 0,86;1,97 3,10;1,97 7,03;1,96 7,15;1,95 9,61;1,95 97,58;1,95 187,06;1,94 266,43;1,94 180,73;1,93 95,01;1,92 2,43;1,91 1,80;1,91 4,72;1,90 3,22;1,89 4,75;1,89 3,08;1,89 4,00;1,88 1,56;1,83 0,65;1,83 1,14;1,83 1,65;1,82 1,18;1,82 0,65;1,27 0,65;1,22 0,82;1,20 1,62;1,19 0,82;1,13 1,63;1,11 3,31;1,10 1,67;0,91 1,01;0,01 2,68;0,00 80,69;-0,01 3,13 |
| Beispiel Nr. 34 [CD ₃CN] 8,69 1,30;8,17 0,45;8,16 0,47;8,15 0,48;8,15 0,47;8,12 3,61;8,12 3,71;8,11 3,65;8,11 3,76;7,98 3,81;7,48 0,41;7,47 0,51;7,46 0,66;7,45 0,64;7,42 0,61;7,41 4,11;7,41 1,96;7,40 4,66;7,40 3,21;7,39 2,80;7,39 5,25;7,38 2,22;7,38 5,01;7,37 2,16;7,37 1,89;7,37 1,06;7,36 2,20;7,35 4,50;7,35 4,42;7,34 1,60;7,33 11,56;7,33 11,26;7,33 9,87;7,32 1,61;7,32 1,78;7,31 3,19;7,30 1,82;7,30 1,64;7,29 1,43;7,28 2,98;7,28 2,70;7,27 1,21;7,27 1,48;7,26 0,68;7,25 0,32;7,06 0,65;7,05 4,63;7,05 1,95;7,04 0,67;7,03 1,84;7,03 8,62;7,02 2,52;7,01 1,51;7,01 4,20;7,00 0,92;7,00 0,60;6,83 0,47;6,83 0,48;6,82 0,48;6,82 0,48;6,79 3,70;6,79 3,70;6,78 3,61;6,77 3,58;5,45 2,16;5,16 0,51;4,16 3,88;4,14 6,94;4,13 3,95;3,70 16,00;3,61 3,16;2,81 3,48;2,79 6,94;2,78 3,76;2,29 0,35;2,27 0,47;2,16 45,32;2,11 2,47;2,11 2,20;2,10 1,79;2,09 1,57;2,09 2,03;2,08 1,79;2,07 2,94;2,07 2,82;2,06 4,09;2,05 2,72;2,04 3,13;2,04 1,35;2,03 1,66;1,99 0,51;1,97 1,52;1,97 2,82;1,96 74,42;1,96 5,26;1,95 30,94;1,95 58,82;1,94 84,49;1,93 58,28;1,93 29,86;1,88 1,56;1,87 1,23;1,86 3,34;1,85 2,36;1,85 3,27;1,84 2,21;1,83 2,67;1,82 1,00;1,82 1,06;1,79 0,60;1,78 0,37;1,77 0,50;1,77 0,63;1,76 0,57;1,45 0,87;1,28 0,44;1,27 1,35;1,22 0,43;1,20 0,73;1,19 0,40;1,14 0,33;0,91 0,48;0,01 1,02;0,00 26,47;-0,01 1,01 |
| Beispiel Nr. 35 [CD ₃CN] 8,84 0,42;8,66 1,38;8,46 0,84;8,36 0,79;8,34 0,73;8,15 0,57;8,14 0,56;8,11 3,83;8,10 3,74;8,10 3,70;7,99 3,95;7,81 0,42;7,57 0,32;7,54 0,37;7,52 0,39;7,50 0,50;7,48 1,10;7,46 1,16;7,43 0,77;7,40 3,91;7,39 4,30;7,39 4,34;7,38 4,93;7,38 5,93;7,38 4,56;7,37 6,73;7,37 6,83;7,36 4,22;7,36 5,13;7,35 6,55;7,35 6,07;7,34 12,99;7,33 12,57;7,32 3,25;7,31 7,48;7,30 4,40;7,30 6,31;7,30 11,28;7,29 8,22;7,29 12,07;7,28 14,06;7,28 14,65;7,27 5,41;7,25 1,54;7,22 0,83;7,21 1,27;7,20 1,00;7,19 1,02;7,17 0,67;7,16 0,86;7,15 0,77;7,14 1,19;7,12 0,87;7,11 0,62;7,09 0,59;7,09 0,65;7,07 0,59;7,06 0,40;7,02 0,70;7,00 0,50;6,83 0,60;6,82 0,52;6,81 0,58;6,81 0,65;6,78 3,48;6,78 3,43;6,77 3,36;6,77 3,46;5,45 3,76;5,41 0,41;5,16 0,41;4,41 0,49;4,20 0,39;4,19 0,42;4,17 4,24;4,15 7,40;4,14 4,79;4,12 0,58;4,11 0,41;4,08 0,44;4,07 0,67;4,05 0,84;3,80 0,36;3,78 0,40;3,76 3,63;3,70 16,00;3,62 1,09;3,61 2,52;3,60 14,42;3,53 0,40;3,52 0,35;3,52 0,41;3,50 0,37;3,49 0,40;3,48 0,35;3,35 0,76;2,81 3,72;2,80 7,21;2,78 3,82;2,47 0,43;2,47 0,57;2,46 0,44;2,36 0,47;2,34 0,79;2,33 0,72;2,31 0,64;2,30 0,86;2,29 0,93;2,28 1,02;2,15 1291,20;2,11 14,42;2,11 13,14;2,10 10,32;2,09 8,15;2,08 6,61;2,07 5,87;2,07 6,83;2,06 4,98;2,05 5,34;2,04 3,33;2,01 2,18;1,97 10,97;1,96 534,49;1,96 47,42;1,95 285,56;1,95 539,00;1,94 772,69;1,93 533,69;1,93 274,84;1,88 4,03;1,87 5,27;1,86 4,07;1,85 4,82;1,85 3,54;1,84 3,89;1,82 2,12;1,80 1,32;1,79 4,02;1,78 2,61;1,77 4,01;1,77 5,27;1,76 4,18;1,76 2,29;1,75 1,25;1,73 1,07;1,72 1,11;1,69 1,12;1,66 0,84;1,62 0,60;1,61 0,62;1,60 0,68;1,59 0,65;1,57 0,62;1,55 0,74;1,53 0,77;1,51 0,74;1,50 0,81;1,49 0,70;1,48 0,69;1,46 0,50;1,44 0,64;1,43 0,51;1,41 0,57;1,41 0,60;1,39 1,11;1,38 0,61;1,36 0,71;1,34 1,17;1,32 0,94;1,31 0,93;1,29 2,24;1,27 7,40;1,24 1,06;1,24 0,91;1,23 1,11;1,22 1,56;1,22 1,01;1,20 2,69;1,19 1,34;1,18 0,67;1,17 0,70;1,16 0,56;1,15 0,53;1,14 1,30;1,12 0,54;1,10 0,57;1,09 0,47;1,08 0,79;1,06 0,81;1,04 0,48;1,00 0,34;1,00 0,32;0,99 0,38;0,97 0,53;0,95 0,54;0,93 0,44;0,91 3,89;0,89 0,87;0,88 1,31;0,86 0,75;0,84 0,52;0,82 0,39;0,82 0,33;0,79 0,32;0,57 0,32;0,15 1,03;0,01 9,80;0,00 232,46;-0,01 9,70;-0,05 0,33;-0,06 0,33;-0,15 1,01 |
| Beispiel Nr. 36 [CD ₃CN] 19,94 0,65;9,24 1,88;9,22 1,96;8,68 1,19;8,46 1,03;8,32 3,19;8,16 2,28;8,15 2,62;8,14 2,64;8,13 3,02;8,12 1,13;7,98 3,50;7,43 9,12;7,42 9,99;7,41 10,29;7,40 9,29;7,09 2,01;7,07 4,09;7,06 4,23;7,05 4,33;7,04 3,59;7,04 2,02;6,85 5,66;6,84 5,65;6,80 0,77;6,79 0,75;6,67 3,50;6,38 0,58;4,17 8,76;4,16 16,00;4,15 9,11;4,13 0,68;4,13 0,66;4,06 1,22;4,05 1,40;4,04 0,63;3,55 0,62;3,53 0,60;3,52 0,52;2,83 7,78;2,82 15,96;2,81 9,19;2,80 0,96;2,78 0,63;2,74 0,71;2,47 0,58;2,15 1619,61;2,10 3,30;2,09 6,91;2,09 12,26;2,08 10,47;2,08 6,56;2,07 7,89;2,07 3,38;2,06 6,46;2,06 6,45;2,05 8,27;2,05 5,80;2,04 3,05;1,97 7,23;1,97 84,42;1,96 46,87;1,95 58,29;1,95 539,35;1,95 972,69;1,94 1413,07;1,94 974,09;1,93 494,81;1,92 6,87;1,89 3,43;1,89 2,52;1,88 8,56;1,88 5,52;1,87 8,87;1,87 5,57;1,86 7,71;1,85 2,81;1,85 3,39;1,83 3,27;1,83 5,72;1,83 8,02;1,82 5,44;1,82 2,80;1,27 3,78;1,22 1,70;1,20 3,25;1,19 1,65;1,13 1,55;1,11 3,17;1,10 1,61;0,88 0,77;0,87 0,67;0,10 1,64;0,01 12,44;0,00 406,61;-0,01 12,14;-0,10 1,66 |
| Beispiel Nr. 37 [CD ₃CN] 9,22 1,12;9,20 1,14;8,70 0,82;8,54 2,28;8,48 0,72;8,32 1,93;8,13 3,54;8,11 2,20;8,09 1,91;8,07 0,47;8,05 0,51;7,99 2,00;7,84 0,53;7,60 0,75;7,58 0,82;7,44 2,13;7,43 1,98;7,42 2,99;7,42 8,16;7,41 5,64;7,41 5,88;7,41 10,34;7,40 7,94;7,40 10,61;7,40 8,08;7,39 11,23;7,39 2,55;7,38 1,90;7,31 8,44;7,14 0,75;7,12 0,74;7,08 2,41;7,08 1,00;7,07 1,65;7,06 4,42;7,05 1,31;7,04 1,81;7,04 2,28;7,02 0,60;6,85 5,39;6,84 5,68;6,83 5,47;6,83 5,25;6,79 2,24;6,78 1,75;6,77 1,48;6,76 1,18;6,69 2,22;6,68 0,97;6,67 0,88;5,45 10,12;4,19 8,35;4,17 16,00;4,16 6,70;4,16 10,13;4,15 3,31;3,68 0,88;3,29 1,63;3,17 7,82;3,06 7,96;2,96 8,28;2,89 0,74;2,84 7,25;2,82 14,80;2,81 8,89;2,80 2,28;2,77 0,72;2,67 0,55;2,47 0,40;2,46 0,32;2,32 3,06;2,16 501,04;2,11 9,58;2,11 9,73;2,10 11,02;2,09 9,42;2,08 12,19;2,08 9,37;2,07 9,56;2,06 5,49;2,05 5,26;2,02 1,40;1,96 90,22;1,96 17,48;1,95 147,07;1,95 278,25;1,94 400,28;1,93 276,42;1,93 142,35;1,90 5,44;1,90 4,39;1,89 9,51;1,88 7,12;1,87 9,50;1,87 7,02;1,86 8,19;1,84 3,43;1,79 1,13;1,78 1,40;1,77 2,09;1,77 2,80;1,76 2,03;1,76 1,33;1,72 0,56;1,62 0,39;1,60 0,60;1,56 0,43;1,49 0,45;1,49 0,37;1,39 0,40;1,34 0,52;1,27 4,21;1,22 0,52;1,20 0,79;1,19 0,46;1,16 0,41;1,14 1,01;1,06 1,12;1,04 2,04;1,02 1,06;0,91 2,16;0,90 0,48;0,89 0,45;0,88 0,67;0,86 0,45;0,15 0,50;0,01 5,19;0,00 127,20;-0,01 4,91;-0,02 0,64;-0,15 0,52 |
| Beispiel Nr. 38 [CD ₃CN] 8,43 5,82;8,43 4,66;8,09 3,36;8,09 3,45;8,09 3,00;8,09 2,73;8,08 3,45;8,08 3,42;8,07 2,89;8,07 2,57;7,86 0,53;7,85 0,56;7,45 0,50;7,44 3,29;7,43 2,06;7,42 4,25;7,42 3,09;7,42 4,02;7,42 5,23;7,41 4,19;7,40 5,47;7,39 3,65;7,39 0,61;7,33 0,43;7,33 0,45;7,31 1,56;7,31 4,38;7,30 4,18;7,30 2,88;7,29 6,18;7,29 5,81;7,28 0,64;7,07 0,56;7,06 3,68;7,06 1,20;7,05 1,48;7,04 6,62;7,04 1,30;7,02 1,13;7,02 3,05;7,01 0,33;6,67 2,18;6,66 2,39;6,65 4,25;6,65 4,85;6,64 2,38;6,64 2,95;6,62 3,88;6,62 4,25;6,61 3,66;6,61 3,23;6,61 3,23;6,61 3,31;6,60 2,87;6,36 0,48;6,36 0,54;6,35 0,45;6,35 0,55;6,33 0,78;5,45 2,73;4,78 0,38;4,18 2,76;4,17 4,19;4,16 5,20;4,15 6,54;4,15 3,47;4,14 3,50;4,07 0,51;4,05 0,54;3,06 16,00;3,05 14,00;2,97 13,85;2,97 11,10;2,96 1,38;2,89 0,35;2,84 0,36;2,81 2,43;2,81 3,59;2,80 5,03;2,79 5,97;2,78 3,16;2,77 3,43;2,76 0,73;2,16 496,75;2,13 3,97;2,12 2,23;2,11 2,30;2,11 2,49;2,10 2,59;2,10 2,28;2,09 2,34;2,08 3,59;2,07 4,48;2,06 3,86;2,04 1,81;2,04 1,54;1,99 0,78;1,97 5,39;1,96 104,98;1,96 9,99;1,95 72,03;1,95 135,24;1,94 193,59;1,93 132,82;1,93 67,88;1,89 1,67;1,89 2,27;1,88 3,21;1,87 4,48;1,85 4,09;1,85 3,03;1,84 2,96;1,83 1,25;1,83 1,08;1,79 0,75;1,78 0,56;1,77 0,90;1,77 1,23;1,76 0,89;1,76 0,52;1,27 1,10;1,22 0,72;1,20 1,38;1,19 0,73;0,91 1,02;0,01 2,42;0,00 61,98;-0,01 2,38 |
| Beispiel Nr. 39 [CD ₃CN] 8,43 5,24;8,09 3,23;8,09 3,08;8,08 3,22;8,08 3,19;7,77 0,33;7,76 0,35;7,44 0,38;7,43 3,36;7,43 1,23;7,42 3,69;7,42 1,57;7,42 1,56;7,42 3,76;7,41 1,32;7,41 3,55;7,40 0,41;7,13 0,35;7,11 0,65;7,10 0,34;7,06 0,45;7,06 3,80;7,06 1,1,5;7,05 1,26;7,04 6,98;7,04 1,20;7,03 1,12;7,03 3,43;7,02 0,35;6,65 2,80;6,64 3,20;6,64 2,77;6,63 3,12;6,61 3,28;6,61 3,80;6,60 3,33;6,60 2,96;6,30 0,48;4,16 2,66;4,15 4,67;4,14 2,67;4,09 0,44;3,06 16,00;2,97 15,08;2,96 0,39;2,80 2,45;2,79 4,77;2,78 2,61;2,17 1355,00;2,14 0,68;2,09 0,83;2,08 0,66;2,08 1,57;2,07 1,42;2,07 2,38;2,06 2,22;2,06 3,39;2,05 3,14;2,05 2,28;2,04 1,13;1,97 13,95;1,96 12,79;1,95 15,39;1,95 165,58;1,95 329,59;1,94 489,99;1,94 325,35;1,93 155,14;1,93 4,91;1,92 2,32;1,88 1,00;1,87 0,70;1,87 2,23;1,86 1,48;1,86 2,07;1,85 1,53;1,85 1,99;1,84 0,74;1,84 1,68;1,83 1,96;1,83 2,79;1,82 1,99;1,82 1,02;1,44 5,93;1,27 1,08;1,11 0,55;0,91 1,77;0,01 1,11;0,00 43,04;-0,01 1,34 |
| Beispiel Nr. 40 [DMSO-D₆] 10,36 4,02;8,15 4,48;8,13 4,63;8,09 4,10;7,46 0,33;7,46 3,35;7,45 1,36;7,44 3,80;7,43 4,54;7,43 1,68;7,42 4,10;7,41 0,53;7,24 0,47;7,24 4,14;7,23 1,29;7,22 1,46;7,21 7,43;7,21 1,47;7,20 1,15;7,19 3,52;7,18 0,34;6,76 3,13;6,76 3,12;6,75 3,10;6,74 3,15;4,20 2,51;4,18 4,06;4,16 2,62;3,36 405,89;3,34 0,50;3,06 2,19;3,04 3,78;3,02 2,66;2,67 0,72;2,65 2,08;2,63 2,82;2,61 1,78;2,59 0,58;2,52 17,00;2,52 23,32;2,51 16,38;2,40 1,88;2,39 6,33;2,37 6,52;2,35 2,11;2,00 0,40;1,10 0,39;1,07 7,47;1,05 16,00;1,03 7,16 |
| Beispiel Nr. 41 [DMSO-D₆] 8,44 5,92;8,43 3,68;8,43 3,83;8,42 6,02;7,28 4,34;7,27 2,33;7,26 6,88;7,19 5,40;7,17 3,72;7,15 6,23;7,15 3,84;7,14 3,89;7,14 5,91;4,18 2,47;4,16 3,77;4,14 2,52;4,10 0,66;4,06 0,64;4,04 1,87;4,02 1,90;4,00 0,65;3,31 284,17;3,07 2,21;3,06 3,49;3,04 2,58;2,89 0,82;2,73 0,65;2,67 0,36;2,67 0,46;2,67 0,34;2,64 0,79;2,62 2,05;2,61 2,87;2,59 1,80;2,57 0,72;2,54 0,64;2,51 24,14;2,50 45,26;2,50 59,28;2,50 40,96;2,49 19,54;2,33 16,00;1,99 8,22;1,91 0,81;1,19 2,27;1,17 4,49;1,16 2,23;1,07 0,48;0,92 0,40;0,01 0,62;0,00 13,23;-0,01 0,53 |
| Beispiel Nr. 42 [DMSO-D₆] 8,73 10,76;8,73 7,01;8,72 7,15;8,72 11,09;8,48 14,87;8,48 9,20;8,47 9,36;8,47 15,18;8,43 0,36;8,42 0,35;7,83 12,62;7,83 8,14;7,82 7,99;7,82 12,12;7,72 3,32;7,70 12,43;7,67 2,99;7,65 6,03;7,63 4,72;7,61 4,80;7,59 1,74;7,56 0,33;7,26 0,45;7,18 16,00;7,18 9,84;7,17 9,90;7,17 15,59;7,15 0,51;7,15 0,33;7,13 0,42;4,22 5,88;4,21 8,79;4,19 6,03;4,16 0,35;4,10 0,74;4,04 0,45;4,02 0,46;3,91 0,50;3,61 0,33;3,60 0,34;3,59 0,37;3,56 0,39;3,54 0,47;3,53 0,47;3,51 0,57;3,48 0,62;3,47 0,70;3,31 2137,05;3,29 34,04;3,08 5,11;3,07 8,13;3,05 6,08;2,89 0,32;2,73 0,39;2,68 0,95;2,67 1,72;2,67 2,34;2,67 2,74;2,64 4,95;2,63 6,91;2,61 4,43;2,59 1,89;2,54 3,03;2,52 8,68;2,51 111,92;2,51 208,40;2,50 272,43;2,50 187,67;2,49 89,14;2,34 0,67;2,33 1,40;2,33 2,48;2,18 0,46;2,07 1,06;1,99 1,30;1,91 1,87;1,24 0,78;1,19 0,39;1,18 0,79;1,16 0,49;1,07 2,34;0,89 0,33;0,00 1,84 |
| Beispiel Nr. 43 [DMSO-D₆] 8,55 3,50;8,54 2,14;8,54 2,20;8,53 3,62;7,95 1,92;7,32 1,62;7,31 0,73;7,30 1,85;7,30 2,22;7,29 0,85;7,28 2,01;7,15 2,10;7,14 0,72;7,13 0,95;7,13 4,57;7,12 4,66;7,12 2,89;7,11 2,49;7,11 4,14;7,10 1,97;4,35 1,40;4,34 1,24;4,33 1,32;3,33 69,85;2,89 16,00;2,73 13,14;2,73 12,88;2,72 1,37;2,70 1,46;2,51 4,08;2,51 7,74;2,50 10,22;2,50 7,17;2,49 3,53;1,91 2,30;1,85 1,02;1,84 0,96;1,77 0,92;1,76 1,09;1,65 0,89;1,63 1,10 |
| Beispiel Nr. 44 [DMSO-D₆] 8,73 2,30;8,73 1,57;8,72 1,56;8,72 2,40;8,47 11,10;8,47 7,35;8,46 7,60;8,46 11,41;8,44 0,39;8,33 0,70;8,31 0,73;7,95 0,47;7,91 0,45;7,83 2,92;7,83 1,97;7,82 1,85;7,82 2,75;7,60 0,35;7,59 0,34;7,58 0,34;7,56 0,33;7,55 0,40;7,54 0,40;7,53 0,42;7,53 0,43;7,51 0,37;7,49 0,40;7,47 0,36;7,44 1,68;7,43 2,92;7,42 3,90;7,41 4,18;7,39 2,91;7,38 1,26;7,36 0,53;7,35 0,99;7,33 0,52;7,32 0,43;7,31 0,42;7,25 0,38;7,24 0,48;7,21 6,27;7,21 7,51;7,19 10,12;7,18 6,82;7,18 15,23;7,17 9,62;7,16 10,33;7,16 16,00;7,15 2,33;7,14 1,12;7,12 0,61;7,12 0,85;4,22 0,38;4,21 5,27;4,19 8,07;4,17 5,51;4,10 1,27;4,04 0,44;4,02 0,49;4,00 0,33;3,91 2,04;3,62 0,32;3,58 0,38;3,58 0,38;3,57 0,41;3,56 0,47;3,54 0,55;3,52 0,62;3,51 0,74;3,44 1,00;3,32 2064,37;3,22 0,68;3,15 0,35;3,07 4,82;3,06 7,57;3,04 5,62;2,89 2,26;2,73 1,91;2,67 1,46;2,67 1,20;2,66 0,89;2,65 1,78;2,63 4,63;2,62 6,32;2,60 4,05;2,58 1,71;2,56 0,76;2,54 1,97;2,51 82,20;2,51 153,58;2,50 201,23;2,50 140,41;2,49 68,02;2,33 0,90;2,33 1,28;2,32 0,88;2,28 0,42;2,07 0,72;1,99 1,57;1,91 14,77;1,66 0,51;1,61 0,45;1,40 5,58;1,37 0,36;1,30 0,41;1,28 0,34;1,26 0,37;1,24 1,07;1,19 0,65;1,18 1,13;1,16 5,74;1,12 0,42;1,09 0,77;1,07 15,92;1,06 0,96;1,04 0,62;0,91 0,32;0,89 0,42;0,85 0,42;0,83 0,37;0,01 0,36;0,00 6,83 |
| Beispiel Nr. 45 [DMSO-D₆] 8,12 0,72;8,10 0,72;7,45 0,62;7,43 0,71;7,42 0,80;7,42 0,32;7,41 0,71;7,26 0,77;7,24 0,32;7,24 1,31;7,22 0,58;7,07 0,42;7,06 0,33;7,06 0,32;7,05 0,40;6,85 0,92;4,20 0,54;4,18 0,82;4,17 0,54;3,32 27,82;3,12 0,48;3,10 0,77;3,08 0,56;2,64 0,45;2,62 0,62;2,60 0,39;2,51 2,57;2,51 4,72;2,50 6,09;2,50 4,24;2,49 2,06;1,99 0,58;1,18 0,33;1,16 0,93;1,07 16,00;0,00 0,34 |
| Beispiel Nr. 46 [DMSO-D₆] 8,48 4,80;8,47 3,50;8,46 3,35;8,46 4,96;7,83 0,35;7,82 0,35;7,58 2,60;7,57 3,11;7,57 2,95;7,56 3,23;7,55 0,43;7,47 2,82;7,47 3,29;7,46 2,90;7,46 2,71;7,45 0,41;7,22 6,37;7,21 4,15;7,21 4,08;7,20 6,43;7,16 0,33;7,12 3,22;7,12 3,27;7,11 3,16;7,10 3,15;7,07 0,34;4,19 0,38;4,17 2,83;4,15 4,15;4,14 2,89;4,10 0,51;4,06 1,24;4,04 3,65;4,02 3,67;4,00 1,27;3,31 237,31;3,06 2,38;3,04 3,72;3,02 2,85;2,67 0,41;2,66 0,33;2,65 0,36;2,64 1,07;2,62 2,39;2,60 3,18;2,58 2,01;2,57 0,84;2,54 0,51;2,51 16,14;2,50 30,00;2,50 39,24;2,50 27,23;2,49 13,12;1,99 16,00;1,91 5,55;1,24 0,51;1,19 4,51;1,17 8,85;1,16 4,44;1,07 0,50;0,06 0,61;0,01 0,39;0,00 7,70 |
| Beispiel Nr. 47 [CD ₃CN] 8,89 2,33;8,86 0,35;8,20 5,77;8,19 9,08;8,18 6,36;8,18 6,14;8,18 5,51;8,14 7,12;8,06 0,35;7,58 4,64;7,58 4,85;7,57 6,50;7,57 6,22;7,50 5,90;7,49 5,94;7,49 4,73;7,48 4,25;7,47 1,28;7,46 6,10;7,46 2,97;7,45 6,65;7,44 6,96;7,43 2,86;7,43 6,45;7,42 1,11;7,40 0,39;7,30 0,33;7,08 6,81;7,07 2,15;7,06 2,74;7,06 12,48;7,05 2,65;7,04 2,15;7,03 5,97;7,03 0,82;6,85 5,42;6,85 5,42;6,84 5,34;6,84 5,28;5,45 16,00;4,19 5,51;4,17 9,99;4,16 5,76;4,09 0,35;4,07 1,06;4,05 1,10;4,03 0,36;3,61 0,33;2,88 5,15;2,86 10,23;2,85 5,40;2,47 0,38;2,46 0,36;2,40 0,38;2,35 0,42;2,28 0,60;2,27 0,68;2,25 0,83;2,15 1423,35;2,11 8,07;2,11 8,55;2,10 7,55;2,09 8,22;2,09 5,22;2,08 5,66;2,06 3,05;2,04 1,40;2,03 1,38;2,00 2,03;1,97 11,74;1,96 113,50;1,96 35,82;1,95 189,71;1,95 349,30;1,94 485,27;1,93 334,49;1,93 171,63;1,90 7,16;1,90 5,07;1,89 5,99;1,87 4,65;1,86 1,99;1,82 0,69;1,79 0,92;1,78 1,43;1,77 2,41;1,77 2,96;1,76 2,33;1,76 1,31;1,72 0,66;1,69 0,94;1,67 0,68;1,65 0,63;1,62 0,57;1,59 0,41;1,57 0,35;1,55 0,33;1,51 0,41;1,50 1,20;1,49 0,67;1,47 0,35;1,45 0,73;1,45 0,39;1,44 0,42;1,42 0,35;1,40 0,34;1,39 0,32;1,36 0,32;1,35 0,34;1,34 0,40;1,32 0,33;1,32 1,24;1,27 2,73;1,23 0,42;1,22 1,69;1,20 3,29;1,19 1,80;1,17 0,58;1,13 0,48;1,11 0,63;1,10 0,46;1,07 0,37;1,03 0,36;1,02 0,35;0,98 0,47;0,95 0,49;0,91 1,72;0,88 0,60;0,86 0,57;0,00 29,99 |
| Beispiel Nr. 48 [DMSO-D₆] 7,85 2,31;7,83 2,30;7,44 0,61;7,42 16,00;7,41 0,46;7,40 0,41;7,40 0,42;6,27 2,89;6,23 1,95;6,22 1,68;6,21 2,13;6,21 2,14;6,21 1,50;6,19 1,33;4,17 1,59;4,15 2,49;4,13 1,60;4,04 0,56;4,02 0,55;3,89 0,54;3,87 0,84;3,86 0,81;3,84 0,52;3,57 0,37;3,41 0,33;3,39 0,47;3,38 0,63;3,31 414,21;3,29 3,89;2,99 5,49;2,98 2,28;2,96 1,59;2,67 0,37;2,63 0,53;2,61 1,35;2,59 1,82;2,57 1,22;2,55 0,58;2,54 0,53;2,51 20,36;2,50 37,33;2,50 48,05;2,50 33,38;2,49 16,08;2,07 0,51;1,99 2,34;1,24 0,36;1,19 0,72;1,17 1,41;1,16 0,79;1,10 15,32;1,08 15,16;1,04 0,40;1,01 0,60;0,99 0,55;0,00 2,20 |
| Beispiel Nr. 50 [CD ₃CN] 9,19 0,34;8,14 1,01;8,13 1,01;8,07 1,21;7,41 0,71;7,41 0,47;7,41 1,01;7,40 1,11;7,40 1,05;7,32 0,37;7,32 0,37;7,31 1,42;7,30 2,07;7,30 2,07;6,81 0,79;6,80 0,79;6,80 0,78;6,80 0,77;4,18 0,91;4,17 1,68;4,16 0,96;4,02 0,46;2,84 0,86;2,83 1,73;2,82 0,94;2,21 0,34;2,21 0,36;2,19 434,90;2,16 0,56;2,10 0,58;2,09 0,52;2,09 1,02;2,08 0,56;2,08 1,60;2,07 0,33;2,06 0,43;2,05 0,63;2,05 0,43;1,97 1,35;1,97 168,54;1,96 5,33;1,95 5,13;1,95 41,91;1,95 76,84;1,94 112,13;1,94 75,93;1,93 38,25;1,93 0,78;1,90 0,36;1,89 0,81;1,88 0,53;1,88 0,85;1,87 0,52;1,87 0,72;1,85 0,99;1,83 0,46;1,83 0,67;1,82 0,46;1,41 16,00;1,27 0,48;0,00 5,09 |
| Beispiel Nr. 51 [CD ₃CN] 8,91 2,39;8,19 12,95;8,19 8,38;8,18 7,52;8,18 9,08;8,18 7,33;8,16 6,98;8,15 7,62;8,15 7,95;7,58 6,53;7,58 6,42;7,57 8,01;7,57 7,72;7,50 7,61;7,49 7,68;7,49 6,31;7,49 6,13;7,47 0,34;7,46 0,33;7,44 1,08;7,44 7,16;7,43 4,64;7,43 4,96;7,43 2,36;7,43 9,55;7,42 8,66;7,42 0,90;7,41 0,36;7,34 1,01;7,33 0,69;7,33 2,74;7,32 1,71;7,32 8,28;7,32 6,68;7,31 15,75;7,31 16,00;7,30 2,36;7,30 2,28;7,30 1,39;7,29 0,41;7,29 0,37;6,85 6,79;6,84 6,68;6,84 6,81;6,83 6,76;5,45 0,63;4,19 5,98;4,18 10,88;4,17 6,24;4,06 0,69;4,05 0,72;3,27 0,53;2,88 5,68;2,87 11,22;2,86 6,08;2,19 573,93;2,12 2,37;2,11 1,86;2,11 3,96;2,10 3,63;2,10 5,91;2,09 3,61;2,09 4,37;2,08 1,60;2,08 5,98;2,06 1,13;2,06 1,91;2,05 2,65;2,05 1,83;2,04 1,02;1,99 0,35;1,97 7,01;1,97 691,81;1,96 14,81;1,95 15,36;1,95 162,44;1,95 294,97;1,94 434,82;1,94 299,80;1,93 150,79;1,93 4,41;1,93 2,05;1,91 2,19;1,91 1,50;1,90 5,02;1,90 3,20;1,89 4,84;1,89 3,04;1,88 4,39;1,88 1,30;1,87 1,63;1,85 3,96;1,84 0,96;1,83 1,78;1,83 2,54;1,82 1,74;1,82 0,93;1,38 0,72;1,34 0,34;1,28 0,72;1,27 2,14;1,22 1,05;1,20 1,81;1,19 0,98;1,11 0,48;0,91 1,94;0,88 0,47;0,00 7,31 |
| Beispiel Nr. 52 [DMSO-D₆] 7,83 2,30;7,82 2,30;7,45 2,09;7,45 0,96;7,44 2,42;7,43 2,61;7,42 1,06;7,42 2,30;7,22 0,43;7,21 2,47;7,21 0,87;7,19 1,08;7,19 4,29;7,18 1,00;7,17 0,78;7,17 1,98;6,26 2,96;6,22 2,03;6,21 1,78;6,20 2,00;6,20 1,79;6,18 1,36;6,16 1,36;4,16 1,59;4,14 2,51;4,13 1,61;3,88 0,54;3,86 0,83;3,84 0,82;3,83 0,54;3,31 221,49;3,29 1,63;3,00 1,39;2,98 2,33;2,96 1,66;2,63 0,51;2,61 1,36;2,59 1,83;2,57 1,18;2,55 0,48;2,51 9,30;2,50 17,10;2,50 22,16;2,50 15,45;2,49 7,50;2,08 11,04;1,99 1,28;1,19 0,41;1,18 0,74;1,16 0,39;1,10 16,00;1,08 15,81;1,01 0,43;0,99 0,41;0,00 1,00 |
| Beispiel Nr. 53 [DMSO-D₆] 7,86 8,61;7,85 8,75;7,66 0,52;7,66 0,52;7,66 0,61;7,65 0,54;7,54 0,61;7,53 0,52;7,53 0,75;7,52 0,74;7,50 8,18;7,49 9,36;7,48 8,86;7,48 9,40;7,42 0,67;7,42 0,63;7,41 0,58;7,41 0,61;7,36 8,91;7,35 9,69;7,35 8,61;7,35 7,93;7,14 10,18;7,13 9,95;7,12 9,72;7,12 9,31;6,33 16,00;6,33 13,52;6,32 6,73;6,31 9,35;6,31 7,01;6,30 1,55;5,88 0,56;5,84 13,46;4,17 0,38;4,12 7,18;4,11 13,20;4,09 7,17;4,06 0,97;4,05 1,37;4,04 0,78;4,03 0,88;4,02 0,61;4,02 0,34;4,01 0,39;4,00 0,42;3,99 0,37;3,96 0,34;3,93 0,41;3,92 0,41;3,91 0,40;3,90 0,47;3,88 0,42;3,87 0,41;3,86 0,38;3,82 0,45;3,82 0,51;3,81 0,49;3,80 0,45;3,78 0,48;3,75 0,59;3,69 0,67;3,68 0,63;3,63 0,81;3,61 0,89;3,59 0,96;3,57 3,27;3,55 1,27;3,51 1,52;3,33 3424,42;3,20 0,61;3,19 0,55;3,18 0,67;3,16 0,36;2,77 0,66;2,75 1,03;2,74 0,84;2,71 6,56;2,69 13,04;2,68 7,88;2,61 0,48;2,61 0,51;2,54 2,77;2,51 94,24;2,51 172,97;2,50 224,40;2,50 156,69;2,49 76,49;2,44 0,38;2,33 1,07;2,33 1,44;2,32 1,04;2,09 0,40;2,07 3,85;2,06 0,39;2,04 1,93;2,02 4,58;2,01 5,21;2,01 6,80;2,00 5,50;1,99 5,59;1,99 4,48;1,98 2,41;1,96 0,60;1,91 0,51;1,81 6,10;1,80 5,44;1,80 6,62;1,78 5,12;1,40 0,32;1,24 0,47;1,19 0,47;1,18 0,79;1,16 0,52;0,00 3,11 |
| Beispiel Nr. 54 [DMSO-D₆] 8,13 8,66;8,12 8,74;8,10 0,33;7,92 0,41;7,90 0,41;7,75 0,34;7,47 2,03;7,45 4,39;7,43 4,74;7,43 3,40;7,41 2,68;7,40 0,68;7,36 0,35;7,26 0,41;7,24 1,76;7,24 2,49;7,23 3,23;7,22 16,00;7,21 14,95;7,20 7,22;7,19 10,48;7,19 5,28;7,12 0,63;7,10 4,07;7,09 5,29;7,09 4,31;7,08 4,10;7,08 4,89;7,07 3,60;6,89 11,11;6,39 0,42;6,39 0,46;4,21 6,84;4,20 10,38;4,18 6,91;3,90 0,73;3,51 0,37;3,46 0,40;3,44 0,39;3,43 0,51;3,42 0,53;3,30 747,94;3,28 10,29;3,17 0,35;3,12 6,19;3,10 9,77;3,08 7,05;2,91 0,42;2,67 1,55;2,66 1,55;2,66 2,44;2,64 5,91;2,62 8,16;2,60 5,39;2,58 2,32;2,51 81,79;2,50 146,04;2,50 185,15;2,50 128,60;2,33 0,97;2,33 1,28;2,29 0,38;2,27 0,34;2,07 1,84;1,99 0,44;1,40 1,95;1,35 0,33;1,30 1,39;1,26 0,42;1,24 1,25;1,16 0,97;1,07 4,07;0,00 32,19;-0,01 1,43 |
| Beispiel Nr. 55 [CD ₃CN] 9,18 0,36;8,16 0,92;8,15 0,94;8,06 1,15;7,44 0,99;7,43 0,45;7,42 1,08;7,41 1,07;7,41 0,47;7,40 0,97;7,07 1,12;7,05 1,92;7,03 0,35;7,02 0,89;6,82 0,87;6,82 0,79;6,81 0,83;6,80 0,81;4,18 0,90;4,16 1,62;4,15 0,89;3,95 2,02;2,84 0,91;2,83 1,69;2,81 0,91;2,47 0,33;2,16 856,19;2,13 4,46;2,12 1,58;2,11 1,73;2,11 1,82;2,10 1,68;2,09 1,31;2,08 1,44;2,08 1,12;2,07 1,19;2,05 0,84;2,04 0,69;1,96 389,96;1,95 62,01;1,95 106,27;1,94 145,13;1,93 99,91;1,93 51,45;1,89 1,30;1,88 0,96;1,88 1,09;1,86 0,82;1,79 2,20;1,78 0,40;1,77 0,59;1,77 0,86;1,76 0,60;1,76 0,34;1,41 16,00;1,27 0,40;0,91 0,75;0,01 0,69;0,00 15,01;-0,01 0,74 |
| Beispiel Nr. 56 [DMSO-D₆] 7,86 2,18;7,85 2,22;7,42 0,61;7,41 0,73;7,40 1,20;7,39 1,23;7,39 0,86;7,38 0,78;7,25 1,11;7,25 1,66;7,25 1,28;7,24 1,01;7,24 1,46;7,24 1,10;7,20 0,76;7,19 0,79;7,19 0,98;7,19 0,84;7,18 0,73;7,18 0,82;7,17 0,97;7,17 0,92;7,17 0,79;7,17 0,69;7,16 0,46;7,16 0,44;7,15 1,12;7,15 0,80;7,14 0,54;7,14 0,55;7,14 0,40;7,13 0,38;6,28 2,62;6,26 1,27;6,25 1,32;6,24 1,91;6,23 1,72;6,23 1,86;6,23 1,74;4,17 1,54;4,16 2,37;4,15 1,59;4,03 0,41;4,02 0,41;3,88 0,36;3,87 0,57;3,86 0,58;3,85 0,37;3,35 80,05;3,33 1,48;2,99 1,35;2,98 2,08;2,96 1,57;2,62 0,62;2,60 1,27;2,59 1,73;2,58 1,05;2,57 0,37;2,51 5,24;2,51 11,62;2,50 16,15;2,50 11,61;2,50 5,26;2,08 0,46;1,99 1,79;1,19 0,53;1,17 1,01;1,16 0,50;1,10 16,00;1,09 15,96;1,01 0,72;1,00 0,72;0,00 6,89 |
| Beispiel Nr. 57 [CD ₃CN] 8,70 7,26;8,70 4,26;8,69 4,42;8,68 7,66;8,67 0,53;8,65 0,38;8,63 0,56;8,62 0,39;8,59 1,12;8,58 0,71;8,57 0,83;8,57 1,19;7,57 0,54;7,55 0,66;7,50 8,38;7,49 4,92;7,49 4,96;7,48 8,26;7,42 1,10;7,42 0,64;7,41 1,01;7,40 0,43;7,39 0,58;7,37 0,41;7,26 0,63;7,25 0,58;6,80 6,76;6,79 7,20;6,78 0,73;6,77 1,57;6,76 1,09;6,48 6,83;6,47 6,42;6,45 0,32;6,44 0,33;6,44 0,33;6,43 0,35;6,39 0,99;6,38 0,99;5,48 5,89;5,00 2,53;4,99 2,68;4,97 2,81;4,97 2,65;4,68 0,43;4,68 0,51;4,67 0,43;4,67 0,36;4,51 0,78;4,50 1,76;4,49 0,99;4,48 1,61;4,47 3,31;4,45 1,63;4,42 1,76;4,40 1,90;4,39 2,38;4,38 2,51;4,37 1,06;4,36 1,13;4,35 1,01;4,34 0,93;4,33 0,64;4,32 0,68;4,31 0,50;4,30 0,46;4,29 0,35;4,27 0,62;4,25 0,35;4,12 0,52;4,10 1,47;4,08 1,56;4,06 0,56;3,58 0,33;3,57 0,41;3,55 0,39;3,46 0,40;3,44 0,41;3,18 0,62;3,16 0,59;3,13 0,43;3,12 0,48;2,88 0,58;2,88 0,66;2,87 1,17;2,86 1,13;2,86 0,61;2,85 1,18;2,84 0,94;2,83 1,75;2,83.1,72;2,82 0,84;2,81 0,75;2,77 0,95;2,75 0,96;2,75 1,09;2,73 1,53;2,72 0,94;2,72 0,64;2,71 1,35;2,69 0,64;2,69 0,62;2,67 0,45;2,63 16,00;2,42 0,34;2,39 0,37;2,36 0,36;2,35 0,65;2,34 0,60;2,33 0,49;2,32 0,7.5;2,30 1,06;2,25 144,20;2,24 210,43;2,16 1,66;2,15 0,72;2,14 0,91;2,14 1,06;2,13 0,75;2,12 0,55;2,01 2,16;2,00 8,74;1,99 191,01;1,99 6,63;1,98 40,96;1,98 75,96;1,97 108,11;1,96 73,24;1,96 37,20;1,83 0,41;1,82 1,25;1,81 0,44;1,80 0,62;1,80 0,77;1,79 0,54;1,79 0,33;1,30 1,21;1,28 0,49;1,25 2,02;1,23 3,66;1,21 2,02;1,19 0,34;1,00 1,36;0,98 2,76;0,97 1,40;0,96 1,39;0,94 2,94;0,92 1,34;0,03 0,38 |
| Beispiel Nr. 58 [CD ₃CN] 8,64 2,38;8,18 9,09;8,17 9,01;8,04 4,38;7,36 6,82;7,35 8,54;7,35 7,26;7,34 9,17;7,31 8,37;7,31 8,80;7,30 7,04;7,30 6,78;7,18 8,82;7,18 8,56;7,17 8,20;7,17 7,67;6,90 8,19;6,89 8,10;6,89 8,21;6,89 7,98;5,45 0,99;4,15 8,52;4,14 15,37;4,13 8,72;2,78 8,12;2,77 16,00;2,76 8,68;2,21 0,60;2,20 0,48;2,19 1,42;2,16 872,77;2,14 1,02;2,10 76,19;2,09 2,51;2,08 1,78;2,08 5,50;2,07 4,41;2,07 7,95;2,06 5,30;2,06 7,22;2,05 3,46;2,05 2,48;2,05 2,83;1,99 0,42;1,97 2,84;1,97 264,26;1,96 15,21;1,95 17,71;1,95 146,93;1,95 270,28;1,94 396,53;1,94 268,16;1,93 133,53;1,92 0,44;1,92 0,39;1,91 0,38;1,87 2,67;1,87 1,70;1,86 6,86;1,86 4,14;1,85 7,01;1,85 3,94;1,84 6,02;1,84 1,64;1,83 2,87;1,83 2,29;1,82 1,52;1,82 0,81;1,27 0,69;0,01 0,95;0,00 31,85;-0,01 0,87 |
| Beispiel Nr. 59 [CD ₃CN] 8,57 1,21;8,17 3,52;8,16 3,62;8,07 3,46;7,36 2,32;7,35 2,95;7,35 2,69;7,34 3,13;7,31 3,03;7,31 3,45;7,30 2,75;7,30 2,66;7,18 3,18;7,18 3,36;7,17 2,94;7,17 3,01;6,89 2,95;6,88 3,11;6,88 2,90;6,87 3,04;5,45 3,47;4,15 3,33;4,14 6,15;4,13 3,44;4,08 0,99;4,06 3,02;4,05 3,07;4,04 1,02;2,79 3,18;2,78 6,32;2,77 3,41;2,42 2,26;2,41 6,99;2,40 7,18;2,39 2,42;2,16 57,06;2,09 1,30;2,08 5,07;2,07 3,51;2,06 2,13;2,06 2,86;2,05 1,64;2,05 1,49;2,04 0,56;1,99 0,67;1,98 0,63;1,97 19,43;1,97 586,60;1,96 10,34;1,96 8,32;1,95 9,95;1,95 65,34;1,95 118,42;1,94 170,03;1,94 118,14;1,93 60,29;1,88 1,04;1,87 0,72;1,87 2,74;1,86 1,77;1,86 2,85;1,85 4,62;1,85 2,57;1,84 0,88;1,83 0,70;1,83 1,00;1,82 0,68;1,82 0,34;1,27 0,36;1,22 3,56;1,20 7,06;1,19 3,52;1,13 7,88;1,12 16,00;1,11 7,61;0,01 0,42;0,00 13,95;-0,01 0,45 |
| Beispiel Nr. 60 [CD ₃CN] 8,75 0,39;8,21 1,41;8,21 1,42;8,21 1,39;8,21 1,46;8,08 1,43;7,36 1,16;7,36 1,43;7,35 1,37;7,35 1,65;7,31 1,36;7,31 1,50;7,31 1,29;7,31 1,25;7,18 1,54;7,18 1,49;7,17 1,37;7,17 1,33;6,95 1,37;6,95 1,37;6,94 1,33;6,94 1,34;5,45 0,77;4,15 1,26;4,14 2,29;4,13 1,34;4,08 0,55;4,06 1,68;4,05 1,67;4,04 0,57;3,98 8,38;3,46 16,00;2,80 1,20;2,79 2,38;2,78 1,30;2,17 14,59;2,09 0,45;2,08 0,35;2,08 0,83;2,07 0,75;2,07 1,25;2,06 0,79;2,06 1,02;2,06 0,51;2,05 0,45;2,05 0,53;2,05 0,53;1,97 7,67;1,97 16,34;1,96 2,15;1,95 2,09;1,95 19,24;1,95 34,64;1,94 51,63;1,94 35,91;1,93 18,25;1,92 0,38;1,88 0,42;1,87 1,05;1,86 0,67;1,86 1,03;1,85 0,68;1,85 0,97;1,84 0,33;1,84 0,39;1,83 0,34;1,22 2,21;1,20 4,18;1,19 2,18;0,00 3,45 |
| Beispiel Nr. 61 [DMSO-D₆] 8,10 4,76;8,10 4,86;7,43 0,48;7,42 0,85;7,42 0,49;7,42 0,91;7,41 2,11;7,41 2,38;7,41 3,51;7,40 15,14;7,40 16,00;7,39 3,43;7,39 2,45;7,38 2,55;7,38 0,42;7,38 0,38;7,37 0,60;7,07 1,64;7,07 2,34;7,07 1,70;7,07 1,69;7,06 2,25;7,06 1,58;6,85 5,37;4,20 3,12;4,19 4,71;4,18 3,22;3,38 0,58;3,38 0,77;3,38 0,56;3,37 1,26;3,35 992,34;3,33 3,42;3,32 0,41;3,12 2,81;3,11 4,11;3,10 3,22;2,64 0,92;2,63 2,39;2,62 3,93;2,61 1,27;2,61 2,17;2,59 0,79;2,54 0,38;2,52 1,03;2,52 1,29;2,52 1,27;2,51 40,31;2,51 89,93;2,50 125,16;2,50 90,10;2,50 40,68;2,39 0,56;2,39 0,74;2,38 0,53;2,08 0,90;1,99 0,69;1,17 0,38;0,00 5,87 |
| Beispiel Nr. 62 [CD ₃CN] 8,54 13,69;8,54 7,82;8,53 8,02;8,53 13,64;7,31 6,31;7,31 6,26;7,30 6,64;7,30 6,44;7,26 16,00;7,26 9,18;7,25 9,20;7,25 15,58;6,95 6,21;6,94 7,63;6,94 5,84;6,93 7,53;6,89 7,27;6,89 7,53;6,89 6,07;6,89 5,75;5,45 2,59;4,15 6,33;4,14 11,20;4,13 6,60;2,75 6,07;2,74 11,71;2,73 6,51;2,20 0,49;2,19 0,68;2,19 0,60;2,17 523,59;2,16 0,50;2,08 1,91;2,08 1,37;2,07 3,62;2,07 3,10;2,06 5,83;2,06 3,58;2,05 5,08;2,05 1,37;2,04 2,30;1,97 0,32;1,97 28,74;1,96 5,43;1,95 6,64;1,95 58,15;1,95 105,93;1,94 157,71;1,94 107,34;1,93 52,87;1,93 1,19;1,92 0,46;1,86 2,22;1,86 1,22;1,85 5,44;1,85 2,99;1,84 4,53;1,84 4,86;1,84 2,86;1,83 4,94;1,83 1,28;1,83 1,13;1,83 1,27;1,82 1,81;1,82 0,35;1,27 0,42;1,13 0,59;0,00 5,72 |
| Beispiel Nr. 63 [CD ₃CN] 8,58 0,51;8,18 1,70;8,17 1,56;8,17 1,71;8,08 1,75;7,36 1,22;7,35 1,55;7,35 1,37;7,35 1,66;7,31 1,61;7,31 1,70;7,30 1,39;7,30 1,27;7,18 1,68;7,18 1,60;7,17 1,55;7,17 1,50;6,89 1,47;6,88 1,39;6,88 1,44;6,87 1,39;5,45 0,44;4,15 1,52;4,14 2,78;4,13 1,60;3,61 0,56;2,79 1,41;2,78 2,80;2,77 1,52;2,66 0,32;2,65 0,84;2,64 1,14;2,63 0,87;2,62 0,35;2,18 415,81;2,09 0,42;2,09 0,43;2,09 0,32;2,08 0,91;2,08 0,82;2,08 0,79;2,07 1,41;2,07 0,83;2,06 1,19;2,06 0,60;2,05 0,70;2,05 0,53;1,97 0,53;1,97 60,62;1,96 3,38;1,95 3,70;1,95 32,80;1,95 60,37;1,94 88,12;1,94 59,46;1,93 30,14;1,93 0,49;1,88 0,47;1,87 1,20;1,86 0,76;1,86 1,20;1,85 0,74;1,85 1,08;1,84 0,39;1,83 0,36;1,83 0,51;1,82 0,35;1,27 0,49;1,15 16,00;1,14 15,84;0,97 0,53;0,95 0,51;0,00 5,59 |
| Beispiel Nr. 64 [CD ₃CN] 8,58 0,47;8,18 1,49;8,18 1,55;8,17 1,50;8,17 1,65;8,08 1,29;7,35 1,32;7,35 1,75;7,35 1,48;7,34 1,87;7,31 1,71;7,31 1,85;7,30 1,47;7,30 1,47;7,18 1,66;7,18 1,65;7,17 1,52;7,17 1,51;6,90 1,52;6,89 1,52;6,89 1,50;6,88 1,53;4,15 1,33;4,14 2,44;4,13 1,44;3,27 0,36;2,79 1,29;2,78 2,52;2,77 1,39;2,25 3,64;2,24 4,55;2,16 187,11;2,13 0,83;2,11 0,87;2,10 0,88;2,09 0,86;2,09 0,78;2,08 1,18;2,08 1,28;2,07 0,83;2,07 1,37;2,06 0,88;2,06 1,30;2,06 1,17;2,05 1,39;2,05 1,12;2,04 0,53;1,97 1,48;1,97 125,29;1,96 8,47;1,95 8,35;1,95 77,11;1,95 141,39;1,94 210,88;1,94 144,04;1,93 71,86;1,93 2,18;1,92 0,89;1,88 0,44;1,87 1,10;1,86 0,71;1,86 1,05;1,85 1,13;1,85 1,03;1,84 0,40;1,84 0,41;1,84 0,55;1,83 0,90;1,83 1,21;1,82 0,83;1,82 0,43;1,48 0,34;1,44 0,33;1,27 0,65;1,22 0,35;1,20 0,41;0,97 16,00;0,95 15,50;0,94 0,61;0,93 0,57;0,01 1,30;0,00 44,51;-0,01 1,22 |
| Beispiel Nr. 65 [CD ₃CN] 8,88 2,87;8,18 9,34;8,18 9,71;8,17 9,31;8,17 9,80;8,04 8,90;7,35 8,26;7,35 9,82;7,34 8,90;7,34 10,57;7,30 9,48;7,30 10,16;7,29 8,16;7,29 7,89;7,18 10,24;7,17 10,03;7,17 9,49;7,17 9,10;6,88 9,46;6,88 9,25;6,87 9,12;6,87 9,28;5,45 11,62;4,14 8,65;4,13 15,38;4,12 8,87;4,06 0,38;4,05 0,36;3,28 1,09;3,27 1,01;2,89 1,16;2,77 1,60;2,77 8,04;2,76 16,00;2,75 8,74;2,20 0,51;2,19 1,79;2,18 1,59;2,18 3,31;2,16 2864,50;2,15 3,12;2,14 1,52;2,14 1,10;2,13 0,90;2,12 0,38;2,08 2,30;2,08 1,74;2,07 4,95;2,07 4,48;2,06 9,35;2,06 6,41;2,05 8,65;2,05 4,98;2,04 2,78;2,04 2,75;1,98 0,34;1,97 2,80;1,97 167,69;1,96 23,95;1,95 28,58;1,95 315,03;1,95 583,14;1,94 830,07;1,94 575,58;1,93 291,99;1,93 9,01;1,92 4,13;1,92 1,05;1,91 0,67;1,91 0,52;1,87 2,78;1,86 1,82;1,86 6,98;1,85 4,46;1,85 6,61;1,84 4,17;1,84 6,37;1,83 4,78;1,83 6,13;1,82 3,54;1,82 1,79;1,81 1,32;1,80 2,76;1,80 2,97;1,79 1,75;1,79 5,06;1,78 3,17;1,78 2,92;1,77 1,47;1,27 1,88;1,22 0,46;1,20 0,85;1,19 0,59;0,89 2,33;0,89 1,94;0,88 8,28;0,88 12,08;0,87 7,33;0,87 6,18;0,87 11,16;0,86 4,31;0,85 1,64;0,85 2,10;0,84 4,76;0,84 11,77;0,83 6,52;0,83 5,42;0,83 12,12;0,82 6,09;0,82 2,49;0,81 2,31;0,10 1,08;0,01 8,35;0,00 298,98;-0,01 9,20;-0,10 1,12 |
| Beispiel Nr. 66 [CD ₃CN] 9,02 0,50;8,21 1,75;8,21 1,81;8,20 1,85;8,20 1,77;8,09 1,58;7,36 1,83;7,36 2,23;7,35 1,86;7,35 2,53;7,31 2,04;7,31 2,30;7,31 1,87;7,31 1,70;7,18 2,27;7,18 2,32;7,18 2,16;7,17 2,09;6,94 2,04;6,93 1,96;6,93 1,94;6,93 1,96;5,45 16,00;4,26 0,49;4,25 0,56;4,25 0,51;4,24 0,51;4,15 1,60;4,14 2,78;4,13 1,68;4,06 0,58;4,05 0,57;4,01 0,58;4,00 0,56;2,80 1,39;2,79 2,75;2,78 1,56;2,19 0,45;2,18 0,47;2,18 0,72;2,16 404,88;2,14 0,40;2,09 0,46;2,09 0,36;2,08 0,94;2,08 1,11;2,07 1,54;2,07 0,96;2,06 1,13;2,06 1,10;2,05 1,53;2,05 0,81;2,04 0,42;1,97 2,83;1,97 39,03;1,96 0,53;1,96 4,80;1,95 5,85;1,95 65,22;1,95 118,44;1,94 182,94;1,94 127,17;1,93 61,91;1,93 1,84;1,92 0,80;1,88 0,55;1,88 0,35;1,87 1,35;1,87 0,81;1,86 1,27;1,86 0,77;1,85 1,34;1,84 0,34;1,84 0,41;1,84 0,45;1,83 0,76;1,83 1,01;1,82 0,72;1,82 0,39;1,39 9,59;1,37 9,73;1,27 0,44;1,22 0,91;1,20 1,64;1,19 0,84;1,11 0,63;0,01 1,73;0,00 66,64;-0,01 1,82 |
| Beispiel Nr. 67 [CD ₃CN] 8,16 1,25;8,16 1,28;8,15 1,33;8,15 1,26;8,12 0,43;7,82 1,39;7,36 1,12;7,36 1,38;7,35 1,27;7,35 1,53;7,31 1,34;7,31 1,40;7,31 1,20;7,31 1,14;7,19 1,41;7,18 1,40;7,18 1,30;7,18 1,28;6,87 1,31;6,87 1,29;6,86 1,28;6,86 1,27;5,45 2,80;4,15 1,16;4,14 2,10;4,13 1,22;4,06 0,49;4,05 0,49;3,70 16,00;2,79 1,12;2,78 2,19;2,77 1,21;2,16 399,73;2,15 0,76;2,14 0,32;2,09 0,33;2,08 0,67;2,08 0,77;2,07 1,08;2,07 0,67;2,06 0,84;2,06 0,85;2,05 1,14;2,05 0,62;2,04 0,35;1,97 2,44;1,97 33,49;1,96 3,95;1,95 4,93;1,95 52,98;1,95 96,11;1,94 144,50;1,94 99,18;1,93 49,40;1,93 1,38;1,92 0,61;1,88 0,40;1,87 0,98;1,86 0,61;1,86 0,93;1,85 0,58;1,85 0,86;1,84 0,36;1,84 0,35;1,83 0,61;1,83 0,82;1,82 0,58;1,22 0,64;1,20 1,26;1,19 0,62;1,11 0,51;0,01 1,41;0,00 49,91;-0,01 1,40 |
| Beispiel Nr. 68 [CD ₃CN] 9,20 0,37;8,21 0,99;8,20 1,02;8,10 1,30;7,36 0,69;7,36 0,82;7,35 0,75;7,35 0,85;7,31 0,87;7,31 0,95;7,30 0,80;7,30 0,76;7,18 0,91;7,18 0,92;7,17 0,84;7,17 0,86;6,93 0,83;6,92 0,87;6,92 0,81;6,91 0,85;4,15 0,93;4,14 1,71;4,13 0,97;3,93 0,80;2,80 0,86;2,79 1,74;2,78 0,97;2,16 367,05;2,08 0,61;2,08 0,53;2,07 0,92;2,07 0,57;2,06 0,71;2,06 0,58;2,06 0,77;2,05 1,08;2,05 0,65;1,97 12,84;1,96 6,06;1,95 8,11;1,95 62,38;1,95 113,23;1,94 163,35;1,94 112,62;1,93 58,71;1,87 0,77;1,87 0,51;1,86 0,80;1,85 0,66;1,83 0,37;1,83 0,65;1,83 0,95;1,82 0,63;1,82 0,36;1,42 16,00;1,27 0,32;1,11 0,62;0,01 1,19;0,00 33,11;-0,01 1,31 |
| Beispiel Nr. 69 [DMSO-D₆] 7,82 0,47;7,80 0,44;7,49 0,39;7,47 0,45;7,47 0,50;7,45 0,44;7,29 0,47;7,27 0,34;7,27 0,84;7,24 0,73;6,73 0,49;6,72 0,50;6,57 0,33;6,55 0,33;4,22 0,34;4,20 0,50;4,18 0,37;4,07 0,35;3,93 4,17;3,09 0,40;2,51 5,19;2,51 10,41;2,50 13,89;2,50 10,12;2,50 5,02;1,30 6,27;1,16 2,70;1,07 16,00;0,00 0,54 |
| Beispiel Nr. 70 [DMSO-D₆] 8,10 0,52;8,10 0,52;7,82 2,25;7,81 2,31;7,42 1,96;7,42 2,86;7,42 0,85;7,41 1,82;7,41 3,98;7,41 3,27;7,40 2,21;7,40 1,84;7,40 0,66;7,39 0,36;7,37 1,11;7,37 1,87;7,36 0,63;7,35 3,83;7,35 1,68;7,34 0,99;7,34 2,06;7,34 0,34;7,33 0,88;7,32 1,67;7,32 0,89;7,32 0,56;7,31 1,81;7,31 0,37;7,30 0,54;6,85 0,58;6,28 2,55;6,28 2,60;6,21 1,35;6,21 2,25;6,21 1,97;6,20 3,14;6,20 2,40;4,20 0,34;4,19 0,52;4,18 0,37;4,16 1,52;4,15 2,36;4,14 1,57;4,05 0,33;4,03 1,02;4,02 1,02;4,01 0,34;3,85 0,35;3,84 0,55;3,83 0,56;3,82 0,35;3,35 117,16;3,33 1,19;3,11 0,45;3,10 0,35;2,99 1,35;2,98 2,04;2,97 1,56;2,62 0,75;2,61 0,35;2,60 1,31;2,59 1,72;2,58 1,04;2,57 0,36;2,51 8,24;2,51 18,33;2,50 25,54;2,50 18,54;2,50 8,47;1,99 4,60;1,19 1,27;1,17 2,51;1,16 1,32;1,09 16,00;1,08 15,90;1,06 0,33;1,01 0,95;0,99 0,95;0,00 9,01 |
| Beispiel Nr. 71 [DMSO-D₆] 7,83 1,95;7,82 2,02;7,45 1,91;7,44 0,78;7,43 2,20;7,43 2,45;7,42 0,91;7,41 2,23;7,22 2,37;7,21 0,75;7,20 0,82;7,19 4,23;7,19 0,89;7,18 0,68;7,17 2,00;6,44 0,61;6,43 1,27;6,41 0,63;6,25 2,65;6,25 2,67;6,25 1,32;6,24 1,87;6,23 1,40;4,16 1,37;4,15 2,19;4,13 1,43;4,04 0,48;4,02 0,48;3,35 24,21;3,35 25,51;3,34 49,43;3,31 1,33;3,00 1,20;2,99 2,03;2,97 1,49;2,94 1,39;2,92 2,43;2,91 1,78;2,89 0,36;2,63 0,45;2,61 1,13;2,59 1,54;2,57 0,97;2,55 0,32;2,51 6,42;2,51 13,24;2,50 17,92;2,50 13,08;2,49 6,27;1,99 2,16;1,75 0,35;1,74 0,69;1,72 0,88;1,70 0,73;1,69 0,40;1,24 0,59;1,19 0,61;1,17 1,19;1,16 0,59;0,99 0,39;0,98 0,37;0,85 16,00;0,83 15,38;0,82 2,16;0,81 0,47;0,81 0,63;0,81 1,69;0,00 5,51 |
| Beispiel Nr. 72 [DMSO-D₆] 8,02 0,59;7,81 1,82;7,80 1,87;7,43 1,31;7,42 1,62;7,42 0,65;7,40 2,85;7,40 2,69;7,37 0,84;7,37 1,05;7,36 0,52;7,35 2,78;7,35 1,13;7,33 2,72;7,33 2,04;7,32 1,03;7,32 0,59;7,31 0,98;6,42 0,53;6,41 1,10;6,39 0,56;6,29 2,10;6,24 1,50;6,24 1,34;6,23 1,48;6,23 1,35;4,17 1,22;4,15 1,94;4,13 1,27;4,06 0,88;4,04 2,69;4,02 2,72;4,00 0,91;3,35 35,16;3,34 65,44;3,32 1,52;3,01 1,08;2,99 1,81;2,97 1,31;2,94 1,23;2,92 2,13;2,91 1,69;2,90 1,81;2,89 0,75;2,82 0,42;2,80 0,50;2,79 0,37;2,63 0,37;2,61 1,03;2,59 1,39;2,58 0,86;2,52 0,33;2,51 7,77;2,51 15,73;2,50 21,07;2,50 15,30;2,49 7,23;1,99 12,05;1,76 0,32;1,74 0,63;1,72 0,81;1,71 0,70;1,69 0,52;1,67 0,44;1,65 0,33;1,40 0,52;1,24 1,00;1,22 0,42;1,19 3,46;1,18 1,18;1,17 6,92;1,16 3,35;1,05 0,42;1,03 0,42;0,99 0,72;0,98 0,63;0,87 0,69;0,85 16,00;0,83 15,89;0,82 3,66;0,81 0,72;0,81 1,07;0,81 2,83;0,00 1,75 |
| Beispiel Nr. 73 [DMSO-D₆] 7,85 2,33;7,85 2,30;7,84 2,36;7,84 2,38;7,42 0,66;7,41 0,87;7,40 1,28;7,39 1,28;7,39 0,89;7,38 0,77;7,26 1,21;7,25 1,73;7,25 1,38;7,24 1,08;7,24 1,49;7,24 1,16;7,19 0,68;7,19 1,03;7,18 0,90;7,17 1,34;7,17 1,21;7,17 1,44;7,17 1,19;7,16 1,26;7,16 0,98;7,15 0,78;7,15 0,60;7,14 0,60;7,14 0,42;7,14 0,38;6,49 0,69;6,48 1,44;6,47 0,71;6,29 2,07;6,27 1,79;6,27 1,49;6,26 1,74;6,26 1,52;4,17 1,58;4,16 2,46;4,15 1,69;4,05 0,32;4,03 0,97;4,02 0,99;4,01 0,32;3,37 166,66;3,35 0,70;2,99 1,43;2,98 2,18;2,97 1,67;2,95 1,08;2,94 1,81;2,93 1,12;2,62 0,54;2,61 1,28;2,59 1,84;2,58 1,13;2,57 0,40;2,51 4,37;2,51 9,64;2,51 13,51;2,50 9,63;2,50 4,35;1,99 4,49;1,75 0,32;1,74 0,65;1,73 0,83;1,72 0,68;1,71 0,35;1,24 0,42;1,19 1,32;1,17 2,61;1,16 1,23;0,85 16,00;0,85 3,05;0,84 15,75;0,84 2,67;0,82 0,39;0,82 1,46;0,81 1,40;0,00 3,26 |
| Beispiel Nr. 74 [DMSO-D₆] 8,45 5,48;8,44 5,69;7,44 0,44;7,43 4,66;7,43 1,82;7,42 5,17;7,41 6,28;7,40 2,07;7,40 5,73;7,39 0,66;7,25 0,64;7,24 5,99;7,23 1,71;7,22 1,89;7,22 10,60;7,21 1,92;7,20 1,58;7,19 4,90;7,19 0,48;7,15 6,49;7,15 4,39;7,14 4,32;7,14 6,40;5,76 1,18;4,49 1,07;4,48 2,04;4,46 2,07;4,44 1,11;3,93 1,99;3,34 110,22;3,31 0,83;3,13 0,58;3,12 0,69;3,11 0,71;3,10 0,80;3,09 1,24;3,08 1,41;3,07 1,34;3,06 1,27;3,02 1,14;3,01 1,49;3,00 1,73;2,99 1,94;2,97 0,70;2,96 0,86;2,95 0,78;2,84 0,64;2,83 0,63;2,83 0,77;2,82 0,84;2,81 1,32;2,80 1,09;2,79 1,26;2,78 0,87;2,77 0,64;2,76 0,49;2,51 14,76;2,50 20,10;2,24 0,60;2,22 1,23;2,21 0,78;2,20 1,16;2,20 1,26;2,19 1,31;2,18 1,06;2,17 0,83;2,17 1,02;2,15 0,50;1,99 0,41;1,49 16,00;1,47 15,92;1,16 0,38;1,07 11,85;0,00 4,14 |
| Beispiel Nr. 75 [CD ₃CN] 8,21 0,50;8,21 0,51;8,20 0,50;8,20 0,51;8,10 0,43;7,30 0,50;7,30 0,54;7,29 0,52;7,29 0,55;6,96 0,54;6,96 0,59;6,96 0,44;6,96 0,45;6,95 0,61;6,95 0,85;6,95 0,87;6,94 0,74;6,94 0,41;6,93 0,66;6,93 0,42;5,45 7,73;4,14 0,46;4,13 0,83;4,12 0,49;2,76 0,44;2,75 0,86;2,74 0,47;2,43 0,36;2,42 1,14;2,40 1,17;2,39 0,38;2,17 123,39;2,06 0,44;2,06 0,43;2,05 0,34;2,05 0,52;2,05 0,35;1,97 0,72;1,97 14,22;1,96 2,29;1,95 2,60;1,95 24,64;1,95 46,24;1,94 66,64;1,94 44,02;1,93 22,38;1,93 0,57;1,86 0,38;1,85 0,36;1,84 0,35;1,83 0,42;1,20 0,39;1,20 0,40;1,13 16,00;1,12 2,98;1,11 1,38;0,00 2,77 |
| Beispiel Nr. 76 [DMSO-D₆] 10,36 0,59;8,14 0,61;8,14 0,66;8,13 0,62;8,12 0,70;8,09 0,59;7,45 0,57;7,44 0,64;7,43 0,73;7,42 0,66;7,23 0,70;7,21 1,25;7,18 0,59;6,76 0,54;6,75 0,53;6,74 0,53;6,74 0,55;3,94 2,56;3,35 55,42;2,51 4,65;2,51 6,42;2,51 4,32;2,38 1,01;2,36 1,04;2,34 0,34;2,00 0,58;1,49 1,72;1,48 1,72;1,18 0,33;1,08 16,00;1,06 1,28;1,04 2,83;1,02 1,21 |
| Beispiel Nr. 77 [DMSO-D₆] 10,39 0,32;8,13 0,37;8,12 0,73;7,43 0,35;7,20 0,55;5,76 1,66;3,96 2,81;3,35 11,07;2,51 1,15;2,51 2,51;2,50 3,45;2,50 2,49;2,50 1,13;1,48 0,85;1,47 0,85;1,12 0,36;1,11 0,35;1,07 16,00;1,06 2,11;0,00 0,42 |
| Beispiel Nr. 78 [DMSO-D₆] 10,72 0,46;8,14 0,47;8,12 0,49;8,07 0,51;7,44 0,41;7,43 0,47;7,42 0,53;7,41 0,48;7,22 0,50;7,20 0,88;7,17 0,41;6,74 0,37;6,73 0,36;6,73 0,37;6,72 0,37;3,93 0,93;3,34 6,37;3,34 9,96;3,34 8,90;2,51 1,52;2,51 2,89;2,50 3,94;2,50 2,73;2,50 1,34;1,48 1,28;1,46 1,27;1,07 16,00;0,79 1,33;0,77 1,24;0,00 0,67 |
| Beispiel Nr. 79 [DMSO-D₆] 10,23 0,80;8,05 0,76;8,05 0,74;8,04 0,79;8,04 0,77;7,87 0,54;7,26 0,65;7,25 0,74;7,24 0,76;7,23 0,69;7,04 0,73;7,03 1,35;7,01 0,64;6,64 0,63;6,64 0,61;6,64 0,62;6,63 0,63;3,93 0,49;3,93 1,05;3,92 0,51;3,19 16,00;2,76 0,50;2,75 0,99;2,74 0,47;2,35 3,21;2,35 7,13;2,35 9,90;2,35 7,10;2,34 3,17;2,10 1,14;2,09 1,16;1,58 8,13;0,30 0,75;0,30 0,77;0,29 0,35;0,29 0,35;0,29 0,73;0,28 0,75;0,01 0,78;0,01 0,81;0,00 0,75;0,00 0,81;-0,16 0,69 |
| Beispiel Nr. 80 [CD ₃CN] 10,78 0,46;10,24 0,42;8,66 7,23;8,64 7,23;8,56 0,78;8,55 0,99;8,54 0,50;8,52 0,46;8,50 1,12;8,49 1,22;8,48 1,11;8,47 1,13;8,45 1,49;8,44 1,80;7,83 0,85;7,82 1,53;7,80 0,94;7,44 8,66;7,44 5,44;7,43 5,20;7,43 8,55;7,34 1,02;7,33 1,03;7,13 0,76;7,12 0,93;7,11 1,25;7,09 1,38;7,05 0,50;7,03 1,50;7,02 1,32;6,96 1,37;6,84 6,20;6,83 6,98;6,82 1,04;6,81 0,92;6,66 6,81;6,65 6,68;6,64 1,44;6,63 0,68;6,61 0,86;6,60 0,70;6,58 0,97;6,57 0,72;6,37 0,48;6,22 0,54;5,56 0,75;5,48 0,44;5,39 2,05;5,38 4,01;5,37 2,06;5,34 0,58;5,31 0,67;4,47 0,45;4,42 1,75;4,40 2,12;4,39 2,45;4,38 2,41;4,37 1,98;4,36 0,48;4,13 0,61;4,12 1,91;4,10 1,76;4,10 1,00;4,08 2,19;4,07 2,00;4,05 1,55;4,04 1,16;2,75 0,56;2,72 0,95;2,68 0,49;2,59 0,55;2,58 0,55;2,56 0,94;2,55 1,28;2,54 1,18;2,53 1,34;2,51 1,45;2,49 1,26;2,48 1,06;2,46 0,69;2,44 0,57;2,40 0,78;2,40 0,84;2,38 0,98;2,37 1,19;2,35 1,95;2,34 1,99;2,31 4,71;2,30 7,50;2,29 7,23;2,26 949,77;2,25 2035,07;2,18 2,50;2,16 3,88;2,15 1,77;2,14 2,57;2,14 2,95;2,13 2,32;2,11 0,47;2,09 0,46;2,03 0,54;1,99 450,00;1,98 121,96;1,98 222,61;1,97 311,09;1,96 209,77;1,96 105,49;1,82 2,57;1,81 0,73;1,80 1,11;1,80 1,74;1,79 1,19;1,79 0,83;1,34 0,55;1,30 3,25;1,26 0,43;1,25 0,68;1,23 1,24;1,22 0,44;1,22 0,70;1,16 16,00;0,98 0,44;0,94 0,70;0,91 0,53;0,87 0,51 |
| Beispiel Nr. 81 [DMSO-D₆] 7,89 3,88;7,88 4,32;7,43 0,33;7,42 3,60;7,42 4,28;7,41 3,93;7,40 4,52;6,98 3,00;6,97 4,31;6,97 3,09;6,96 4,57;6,94 4,63;6,94 5,58;6,93 3,14;6,93 3,25;6,60 0,32;6,39 0,37;6,38 4,47;6,37 16,00;5,99 0,36;5,91 0,49;5,88 7,99;5,85 0,41;5,75 0,38;5,53 0,34;4,12 3,02;4,10 5,56;4,09 3,00;4,04 0,81;4,02 0,77;4,00 0,37;3,34 1573,87;3,28 0,75;3,26 0,44;3,23 0,34;2,68 3,14;2,67 6,30;2,65 2,91;2,54 0,46;2,52 1,95;2,51 157,17;2,50 217,14;2,50 157,72;2,44 0,33;2,33 1,30;2,07 1,41;2,01 2,29;2,00 2,89;1,99 5,53;1,82 1,08;1,80 2,52;1,79 2,74;1,77 2,02;1,58 0,71;1,40 0,36;1,19 0,88;1,17 1,69;1,16 0,88;0,89 0,36;0,00 7,16 |
| Beispiel Nr. 82 [DMSO-D₆] 9,95 1,49;8,23 1,63;8,22 1,70;7,95 1,50;7,42 1,19;7,40 1,36;7,40 1,64;7,39 0,60;7,38 1,45;7,20 1,50;7,18 2,71;7,16 1,26;6,87 1,20;6,87 1,22;6,86 1,20;6,86 1,23;4,09 0,94;4,08 2,08;4,06 1,32;4,03 5,64;3,37 1,07;3,34 65,63;3,34 63,22;3,33 84,69;3,33 101,81;2,93 0,98;2,91 1,98;2,90 0,92;2,52 0,45;2,51 31,47;2,50 42,95;2,50 30,28;1,73 16,00;1,40 4,84;1,07 1,52;0,00 1,73 |
| Beispiel Nr. 83 [DMSO-D₆] 10,41 1,81;8,20 1,62;8,19 1,66;8,02 2,10;7,42 1,21;7,40 1,39;7,40 1,75;7,38 1,52;7,20 1,48;7,18 2,68;7,15 1,26;6,78 1,11;6,77 1,28;6,76 1,11;6,76 1,29;4,09 0,99;4,08 2,18;4,07 1,07;3,32 81,59;2,92 1,07;2,90 2,13;2,89 1,02;2,75 0,64;2,73 0,86;2,71 0,65;2,51 22,25;2,50 30,78;2,50 24,82;2,07 0,35;1,73 16,00;1,09 0,36;1,07 9,66;1,05 9,53;0,00 5,33 |
| Beispiel Nr. 84 [DMSO-D₆] 10,78 1,74;8,21 1,64;8,20 1,70;7,98 1,96;7,41 1,28;7,40 0,55;7,40 1,55;7,39 1,63;7,37 1,46;7,20 1,58;7,17 2,75;7,15 1,24;6,79 1,29;6,78 1,13;6,77 1,25;6,77 1,11;5,76 0,71;4,08 0,95;4,07 2,10;4,05 0,99;3,32 51,77;2,90 0,99;2,88 2,00;2,87 0,94;2,52 0,35;2,51 23,23;2,50 29,15;2,50 20,09;2,00 0,49;1,99 0,75;1,97 0,49;1,72 16,00;1,40 2,00;0,79 2,23;0,78 2,32;0,77 4,47;0,00 5,87 |
| Beispiel Nr. 85 [CD ₃CN] 18,32 0,43;17,36 0,42;12,09 0,40;9,25 0,43;8,50 3,39;8,49 2,77;8,49 3,49;8,47 0,53;8,45 0,59;7,40 1,90;7,39 2,19;7,38 2,41;7,37 1,97;7,23 3,83;7,22 2,82;7,22 2,77;7,21 3,70;7,15 0,43;7,12 0,71;7,11 0,69;7,08 2,43;7,05 3,86;7,03 2,00;5,45 1,04;4,38 1,72;4,34 0,70;4,30 0,73;4,18 0,41;4,17 0,45;4,07 0,67;4,05 0,62;4,03 0,84;4,03 0,95;4,01 0,51;3,99 0,59;3,81 0,41;3,45 0,41;3,28 0,52;3,24 16,00;2,81 0,57;2,60 3,11;2,47 0,43;2,41 0,41;2,39 0,42;2,29 1,62;2,27 2,00;2,25 2,03;2,18 1032,82;2,12 0,98;2,11 1,48;2,11 1,68;2,10 1,14;2,08 0,49;2,06 0,46;2,05 0,49;1,96 24,10;1,95 101,96;1,95 188,93;1,94 255,59;1,93 181,67;1,93 92,41;1,88 0,44;1,86 0,49;1,86 0,42;1,79 0,62;1,78 1,09;1,77 1,92;1,76 1,72;1,72 0,76;1,69 0,69;1,69 0,57;1,66 0,41;1,57 0,41;1,56 0,51;1,55 0,50;1,54 0,60;1,51 0,50;1,41 0,47;1,37 0,50;1,36 0,78;1,34 0,87;1,27 12,86;1,21 0,80;1,18 0,53;1,16 0,50;1,14 0,78;1,11 0,46;1,09 0,51;1,08 0,40;0,93 0,41;0,92 0,73;0,90 1,27;0,88 1,91;0,86 1,56;0,82 0,51;0,81 0,51;0,00 3,40 |
| Beispiel Nr. 86 [DMSO-D₆] 10,41 1,53;8,20 1,66;8,19 1,69;8,00 1,58;7,42 1,29;7,40 1,40;7,39 1,72;7,39 0,62;7,38 1,46;7,20 1,71;7,18 2,86;7,15 1,37;6,79 1,12;6,79 1,32;6,78 1,24;6,78 1,22;5,76 0,61;4,09 1,02;4,08 1,99;4,06 1,04;4,04 1,21;4,02 1,19;4,00 0,38;3,93 0,53;3,38 0,38;3,36 0,74;3,33 1004,40;3,30 3,22;3,28 0,49;3,27 0,42;2,91 0,90;2,90 1,81;2,88 0,90;2,68 1,17;2,67 1,62;2,67 1,21;2,54 0,84;2,52 2,75;2,52 3,94;2,51 95,26;2,51 187,51;2,50 261,60;2,50 181,42;2,49 89,34;2,39 0,76;2,38 2,58;2,36 2,50;2,34 1,29;2,33 1,34;2,33 1,70;2,32 1,15;2,07 2,93;1,99 5,51;1,73 16,00;1,71 0,52;1,40 0,44;1,19 1,42;1,17 3,01;1,16 1,33;1,07 2,92;1,05 3,01;1,03 6,50;1,01 2,68;0,86 4,57;0,00 5,61 |
| Beispiel Nr. 87 [CD ₃CN] 8,67 2,40;8,21 9,20;8,21 9,26;8,21 9,38;8,20 9,13;8,08 4,62;7,30 8,59;7,30 9,02;7,29 8,95;7,29 9,32;7,29 0,48;7,28 0,35;7,28 0,35;6,97 9,37;6,97 9,17;6,96 9,32;6,96 9,34;6,96 6,59;6,95 7,46;6,95 12,63;6,95 11,85;6,94 12,62;6,93 6,71;6,93 11,57;6,93 6,58;6,43 0,40;5,45 4,58;4,14 8,54;4,13 15,44;4,12 8,96;4,11 0,39;2,75 8,14;2,74 16,00;2,73 8,72;2,72 0,46;2,71 0,59;2,21 0,48;2,20 0,38;2,19 0,67;2,16 598,75;2,14 1,14;2,13 0,63;2,13 0,42;2,11 79,56;2,08 2,44;2,08 1,88;2,07 5,10;2,07 4,65;2,06 8,52;2,06 3,96;2,06 5,02;2,05 5,54;2,05 6,21;2,04 2,64;2,04 2,94;2,00 0,40;1,97 1,57;1,97 17,47;1,96 11,11;1,95 15,22;1,95 144,24;1,95 260,73;1,94 388,20;1,94 269,48;1,93 134,42;1,93 4,26;1,93 2,06;1,92 0,65;1,86 2,84;1,86 1,84;1,85 7,15;1,85 4,38;1,84 6,97;1,84 4,29;1,83 6,51;1,83 3,64;1,82 3,80;1,82 1,08;1,28 0,59;1,27 2,87;1,22 0,42;1,20 0,74;1,19 0,40;0,88 0,55;0,87 0,34;0,01 1,64;0,00 50,02;-0,01 1,37 |
| Beispiel Nr. 88 [CD ₃CN] 8,59 0,45;8,21 1,46;8,21 1,54;8,20 1,52;8,20 1,53;8,11 1,54;8,11 1,55;7,30 1,40;7,30 1,54;7,30 1,57;7,29 1,58;6,97 1,58;6,96 1,56;6,96 1,60;6,96 2,05;6,95 1,34;6,95 2,26;6,95 2,07;6,94 2,25;6,94 1,09;6,93 2,03;6,93 1,11;5,45 12,41;4,14 1,33;4,13 2,39;4,12 1,37;3,28 1,31;3,27 1,31;2,76 1,26;2,75 2,48;2,74 1,35;2,66 0,78;2,65 1,07;2,63 0,81;2,62 0,32;2,17 110,60;2,08 0,40;2,07 0,80;2,07 0,74;2,07 1,25;2,06 0,69;2,06 0,79;2,06 1,06;2,06 0,99;2,05 1,21;2,05 0,81;2,04 0,71;1,97 0,43;1,97 7,43;1,96 3,90;1,95 5,42;1,95 49,59;1,95 95,69;1,94 142,35;1,94 95,75;1,93 46,25;1,93 1,58;1,93 0,81;1,92 0,39;1,87 0,49;1,87 0,32;1,86 1,11;1,86 0,69;1,85 1,05;1,85 0,65;1,84 0,97;1,84 0,52;1,83 0,71;1,83 0,87;1,82 0,58;1,44 10,41;1,27 1,05;1,16 16,00;1,15 0,63;1,14 15,64;1,11 0,41;0,01 0,64;0,00 19,73;-0,01 0,54 |
| Beispiel Nr. 89 [CD ₃CN] 8,91 3,39;8,22 9,14;8,22 9,10;8,21 9,33;8,21 9,05;8,08 10,05;7,30 8,23;7,29 8,04;7,29 8,42;7,29 8,46;6,96 8,77;6,96 8,85;6,95 9,07;6,95 12,85;6,95 7,54;6,94 12,50;6,94 11,34;6,94 12,05;6,93 6,06;6,93 10,26;6,92 5,85;5,45 14,46;4,13 8,67;4,12 15,55;4,11 8,69;2,74 8,22;2,73 16,00;2,72 8,58;2,30 0,35;2,17 733,24;2,14 1,75;2,12 0,52;2,07 2,55;2,07 2,23;2,06 5,54;2,06 5,23;2,05 8,79;2,05 6,31;2,05 6,79;2,04 2,15;2,03 2,85;1,97 48,89;1,96 14,99;1,95 20,59;1,95 148,87;1,95 268,38;1,94 374,42;1,94 263,39;1,93 136,32;1,93 2,36;1,92 1,21;1,92 1,06;1,91 0,88;1,91 0,69;1,90 0,46;1,89 0,46;1,88 0,36;1,86 2,88;1,85 2,17;1,85 7,14;1,84 4,76;1,84 7,57;1,83 5,88;1,83 8,22;1,82 3,28;1,82 2,04;1,82 2,67;1,81 2,79;1,80 2,99;1,80 2,08;1,79 5,00;1,79 3,14;1,78 2,80;1,77 1,41;1,44 1,74;1,28 0,80;1,27 4,07;1,16 0,35;1,14 0,33;1,06 1,45;1,05 1,34;0,89 2,75;0,89 2,48;0,88 7,97;0,88 12,18;0,88 8,02;0,88 6,93;0,87 10,69;0,87 4,10;0,87 4,76;0,86 1,63;0,85 2,17;0,85 4,92;0,84 10,80;0,84 6,31;0,83 5,69;0,83 11,40;0,82 5,69;0,82 2,53;0,82 2,22;0,01 1,88;0,00 46,53;-0,01 1,45 |
| Beispiel Nr. 90 [DMSO-D₆] 10,49 1,22;8,21 1,56;8,21 1,61;7,96 0,72;7,41 1,30;7,40 0,59;7,40 1,54;7,39 1,50;7,39 0,70;7,38 1,34;7,20 1,47;7,19 0,51;7,18 2,64;7,17 0,54;7,17 1,23;6,82 1,23;6,82 1,13;6,81 1,21;6,81 1,10;5,77 4,47;4,08 1,10;4,07 2,25;4,06 1,10;4,03 0,47;4,02 0,47;3,39 0,37;3,35 38,82;3,33 0,66;2,90 1,09;2,89 2,14;2,88 1,03;2,51 12,38;2,50 16,01;2,50 11,43;2,09 0,94;2,05 7,29;1,99 2,01;1,76 0,40;1,73 16,00;1,48 0,32;1,19 0,55;1,17 1,08;1,16 0,54;0,00 5,33 |
| Beispiel Nr. 91 [DMSO-D₆] 10,46 2,46;8,21 2,38;8,21 2,35;8,20 2,49;8,20 2,44;8,05 2,44;7,42 2,02;7,41 0,89;7,41 2,31;7,40 2,39;7,40 1,02;7,39 2,19;7,20 2,30;7,20 0,79;7,19 0,99;7,19 4,25;7,18 0,97;7,18 0,78;7,17 2,03;6,78 1,96;6,78 1,90;6,78 1,93;6,77 1,92;5,61 7,16;4,13 1,64;4,12 3,53;4,11 1,69;4,05 0,66;4,03 1,98;4,02 2,01;4,01 0,67;3,39 0,73;3,35 19,15;3,33 0,55;2,98 1,50;2,97 2,97;2,96 1,43;2,76 0,33;2,75 0,82;2,74 1,11;2,72 0,83;2,71 0,33;2,52 0,33;2,52 0,35;2,51 8,34;2,51 17,69;2,50 24,06;2,50 17,58;2,50 8,24;1,99 8,76;1,91 0,81;1,40 0,47;1,19 2,41;1,17 4,69;1,16 2,38;1,12 1,45;1,11 1,44;1,06 16,00;1,05 15,89;0,00 6,78 |
| Beispiel Nr. 92 [CD ₃CN] 8,56 1,50;8,38 0,47;8,37 0,48;8,13 2,13;8,12 2,14;7,95 2,64;7,46 1,92;7,46 0,87;7,46 2,19;7,45 2,17;7,44 0,97;7,44 1,94;7,38 0,38;7,38 0,35;7,38 0,37;7,37 0,33;7,09 2,05;7,09 0,71;7,08 3,83;7,07 0,87;7,06 1,82;6,75 1,72;6,75 1,62;6,74 1,71;6,74 1,55;4,88 8,24;4,21 1,04;4,20 2,67;4,19 2,73;4,17 2,85;4,16 2,88;4,15 1,12;2,65 0,39;2,64 1,01;2,63 1,35;2,61 1,02;2,60 0,41;2,16 55,53;1,97 0,92;1,97 2,31;1,96 1,61;1,95 14,67;1,95 25,87;1,94 36,51;1,94 25,07;1,93 12,62;1,20 3,58;1,19 3,51;1,14 16,00;1,13 15,75;0,00 1,38 |
| Beispiel Nr. 93 [CD ₃CN] 8,90 1,86;8,56 0,37;8,17 4,01;8,15 4,09;7,94 5,40;7,92 0,48;7,89 0,48;7,49 3,82;7,49 1,74;7,48 4,32;7,47 4,68;7,46 2,09;7,46 4,31;7,25 0,33;7,22 0,68;7,20 0,37;7,12 4,26;7,10 7,95;7,07 3,80;6,78 3,58;6,78 3,49;6,77 3,53;6,76 3,47;4,88 16,00;4,78 1,63;4,23 1,66;4,22 4,51;4,21 6,00;4,19 6,12;4,18 4,37;4,17 5,30;4,16 2,29;4,15 0,93;4,14 0,54;4,12 0,79;4,10 2,19;4,08 2,21;4,06 0,77;2,50 0,52;2,48 0,53;2,31 0,33;2,27 0,33;2,25 0,52;2,19 929,12;2,15 0,91;2,14 1,08;2,14 1,41;2,13 0,86;2,00 10,09;1,99 10,55;1,98 80,75;1,98 150,71;1,97 207,03;1,96 142,18;1,96 72,27;1,84 0,56;1,82 1,27;1,82 1,45;1,80 2,81;1,80 1,80;1,79 2,21;1,78 1,52;1,77 0,69;1,47 2,07;1,37 0,36;1,30 1,25;1,25 2,61;1,23 5,20;1,22 2,74;1,20 1,07;1,18 0,53;1,17 0,67;0,94 0,53;0,92 0,75;0,91 2,85;0,90 5,74;0,90 4,91;0,89 4,64;0,88 3,17;0,87 5,35;0,86 2,66;0,86 3,23;0,85 4,72;0,84 2,34;0,83 0,76 |
| Beispiel Nr. 94 [DMSO-D₆] 9,97 4,26;8,24 4,07;8,23 4,20;7,98 3,81;7,43 0,37;7,42 3,59;7,42 1,48;7,41 4,07;7,40 4,53;7,39 1,69;7,39 4,24;7,38 0,55;7,21 0,53;7,21 4,35;7,20 1,36;7,19 1,49;7,18 7,75;7,18 1,52;7,17 1,17;7,16 3,53;7,15 0,39;6,89 3,28;6,88 3,23;6,87 3,22;6,87 3,24;5,61 12,18;4,13 2,51;4,12 5,71;4,10 2,63;4,03 16,00;3,34 42,63;3,32 250,73;3,30 0,69;3,00 2,35;2,98 4,86;2,97 2,24;2,67 0,59;2,67 0,43;2,52 1,00;2,51 33,04;2,51 62,61;2,50 85,72;2,50 59,64;2,49 29,53;2,33 0,37;2,33 0,53;2,32 0,35;2,07 1,55;1,99 0,34;1,91 0,44;1,87 0,56;1,07 0,36;0,96 0,64;0,01 0,40;0,00 13,78;-0,01 0,38 |
| Beispiel Nr. 95 [DMSO-D₆] 10,42 2,84;8,21 2,90;8,21 2,86;8,20 3,02;8,20 3,01;8,03 2,86;7,42 2,54;7,42 1,00;7,41 2,90;7,40 3,25;7,39 1,16;7,39 2,96;7,38 0,36;7,20 3,14;7,20 0,93;7,19 1,01;7,18 5,61;7,18 1,06;7,16 0,86;7,16 2,56;6,81 2,42;6,80 2,38;6,79 2,39;6,79 2,43;5,61 8,42;4,13 1,69;4,11 3,95;4,10 1,79;4,06 0,78;4,04 2,39;4,02 2,41;4,00 0,80;3,93 2,78;3,33 126,38;2,98 1,62;2,97 3,35;2,95 1,51;2,52 0,40;2,51 9,40;2,51 18,18;2,50 25,24;2,50 17,35;2,49 8,54;2,40 1,35;2,38 4,63;2,36 4,76;2,34 1,49;2,07 0,63;1,99 10,81;1,91 0,45;1,19 3,00;1,17 6,01;1,16 2,94;1,07 16,00;1,05 5,63;1,03 12,51;1,01 5,45;0,00 0,89 |
| Beispiel Nr. 96 [DMSO-D₆] 10,48 2,56;8,24 2,60;8,24 2,54;8,23 2,68;8,23 2,65;7,95 1,94;7,34 2,22;7,33 0,88;7,33 2,49;7,32 2,64;7,32 1,03;7,31 2,43;7,14 2,52;7,14 0,78;7,13 0,94;7,13 4,65;7,13 0,90;7,12 0,76;7,11 2,18;6,76 2,19;6,76 2,12;6,75 2,17;6,75 2,16;4,35 1,64;4,34 1,45;4,33 1,65;3,96 1,90;3,36 141,41;3,34 0,62;2,73 1,46;2,73 1,32;2,72 1,51;2,52 0,33;2,51 9,66;2,51 21,18;2,50 29,04;2,50 20,95;2,50 9,52;2,40 1,22;2,39 0,37;2,38 3,83;2,37 3,84;2,36 1,23;1,99 0,53;1,84 1,22;1,83 1,06;1,75 1,19;1,63 0,91;1,62 1,12;1,40 6,79;1,07 16,00;1,04 5,31;1,03 11,30;1,025,23 |
| Beispiel Nr. 97 [DMSO-D₆] 10,47 2,11;8,24 2,00;8,23 2,04;8,23 1,90;7,96 2,00;7,34 1,66;7,34 0,74;7,33 1,90;7,33 1,94;7,32 0,81;7,32 1,74;7,14 1,96;7,14 0,67;7,13 0,81;7,13 3,50;7,13 0,75;7,12 0,62;7,11 1,64;6,76 1,62;6,76 1,55;6,75 1,58;6,75 1,56;4,35 1,33;4,34 1,18;4,33 1,28;3,96 2,28;3,38 0,37;3,35 68,69;3,33 0,53;2,76 0,36;2,75 0,87;2,74 1,90;2,73 1,66;2,72 1,32;2,71 0,48;2,53 0,48;2,52 0,57;2,52 0,64;2,51 6,76;2,51 14,02;2,50 18,81;2,50 13,44;2,50 6,20;1,84 1,02;1,83 0,89;1,75 0,99;1,62 0,93;1,40 3,72;1,09 0,35;1,07 16,00;1,07 14,01;1,05 12,94;0,00 1,82 |
| Beispiel Nr. 98 [DMSO-D₆] 10,78 2,37;8,26 2,53;8,26 2,44;8,25 2,62;8,25 2,55;7,92 1,83;7,33 0,85;7,33 0,78;7,32 16,00;7,32 5,70;7,31 7,18;7,31 2,92;7,30 1,40;7,30 2,88;7,29 0,33;7,25 0,72;7,25 0,93;7,24 1,02;7,24 1,13;7,23 0,80;7,23 0,65;7,22 0,32;7,13 0,34;7,13 2,57;7,12 0,84;7,12 1,04;7,11 4,65;7,11 0,94;7,10 0,83;7,10 2,17;6,77 2,17;6,77 2,07;6,77 2,14;6,76 2,13;5,77 11,89;4,33 1,63;4,32 1,46;4,32 1,59;4,03 0,81;4,02 0,82;3,70 6,48;3,57 0,97;3,36 45,81;2,72 1,46;2,71 1,33;2,70 1,44;2,53 0,35;2,52 0,45;2,52 0,53;2,51 6,82;2,51 14,16;2,50 19,07;2,50 13,75;2,50 6,17;1,99 3,67;1,82 1,22;1,81 1,06;1,73 1,20;1,59 1,14;1,19 0,98;1,17 2,00;1,16 0,97;1,07 1,30;0,00 3,09 |
| Beispiel Nr. 99 [DMSO-D₆] 10,50 1,50;8,24 1,69;8,23 1,71;8,23 1,72;7,92 1,25;7,34 1,48;7,34 0,65;7,33 1,70;7,32 1,97;7,31 0,70;7,31 1,80;7,14 1,85;7,14 0,60;7,13 0,64;7,12 3,28;7,12 0,71;7,10 0,52;7,10 1,52;6,77 1,42;6,77 1,39;6,76 1,40;6,76 1,44;4,35 1,05;4,34 0,96;4,33 1,04;4,06 1,13;4,04 3,57;4,02 3,64;4,00 1,21;3,33 60,54;3,32 111,85;2,73 0,95;2,71 1,01;2,52 0,47;2,51 30,09;2,50 41,12;2,50 28,24;2,06 10,48;1,99 16,00;1,91 0,49;1,84 0,80;1,75 1,66;1,62 0,82;1,36 0,78;1,19 4,25;1,17 8,53;1,16 4,17;0,00 1,16 |
| Beispiel Nr. 100 [DMSO-D₆] 10,42 3,63;8,18 3,78;8,17 3,79;8,16 3,92;8,16 3,96;7,92 3,77;7,41 2,07;7,40 2,09;7,40 1,94;7,39 1,43;7,39 2,12;7,39 5,47;7,38 5,79;7,37 1,31;7,36 2,14;7,36 1,25;7,35 4,99;7,35 2,26;7,34 2,59;7,34 5,94;7,33 5,57;7,32 1,35;7,32 0,88;6,73 3,10;6,73 3,06;6,72 3,07;6,71 3,13;4,87 10,04;4,23 1,22;4,22 3,22;4,21 3,13;4,17 3,20;4,16 3,49;4,15 1,27;4,06 0,39;4,04 1,20;4,02 1,21;4,00 0,42;3,93 1,21;3,34 44,41;3,34 47,95;3,33 89,77;2,52 0,48;2,51 20,91;2,50 28,27;2,40 1,77;2,38 6,05;2,36 6,24;2,34 1,95;1,99 5,34;1,19 1,45;1,17 2,92;1,16 1,40;1,11 0,32;1,09 0,73;1,07 9,81;1,06 7,28;1,04 16,00;1,02 6,97;0,00 0,79 |
| Beispiel Nr. 101 [DMSO-D₆] 10,69 1,52;10,64 0,61;8,21 0,62;8,20 1,49;8,20 1,49;8,20 0,78;8,20 0,72;8,19 1,52;8,19 1,49;7,97 2,12;7,41 1,69;7,40 0,70;7,39 1,94;7,39 2,11;7,38 0,79;7,37 1,88;7,19 1,61;7,19 0,60;7,19 0,80;7,18 0,60;7,17 2,87;7,16 1,31;7,16 0,52;7,15 1,33;7,14 0,59;6,82 0,50;6,81 0,47;6,80 0,48;6,80 0,48;6,78 1,22;6,77 1,19;6,77 1,19;6,76 1,24;5,76 8,22;4,08 1,03;4,07 1,08;4,07 1,95;4,06 0,85;4,05 0,89;4,04 0,85;4,02 0,81;3,33 37,17;3,33 70,41;3,30 0,54;2,91 0,48;2,90 0,53;2,89 0,93;2,87 1,73;2,86 0,82;2,52 0,41;2,51 16,66;2,50 22,81;2,50 15,48;1,99 3,69;1,76 0,61;1,75 0,69;1,73 8,51;1,72 16,00;1,24 0,41;1,23 0,47;1,22 0,54;1,21 0,55;1,20 0,53;1,20 0,61;1,19 1,46;1,18 0,48;1,18 2,27;1,17 0,34;1,16 0,36;1,16 1,09;1,09 0,71;1,08 4,37;1,06 3,62;1,05 2,38;1,04 1,77;1,03 0,87;1,02 0,36;0,99 0,42;0,98 0,59;0,97 0,71;0,96 0,56;0,95 0,41;0,66 0,35;0,65 0,40;0,64 0,39;0,63 0,51;0,63 0,47;0,63 0,40;0,62 0,38;0,00 5,15 |
| Beispiel Nr. 102 [DMSO-D₆] 10,29 1,60;8,19 1,50;8,19 1,51;8,18 1,53;8,18 1,56;8,03 1,33;7,42 1,35;7,41 0,56;7,41 1,55;7,40 1,71;7,39 0,64;7,38 1,53;7,20 1,65;7,20 0,53;7,18 0,59;7,18 2,91;7,17 0,59;7,16 0,46;7,16 1,35;6,78 1,25;6,78 1,23;6,77 1,23;6,77 1,27;5,76 0,49;4,10 0,87;4,08 1,95;4,07 0,91;3,36 0,55;3,34 1,05;3,33 44,43;3,30 0,46;2,92 0,91;2,91 1,84;2,90 0,86;2,50 15,50;2,20 0,55;2,19 0,50;2,17 0,79;2,17 1,00;2,15 0,63;2,15 0,91;2,13 0,45;2,11 0,33;2,11 0,56;2,09 0,68;2,08 0,95;2,07 0,78;2,07 0,61;2,06 0,76;1,99 0,33;1,91 0,49;1,89 0,68;1,87 0,33;1,78 0,47;1,77 0,34;1,77 0,34;1,76 0,43;1,73 16,00;0,00 3,15 |
| Beispiel Nr. 103 [DMSO-D₆] 7,98 0,34;7,97 0,34;7,85 5,61;7,84 1,67;7,84 1,71;7,83 5,91;7,45 0,45;7,44 4,48;7,44 1,84;7,43 5,08;7,42 5,87;7,41 2,08;7,41 5,39;7,40 0,73;7,21 0,67;7,20 5,45;7,20 1,72;7,19 1,86;7,18 9,84;7,18 2,00;7,16 1,51;7,16 4,62;7,15 0,46;6,64 0,42;6,29 2,62;6,28 5,68;6,28 6,58;6,27 13,56;6,04 0,46;5,85 8,08;5,76 1,39;5,58 16,00;4,10 3,26;4,09 7,56;4,07 3,42;3,93 0,53;3,57 1,58;3,32 103,95;3,30 1,31;2,94 3,07;2,92 6,41;2,91 2,91;2,67 0,35;2,52 0,59;2,51 39,53;2,50 53,86;2,50 37,13;2,33 0,37;2,07 0,43;1,07 2,95;0,00 2,93 |
| Beispiel Nr. 104 [CD ₃CN] 8,58 1,44;8,56 0,60;8,16 3,63;8,15 3,72;7,96 4,63;7,50 3,50;7,49 1,49;7,49 3,97;7,48 4,16;7,47 1,78;7,46 3,83;7,13 3,89;7,10 7,32;7,09 1,44;7,08 3,43;6,78 3,31;6,78 3,16;6,77 3,26;6,77 3,08;4,90 14,71;4,24 1,43;4,23 4,05;4,22 5,38;4,20 5,60;4,19 3,76;4,18 4,54;4,17 1,66;4,12 0,51;4,10 1,66;4,08 1,64;4,06 0,56;2,50 0,43;2,48 0,48;2,45 2,32;2,43 7,17;2,41 7,38;2,39 2,50;2,19 450,42;2,17 0,90;2,16 0,47;2,15 0,37;2,14 0,56;2,14 0,58;2,13 0,38;2,00 7,36;1,99 4,85;1,98 38,06;1,98 71,36;1,97 98,93;1,96 67,61;1,96 34,11;1,80 0,38;1,80 0,57;1,79 0,36;1,47 0,57;1,37 0,40;1,30 0,57;1,25 1,88;1,23 3,77;1,22 2,02;1,20 0,97;1,18 0,50;1,16 7,95;1,14 16,00;1,12 7,43 |
| Beispiel Nr. 105 [DMSO-D₆] 10,39 1,59;8,21 1,54;8,21 1,53;8,20 1,60;8,20 1,59;7,98 1,54;7,41 1,31;7,41 0,53;7,40 1,55;7,39 1,69;7,38 0,62;7,38 1,53;7,19 1,63;7,19 0,51;7,17 0,58;7,17 2,90;7,16 0,57;7,15 0,44;7,15 1,32;6,82 1,23;6,82 1,22;6,81 1,24;6,81 1,25;4,09 0,87;4,08 1,94;4,06 0,92;3,32 71,04;3,30 1,70;2,92 0,91;2,91 1,86;2,90 0,88;2,89 1,45;2,73 0,97;2,52 0,55;2,52 0,83;2,51 18,44;2,51 35,30;2,50 48,54;2,50 33,69;2,49 16,77;2,24 2,35;2,22 2,85;2,07 0,65;2,07 0,36;2,04 0,51;2,02 0,64;2,00 0,50;1,73 16,00;1,40 3,01;0,90 1,42;0,90 11,40;0,89 1,97;0,88 11,08;0,86 0,37;0,01 0,41;0,00 13,55;-0,01 0,41 |
| Beispiel Nr. 106 [DMSO-D₆] 10,47 2,01;8,22 2,45;8,22 2,58;8,21 2,55;8,21 2,67;7,98 1,71;7,42 2,27;7,41 0,90;7,40 2,59;7,40 2,96;7,39 1,05;7,38 2,67;7,21 0,33;7,20 2,82;7,20 0,89;7,18 0,92;7,18 5,07;7,17 0,97;7,16 0,76;7,16 2,31;6,84 2,14;6,83 2,11;6,83 2,11;6,82 2,15;5,76 0,91;5,61 7,37;4,12 1,53;4,11 3,50;4,09 1,56;3,34 47,42;3,33 44,00;3,33 52,21;3,32 49,40;3,30 0,61;2,97 1,42;2,96 2,95;2,95 1,37;2,52 0,49;2,52 0,70;2,51 15,64;2,51 28,39;2,50 39,07;2,50 26,13;2,07 0,35;2,05 16,00;0,01 0,46;0,00 16,26;-0,01 0,48 |
| Beispiel Nr. 107 [DMSO-D₆] 10,78 3,48;8,22 3,72;8,22 3,74;8,21 3,85;8,20 3,83;8,01 3,86;7,42 0,40;7,41 3,36;7,41 1,38;7,40 3,88;7,39 4,20;7,38 1,52;7,38 3,81;7,37 0,44;7,21 0,48;7,20 4,15;7,20 1,26;7,18 1,41;7,18 7,38;7,17 1,36;7,16 1,15;7,16 3,36;6,80 3,22;6,79 3,12;6,78 3,18;6,78 3,20;5,76 16,00;5,60 10,42;4,12 2,14;4,10 4,91;4,09 2,24;3,34 50,57;3,33 51,49;3,33 57,39;3,33 66,01;3,30 0,71;3,18 0,34;2,96 2,03;2,95 4,15;2,93 1,90;2,52 0,57;2,52 0,84;2,51 18,33;2,51 32,88;2,50 45,30;2,50 29,95;2,49 15,00;2,33 0,32;2,02 0,35;2,01 1,10;1,99 1,74;1,98 1,10;1,96 0,38;1,21 0,32;1,17 0,51;0,79 6,19;0,77 10,98;0,63 0,39;0,63 0,43;0,62 0,35;0,01 0,48;0,00 18,65;-0,01 0,57 |
| Beispiel Nr. 108 [DMSO-D₆] 10,73 3,94;10,68 1,45;8,22 1,61;8,22 1,60;8,21 4,05;8,20 3,86;8,00 4,82;7,41 0,52;7,41 4,10;7,40 1,94;7,40 4,85;7,39 5,05;7,39 2,22;7,38 4,37;7,38 0,62;7,20 0,49;7,20 3,58;7,19 1,37;7,19 2,37;7,18 6,74;7,18 1,79;7,17 3,11;7,17 3,42;7,16 0,72;7,16 1,27;6,83 1,20;6,83 1,17;6,82 1,19;6,82 1,18;6,79 2,99;6,79 2,92;6,78 2,98;6,78 2,98;5,77 16,00;5,60 11,84;5,59 0,42;4,12 0,79;4,11 3,46;4,10 5,95;4,09 2,70;3,35 80,58;3,33 0,60;2,99 0,53;2,98 1,15;2,97 1,19;2,96 0,65;2,95 2,42;2,94 4,69;2,93 2,26;2,52 0,34;2,51 10,03;2,51 21,21;2,51 28,69;2,50 21,16;2,50 10,09;2,01 0,37;2,00 0,51;1,99 0,65;1,99 0,37;1,76 0,70;1,76 1,31;1,75 1,37;1,74 1,30;1,74 0,75;1,25 0,45;1,25 0,60;1,24 0,80;1,23 0,79;1,23 0,88;1,22 0,86;1,22 0,88;1,21 1,25;1,21 1,18;1,20 1,31;1,20 1,23;1,19 1,11;1,19 0,86;1,18 0,76;1,18 0,35;1,10 0,59;1,09 0,56;1,08 0,42;1,07 11,46;1,06 10,13;1,05 5,88;1,04 5,04;1,04 0,41;1,03 0,48;1,03 2,32;1,02 0,89;0,99 0,99;0,98 1,49;0,97 1,79;0,96 1,40;0,96 0,95;0,93 0,40;0,93 0,49;0,92 0,71;0,91 0,69;0,91 0,52;0,90 0,35;0,82 0,34;0,75 0,38;0,74 0,58;0,74 0,54;0,74 0,50;0,73 0,52;0,73 0,51;0,73 0,34;0,65 0,93;0,65 1,05;0,64 1,16;0,64 1,39;0,64 1,30;0,63 1,05;0,63 1,10;0,62 0,78;0,00 5,93 |
| Beispiel Nr. 109 [DMSO-D₆] 10,33 5,34;8,20 5,37;8,19 5,33;8,19 5,42;8,06 3,12;7,42 0,40;7,42 0,82;7,42 4,50;7,41 2,07;7,41 5,13;7,40 5,43;7,40 2,23;7,39 4,85;7,39 0,61;7,21 0,68;7,20 5,15;7,20 1,88;7,19 2,14;7,19 9,54;7,18 1,67;7,17 4,49;7,17 0,52;6,79 4,34;6,79 4,34;6,78 4,32;6,78 4,35;5,62 16,00;4,13 3,67;4,12 7,87;4,11 3,71;3,58 0,37;3,37 0,76;3,35 338,69;3,33 1,44;3,33 2,71;2,99 3,38;2,98 6,68;2,97 3,15;2,95 0,62;2,95 0,68;2,94 0,46;2,93 0,46;2,62 0,55;2,62 0,76;2,61 0,56;2,52 1,45;2,52 1,93;2,52 2,31;2,51 41,63;2,51 87,11;2,50 117,52;2,50 85,88;2,50 39,66;2,39 0,47;2,39 0,67;2,38 0,47;2,20 0,73;2,20 0,46;2,19 2,33;2,18 1,90;2,17 2,94;2,17 3,52;2,16 2,17;2,15 3,09;2,14 0,73;2,14 1,04;2,10 0,57;2,09 1,04;2,09 1,34;2,08 1,37;2,08 3,15;2,07 3,16;2,07 2,22;2,07 2,30;2,06 2,91;2,06 1,78;2,05 0,84;2,05 0,87;2,04 0,88;2,04 0,72;2,00 0,42;1,99 0,61;1,98 0,35;1,98 0,54;1,94 0,49;1,93 1,25;1,93 0,85;1,91 1,89;1,91 1,80;1,90 1,21;1,90 2,26;1,88 1,25;1,87 0,41;1,86 0,36;1,86 0,35;1,84 0,48;1,80 0,41;1,79 0,69;1,78 0,85;1,78 1,44;1,77 0,99;1,77 0,91;1,77 0,88;1,76 1,21;1,75 0,72;1,74 0,47;1,74 0,35;1,73 0,32;1,49 0,50;1,31 0,53;1,23 0,38;1,08 0,34;1,07 0,39;0,01 0,98;0,00 25,43;-0,01 0,78 |
| Beispiel Nr. 110 [DMSO-D₆] 10,40 1,02;8,16 0,99;8,16 1,04;8,15 1,03;8,15 1,07;8,09 0,98;7,40 0,88;7,40 0,35;7,39 1,01;7,38 1,15;7,37 0,41;7,37 1,04;7,20 1,09;7,18 0,35;7,17 1,95;7,17 0,38;7,15 0,90;6,76 0,85;6,76 0,83;6,75 0,84;6,75 0,84;4,04 0,84;4,02 0,85;3,94 2,79;3,34 31,11;2,51 4,74;2,50 6,50;2,50 4,43;2,40 0,42;2,38 1,45;2,36 1,59;2,35 0,53;1,99 3,78;1,40 0,53;1,19 1,01;1,18 2,06;1,16 3,63;1,14 2,65;1,07 16,00;1,06 2,01;1,04 4,34;1,02 1,90 |
| Beispiel Nr. 111 [DMSO-D₆] 10,39 0,56;8,16 0,55;8,16 0,56;8,15 0,56;8,15 0,59;8,09 0,60;7,40 0,48;7,39 0,55;7,38 0,62;7,37 0,57;7,20 0,59;7,17 1,06;7,15 0,49;6,76 0,47;6,75 0,46;6,74 0,46;6,74 0,48;3,94 2,46;3,34 27,70;2,51 3,07;2,50 4,21;2,50 2,82;1,99 0,34;1,16 1,44;1,15 1,43;1,07 16,00;1,05 2,92 |
| Beispiel Nr. 112 [DMSO-D₆] 10,41 2,31;8,22 2,22;8,21 2,29;8,01 2,21;7,83 0,35;7,82 0,36;7,43 0,41;7,42 2,11;7,41 0,83;7,40 2,39;7,39 2,48;7,39 0,93;7,38 2,23;7,34 0,56;7,33 0,43;7,31 1,42;7,31 1,82;7,29 1,20;7,27 0,49;7,25 0,41;7,20 0,81;7,20 2,57;7,19 0,92;7,18 1,44;7,18 4,29;7,17 0,99;7,16 0,89;7,15 1,94;6,84 1,77;6,83 1,75;6,82 1,75;6,82 1,81;6,26 0,36;6,26 0,42;6,25 0,61;5,84 0,50;5,61 6,29;4,13 1,29;4,11 2,96;4,10 1,37;4,05 0,41;3,70 2,22;3,34 70,98;2,99 1,23;2,98 2,51;2,96 1,15;2,86 0,38;2,52 0,51;2,52 0,76;2,51 17,53;2,51 34,04;2,50 47,12;2,50 33,07;2,49 16,51;2,24 3,42;2,22 4,15;2,06 0,42;2,04 0,77;2,02 0,91;2,01 0,73;1,99 0,41;1,91 0,43;1,87 0,92;1,71 3,25;0,90 16,00;0,88 15,65;0,86 1,17;0,00 1,83 |
| Beispiel Nr. 113 [CD ₃CN] 8,59 1,60;8,56 0,71;8,56 0,71;8,38 0,65;8,38 0,67;8,37 0,68;8,37 0,68;8,14 4,03;8,14 4,16;8,13 4,11;8,13 4,21;7,95 3,76;7,68 0,33;7,67 0,33;7,47 0,47;7,47 4,23;7,46 1,73;7,46 4,60;7,45 4,82;7,45 1,84;7,44 4,47;7,44 0,54;7,39 0,62;7,39 0,64;7,39 0,64;7,38 0,64;7,10 0,55;7,09 4,60;7,09 1,51;7,08 1,78;7,08 8,87;7,08 1,79;7,07 1,48;7,06 4,33;7,06 0,49;6,76 3,77;6,76 3,90;6,76 3,90;6,75 3,80;5,45 2,55;4,88 16,00;4,21 1,80;4,20 4,70;4,19 4,88;4,17 4,86;4,17 5,10;4,16 3,79;4,16 5,61;4,15 2,15;4,15 1,96;4,08 0,34;4,06 0,97;4,05 0,98;4,04 0,34;2,34 1,48;2,33 1,52;2,27 9,09;2,26 9,27;2,18 87,47;2,09 31,03;2,08 0,55;1,97 4,58;1,97 3,02;1,96 2,10;1,96 2,73;1,95 21,51;1,95 39,41;1,94 55,04;1,94 37,15;1,94 19,43;1,34 1,11;1,22 1,29;1,20 2,46;1,20 0,81;1,19 1,28;1,14 1,03;1,13 0,87;1,11 0,33;1,09 0,39;1,08 0,47;1,08 0,48;1,07 0,94;1,07 0,97;1,07 0,91;1,07 0,68;1,06 0,64;1,06 1,70;1,06 0,62;1,06 0,69;1,05 0,95;1,05 0,96;1,05 1,04;1,04 0,49;1,04 0,54;1,04 0,41;0,58 0,78;0,58 0,74;0,58 0,35;0,57 0,38;0,57 0,84;0,57 0,75;0,56 1,73;0,55 4,37;0,55 4,57;0,54 2,10;0,54 2,18;0,54 4,56;0,53 4,30;0,53 1,75;0,26 0,75;0,26 0,74;0,25 0,76;0,25 0,83;0,24 0,33;0,23 1,51;0,22 4,61;0,22 4,58;0,21 4,27;0,21 4,75;0,20 1,33;0,00 6,10 |
| Beispiel Nr. 114 [CD ₃CN] 8,59 2,54;8,59 1,53;8,58 1,54;8,58 2,57;7,37 3,17;7,37 1,88;7,36 1,84;7,36 3,12;6,82 2,59;6,81 2,86;6,67 2,84;6,67 2,67;4,32 0,65;4,32 1,32;4,31 0,57;4,28 0,34;4,28 0,33;4,27 0,33;4,27 0,32;4,26 0,42;4,26 0,38;4,25 0,37;4,02 0,40;4,01 0,53;4,00 0,47;4,00 0,43;3,99 0,57;3,99 0,50;3,98 0,37;3,97 0,42;3,17 16,00;2,24 0,62;2,23 0,43;2,23 0,40;2,23 0,38;2,21 0,39;2,21 0,40;2,20 0,45;2,20 0,37;2,18 0,36;2,16 394,92;2,15 0,79;2,14 0,54;2,14 0,85;2,06 0,40;2,06 0,74;2,05 1,10;2,05 0,75;2,04 0,38;1,98 0,34;1,97 0,52;1,97 9,52;1,96 5,44;1,95 7,79;1,95 72,73;1,95 131,74;1,94 197,34;1,94 136,52;1,93 68,11;1,93 1,17;1,92 0,67;1,92 0,57;1,91 0,34;1,84 0,41;1,83 0,78;1,83 1,10;1,82 0,76;1,82 0,38;1,78 0,44;1,76 0,33;1,75 0,49;1,75 0,48;1,27 1,08;0,01 0,97;0,00 30,82;-0,01 0,89 |
| Beispiel Nr. 115 [DMSO-D₆] 8,20 0,32;7,35 0,40;7,35 0,54;7,32 0,46;7,31 0,41;7,30 0,42;3,93 2,43;3,89 0,85;3,34 21,37;2,51 1,65;2,51 3,53;2,50 4,82;2,50 3,58;2,49 1,79;2,38 0,51;2,36 0,53;1,99 0,94;1,17 0,51;1,16 0,35;1,07 16,00;1,05 0,70;1,03 1,31;1,02 0,60;0,00 0,48 |
| Beispiel Nr. 116 [DMSO-D₆] 10,41 1,98;8,21 1,89;8,19 1,97;8,03 2,31;7,42 1,56;7,41 0,75;7,41 1,83;7,40 2,12;7,39 0,87;7,38 1,88;7,33 0,38;7,20 1,92;7,20 0,75;7,18 0,78;7,18 3,43;7,16 0,60;7,16 1,60;6,78 1,46;6,78 1,51;6,77 1,48;6,77 1,53;5,69 0,34;5,67 1,21;5,66 1,23;5,64 0,36;4,31 0,50;4,30 0,54;4,28 0,65;4,27 0,59;4,06 1,23;4,04 3,73;4,02 3,79;4,00 1,29;3,94 0,33;3,93 0,47;3,93 0,70;3,91 0,72;3,91 0,77;3,89 0,42;3,88 0,34;3,57 0,45;3,32 28,64;3,30 0,55;3,12 0,54;3,10 0,38;3,09 0,40;2,81 0,66;2,81 0,72;2,77 0,82;2,75 0,80;2,73 1,07;2,72 0,80;2,51 7,58;2,51 15,92;2,50 21,76;2,50 16,77;2,49 8,99;1,99 16,00;1,72 5,27;1,71 5,31;1,40 0,42;1,20 0,33;1,19 4,44;1,17 8,71;1,164,36;1,09 0,44;1,07 13,25;1,05 12,16;0,00 3,20 |
| Beispiel Nr. 117 [DMSO-D₆] 8.54 6.04;8.54 6.22;8.34 3.94;8.34 4.00;8.33 4.08;8.33 4.10;7.40 0.44;7.39 4.29;7.38 1.85;7.38 4.83;7.37 5.80;7.36 2.11;7.35 5.24;7.35 0.67;7.26 3.11;7.25 3.29;7.24 3.51;7.23 3.12;7.20 0.65;7.20 5.66;7.19 1.80;7.18 1.92;7.17 10.00;7.17 2.10;7.16 1.59;7.15 4.54;7.14 0.50;4.53 1.17;4.51 2.29;4.49 2.31;4.48, 1.15;3.94 1.70;3.46 0.35;3.41 0.63;3.35 757.79;3.34 820.12;3.01 0.46;3.00 0.61;2.98 0.56;2.97 1.30;2.96 1.51;2.94 1.43;2.93 1.61;2.91 2.04;2.89 1.87;2.87 0.78;2.85 0.84;2.83 0.87;2.82 0.74;2.82 0.92;2.80 1.57;2.79 1.11;2.78 1.40;2.77 0.98;2.76 0.70;2.75 0.51;2.68 0.42;2.68 0.85;2.67 1.18;2.67 0.86;2.66 0.43;2.54 0.57;2.54 0.53;2.53 3.59;2.52 5.35;2.51 62.19;2.51 127.34;2.50 169.68;2.50 125.11;2.49 62.31;2.34 0.43;2.33 0.86;2.33 1.19;2.33 0.89;2.32 0.45;2.23 0.65;2.21 1.34;2.21 0.89;2.20 1.22;2.19 1.37;2.18 1.43;2.17 1.06;2.16 0.90;2.16 1.09;2.14 0.56;2.07 1.40;1.50 16.00;1.49 15.95;1.24 0.51;1.16 2.81;1.07 13.39;0.01 0.78;0.00 25.60 |
| Beispiel Nr. 118 [DMSO-D₆] 8.61 9.62;8.60 9.81;8.44 6.16;8.44 6.08;8.43 6.35;8.43 6.19;7.33 0.68;7.32 6.73;7.31 2.80;7.31 7.54;7.30 9.22;7.29 3.29;7.28 8.37;7.28 1.18;7.26 4.65;7.25 4.96;7.25 5.29;7.23 4.63;7.16 1.02;7.16 9.31;7.15 2.73;7.14 2.89;7.13 16.00;7.13 3.09;7.12 2.41;7.11 7.12;7.10 0.79;5.76 0.46;4.37 5.36;4.36 4.78;4.35 5.03;3.35 80.36;2.65 4.89;2.64 4.16;2.62 5.19;2.52 0.44;2.52 8.56;2.51 18.70;2.51 25.30;2.50 18.15;2.50 8.58;1.85 3.90;1.84 3.74;1.77 3.55;1.76 4.08;1.64 3.24;1.63 4.07;1.26 0.41;1.23 0.52;1.07 1.69;0.00 2.91 |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Wenn auf Grund der Rundung des δ-Wertes auf zwei Nachkommastellen fallweise Signale mit gleichem δ-Wert auftreten, ergeben deren Intensitäten nach Addition dasselbe Bild, das auch in einem Ausdruck eines klassischen NMR's im Bereich dieses δ-Wertes beobachtet werden kann.

### Verwendungsbeispiele

### Beispiel A

**Alternaria-Test (Tomate) / protektiv**

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von ***Alternaria solani*** inokuliert und stehen dann 24h bei 100% rel. Feuchte und 22°C. Anschließend stehen die Pflanzen bei 96% rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 500ppm einen Wirkungsgrad von 70% oder mehr.

Beispiel Nr. [Tabelle 1] (Wirkungsgrad in %):
2 ((94%), 3 (95%), 5 (100%), 6 (95%), 7 (95%), 8 (100%), 9 (100%), 10 (70%), 15 (100%), 19 (100%), 20 (100%), 21 (100%), 22 (95%), 23 (95%), 24 (95%), 25 (100%), 26 (100%), 27 (100%), 28 (100%), 29 (100%), 30 (100%), 31 (94%), 32 (78%), 33 (89%), 34 (100%), 35 (94%), 37 (95%), 38 (95%), 39 (80%), 40 (100%), 41 (70%), 43 (94%), 44 (80%), 45 (95%), 46 (70%), 47 (90%), 50 (95%), 51 (95%), 52 (90%), 54 (80%), 55 (80%), 58 (80%), 59 (70%), 62 (94%), 63 (100%), 64 (100%), 65 (100%), 66 (70%), 67 (80%), 70 (90%), 74 (95%), 75 (95%), 76 (100%), 77 (89%), 82 (89%), 83 (89%), 86 (90%), 87 (80%), 88 (90%), 89 (90%), 90 (95%), 91 (90%), 92 (90%), 93 (95%), 94 (90%), 95 (80%), 96 (90%), 97 (70%), 98 (90%), 99 (80%), 100 (80%), 101 (90%), 102 (95%), 104 (80%), 106 (80%), 108 (80%), 109 (90%), 112 (90%), 113 (90%) 117 (94%).

### Beispiel B

**Botrytis - Test (Bohne) / protektiv**

| | | |
|---|---|---|
| Lösungsmittel : | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

Beispiel Nr. [Tabelle 1] (Wirkungsgrad in %):
2 ((100%), 5 (100%), 7 (95%), 8 (99%), 9 (93%), 15 (82%), 19 (99%), 20 (98%), 21 (98%), 22 (89%), 23 (100%), 24 (76%), 25 (90%), 26 (93%), 27 (92%), 28 (95%), 29 (100%), 30 (100%), 31 (90%), 32 (98%), 33 (98%), 34 (99%), 35 (96%), 40 (100%), 43 (99%), 51 (99%), 52 (78%), 58 (100%), 59 (100%), 63 (94%), 64 (100%), 65 (97%), 74 (100%), 76 (100%), 77 (100%), 78 (100%), 85 (100%), 86 (100%), 87 (100%), 89 (100%), 91 (100%) 117 (100%).

### Beispiel C

**Fusarium graminearum -Test (Gerste) / protektiv**

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen mit einer Sporensuspension von ***Fusarium graminearum*** besprüht.

Die Pflanzen werden in einer Gewächshauskammer unter eine lichtdurchlässige Inkubationshaube bei 22°C und 100 % relativer Luftfeuchtigkeit gestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

Beispiel Nr. [Tabelle 1] (Wirkungsgrad in %):
5 ((100%), 8 (88%), 15 (100%), 19 (100%), 20 (94%), 21 (100%), 22 (100%), 23 (88%), 24 (100%), 25 (94%), 26 (88%), 27 (94%), 28 (100%), 29 (94%), 30 (88%), 31 (100%), 32 (86%), 34 (100%), 36 (93%), 40 (100%), 50 (83%), 55 (83%), 58 (100%), 59 (94%), 63 (83%), 65 (83%), 76 (90%), 77 (90%) 117 (100%).

### Beispiel D

**Fusarium nivale (var.majus) -Test (Weizen) / protektiv**

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen mit einer Sporensuspension von ***Fusarium nivale (var.majus)*** besprüht.

Die Pflanzen werden in einer Gewächshauskammer unter eine lichtdurchlässige Inkubationshaube bei 10°C und 100 % relativer Luftfeuchtigkeit gestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

Beispiel Nr. [Tabelle 1] (Wirkungsgrad in %):
5 ((100%), 8 (100%), 15 (100%), 19 (100%), 20 (100%), 21 (100%), 22 (86%), 23 (86%), 24 (100%), 25 (100%), 26 (86%), 27 (86%), 28 (100%), 29 (86%), 30 (86%), 31 (100%), 32 (86%), 34 (100%), 36 (88%), 37 (86%), 38 (86%), 40 (100%), 43 (80%), 47 (80%), 50 (83%), 55 (100%), 58 (100%), 59 (100%), 63 (83%), 65 (100%), 76 (100%), 77 (100%) 117 (100%).

### Beispiel E

**Leptosphaeria nodorum - Test (Weizen) / protektiv**

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Weizenpflanzen mit der Wirkstoffzubereitung in der angegebenen Aufwandmenge. 1 Tag nach der Behandlung werden die Pflanzen mit einer wäßrigen Sporensuspension von ***Leptosphaeria nodorum*** inokuliert und verbleiben dann 48h bei 100% rel. Luftfeuchte und 22°C. Anschließend werden die Pflanzen in einem Gewächshaus bei 90 % rel. Luftfeuchtigkeit und einer Temperatur von 22°C aufgestellt.

7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 500ppm einen Wirkungsgrad von 70% oder mehr.

Beispiel Nr. [Tabelle 1] (Wirkungsgrad in %):
2 ((80%), 3 (89%), 5 (94%), 6 (70%), 8 (95%), 9 (95%), 10 (80%), 11 (70%), 15 (95%), 19 (95%), 20 (95%), 21 (95%), 22 (80%), 23 (90%), 24 (90%), 25 (95%), 26 (80%), 28 (90%), 29 (90%), 30 (90%), 31 (90%), 32 (90%), 33 (70%), 34 (95%), 35 (90%), 36 (90%), 37 (90%), 40 (95%), 43 (90%), 55 (90%), 58 (94%), 59 (89%), 60 (78%), 62 (78%), 63 (94%), 64 (94%), 65 (94%), 70 (90%), 74 (88%), 76 (89%), 77 (94%), 78 (100%), 82 (88%), 83 (75%), 84 (88%), 85 (75%), 86 (70%), 87 (70%), 89 (80%), 90 (89%), 91 (78%), 92 (90%), 93 (80%), 95 (90%), 96 (90%), 97 (80%), 99 (95%), 100 (70%), 101 (70%), 102 (90%), 107 (90%), 108 (70%), 112 (90%), 113 (78%) 117 (86%).

### Beispiel F

**Phytophthora-Test (Tomate) / protektiv**

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen bei einer Konzentration an Wirkstoff von 500ppm einen Wirkungsgrad von 70% oder mehr.

Beispiel Nr. [Tabelle 1] (Wirkungsgrad in %):
2 ((94%), 3 (90%), 5 (95%), 7 (95%), 8 (95%), 9 (90%), 10 (70%), 11 (80%), 14 (70%), 15 (95%), 17 (80%), 19 (100%), 20 (100%), 21 (90%), 22 (90%), 23 (80%), 24 (90%), 25 (80%), 26 (95%), 27 (80%), 28 (95%), 29 (90%), 30 (80%), 31 (80%), 32 (70%), 33 (70%), 34 (95%), 35 (80%), 36 (80%), 37 (70%), 38 (70%), 39 (80%), 40 (70%), 41 (70%), 43 (70%), 44 (70%), 45 (90%), 46 (70%), 47 (80%), 48 (70%), 52 (95%), 56 (80%), 58 (70%), 62 (80%), 63 (95%), 64 (80%), 65 (90%), 66 (80%), 67 (70%), 69 (70%), 70 (95%), 71 (80%), 74 (90%), 75 (80%), 82 (70%), 83 (70%), 85 (90%), 87 (90%), 88 (80%), 89 (90%), 90 (78%), 92 (80%), 93 (90%), 96 (70%), 113 (70%).

### Beispiel G

**Puccinia triticina -Test (Weizen) / protektiv**

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen mit einer Sporensuspension von Puccinia triticina besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

Beispiel Nr. [Tabelle 1] (Wirkungsgrad in %):
5 ((100%), 8 (100%), 15 (94%), 19 (100%), 19 (95%), 20 (100%), 21 (95%), 22 (75%), 23 (75%), 24 (100%), 25 (88%), 26 (100%), 27 (75%), 28 (100%), 29 (94%), 30 (88%), 31 (94%), 32 (89%), 34 (94%), 36 (78%), 37 (78%), 40 (100%), 43 (100%), 50 (86%), 55 (100%), 58 (100%), 59 (100%), 63 (86%), 65 (86%), 76 (88%), 77 (88%) 117 (100%).

### Beispiel H

**Pyrenophora teres - Test (Gerste) / protektiv**

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gerstenpflanzen mit der Wirkstoffzubereitung in der angegebenen Aufwandmenge. 1 Tag nach der Behandlung werden die Pflanzen mit einer wäßrigen Sporensuspension von ***Pyrenophora teres*** inokuliert und verbleiben dann 48h bei 100% rel. Luftfeuchte und 22°C. Anschließend werden die Pflanzen in einem Gewächshaus bei 80 % rel. Luftfeuchtigkeit und einer Temperatur von 20°C aufgestellt.

7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 500ppm einen Wirkungsgrad von 70% oder mehr.

Beispiel Nr. [Tabelle 1] (Wirkungsgrad in %): 2 (95%), 3 (95%), 5 (100%), 6 (70%), 7 (80%), 8 (100%), 9 (94%), 10 (80%), 11 (70%), 13 (80%), 15 (100%), 19 (100%), 20 (100%), 21 (100%), 22 (95%), 23 (95%), 24 (95%), 25 (100%), 26 (95%), 27 (90%), 28 (100%), 29 (100%), 30 (100%), 31 (100%), 32 (78%), 33 (78%), 34 (94%), 35 (78%), 36 (89%), 37 (100%), 38 (95%), 39 (95%), 40 (95%), 41 (95%), 43 (95%), 44 (90%), 45 (95%), 46 (70%), 47 (90%), 50 (95%), 51 (90%), 52 (80%), 55 (95%), 58 (90%), 59 (90%), 60 (90%), 61 (70%), 62 (95%), 63 (95%), 64 (100%), 65 (95%), 66 (95%), 67 (95%), 70 (80%), 71 (80%), 72 (80%), 73 (70%), 74 (100%), 75 (100%), 78 (95%), 82 (94%), 83 (94%), 84 (94%), 85 (100%), 86 (100%), 87 (90%), 88 (90%), 89 (95%), 90 (95%), 91 (95%), 92 (95%), 93 (100%), 94 (95%), 95 (100%), 96 (100%), 97 (100%), 98 (95%), 99 (95%), 100 (100%), 101 (95%), 102 (95%), 103 (90%), 104 (95%), 105 (95%), 106 (95%), 107 (95%), 108 (95%), 109 (95%), 112 (95%), 113 (94%) 117 (100%).

### Beispiel I

**Septoria tritici-Test (Weizen) / protektiv**

| | | |
|---|---|---|
| Lösungsmittel: | 49 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Sporensuspension von ***Septoria tritici*** besprüht. Die Pflanzen verbleiben 48 Stunden bei 20°C und 100 % relativer Luftfeuchtigkeit in einer Inkubationskabine. Danach werden die Pflanzen für weitere 60 Stunden unter eine Klarsichthaube bei 15°C und 100% relativer Luftfeuchte gestellt.

Die Pflanzen werden in einem Gewächshaus bei einer Temperatur von ca. 15°C und einer relativen Luftfeuchtigkeit von 80 % aufgestellt.

21 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die erfindungsgemäßen nachfolgenden Verbindungen bei einer Konzentration an Wirkstoff von 1000 ppm einen Wirkungsgrad von 70% oder mehr.

Beispiel Nr. [Tabelle 1] (Wirkungsgrad in %):
8 ((100%), 15 (100%), 19 (100%), 21 (100%), 22 (86%), 23 (86%), 24 (100%), 25 (86%), 26 (86%), 28 (100%), 29 (71%), 30 (100%), 31 (100%), 32 (100%), 34 (93%), 36 (100%), 40 (90%), 43 (100%), 47 (100%), 50 (90%), 55 (90%), 58 (89%), 63 (100%), 65 (90%), 76 (100%), 77 (100%) 117 (100%).

### Beispiel J

**Sphaerotheca-Test (Gurke) / protektiv**

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von ***Sphaerotheca fuliginea*** inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen bei einer Konzentration an Wirkstoff von 500ppm einen Wirkungsgrad von 70% oder mehr.

Beispiel Nr. [Tabelle 1] (Wirkungsgrad in %):
2 ((91%), 5 (95%), 7 (90%), 8 (99%), 9 (86%), 10 (94%), 11 (88%), 15 (98%), 19 (98%), 20 (100%), 21 (100%), 22 (90%), 23 (89%), 24 (94%), 25 (94%), 26 (98%), 28 (100%), 29 (83%), 30 (95%), 31 (95%), 33 (95%), 34 (100%), 36 (94%), 40 (94%), 47 (96%), 50 (94%), 55 (95%), 58 (96%), 59 (98%), 60 (90%), 63 (93%), 64 (86%), 65 (100%), 70 (94%), 74 (93%), 75 (95%), 76 (100%), 78 (100%), 82 (95%), 83 (98%), 84 (98%), 85 (85%), 86 (100%), 87 (95%), 88 (95%), 89 (100%), 90 (95%), 91 (100%), 92 (98%), 93 (98%), 94 (93%), 95 (95%), 96 (95%), 97 (100%), 98 (90%), 99 (98%), 100 (95%), 101 (98%), 102 (100%), 104 (93%), 106 (75%), 107 (100%), 108 (95%), 109 (93%), 112 (95%), 113 (98%) 117 (95%).

### Beispiel K

**Uromyces - Test (Bohne) / protektiv**

| | | |
|---|---|---|
| Lösungsmittel : | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Bohnenrosterregers Uromyces appendiculatus inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

Beispiel Nr. [Tabelle 1] (Wirkungsgrad in %):
5 ((98%), 8 (100%), 15 (94%), 19 (94%), 20 (94%), 21 (94%), 22 (94%), 23 (95%), 24 (91%), 25 (94%), 26 (94%), 27 (94%), 28 (95%), 29 (94%), 30 (95%), 31 (94%), 32 (96%), 33 (95%), 34 (95%), 36 (85%), 40 (81%), 40 (75%), 43 (90%), 51 (86%), 63 (75%), 64 (78%), 65 (95%), 76 (95%), 77 (98%), 78 (94%), 85 (85%), 86 (95%), 87 (93%), 89 (95%), 91 (95%) 117 (90%).

### Beispiel L

**Venturia - Test (Apfel) / protektiv**

| | | |
|---|---|---|
| Lösungsmittel : | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers ***Venturia inaequalis*** inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

Beispiel Nr. [Tabelle 1] (Wirkungsgrad in %): 2 (95%), 5 (98%), 7 (76%), 8 (96%), 9 (95%), 15 (94%), 19 (93%), 20 (99%), 21 (99%), 22 (76%), 23 (98%), 24 (83%), 25 (94%), 26 (93%), 27 (89%), 28 (100%), 29 (95%), 30 (99%), 31 (100%), 32 (90%), 33 (95%), 34 (95%), 35 (95%), 40 (94%), 43 (98%), 50 (88%), 51 (74%), 52 (94%), 58 (99%), 59 (97%), 63 (99%), 64 (99%), 65 (96%), 70 (100%), 74 (95%), 76 (100%), 77 (100%), 78 (100%), 86 (95%), 89 (94%), 91 (95%) 117 (100%).

### Beispiel M

**Pyricularia-Test (Reis) / protektiv**

| | | |
|---|---|---|
| Lösungsmittel: | 28,5 | Gew.-Teile Aceton |
| Emulgator: | 1,5 | Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Pyricularia oryzae* inokuliert. Anschließend werden die Pflanzen in einem Gewächshaus bei 100 % relativer Luftfeuchtigkeit und 25°C aufgestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 250 ppm einen Wirkungsgrad von 80% oder mehr.

Beispiel Nr. [Tabelle 1] (Wirkungsgrad in %):
19 ((92%), 20 (92%), 21 (98%), 28 (90%), 29 (95%), 30 (94%), 31 (98%), 34 (95%).

### Beispiel N

**Rhizoctonia Test (Reis) / protektiv**

| | | |
|---|---|---|
| Lösungsmittel: | 28,5 | Gew.-Teile Aceton |
| Emulgator: | 1,5 | Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit Hyphen von *Rhizoctonia solani* inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 250 ppm einen Wirkungsgrad von 80% oder mehr.

Beispiel Nr. [Tabelle 1] (Wirkungsgrad in %):
19 ((100%), 20 (100%), 21 (100%), 28 (100%), 29 (100%), 30 (100%), 31 (100%), 34 (85%).

### Beispiel O

**Cochliobolus Test (Reis) / protektiv**

| | | |
|---|---|---|
| Lösungsmittel: | 28,5 | Gew.-Teile Aceton |
| Emulgator: | 1,5 | Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Cochliobolus miyabeanus* inokuliert. Anschließend werden die Pflanzen in einem Gewächshaus bei 100 % relativer Luftfeuchtigkeit und 25°C aufgestellt.

4 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 250 ppm einen Wirkungsgrad von 80% oder mehr.

Beispiel Nr. [Tabelle 1] (Wirkungsgrad in %):
19 ((96%), 20 (96%), 21 (96%), 28 (95%), 29 (95%), 30 (96%), 31 (95%), 34 (90%).

### Beispiel P

**Gibberella test (Reis) / protektiv**

| | | |
|---|---|---|
| Lösungsmittel: | 28,5 | Gew.-Teile Aceton |
| Emulgator: | 1,5 | Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. 1 Tag nach der Behandlung werden die Pflanzen mit einer wässrigen Sporensuspension von *Gibberella zeae* inokuliert. Anschließend werden die Pflanzen in einem Gewächshaus bei 100 % relativer Luftfeuchtigkeit und 25°C aufgestellt.

5 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 250 ppm einen Wirkungsgrad von 80% oder mehr.

Beispiel Nr. [Tabelle 1] (Wirkungsgrad in %):
28 ((85%), 29 (80%), 31 (80%), 34 (80%).

### Beispiel Q

**Phakopsora test (Sojabohnen) / protektiv**

| | | |
|---|---|---|
| Lösungsmittel: | 28,5 | Gew.-Teile Aceton |
| Emulgator: | 1,5 | Gew.-Teil Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Pflanzen mit der Wirkstoffzubereitung in der angegebenen Aufwandmenge. 1 Tag nach der Behandlung werden die Pflanzen mit einer wässrigen Sporensuspension von *Phakopsora pachyrhizi* inokuliert. Anschließend werden die Pflanzen in einem Gewächshaus bei 80 % relativer Luftfeuchtigkeit und 20°C aufgestellt.

1 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die nachfolgenden erfindungsgemäßen Verbindungen bei einer Konzentration an Wirkstoff von 250 ppm einen Wirkungsgrad von 80% oder mehr.

Beispiel Nr. [Tabelle 1] (Wirkungsgrad in %):
5 ((80%).

### Beispiel R

### Produktion von DON/Acetyl-DON durch Fusarium graminearum

Die Verbindungen wurden in Mikrotiter-Platten in einem DON induzierenden Flüssig-Medium (1g (NH₄)₂HPO₄, 0.2g MgSO₄x7H₂O, 3g KH₂PO₄, 10g Glycerin, 5g NaCl and 40g Saccharose pro Liter) und DMSO (0,5 %) getestet. Die Inokulation erfolgte mit einer konzentrierten SporenSuspension von *Fusarium graminearum* bei einer Endkonzentration von 2000 Sporen/ml.

Die Platte wurde bei hoher Luftfeuchtigkeit 7 Tage lang bei 28 °C inkubiert.

Zu Beginn und nach 3 Tagen wurde eine OD-Messung bei OD620 (mehrfache Messung: 3 x 3 Messungen pro Loch) zur Berechnung der Wachstumshemmung vorgenommen.

Nach 7 Tagen wurden 1 Volumen einer 84/16 Acetonitril/Wasser -Mischung hinzugefügt und aus jedem Loch wurde anschließend eine Probe des flüssigen Mediums entnommen und 1:100 in 10 %igem Acetonitril verdünnt. Die Anteile von DON und Acetyl-DON der Proben wurden mittels HPLC-MS/MS analysiert und die Meßwerte wurden zur Berechnung der Hemmung der DON/AcDON Produktion im Vergleich zu einer wirkstofffreien Kontrolle genutzt.

Die HPLC-MS/MS-Messungen wurden mit folgenden Parametern durchgeführt:
Ionisierungs-Art: ESI negativ
Ionen-Spray Spannung: - 4500 V
Spraygas-Temperatur: 500°C
Dekluster-Potential: - 40 V
Kollisions-Energie: -22eV
Kollisions-Gas: N₂
NMR Spur: 355,0 >264,9;

HPLC Säule: Waters Atlantis T3 (trifunktionelle C18 Bindung, verschlossen)
Partikelgröße: 3µm
Säulenmaße: 50 x 2 mm
Temperatur: 40°C
Lösungsmittel A: Wasser/2.5mM NH₄OAc+0.05% CH₃COOH (v/v)
Lösungsmittel B: Methanol/2.5mM NH₄OAc+0.05% CH₃COOH (v/v)
Durchfluß: 400 µL/Minute
Injektionsvolumen: 11 µL
Gradient:

| Zeit [min] | A% | B% |
|---|---|---|
| 0 | 100 | 0 |
| 0.75 | 100 | 0 |
| 1.5 | 5 | 95 |
| 4 | 5 | 95 |
| 5 | 100 | 0 |
| 10 | 100 | 0 |

### Beispiele für DON-Hemmung

Die folgenden Beispiele zeigten eine Aktivität > 80 % bei der Hemmung von DON/AcDON Produktion bei 50 µM. Die Hemmung des Wachstums von *Fusarium graminearum* der genannten Beispiele variierte von 0 bis 100 % bei 50 µM.

| Beispiel Nr. | % Hemmung von DON/AcDON bei 50 µM | % Hemmung des Pilzwachstums von Fusarium graminearum bei 50 µM |
|---|---|---|
| 2 | 100 | 99 |
| 3 | 94 | 30 |
| 5 | 99 | 100 |
| 6 | 99 | 70 |
| 7 | 99 | 100 |
| 8 | 99 | 100 |
| 9 | 99 | 100 |
| 10 | 100 | 87 |
| 11 | 100 | 92 |
| 13 | 96 | 17 |
| 14 | 93 | 24 |
| 15 | 99 | 88 |
| 16 | 93 | 0 |
| 19 | 100 | 100 |
| 20 | 100 | 95 |
| 21 | 100 | 97 |
| 22 | 100 | 97 |
| 23 | 100 | 97 |
| 24 | 99 | 74 |
| 25 | 99 | 92 |
| 26 | 98 | 57 |
| 27 | 99 | 56 |
| 28 | 99 | 100 |
| 29 | 99 | 100 |
| 30 | 99 | 88 |
| 31 | 99 | 97 |
| 32 | 99 | 94 |
| 33 | 99 | 100 |
| 34 | 99 | 98 |
| 36 | 99 | 94 |
| 37 | 99 | 93 |
| 38 | 99 | 89 |
| 41 | 98 | 100 |
| 42 | 98 | 100 |
| 43 | 98 | 100 |
| 44 | 100 | 61 |
| 45 | 100 | 21 |
| 46 | 100 | 52 |
| 47 | 100 | 76 |
| 50 | 92 | 57 |
| 51 | 100 | 41 |
| 52 | 99 | 19 |
| 54 | 100 | 0 |
| 56 | 92 | 27 |
| 58 | 100 | 100 |
| 59 | 100 | 100 |
| 60 | 100 | 100 |
| 61 | 100 | 0 |
| 62 | 100 | 35 |
| 63 | 100 | 100 |
| 64 | 100 | 100 |
| 65 | 100 | 100 |
| 66 | 98 | 50 |
| 67 | 100 | 60 |

## Patentansprüche

1. Verbindungen der Formel (I), 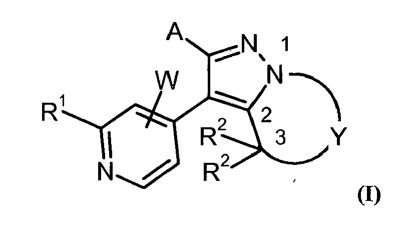 in welcher die Symbole folgende Bedeutungen haben:
Y bildet zusammen mit dem angrenzenden Stickstoffatom "1" und den beiden Kohlenstoffatomen "2" und "3" einen 5- bis 7-gliedrigen nicht aromatischen heterocyclischen Ring, dessen weitere Ringglieder aus der Gruppe C(R²)₂, O, S, NR³, C(R²)=C(R²), C(R²)=N, N=N, C(=O), C(=S), C(=NR⁴), S(=O)ₚ(=NR⁴)_{q} und SiR^{5a}R^{5b} ausgewählt sind;
R² steht jeweils unabhängig voneinander für H, Halogen, Cyano, Hydroxy, -CHO, - NHCHO, -N₃, -N=C=O, -N=C=S, -SH, -C(=O)NH₂, -C(=O)NHCN, -C(=O)OR⁶, - C(=O)NHOR^{6a}, C₁-C₅-Alkyl, C₂-C₅-Alkenyl, C₂-C₅-Alkynyl, C₃-C₆-Cycloalkyl, C₃-C₆-Halocycloalkyl, C₃-C₆-Cycloalkenyl, C₁-C₅-Haloalkyl, C₁-C₅-Alkoxy, C₁-C₅-Haloalkoxy, C₃-C₆-Cycloalkoxy, C₂-C₅-Alkenyloxy, C₃-C₅-Haloalkenyloxy, C₂-C₅-Alkynyloxy, C₂-C₅-Alkylcarbonyl, C₂-C₅-Alkylcarbonyloxy, C₂-C₅-Haloalkylcarbonyloxy, C₃-C₅-Alkoxycarbonylalkoxy, C₁-C₅-Alkylthio, C₁-C₅-Haloalkylthio, C₃-C₆-Cycloalkylthio, C₂-C₅-Alkyl(thiocarbonyl), C₂-C₅-Alkylthio(thiocarbonyl), C₁-C₅-Alkylsulfinyl, C₁-C₅-Haloalkylsulfinyl, C₃-C₆-Cycloalkylsulfinyl, C₁-C₅-Alkylsulfonyl, C₁-C₅-Haloalkylsulfonyl, C₃-C₆-Cycloalkylsulfonyl, C₃-C₅-Trialkylsilyl, C₃-C₅-Halotriallcylsilyl, C₁-C₅-Alkylamino, C₂-C₅-Haloalkylamino, C₃-C₆-Cycloalkylamino, C₂-C₅-Dialkylamino oder C₃-C₅-Halodialkylamino;
R³ steht für H, -CN, -C(=O)NH₂, -C(=O)NHCN, -CHO, -NHCHO, -C(=O)OR⁶, - C(=O)NHOR^{6a}, Hydroxy, C₁-C₅-Alkyl, C₂-C₅-Alkenyl, C₂-C₅-Alkynyl, C₃-C₆-Cycloalkyl, C₃-C₆-Halocycloalkyl, C₃-C₆-Cycloalkenyl, C₄-C₁₀-Cycloalkylalkyl, C₄-C₇-Alkylcycloalkyl, C₅-C₇-Alkylcycloalkylalkyl, C₂-C₅-Haloalkyl, C₁-C₄-Alkylcarbonyl, C₁-C₄-Haloalkylcarbonyl_{;} C₃-C₇-Cycloalkylcarbonyl, C₁-C₆-Alkoxycarbonyl, C₁-C₄-Haloalkoxycarbonyl, C₃-C₆-Cycloalkoxycarbonyl, C₂-C₆-Alkoxyalkylcarbonyl, C₂-C₆-Alkoxyalkoxycarbonyl, C₁-C₆-(Alkylthio)carbonyl, C₁-C₆-Alkoxy(thiocarbonyl), C₁-C₆-Alkyl(thiocarbonyl), C₁-C₆-Alkylthio(thiocarbonyl), C₁-C₆-Alkylaminocarbonyl, C₃-C₆-Cycloalkylaminocarbonyl, C₂-C₆-Dialkylaminocarbonyl, C₁-C₆-Alkylamino(thiocarbonyl), C₂-C₆-Dialkylamino(thiocarbonyl), C₂-C₆-Alkoxy(alkyl)aminocarbonyl, C₁-C₅-Allcoxy, C₁-C₅-Haloalkoxy, C₁-C₅-Alkylthio, C₁-C₅-Haloalkylthio, C₃-C₆-Cycloalkylthio, C₁-C₅-Alkylaminosulfonyl, C₃-C₅-Trialkylsilyl oder C₃-C₅-Halotrialkylsilyl;
R⁴ steht jeweils für H, Cyano, Amino, Hydroxy, C₁-C₆-Alkyl, C₃-C₆-Cycloalkyl, C₁-C₄-Alkylcarbonyl, C₁-C₄-Haloalkylcarbonyl, C₁-C₄-Alkoxy, Phenyl oder Benzoyl;
R^{5a}, R^{5b} stehen unabhängig voneinander für C₁-C₄-Alkyl, C₂-C₅-Alkenyl, C₂-C₅-Alkynyl, C₃-C₅-Cycloalkyl, C₃-C₆-Halocycloalkyl, C₄-C₇-Cycloalkylalkyl, C₄-C₇-Alkylcycloalkyl, C₅-C₇-Alkylcycloalkylalkyl, C₁-C₅-Haloalkyl, C₁-C₅-Alkoxy oder C₁-C₅-Haloalkoxy;
R⁶ steht jeweils für H, C₁-C₆-Alkyl, C₁-C₆-Haloalkyl, C₃-C₆-Cycloalkyl, C₄-C₇-Cycloalkylalkyl, C₄-C₇-Alkylcycloalkyl oder Benzyl;
R^{6a} steht jeweils für C₁-C₆-Alkyl, C₁-C₆-Haloalkyl, C₃-C₆-Cycloalkyl, C₄-C₇-Cycloalkylalkyl oder C₄-C₇-Alkylcycloalkyl;
A steht für einen Phenylring, der gegebenenfalls einfach oder mehrfach mit R⁷ substituiert sein kann oder für einen Thiophenylring der gegebenenfalls einfach oder mehrfach mit R⁸ substituiert sein kann;
R⁷ steht unabhängig voneinander für Halogen, C₁-C₆-Alkyl, C₂-C₆-Alkenyl, C₂-C₆-Alkynyl, C₃-C₆-Cycloalkyl, C₃-C₆-Halocycloalkyl, C₄-C₇-Alkylcycloalkyl, C₁-C₆-Haloalkyl, C₂-C₆-Haloalkenyl, Cyano, Nitro, C₁-C₆-Alkoxy, C₁-C₆-Haloalkoxy, C₁-C₆-Alkylthio, C₁-C₆-Alkylsulfinyl, C₁-C₆-Alkylsulfonyl, C₁-C₆-Haloalkylthio, C₁-C₆-Haloalkylsulfinyl, C₁-C₆-Haloalkylsulfonyl, C₁-C₆-Alkylamino, C₂-C₆-Dialkylamino, C₁-C₆-Alkylcarbonyl, C₁-C₆-Alkoxycarbonyl, C₁-C₆-Alkylaminocarbonyl, C₂-C₆-Dialkylaminocarbonyl oder C₃-C₆-Trialkylsilyl;
R⁸ steht unabhängig voneinander für Halogen, Cyano, C₁-C₃-Alkyl; C₁-C₃-Alkoxy, C₁-C₃-Haloalkyl, C₁-C₃-Haloalkoxy;
R¹ steht für H, Halogen, Cyano, Hydroxy, C₁-C₄-Alkyl, CONR^{9a}R^{9b}, COOH, COOR¹², - NR^{9a}R^{9b}, -N(R^{9b})COR^{9a}, -N(R^{9b})CSR^{9a}, -N(R^{9b})COOR¹², -N(R^{9b})SO₂R¹², -NR¹⁰-NR^{11a}R^{11b}, -S(O)ₘR¹², -OR¹², -N=CR^{13a}R^{13b} oder -NR¹⁰N=CR^{14a}R^{14b};
R^{9a} und R^{11a} stehen unabhängig voneinander jeweils für H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₂-C₆-Alkoxyalkyl, C₃-C₆-Alkoxyalkoxyalkyl, C₃-C₆-Alkoxyalkenyl, C₃-C₆-Alkoxyalkynyl, C₃-C₆-Dialkoxyalkyl, C₄-C₁₀-Trialkoxyalkyl, C₂-C₆-Haloalkoxyalkyl, C₂-C₆-Alkoxyhaloalkyl, C₂-C₆-Haloalkoxyhaloalkyl, C₁-C₆-Hydroxyalkyl, C₁-C₁₀-Cyanoalkyl, C₂-C₆-Alkylthioalkyl, C₂-C₆-Alkylsulfinylalkyl, C₃-C₆-Alkylaminoalkyl, C₃-C₆-Haloalkylaminoalkyl, C₅-C₁₀-Cycloalkylaminoalkyl, C₄-C₁₀-Dialkylaminoalkyl, C₄-C₁₀-Halodialkylaminoalkyl, C₅-C₁₀-Cycloalkyl(alkyl)aminoalkyl, oder -(CR^{15a}R^{15b})ₘR¹⁶;
R^{9b} und R^{11b} stehen unabhängig voneinander jeweils für H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₂-C₆-Alkoxyalkyl, C₃-C₆-Alkoxyalkoxyalkyl, C₃-C₆-Alkoxyalkenyl, C₃-C₆-Alkoxyalkynyl, C₃-C₆-Dialkoxyalkyl, C₄-C₁₀-Trialkoxyalkyl, C₂-C₆-Haloalkoxyalkyl, C₂-C₆-Alkoxyhaloalkyl, C₂-C₆-Haloalkoxyhaloalkyl, C₁-C₆-Hydroxyalkyl, C₁-C₁₀-Cyanoalkyl, C₂-C₆-Alkylthioalkyl, C₂-C₆-Alkylsulfinylalkyl, C₃-C₆-Alkylaminoalkyl, C₃-C₆-Haloalkylaminoalkyl, C₅-C₁₀-Cycloalkylaminoalkyl, C₄-C₁₀-Dialkylaminoalkyl, C₄-C₁₀-Halodialkylaminoalkyl, C₅-C₁₀-Cycloalkyl(alkyl)aminoalkyl, oder -(CR^{15a}R^{15b})ₘR¹⁶;
oder
R^{9a} und R^{9b} bzw. R^{11a} und R^{11b} bilden jeweils mit dem Stickstoffatom oder der (NCO) Einheit oder der (NCS) Einheit, mit dem/ mit der sie verknüpft sind, einen 3 bis 6-gliedrigen Ring, der gegebenenfalls zusätzlich Ringglieder ausgewählt aus der Gruppe O, NR³, C(=O), C(=S), C(=NR⁴), SiR^{5a}R^{5b} und S(=O)ₚ(=NR⁴)_{q}, enthalten kann und der gegebenenfalls an den Ringkohlenstoffatomen mit 1 bis 4 Substituenten ausgewählt aus der Gruppe bestehend aus Halogen, -CN, C₁-C₂-Alkyl und C₁-C₂-Alkoxy substituiert sein kann;
R¹² steht jeweils für C₁-C₁₀-Alkyl, C₂-C₁₀-Alkenyl, C₂-C₁₀-Alkynyl, C₁-C₁₀-Haloalkyl, C₂-C₁₀-Haloalkenyl, C₂-C₁₀-Haloalkynyl, C₂-C₁₀-Alkoxyalkyl, C₃-C₁₀-Alkoxyalkoxyalkyl, C₃-C₁₀-Alkoxyalkenyl, C₃-C₁₀-Akoxyalkynyl, C₃-C₁₀-Dialkoxyalkyl, C₄-C₁₀-Trialkoxyalkyl, C₂-C₁₀-Haloalkoxyalkyl, C₂-C₁₀-Alkoxyhaloalkyl, C₂-C₁₀-Haloalkoxyhaloalkyl, C₂-C₁₀-Hydroxyalkyl, C₂-C₁₀-Cyanolkyl, C₂-C₁₀-Alkylthioalkyl, C₂-C₁₀-Alkylsulfinylalkyl, C₃-C₁₀-Alkylaminoalkyl, C₃-C₁₀-Haloalkylaminoalkyl, C₅-C₁₀-Cycloalkylaminoalkyl, C₄-C₁₀-Dialkylaminoalkyl, C₄-C₁₀-Halodialkylaminoalkyl, C₆-C₁₀-Cycloalkyl(alkyl)-aminoalkyl, oder -(CR^{15a}R^{15b})ₘR¹⁶;
R^{15a}, R^{15b} stehen unabhängig voneinander für H, Halogen, C₁-C₅-Alkyl, C₁-C₅-Haloalkyl oder C₁-C₅-Alkoxy;
oder
ein geminales Paar von R^{15a} und R^{15b} bildet zusammen mit dem Kohlenstoffatom, an das sie gebunden sind, C(=O) oder einen C₃-C₆-Cycloalkylring oder einen C₃-C₆-Halocycloalkyl ring;
R¹⁶ steht für Phenyl, C₃-C₈-Cycloallcyl, C₃-C₈-Cycloalkenyl, einen 5- oder 6-gliedrigen heteroaromatischen Ring oder Naphthalenyl oder ein 8-, 9- oder 10-gliedriges heteroaromatisches bicyclisches Ringsystem; oder einen 5- oder 6-gliedrigen heterocyclischen nicht aromatischen Ring, der gegebenenfalls Ringglieder ausgewählt aus der Gruppe C(=O), C(=S), C(=NR⁴), SiR^{5a}R^{5b} und S(=O)ₚ(=NR⁴)_{q} enthält; jeder Ring oder jedes Ringsystem kann an den Ringkohlenstoffatomen gegebenenfalls substituiert sein mit bis zu 5 Substituenten unabhängig voneinander ausgewählt aus R¹⁷;
R¹⁷ steht jeweils unabhängig voneinander für Halogen, C₁-C₆-Alkyl, C₂-C₆-Alkenyl, C₂-C₆-Alkynyl, C₃-C₆-Cycloalkyl, C₄-C₁₀-Alkylcycloalkyl, C₃-C₆-Halocycloalkyl, C₁-C₆-Haloalkyl, C₂-C₆-Haloalkenyl, Cyano, Nitro, C₁-C₆-Alkoxy, C₃-C₈-Cycloalkoxy, C₃-C₈-Halocycloalkoxy, C₁-C₆-Haloalkoxy, C₁-C₆-Alkylthio, C₁-C₆-Alkylsulfinyl, C₁-C₆-Alkylsulfonyl, C₁-C₆-Haloalkylthio, C₁-C₆-Haloalkylsulfinyl, C₁-C₆-Haloalkylsulfonyl, C₁-C₆-Alkylamino, C₁-C₆-Dialkylamino, C₁-C₆-Alkylcarbonyl, C₁-C₆-Alkoxycarbonyl, C₁-C₆-Alkylaminocarbonyl, C₃-C₆-Dialkylaminocarbonyl, C₃-C₆-Trialkylsilyl, Phenyl, Naphthalenyl oder einen 5- oder 6-gliedrigen heteroaromatischen Ring;
m steht für 0, 1 oder 2;
R¹⁰ steht jeweils für H, C₁-C₅-Alkyl, C₂-C₅-Alkenyl, C₂-C₅-Alkynyl, C₃-C₆-Cycloalkyl, C₃-C₆-Halocycloalkyl, C₁-C₅-Haloalkyl, C₂-C₅-Haloalkenyl, C₂-C₅-Haloalkynyl, C₂-C₅-Alkoxyalkyl, C₂-C₅-Alkylcarbonyl oder C₁-C₅-Alkoxy;
R^{13a}, R^{13b} stehen unabhängig voneinander für H, -CN, -C(=O)OR¹⁸, C₁-C₆-Alkyl, C₂-C₆-Alkenyl, C₂-C₆-Alkynyl, C₃-C₈-Cycloalkyl, C₃-C₈-Halocycloalkyl, C₃-C₈-Cycloalkenyl, C₄-C₁₀-Cycloalkylalkyl, C₄-C₁₀-Alkylcycloalkyl, C₅-C₁₀-Alkylcycloalkylalkyl, C₁-C₆-Alkylamino, C₂-C₆-Dialkylamino, C₂-C₆-Alkylaminoalkyl, C₂-C₆-Haloalkylaminoalkyl, C₄-C₆-Cycloalkylaminoalkyl, C₃-C₆-Dialkylaminoalkyl, C₃-C₆-Halodialkylaminoalkyl, C₅-C₁₀-Cycloalkyl(alkyl)aminoalkyl, C₁-C₆-Alkoxy, C₁-C₆-Haloalkoxy, C₃-C₁₀-Cycloalkoxy, C₁-C₁₀-Alkylthio, C₁-C₁₀-Haloalkylthio, C₃-C₁₀-Cycloalkylthio, C₃-C₁₀-Trialkylsilyl oder C₃-C₁₀-Halotrialkylsilyl, oder Phenyl oder einen 5- oder 6-gliedrigen heteroaromatischen Ring, ein 8-, 9- oder 10-gliedrigen heteroaromatisches bicyclisches Ringsystem, oder einen 5- oder 6-gliedrigen heterocyclischen nicht aromatischen Ring, der gegebenenfalls Ringglieder ausgewählt aus der Gruppe NR³, C(=O), C(=S), C(=NR⁴), SiR^{5a}R^{5b} und S(=O)ₚ(=NR⁴)_{q} enthält; jeder Ring oder jedes Ringsystem kann an den Ringkohlenstoffatomen gegebenenfalls substituiert sein mit 1 bis 5 Substituenten ausgewählt aus der Gruppe bestehend aus C₁-C₃-Alkyl, Halogen, -CN and C₁-C₃-Alkoxy;
oder
R^{13a} und R^{13b} bilden zusammen mit dem Kohlenstoffatom, an das sie gebunden sind, einen 3-bis 6-gliedrigen Ring, besagter Ring kann gegebenenfalls Ringglieder enthalten ausgewählt aus der Gruppe NR³, C(=O), C(=S), C(=NR⁴), SiR^{5a}R^{5b} oder S(=O)ₚ(=NR⁴)_{q} und kann gegebenenfalls an den Ringkohlenstoffatomen mit 1 bis 4 Susbstituenten ausgewählt aus der Gruppe C₁-C₂-Alkyl, Halogen, -CN und C₁-C₂-Alkoxy substituiert sein;
R^{14a}, R^{14b} stehen unabhängig voneinander für H, -CN, -C(=O)OR¹⁸, C₁-C₆-Alkyl, C₂-C₆-Alkenyl, C₂-C₆-Alkynyl, C₃-C₈-Cycloalkyl, C₃-C₈-Halocycloalkyl, C₃-C₈-Cycloalkenyl, C₄-C₁₀-Cycloalkylalkyl, C₄-C₁₀-Alkylcycloalkyl, C₅-C₁₀-Alkylcycloalkylalkyl, C₁-C₆-Alkylamino, C₂-C₆-Dialkylamino, C₂-C₆-Alkylaminoalkyl, C₂-C₆-Haloalkylaminoalkyl, C₄-C₆-Cycloalkylaminoalkyl, C₃-C₆-Dialkylaminoalkyl, C₃-C₆-Halodialkylaminoalkyl, C₅-C₁₀-Cycloalkyl(alkyl)aminoalkyl, C₁-C₆-Alkoxy, C₁-C₆-Haloalkoxy, C₃-C₁-Cycloalkoxy, C₁-C₁₀-Alkylthio, C₁-C₁₀-Haloalkylthio, C₃-C₁₀-Cycloalkylthio, C₃-C₁₀-Trialkylsilyl or C₃-C₁₀-Halotrialkylsilyl; oder Phenyl oder einen 5- oder 6-gliedrigen heteroaromatischen Ring, ein 8-, 9- oder 10-gliedrigen heteroaromatisches bicyclisches Ringsystem, oder einen 5- oder 6-gliedrigen heterocyclischen nicht aromatischen Ring, der gegebenenfalls Ringglieder ausgewählt aus der Gruppe NR³, C(=O), C(=S), C(=NR⁴), SiR^{5a}R^{5b} und S(=O)ₚ(=NR⁴)_{q} enthält; jeder Ring oder jedes Ringsystem kann an den Ringkohlenstoffatomen gegebenenfalls substituiert sein mit 1 bis 5 Substituenten ausgewählt aus der Gruppe bestehend aus C₁-C₃-Alkyl, Halogen, -CN and C₁-C₃-Alkoxy;
oder
R^{14a}, R^{14b} bilden zusammen mit dem Kohlenstoffatom, an das sie gebunden sind, einen 3- bis 6-gliedrigen Ring, besagter Ring kann gegebenenfalls Ringkomponenten enthalten ausgewählt aus der Gruppe NR³, C(=O), C(=S), C(=NR⁴), SiR^{5a}R^{5b} oder S(=O)p(=NR⁴)_{q} und kann gegebenenfalls an den Ringkohlenstoffatomen mit 1 bis 4 Susbstituenten ausgewählt aus der Gruppe C₁-C₂-Alkyl, Halogen, -CN und C₁-C₂-Alkoxy substituiert sein;
p, q stehen unabhängig voneinander für 0, 1 oder 2 vorausgesetzt, dass die Summe von p und q 1 oder 2 ist;
R¹⁸ steht für C₁-C₆-Alkyl, C₁-C₆-Haloalkyl, C₃-C₆-Cycloalkyl, C₄-C₇-Cycloalkylalkyl oder C₄-C₇-Alkylcycloalkyl,
W steht für H
oder
W steht für Halogen, CN, C₁-C₄-Haloalkyl, C₁-C₄-Alkyl, C₁-C₄-Alkoxy
sowie agrochemisch wirksamen Salzen davon.

2. Verbindungen der Formel (**I**) nach Anspruch 1,
in welcher die Symbole folgende Bedeutungen haben,
Y bildet zusammen mit dem angrenzenden Stickstoffatom "1" und den beiden Kohlenstoffatomen "2" und "3" einen 5- bis 7-gliedrigen nicht aromatischen heterocyclischen Ring, dessen weitere Ringglieder aus der Gruppe C(R²)₂, O, S, SO₂, NR³, -C(R²)=C(R²)-, C(=O) und C(=S); ausgewählt sind;
R² steht jeweils unabhängig voneinander für H, Halogen, Cyano, Hydroxy, -CHO, - C(=O)OR⁶, -C(=O)NHOR^{6a}, C₁-C₅-Alkyl, C₂-C₅-Akenyl, C₂-C₅-Alkynyl, C₃-C₆-Cycloalkyl, C₃-C₆-Halocycloalkyl, C₃-C₆-Cycloalkenyl, C₁-C₅-Haloalkyl, C₁-C₅-Alkoxy, C₁-C₅-Haloalkoxy, C₃-C₆-Cycloalkoxy, C₂-C₅-Alkenyloxy, C₃-C₅-Haloalkenyloxy, C₂-C₅-Alkynyloxy, C₂-C₅-Alkylcarbonyl, C₁-C₅-Alkylthio, C₁-C₅-Haloalkylthio oder C₃- C₆-Cycloalkylthio;
R³ steht für H, -CN, -C(=O)NH₂, -C(=O)NHCN, -CHO, -C(=O)OR⁶, -C(=O)NHOR^{6a}, C₁-C₅-Alkyl, C₂-C₅-Haloalkyl, C₁-C₄-Alkylcarbonyl, C₁-C₄-Haloalkylcarbonyl, C₃-C₆-Cycloalkylcarbonyl, C₁-C₄-Alkoxycarbonyl, C₁-C₄-Haloalkoxycarbonyl, C₃-C₆-Cycloalkoxycarbonyl, C₃-C₆-Alkoxyalkylcarbonyl, C₃-C₆-Allcoxyallcoxycarbonyl, C₁-C₄-(Alkylthio)carbonyl, C₁-C₄-Alkoxy(thiocarbonyl), C₁-C₄-Alkyl(thiocarbonyl), C₁-C₄-Alkylthio(thiocarbonyl), C₁-C₄-Alkylaminocarbonyl, C₃-C₆-Cycloalkylaminocarbonyl, C₂-C₆-Dialkylaminocarbonyl, C₂-C₆-Alkylamino(thiocarbonyl), C₂-C₆-Dialkylamino(thiocarbonyl) oder C₃-C₆-Alkoxy(alkyl)aminocarbonyl;
R⁶ steht jeweils für H or C₁-C₄-Alkyl;
R^{6a} steht jeweils für C₁-C₄-Alkyl;
A steht für einen Phenylring der gegebenenfalls einfach oder mehrfach mit R⁷ substituiert sein kann oder für einen Thiophenylring der gegebenenfalls einfach oder mehrfach mit R⁸ substituiert sein kann;
R⁷ steht unabhängig voneinander für Halogen, C₁-C₄-Alkyl, C₁-C₄-Haloalkyl, Cyano, C₁-C₄-Alkoxy, C₁-C₄-Haloalkoxy, C₁-C₄-Alkylthio, C₁-C₄-Alkylsulfinyl, C₁-C₄-Alkylsulfonyl oder C₁-C₄-Haloalkylthio;
R⁸ steht unabhängig voneinander für Halogen, Cyano, C₁-C₃-Alkyl, C₁-C₃-Alkoxy, C₁-C₃-Haloalkyl;
R¹ steht für H, Halogen, C₁-C₃ Alkyl, Cyano, -NR^{9a}R^{9b}, -N(R^{9b})COR^{9a}, -N(R^{9b})CSR^{9a}, -N(R^{9b})COOR¹², -OR¹², -S(O)mR^{6a}, COOR₁₂ oder -CONR^{9a}R^{9b};
R^{9a} steht jeweilsfür H, C₁-C₆-Alkyl, C₂-C₆-Alkenyl, C₂-C₆-Alkynyl, C₁-C₆-Haloalkyl, C₂-C₆-Haloalkenyl, C₂-C₆-Haloalkynyl, C₂-C₆-Alkoxyalkyl, C₃-C₆-Alkoxyalkoxyalkyl, C₃-C₆-Alkoxyalkenyl, C₃-C₆-Alkoxyalkynyl, C₃-C₆-Dialkoxyalkyl, C₂-C₆-Haloalkoxyalkyl, C₂-C₆-Alkoxyhaloalkyl, C₂-C₆-Haloalkoxyhaloalkyl, C₁-C₆-Hydroxyalkyl, C₁-C₆-Cyanoalkyl, C₂-C₆-Alkylthioalkyl, C₂-C₆-Alkylsulfinylalkyl, C₃-C₆-Alkylaminoalkyl, C₃-C₆-Haloalkylaminoalkyl, C₅-C₁₀-Cycloalkylaminoalkyl C₄-C₁₀-Dialkylaminoalkyl, C₄-C₁₀-Halodialkylaminoalkyl oder -(CR^{15a}R^{15b})ₘR¹⁶;
R^{9b} steht jeweils für H, C₁-C₆-Alkyl, C₂-C₆-Alkenyl, C₂-C₆-Alkynyl, C₁-C₆-Haloalkyl or - (CR^{15a}R^{15b})ₘR¹⁶
oder
R^{9a},R^{9b} bilden jeweils zusammen mit dem Stickstoff oder der (NCO) oder der (NCS) Einheit, mit dem/ mit der sie verknüpft sind, einen 3 bis 6-gliedrigen Ring, der gegebenenfalls zusätzlich auch Ringglieder enthalten kann ausgewählt aus der Gruppe NR³, C(=0), C(=S), O und gegebenenfalls an den Ringkohlenstoffatomen substituiuert ist mit 1 bis 4 Substituenten ausgewählt aus der Gruppe bestehend aus Halogen, -CN, C₁-C₂-Alkyl und C₁-C₂-Alkoxy;
R¹² steht jeweils für C₁-C₆-Alkyl, C₂-C₆-Akenyl, C₂-C₆-Alkynyl, C₁-C₆-Haloalkyl, C₂-C₆-Haloalkenyl oder -(CR^{15a}R^{15b})ₘR¹⁶;
R^{15a}, R¹⁵ steht unabhängig voneinander für H, Halogen oder C₁-C₄-Alkyl;
R¹⁶ steht für Phenyl, C₃-C₈-Cycloalkyl, C₃-C₈-Cycloalkenyl, einen 5- oder 6-gliedrigen heteroaromatischen Ring oder Naphthalenyl oder ein 8-, 9- oder 10-gliedriges heteroaromatisches bicyclisches Ringsystem; oder einen 5- oder 6-gliedrigen heterocyclischen nicht aromatischen Ring, der gegebenenfalls Ringglieder ausgewählt aus der Gruppe C(=O), C(=S), C(=NR⁴) enthält; jeder Ring oder jedes Ringsystem kann an den Ringkohlenstoffatomen gegebenenfalls substituiert sein mit bis zu 3 Substituenten unabhängig voneinander ausgewählt von R¹⁷;
R¹⁷ steht jeweils unabhängig voneinander für Halogen, Cyano, C₁-C₆-Alkyl, C₂-C₆-Alkenyl, C₂-C₆₋Alkynyl, C₃-C₆-Cycloalkyl, C₁-C₆-Haloalkyl, C₁-C₆-Alkoxy;
m steht für 0, 1 oder 2,
W steht für H
oder
W steht für Fluor, Chlor, CN, CF₃, Methyl, Ethyl, Methoxy
sowie agrochemisch wirksamen Salzen davon.

3. Verbindungen der Formel (I) nach einem oder mehreren der Ansprüche 1 bis 2,
in welcher die Symbole folgende Bedeutungen haben,
Y bildet zusammen mit dem angrenzenden Stickstoffatom "1" und den beiden Kohlenstoffatomen "2" und "3" einen 5- bis 7-gliedrigen nicht aromatischen heterocyclischen Ring, ausgewählt aus der Gruppe: H-1, H-2, H-3, H-4, H-5, H-6, H-7, H-8, H-9 und H-10 dargestellt in Schema 1, wobei s eine Zahl zwischen 0 und 4 ist; 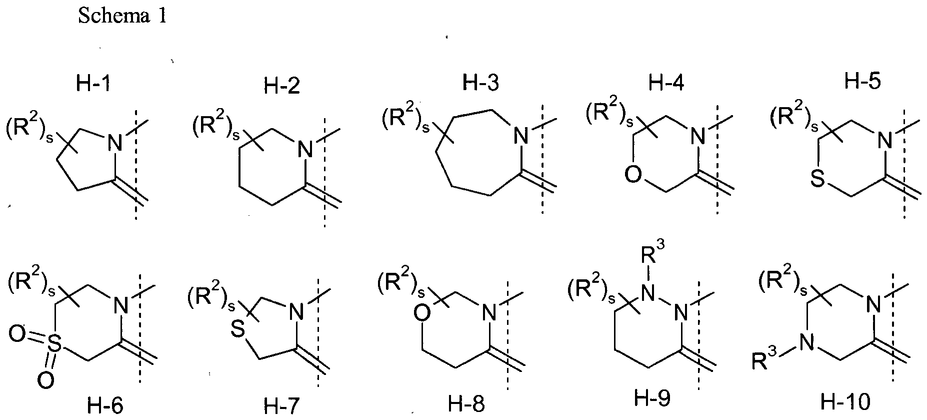
R² steht jeweils unabhängig voneinander für H, F, Cl, Br, I, Cyano, Hydroxy, -CHO, - C(=O)OR⁶, Methyl, Ethyl, iso-Propyl, n-Propyl, Trifluormethyl, Difluormethyl, Dichlormethyl, Pentafluorethyl, Methoxy, Ethoxy, n-Propoxy, iso-Propoxy, Trifluormethoxy, Difluormethoxy, oder Acetyl, Propionyl, Isobutyryl, 2,2-Dimethylpropanoyl;
R³ steht für H, -CHO, Methyl, Ethyl, iso-Propyl, n-Propyl, Acetyl, Propionyl, Isobutyryl, 2,2-Dimethylpropanoyl, Trifluoracetyl, Difluoracetyl, CH₃OC(O), CH₃CH₂C(O), (CH₃)₂CHC(O) oder CF₃OC(O), CF₂HOC(O);
R⁶ steht jeweilsfür H, Methyl, Ethyl, iso-Propyl, n-Propyl;
A steht für einen Phenyl -oder Thiophenring, der gegebenenfalls mit Resten ausgewählt aus F, Cl, Br, I, Cyano, Methyl, Ethyl, iso-Propyl, n-Propyl, Trifluormethyl, Difluormethyl, Dichlormethyl, Pentafluorethyl, oder Methoxy, Ethoxy, n-Propoxy, iso-Propoxy substituiert ist;
R¹ steht für H, F, Cl, Br, I, CH₃, S(O)ₘMe, -NR^{9a}R^{9b}, N(R^{9b})COR^{9a}, N(R^{9b})COOR¹²;
R^{9a} steht jeweils für H, Methyl, Ethyl, iso-Propyl, n-Propyl, n-Butyl, sec-Butyl, tert-Butyl, iso-Butyl, n-Pentyl, n-Hexyl" -CH=CH₂, -CH₂CH=CH₂, -CH=CHCH₃, - CH₂C=CH, -C≡CH" Trifluormethyl, Difluormethyl, Dichlormethyl, Pentafluorethyl, Methoxymethyl, Ethoxymethyl, Methoxyethyl, tert-Butoxymethyl, Hydroxymethyl, 1-Hydroxyethyl, 2-Hydroxypropan-2-yl oder -(CH₂)ₘR¹⁶;
R^{9b} steht jeweils für H, Methyl, Ethyl, iso-Propyl, n-Propyl, n-Butyl, sec-Butyl, tert-Butyl, iso-Butyl, n-Pentyl, n-Hexyl, -CH₂CH=CH₂, -CH=CHCH₃, -CH₂C≡CH, - C=CH;
R^{9a},R^{9b} bilden jeweils zusammen mit dem Stickstoff, mit dem sie verknüpft sind, einen 5 bis 6-gliedrigen Ring welcher gegebenenfalls an den Ringkohlenstoffatomen substituiert ist mit 1 bis 2 Substituenten ausgewählt aus der Gruppe bestehend aus F, Cl, Br, I, -CN and Methyl, Ethyl;
R¹² steht für Methyl, Ethyl, iso-Propyl, n-Propyl, n-Butyl, sec-Butyl, tert-Butyl, iso-Butyl, -CH₂CH=CH₂, -CH=CHCH₃, CH₂C≡CH, -C≡CH, Trifluormethyl, Difluormethyl, Dichlormethyl, oder -(CH₂)ₘR¹⁶;
R¹⁶ ist steht für cyclopropyl, cyclobutyl, cyclopentyl, cyclohexyl, cyclopentenyl, cyclohexenyl, Phenyl oder Thienyl, von denen ein jeder gegebenenfalls mit bis zu 2 Resten ausgewählt aus der Gruppe R¹⁷ substituiert sein kann;
R¹⁷ steht jeweils unabhängig voneinander für F, Cl, Br, I , Methyl, Ethyl, iso-Propyl, n-Propyl, n-Butyl, sec-Butyl, tert-Butyl, iso-Butyl, Trifluormethyl, Difluormethyl, Dichlormethyl, Methoxy, Ethoxy oder Cyano;
m ist 0, 1 und 2,
W steht für H
oder
W steht für Fluor, Chlor, CN, CF₃, Methyl, Ethyl
sowie agrochemisch wirksamen Salzen davon als Fungizide.

4. Verbindungen der Formel (I) nach einem oder mehreren der Ansprüche 1 bis 3,
in welcher die Symbole folgende Bedeutungen haben:
Y bildet zusammen mit dem angrenzenden Stickstoffatom "1" und den beiden Kohlenstoffatomen "2" und "3" einen 5- bis 7-gliedrigen nicht aromatischen heterocyclischen Ring, ausgewählt aus der Gruppe: H-1, H-2, H-3, H-4, H-5 and H-8, dargestellt in Schema 2, wobei s eine Zahl zwischen 0 und 4 ist; 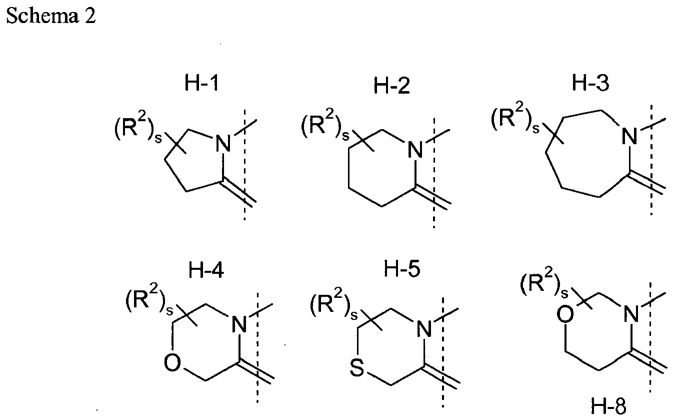
R² steht jeweils unabhängig voneinander für H, Fluor, Chlor, Cyano, CF₃, Methyl, oder Methoxy;
A steht für einen Phenyl -oder Thiophenring, der gegebenenfalls mit Resten ausgewählt aus F, Cl, Cyano, CH₃, CF₃ substituiert ist;
R¹ steht für H, Fluor, Chlor, S(O)ₘMe, NR^{9a}R^{9b}, N(R^{9b})COR^{9a}, N(R^{9b})COOR¹²;
R^{9a} steht jeweils für H, Methyl, Ethyl, n-Propyl, iso-Propyl, n-Butyl, iso-Butyl, sec-Butyl, tert-Butyl, n-Pentyl, n-Hexyl, Methoxymethyl, Ethoxymethyl, Ethoxyethyl, Methoxyethyl, Methoxypropyl, Hydroxymethyl, 1-Hydroxyethyl, 2-Hydroxyethyl, Hydroxypropyl oder -(C_{H2})ₘR¹⁶;
R^{9b} steht jeweils für H, Methyl, Ethyl, n-Propyl, iso-Propyl, n-Butyl, Allyl oder Propargyl;
R¹² steht für Methyl, Ethyl, n-Propyl, iso-Propyl, t-Butyl, Allyl, Propargyl oder - (CH₂)ₘR¹⁶;
R¹⁶ steht für Cyclopropyl, Cyclopentyl, Cyclobutyl, Cyclohexyl, Thienyl oder Phenyl, von denen ein jeder gegebenenfalls mit einem Rest ausgewählt aus der Gruppe R¹⁷ substituiert sein kann;
m steht für 0, 1 oder 2;
R¹⁷ steht für Methyl, Ethyl, Fluor, Chlor, CF₃, OMe, Cyano;
W steht für H,
oder
W steht für Fluor, Chlor, Cyano;
sowie agrochemisch wirksamen Salzen davon als Fungizide.

5. Nicht therapeutisches Verfahren zur Bekämpfung phytopathogener und Mykotoxin produzierender Pilze, **dadurch gekennzeichnet, dass** man bicyclische Pyridinylpyrazole der Formel (**I**) gemäß einem oder mehreren der Ansprüche 1 bis 4 auf die Pilze und/oder deren Lebensraum ausbringt.

6. Mittel zur Bekämpfung phytopathogener und Mykotoxin produzierender Pilze, **gekennzeichnet durch** einen Gehalt an mindestens einem bicyclischen Pyridinylpyrazol der Formel **(I)** gemäß einem oder mehreren der Ansprüche 1 bis 4 neben Streckmitteln und/oder oberflächenaktiven Stoffen.

7. Verwendung von bicyclischen Pyridinylpyrazole der Formel **(I),** gemäß einem oder mehreren der Ansprüche 1 bis 4 zur Bekämpfung von pflanzenpathogenen Schadpilzen.

8. Verfahren zur Herstellung von Mitteln zur Bekämpfung von pflanzenpathogenen Schadpilzen, **dadurch gekennzeichnet, dass** man bicyclische Pyridinylpyrazole der Formel **(I)** gemäß einem oder mehreren der Ansprüche 1 bis 4 mit Streckmitteln und/oder oberflächenaktiven Stoffen vermischt.

9. Verwendung von Verbindungen der Formel **(I)** gemäß einem oder mehreren der Ansprüche 1 bis 4 zur Behandlung von transgenen Pflanzen.

10. Verwendung von Verbindungen der Formel **(I)** gemäß einem oder mehreren der Ansprüche 1 bis 4 zur Behandlung von Saatgut sowie von Saatgut transgener Pflanzen.

## Claims

1. Compounds of the formula **(I)** 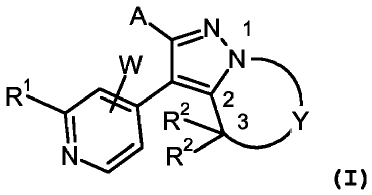 in which the symbols have the following meanings:
Y together with the adjacent nitrogen atom "1" and the two carbon atoms "2" and "3" forms a 5- to 7-membered non-aromatic heterocyclic ring whose further ring members are selected from the group consisting of C(R²)₂, O, S, NR³, C(R²)=C(R²), C(R²)=N, N=N, C(=O), C(=S), C(=NR⁴), S (=O)ₚ(=NR⁴)_{q} and SiR^{5a}R^{5b};
R² represent in each case independently of one another H, halogen, cyano, hydroxyl, -CHO, - NHCHO, -N₃, -N=C=O, -N=C=S, -SH, -C(=O)NH₂, - C(=O)NHCN, -C(=O)OR⁶, -C (=O)NHOR^{6a}, C₁-C₅-alkyl, C₂-C₅-alkenyl, C₂-C₅-alkynyl, C₃-C₆-cycloalkyl, C₃-C₆-halocycloalkyl, C₃-C₆-cycloalkenyl, C₁-C₅-haloalkyl, C₁-C₅-alkoxy, C₁-C₅-haloalkoxy, C₃-C₆-cycloalkoxy, C₂-C₅-alkenyloxy, C₃-C₅-haloalkenyloxy, C₂-C₅-alkynyloxy, C₂-C₅-alkylcarbonyl, C₂-C₅-alkylcarbonyloxy, C₂-C₅-haloalkylcarbonyloxy, C₃-C₅-alkoxycarbonylalkoxy, C₁-C₅-alkylthio, C₁-C₅-haloalkylthio, C₃-C₆-cycloalkylthio, C₂-C₅-alkyl(thiocarbonyl), C₂-C₅-alkylthio(thiocarbonyl), C₁-C₅-alkylsulphinyl, C₁-C₅-haloalkylsulphinyl, C₃-C₆-cycloalkylsulphinyl, C₁-C₅-alkylsulphonyl, C₁-C₅-haloalkylsulphonyl, C₃-C₆-cycloalkylsulphonyl, C₃-C₅-trialkylsilyl, C₃-C₅-halotrialkylsilyl, C₁-C₅-alkylamino, C₂-C₅-haloalkylamino, C₃-C₆-cycloalkylamino, C₂-C₅-dialkylamino or C₃-C₅-halodialkylamino;
R³ represents H, -CN, -C(=O)NH₂, -C(=O)NHCN, -CHO, -NHCHO, -C(=O)OR⁶, -C(=O)NHOR^{6a}, hydroxyl, C₁-C₅-alkyl, C₂-C₅-alkenyl, C₂-C₅-alkynyl, C₃-C₆-cycloalkyl, C₃-C₆-halocycloalkyl, C₃-C₆-cycloalkenyl, C₄-C₁₀-cycloalkylalkyl, C₄-C₇-alkylcycloalkyl, C₅-C₇-alkylcycloalkylalkyl, C₂-C₅-haloalkyl, C₁-C₄-alkylcarbonyl, C₁-C₄-haloalkylcarbonyl, C₃-C₇-cycloalkylcarbonyl, C₁-C₆-alkoxycarbonyl, C₁-C₄-haloalkoxycarbonyl, C₃-C₆-cycloalkoxycarbonyl, C₂-C₆-alkoxyalkylcarbonyl, C₂-C₆-alkoxyalkoxycarbonyl, C₁-C₆-(alkylthio) carbonyl, C₁-C₆-alkoxy(thiocarbonyl), C₁-C₆-alkyl(thiocarbonyl), C₁-C₆-alkylthio(thiocarbonyl), C₁-C₆-alkylaminocarbonyl, C₃-C₆-cycloalkylaminocarbonyl, C₂-C₆-dialkylaminocarbonyl, C₁-C₆-alkylamino(thiocarbonyl), C₂-C₆-dialkylamino(thiocarbonyl), C₂-C₆-alkoxy(alkyl)aminocarbonyl, C₁-C₅-alkoxy, C₁-C₅-haloalkoxy, C₁-C₅-alkylthio, C₁-C₅-haloalkylthio, C₃-C₆-cycloalkylthio, C₁-C₅-alkylaminosulphonyl, C₃-C₅-trialkylsilyl or C₃-C₅-halotrialkylsilyl;
R⁴ in each case represents H, cyano, amino, hydroxyl, C₁-C₆-alkyl, C₃-C₆-cycloalkyl, C₁-C₄-alkylcarbonyl, C₁-C₄-haloalkylcarbonyl, C₁-C₄-alkoxy, phenyl or benzoyl;
R^{5a}, R^{5b} independently of one another represent C₁-C₄-alkyl, C₂-C₅-alkenyl, C₂-C₅-alkynyl, C₃-C₅-cycloalkyl, C₃-C₆-halocycloalkyl, C₄-C₇-cycloalkylalkyl, C₄-C₇-alkylcycloalkyl, C₅-C₇-alkylcycloalkylalkyl, C₁-C₅-haloalkyl, C₁-C₅-alkoxy or C₁-C₅-haloalkoxy;
R⁶ in each case represents H, C₁-C₆-alkyl, C₁-C₆-haloalkyl, C₃-C₆-cycloalkyl, C₄-C₇-cycloalkylalkyl, C₄-C₇-alkylcycloalkyl or benzyl;
R^{6a} in each case represents C₁-C₆-alkyl, C₁-C₆-haloalkyl, C₃-C₆-cycloalkyl, C₄-C₇-cycloalkylalkyl or C₄-C₇-alkylcycloalkyl;
A represents a phenyl ring which may optionally be mono- or polysubstituted by R⁷ or represents a thiophenyl ring which may optionally be mono- or polysubstituted by R⁸;
R⁷ independently of one another represent halogen, C₁-C₆-alkyl, C₂-C₆-alkenyl, C₂-C₆-alkynyl, C₃-C₆-cycloalkyl, C₃-C₆-halocycloalkyl, C₄-C₇-alkylcycloalkyl, C₁-C₆-haloalkyl, C₂-C₆-haloalkenyl, cyano, nitro, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl, C₁-C₆-alkylamino, C₂-C₆-dialkylamino, C₁-C₆-alkylcarbonyl, C₁-C₆-alkoxycarbonyl, C₁-C₆-alkylaminocarbonyl, C₂-C₆-dialkylaminocarbonyl or C₃-C₆-trialkylsilyl;
R⁸ independently of one another represent halogen, cyano, C₁-C₃-alkyl; C₁-C₃-alkoxy, C₁-C₃-haloalkyl, C₁-C₃-haloalkoxy;
R¹ represents H, halogen, cyano, hydroxyl, C₁-C₄-alkyl, CONR^{9a}R^{9b}, COOH, COOR¹², -NR^{9a}R^{9b}, - N (R^{9b}) COR^{9a}, -N (R^{9b}) CSR^{9a}, -N (R^{9b}) COOR¹², - N (R^{9b}) SO₂R¹², -NR¹⁰-NR^{11a}R^{11b}, -S (O)ₘR¹², -OR¹², - N=CR^{13a}R^{13b} or -NR¹⁰N=CR^{14a}R^{14b};
R^{9a} and R^{11a} independently of one another each represent H, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₂-C₆-alkoxyalkyl, C₃-C₆-alkoxyalkoxyalkyl, C₃-C₆-alkoxyalkenyl, C₃-C₆-alkoxyalkynyl, C₃-C₆-dialkoxyalkyl, C₄-C₁₀-trialkoxyalkyl, C₂-C₆-haloalkoxyalkyl, C₂-C₆-alkoxyhaloalkyl, C₂-C₆-haloalkoxyhaloalkyl, C₁-C₆-hydroxyalkyl, C₁-C₁₀-cyanoalkyl, C₂-C₆-alkylthioalkyl, C₂-C₆-alkylsulphinylalkyl, C₃-C₆-alkylaminoalkyl, C₃-C₆-haloalkylaminoalkyl, C₅-C₁₀-cycloalkylaminoalkyl, C₄-C₁₀-dialkylaminoalkyl, C₄-C₁₀-halodialkylaminoalkyl, C₅-C₁₀-cycloalkyl (alkyl) aminoalkyl or - (CR^{15a}R^{15b})ₘR¹⁶;
R^{9b} and R^{11b} independently of one another each represent H, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₂-C₆-alkoxyalkyl, C₃-C₆-alkoxyalkoxyalkyl, C₃-C₆-alkoxyalkenyl, C₃-C₆-alkoxyalkynyl, C₃-C₆-dialkoxyalkyl, C₄-C₁₀-trialkoxyalkyl, C₂-C₆-haloalkoxyalkyl, C₂-C₆-alkoxyhaloalkyl, C₂-C₆-haloalkoxyhaloalkyl, C₁-C₆-hydroxyalkyl, C₁-C₁₀-cyanoalkyl, C₂-C₆-alkylthioalkyl, C₂-C₆-alkylsulphinylalkyl, C₃-C₆-alkylaminoalkyl, C₃-C₆-haloalkylaminoalkyl, C₅-C₁₀-cycloalkylaminoalkyl, C₄-C₁₀-dialkylaminoalkyl, C₄-C₁₀-halodialkylaminoalkyl, C₅-C₁₀-cycloalkyl (alkyl) aminoalkyl or - (CR^{15a}R^{15b})ₘR¹⁶;
or
R^{9a} and R^{9b} or R^{11a} and R^{11b} in each case together with the nitrogen atom or the (NCO) unit or the (NCS) unit to which they are attached form a 3-to 6-membered ring which may optionally additionally comprise ring members selected from the group consisting of O, NR³, C(=O), C(=S), C (=NR⁴), SiR^{5a}R^{5b} and S(=O)ₚ(=NR⁴)_{q} and which may optionally be substituted at the ring carbon atoms by 1 to 4 substituents selected from the group consisting of halogen, -CN, C₁-C₂-alkyl and C₁-C₂-alkoxy;
R¹² in each case represents C₁-C₁₀-alkyl, C₂-C₁₀-alkenyl, C₂-C₁₀-alkynyl, C₁-C₁₀-haloalkyl, C₂-C₁₀-haloalkenyl, C₂-C₁₀-haloalkynyl, C₂-C₁₀-alkoxyalkyl, C₃-C₁₀-alkoxyalkoxyalkyl, C₃-C₁₀-alkoxyalkenyl, C₃-C₁₀-alkoxyalkynyl, C₃-C₁₀-dialkoxyalkyl, C₄-C₁₀-trialkoxyalkyl, C₂-C₁₀-haloalkoxyalkyl, C₂-C₁₀-alkoxyhaloalkyl, C₂-C₁₀-haloalkoxyhaloalkyl, C₂-C₁₀-hydroxyalkyl, C₂-C₁₀-cyanoalkyl, C₂-C₁₀-alkylthioalkyl, C₂-C₁₀-alkylsulphinylalkyl, C₃-C₁₀-alkylaminoalkyl, C₃-C₁₀-haloalkylaminoalkyl, C₅-C₁₀-cycloalkylaminoalkyl, C₄-C₁₀-dialkylaminoalkyl, C₄-C₁₀-halodialkylaminoalkyl, C₆-C₁₀-cycloalkyl (alkyl) -aminoalkyl or - (CR^{15a}R^{15b})ₘR¹⁶;
R^{15a}, R^{15b} independently of one another represent H, halogen, C₁-C₅-alkyl, C₁-C₅-haloalkyl or C₁-C₅-alkoxy;
or
a geminal pair of R^{15a} and R^{15b} together with the carbon atom to which it is attached forms C(=O) or a C₃-C₆-cycloalkyl ring or a C₃-C₆-halocycloalkyl ring;
R¹⁶ represents phenyl, C₃-C₈-cycloalkyl, C₃-C₈-cycloalkenyl, a 5- or 6-membered heteroaromatic ring or naphthalenyl or an 8-, 9- or 10-membered heteroaromatic bicyclic ring system; or a 5- or 6-membered heterocyclic non-aromatic ring which optionally contains ring members selected from the group consisting of C(=O), C(=S), C(=NR⁴), SiR^{5a}R^{5b} and S(=O)ₚ(=NR⁴)_{q}; where each ring or each ring system may optionally be substituted at the ring carbon atoms by up to 5 substituents independently of one another selected from R¹⁷;
R¹⁷ in each case independently of one another represent halogen, C₁-C₆-alkyl, C₂-C₆-alkenyl, C₂-C₆-alkynyl, C₃-C₆-cycloalkyl, C₄-C₁₀-alkylcycloalkyl, C₃-C₆-halocycloalkyl, C₁-C₆-haloalkyl, C₂-C₆-haloalkenyl, cyano, nitro, C₁-C₆-alkoxy, C₃-C₈-cycloalkoxy, C₃-C₈-halocycloalkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl, C₁-C₆-alkylamino, C₁-C₆-dialkylamino, C₁-C₆-alkylcarbonyl, C₁-C₆-alkoxycarbonyl, C₁-C₆-alkylaminocarbonyl, C₃-C₆-dialkylaminocarbonyl, C₃-C₆-trialkylsilyl, phenyl, naphthalenyl or a 5- or 6-membered heteroaromatic ring;
m represents 0, 1 or 2;
R¹⁰ in each case represents H, C₁-C₅-alkyl, C₂-C₅-alkenyl, C₂-C₅-alkynyl, C₃-C₆-cycloalkyl, C₃-C₆-halocycloalkyl, C₁-C₅-haloalkyl, C₂-C₅-haloalkenyl, C₂-C₅-haloalkynyl, C₂-C₅-alkoxyalkyl, C₂-C₅-alkylcarbonyl or C₁-C₅-alkoxy;
R^{13a}, R^{13b} independently of one another represent H, -CN, -C(=O)OR¹⁸, C₁-C₆-alkyl, C₂-C₆-alkenyl, C₂-C₆-alkynyl, C₃-C₈-cycloalkyl, C₃-C₈-halocycloalkyl, C₃-C₈-cycloalkenyl, C₄-C₁₀-cycloalkylalkyl, C₄-C₁₀-alkylcycloalkyl, C₅-C₁₀-alkylcycloalkylalkyl, C₁-C₆-alkylamino, C₂-C₆-dialkylamino, C₂-C₆-alkylaminoalkyl, C₂-C₆-haloalkylaminoalkyl, C₄-C₆-cycloalkylaminoalkyl, C₃-C₆-dialkylaminoalkyl, C₃-C₆-halodialkylaminoalkyl, C₅-C₁₀-cycloalkyl(alkyl)aminoalkyl, C₁-C₆-alkoxy, C₁-C₆-haloalkoxy, C₃-C₁₀-cycloalkoxy, C₁-C₁₀-alkylthio, C₁-C₁₀-haloalkylthio, C₃-C₁₀-cycloalkylthio, C₃-C₁₀-trialkylsilyl or C₃-C₁₀-halotrialkylsilyl, or phenyl or a 5- or 6-membered heteroaromatic ring, an 8-, 9- or 10-membered heteroaromatic bicyclic ring system, or a 5- or 6-membered heterocyclic non-aromatic ring which optionally contains ring members selected from the group consisting of NR³, C(=O), C(=S), C(=NR⁴), SiR^{5a}R^{5b} and S(=O)ₚ(=NR⁴)_{q}; where each ring or each ring system may optionally be substituted at the ring carbon atoms by 1 to 5 substituents selected from the group consisting of C₁-C₃-alkyl, halogen, -CN and C₁-C₃-alkoxy;
or
R^{13a} and R^{13b} together with the carbon atom to which they are attached form a 3- to 6-membered ring, where said ring may optionally contain ring members selected from the group consisting of NR³, C(=O), C(=S), C(=NR⁴), SiR^{5a}R^{5b} and S(=O)ₚ(=NR⁴)_{q} and may optionally be substituted at the ring carbon atoms by 1 to 4 substituents selected from the group consisting of C₁-C₂-alkyl, halogen, -CN and C₁-C₂-alkoxy;
R^{14a}, R^{14b} independently of one another represent H, -CN, -C(=O)OR¹⁸, C₁-C₆-alkyl, C₂-C₆-alkenyl, C₂-C₆-alkynyl, C₃-C₈-cycloalkyl, C₃-C₈-halocycloalkyl, C₃-C₈-cycloalkenyl, C₄-C₁₀-cycloalkylalkyl, C₄-C₁₀-alkylcycloalkyl, C₅-C₁₀-alkylcycloalkylalkyl, C₁-C₆-alkylamino, C₂-C₆-dialkylamino, C₂-C₆-alkylaminoalkyl, C₂-C₆-haloalkylaminoalkyl, C₄-C₆-cycloalkylaminoalkyl, C₃-C₆-dialkylaminoalkyl, C₃-C₆-halodialkylaminoalkyl, C₅-C₁₀-cycloalkyl(alkyl)aminoalkyl, C₁-C₆-alkoxy, C₁-C₆-haloalkoxy, C₃-C₁₀-cycloalkoxy, C₁-C₁₀-alkylthio, C₁-C₁₀-haloalkylthio, C₃-C₁₀-cycloalkylthio, C₃-C₁₀-trialkylsilyl or C₃-C₁₀-halotrialkylsilyl; or phenyl or a 5- or 6-membered heteroaromatic ring, an 8-, 9- or 10-membered heteroaromatic bicyclic ring system, or a 5- or 6-membered heterocyclic non-aromatic ring which optionally contains ring members selected from the group consisting of NR³, C(=O), C(=S), C(=NR⁴), SiR^{5a}R^{5b} and S(=O)ₚ(=NR⁴)_{q}; where each ring or each ring system may optionally be substituted at the ring carbon atoms by 1 to 5 substituents selected from the group consisting of C₁-C₃-alkyl, halogen, -CN and C₁-C₃-alkoxy;
or
R^{14a}, R^{14b} together with the carbon atom to which they are attached form a 3- to 6-membered ring, where said ring may optionally contain ring components selected from the group consisting of NR³, C(=O), C(=S), C(=NR⁴), SiR^{5a}R^{5b} and S(=O)ₚ(=NR⁴)_{q} and may optionally be substituted at the ring carbon atoms by 1 to 4 substituents selected from the group consisting of C₁-C₂-alkyl, halogen, -CN and C₁-C₂-alkoxy;
p, q independently of one another represent 0, 1 or 2, provided the sum of p and q is 1 or 2;
R¹⁸ represents C₁-C₆-alkyl, C₁-C₆-haloalkyl, C₃-C₆-cycloalkyl, C₄-C₇-cycloalkylalkyl or C₄-C₇-alkylcycloalkyl,
W represents H
or
W represents halogen, CN, C₁-C₄-haloalkyl, C₁-C₄-alkyl, C₁-C₄-alkoxy
and agrochemically active salts thereof.

2. Compounds of the formula (**I**) according to Claim 1, in which the symbols have the following meanings:
Y together with the adjacent nitrogen atom "1" and the two carbon atoms "2" and "3" forms a 5- to 7-membered non-aromatic heterocyclic ring whose further ring members are selected from the group consisting of C(R²)₂, O, S, SO₂, NR³, -C(R²)=C(R²)-, C(=O) and C(=S);
R² represent in each case independently of one another H, halogen, cyano, hydroxyl, -CHO, -C(=O)OR⁶, -C(=O)NHOR^{6a}, C₁-C₅-alkyl, C₂-C₅-alkenyl, C₂-C₅-alkynyl, C₃-C₆-cycloalkyl, C₃-C₆-halocycloalkyl, C₃-C₆-cycloalkenyl, C₁-C₅-haloalkyl, C₁-C₅-alkoxy, C₁-C₅-haloalkoxy, C₃-C₆-cycloalkoxy, C₂-C₅-alkenyloxy, C₃-C₅-haloalkenyloxy, C₂-C₅-alkynyloxy, C₂-C₅-alkylcarbonyl, C₁-C₅-alkylthio, C₁-C₅-haloalkylthio or C₃-C₆-cycloalkylthio;
R³ represents H, -CN, -C(=O)NH₂, -C(=O)NHCN, -CHO, -C (=O) OR⁶, -C (=O) NHOR^{6a}, C₁-C₅-alkyl, C₂-C₅-haloalkyl, C₁-C₄-alkylcarbonyl, C₁-C₄-haloalkylcarbonyl, C₃-C₆-cycloalkylcarbonyl, C₁-C₄-alkoxycarbonyl, C₁-C₄-haloalkoxycarbonyl, C₃-C₆-cycloalkoxycarbonyl, C₃-C₆-alkoxyalkylcarbonyl, C₃-C₆-alkoxyalkoxycarbonyl, C₁-C₄-(alkylthio) carbonyl, C₁-C₄-alkoxy(thiocarbonyl), C₁-C₄-alkyl(thiocarbonyl), C₁-C₄-alkylthio(thiocarbonyl), C₁-C₄-alkylaminocarbonyl, C₃-C₆-cycloalkylaminocarbonyl, C₂-C₆-dialkylaminocarbonyl, C₂-C₆-alkylamino(thiocarbonyl), C₂-C₆-dialkylamino(thiocarbonyl) or C₃-C₆-alkoxy(alkyl)aminocarbonyl;
R⁶ in each case represents H or C₁-C₄-alkyl;
R^{6a} in each case represents C₁-C₄-alkyl;
A represents a phenyl ring which may optionally be mono- or polysubstituted by R⁷ or represents a thiophenyl ring which may optionally be mono- or polysubstituted by R⁸;
R⁷ independently of one another represent halogen, C₁-C₄-alkyl, C₁-C₄-haloalkyl, cyano, C₁-C₄-alkoxy, C₁-C₄-haloalkoxy, C₁-C₄-alkylthio, C₁-C₄-alkylsulphinyl, C₁-C₄-alkylsulphonyl or C₁-C₄-haloalkylthio;
R⁸ independently of one another represent halogen, cyano, C₁-C₃-alkyl, C₁-C₃-alkoxy, C₁-C₃-haloalkyl;
R¹ represents H, halogen, C₁-C₃-alkyl, cyano, - NR^{9a}R^{9b}, -N(R^{9b})COR^{9a}, -N(R^{9b})CSR^{9a}, -N(R^{9b})COOR¹², -OR¹², -S(O)ₘR^{6a}, COOR¹² or -CONR^{9a}R^{9b};
R^{9a} in each case represents H, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₂-C₆-alkoxyalkyl, C₃-C₆-alkoxyalkoxyalkyl, C₃-C₆-alkoxyalkenyl, C₃-C₆-alkoxyalkynyl, C₃-C₆-dialkoxyalkyl, C₂-C₆-haloalkoxyalkyl, C₂-C₆-alkoxyhaloalkyl, C₂-C₆-haloalkoxyhaloalkyl, C₁-C₆-hydroxyalkyl, C₁-C₆-cyanoalkyl, C₂-C₆-alkylthioalkyl, C₂-C₆-alkylsulphinylalkyl, C₃-C₆-alkylaminoalkyl, C₃-C₆-haloalkylaminoalkyl, C₅-C₁₀-cycloalkylaminoalkyl, C₄-C₁₀-dialkylaminoalkyl, C₄-C₁₀-halodialkylaminoalkyl or -(CR^{15a}R^{15b})ₘR¹⁶;
R^{9b} in each case represents H, C₁-C₆-alkyl, C₂-C₆-alkenyl, C₂-C₆-alkynyl, C₁-C₆-haloalkyl or - (CR^{15a}R^{15b})ₘR¹⁶;
or
R^{9a}, R^{9b} in each case together with the nitrogen atom or the (NCO) or the (NCS) unit to which they are attached form a 3- to 6-membered ring which may optionally additionally also comprise ring members selected from the group consisting of NR³, C(=O), C(=S), O and which is optionally substituted at the ring carbon atoms by 1 to 4 substituents selected from the group consisting of halogen, -CN, C₁-C₂-alkyl and C₁-C₂-alkoxy;
R¹² in each case represents C₁-C₆-alkyl, C₂-C₆-alkenyl, C₂-C₆-alkynyl, C₁-C₆-haloalkyl, C₂-C₆-haloalkenyl or -(CR^{15a}R^{15b})ₘR¹⁶;
R^{15a}, R^{15b} independently of one another represent H, halogen or C₁-C₄-alkyl;
R¹⁶ represents phenyl, C₃-C₈-cycloalkyl, C₃-C₈-cycloalkenyl, a 5- or 6-membered heteroaromatic ring or naphthalenyl or an 8-, 9- or 10-membered heteroaromatic bicyclic ring system; or a 5- or 6-membered heterocyclic non-aromatic ring which optionally contains ring members selected from the group consisting of C(=O), C(=S), C(=NR⁴); where each ring or each ring system may optionally be substituted at the ring carbon atoms by up to 3 substituents independently of one another selected from R¹⁷;
R¹⁷ in each case independently of one another represent halogen, cyano, C₁-C₆-alkyl, C₂-C₆-alkenyl, C₂-C₆-alkynyl, C₃-C₆-cycloalkyl, C₁-C₆-haloalkyl, C₁-C₆-alkoxy;
m represents 0, 1 or 2,
W represents H
or
W represents fluorine, chlorine, CN, CF₃, methyl, ethyl, methoxy
and agrochemically active salts thereof.

3. Compounds of the formula **(I)** according to Claim 1 or 2
in which the symbols have the following meanings:
Y together with the adjacent nitrogen atom "1" and the two carbon atoms "2" and "3" forms a 5- to 7-membered non-aromatic heterocyclic ring selected from the group consisting of: H-1, H-2, H-3, H-4, H-5, H-6, H-7, H-8, H-9 and H-10 shown in Scheme 1, where s is a number from 0 to 4; 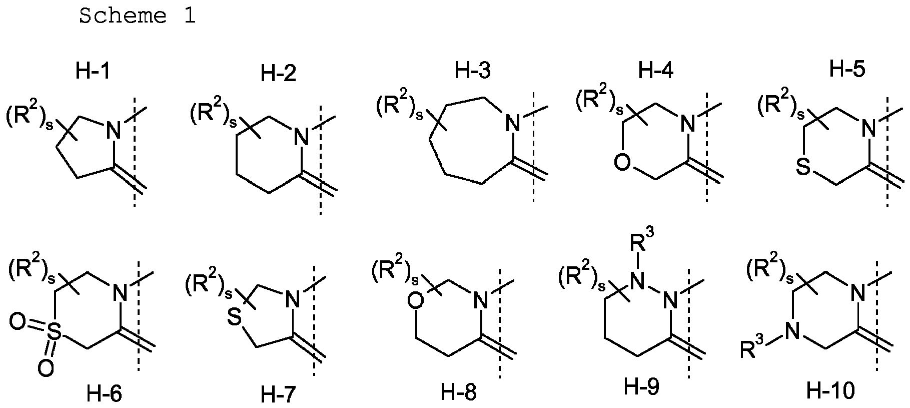
R² in each case independently of one another represent H, F, Cl, Br, I, cyano, hydroxyl, - CHO, -C(=O)OR⁶, methyl, ethyl, isopropyl, n-propyl, trifluoromethyl, difluoromethyl, dichloromethyl, pentafluoroethyl, methoxy, ethoxy, n-propoxy, isopropoxy, trifluoromethoxy, difluoromethoxy or acetyl, propionyl, isobutyryl, 2,2-dimethylpropanoyl;
R³ represents H, -CHO, methyl, ethyl, isopropyl, n-propyl, acetyl, propionyl, isobutyryl, 2,2-dimethylpropanoyl, trifluoroacetyl, difluoroacetyl, CH₃OC(O), CH₃CH₂C(O), (CH₃)₂CHC(O) or CF₃OC(O), CF₂HOC(O);
R⁶ in each case represents H, methyl, ethyl, isopropyl, n-propyl;
A represents a phenyl or thiophene ring which is optionally substituted by radicals selected from the group consisting of F, Cl, Br, I, cyano, methyl, ethyl, isopropyl, n-propyl, trifluoromethyl, difluoromethyl, dichloromethyl, pentafluoroethyl or methoxy, ethoxy, n-propoxy, isopropoxy;
R¹ represents H, F, Cl, Br, I, CH₃, S(O)ₘMe, -NR^{9a}R^{9b}, N (R^{9b}) COR^{9a}, N (R^{9b}) COOR¹²;
R^{9a} in each case represents H, methyl, ethyl, isopropyl, n-propyl, n-butyl, sec-butyl, tert-butyl, isobutyl, n-pentyl, n-hexyl, -CH=CH₂, - CH₂CH=CH₂, -CH=CHCH₃, -CH₂C≡CH, -C≡CH, trifluoromethyl, difluoromethyl, dichloromethyl, pentafluoroethyl, methoxymethyl, ethoxymethyl, methoxyethyl, tert-butoxymethyl, hydroxymethyl, 1-hydroxyethyl, 2-hydroxypropan-2-yl or - (CH₂)ₘR¹⁶;
R^{9b} in each case represents H, methyl, ethyl, isopropyl, n-propyl, n-butyl, sec-butyl, tert-butyl, isobutyl, n-pentyl, n-hexyl, - CH₂CH=CH₂, -CH=CHCH₃, -CH₂C≡CH, -C≡CH;
R^{9a}, R^{9b} in each case together with the nitrogen to which they are attached form a 5- or 6-membered ring which is optionally substituted at the ring carbon atoms by 1 or 2 substituents selected from the group consisting of F, Cl, Br, I, -CN and methyl, ethyl;
R¹² represents methyl, ethyl, isopropyl, n-propyl, n-butyl, sec-butyl, tert-butyl, isobutyl, -CH₂CH=CH₂, -CH=CHCH₃, CH₂C≡CH, -C≡CH, trifluoromethyl, difluoromethyl, dichloromethyl or -(CH₂)ₘR¹⁶;
R¹⁶ represents cyclopropyl, cyclobutyl, cyclopentyl, cyclohexyl, cyclopentenyl, cyclohexenyl, phenyl or thienyl, each of which may optionally be substituted by up to 2 radicals selected from the group R¹⁷;
R¹⁷ in each case independently of one another represent F, Cl, Br, I, methyl, ethyl, isopropyl, n-propyl, n-butyl, sec-butyl, tert-butyl, isobutyl, trifluoromethyl, difluoromethyl, dichloromethyl, methoxy, ethoxy or cyano;
m represents 0, 1 and 2,
W represents H
or
W represents fluorine, chlorine, CN, CF₃, methyl, ethyl
and agrochemically active salts thereof as fungicides.

4. Compounds of the formula **(I)** according to one or more of Claims 1 to 3
in which the symbols have the following meanings:
Y together with the adjacent nitrogen atom "1" and the two carbon atoms "2" and "3" forms a 5- to 7-membered non-aromatic heterocyclic ring selected from the group consisting of: H-1, H-2, H-3, H-4, H-5 and H-8 shown in Scheme 2, where s is a number from 0 to 4; 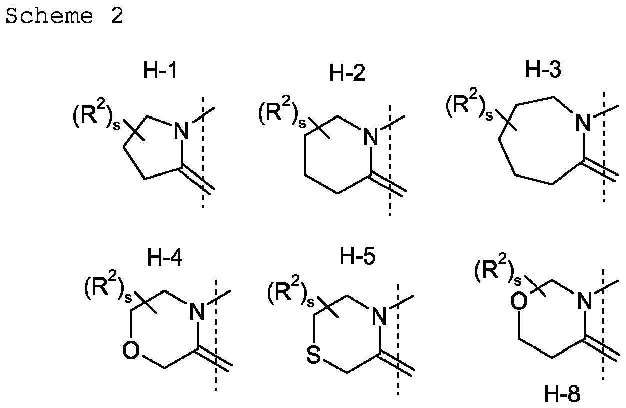
R² in each case independently of one another represent H, fluorine, chlorine, cyano, CF₃, methyl or methoxy;
A represents a phenyl or thiophene ring which is optionally substituted by radicals selected from the group consisting of F, Cl, cyano, CH₃, CF₃;
R¹ represents H, fluorine, chlorine, S(O)ₘMe, NR^{9a}R^{9b}, N(R^{9b})COR^{9a}, N(R^{9b})COOR¹²;
R^{9a} in each case represents H, methyl, ethyl, n-propyl, isopropyl, n-butyl, isobutyl, sec-butyl, tert-butyl, n-pentyl, n-hexyl, methoxymethyl, ethoxymethyl, ethoxyethyl, methoxyethyl, methoxypropyl, hydroxymethyl, 1-hydroxyethyl, 2-hydroxyethyl, hydroxypropyl or -(CH₂)ₘR¹⁶;
R^{9b} in each case represents H, methyl, ethyl, n-propyl, isopropyl, n-butyl, allyl or propargyl;
R¹² represents methyl, ethyl, n-propyl, isopropyl, t-butyl, allyl, propargyl or - (CH₂)ₘR¹⁶;
R¹⁶ represents cyclopropyl, cyclopentyl, cyclobutyl, cyclohexyl, thienyl or phenyl, each of which may be substituted by a radical selected from the group R¹⁷;
m represents 0, 1 or 2;
R¹⁷ represents methyl, ethyl, fluorine, chlorine, CF₃, OMe, cyano;
W represents H,
or
W represents fluorine, chlorine, cyano;
and agrochemically active salts thereof as fungicides.

5. Nontherapeutic method for controlling phytopathogenic and mycotoxin-producing fungi, **characterized in that** bicyclic pyridinylpyrazoles of the formula **(I)** according to one or more of Claims 1 to 4 are applied to the fungi and/or their habitat.

6. Composition for controlling phytopathogenic and mycotoxin-producing fungi, **characterized in that** it comprises at least one bicyclic pyridinylpyrazole of the formula **(I)** according to one or more of Claims 1 to 4, in addition to extenders and/or surfactants.

7. Use of bicyclic pyridinylpyrazoles of the formula **(I)** according to one or more of Claims 1 to 4 for controlling phytopathogenic harmful fungi.

8. Process for preparing compositions for controlling phytopathogenic harmful fungi, **characterized in that** bicyclic pyridinylpyrazoles of the formula **(I)** according to one or more of Claims 1 to 4 are mixed with extenders and/or surfactants.

9. Use of compounds of the formula **(I)** according to one or more of Claims 1 to 4 for treating transgenic plants.

10. Use of compounds of the formula **(I)** according to one or more of Claims 1 to 4 for treating seed and seed of transgenic plants.

## Revendications

1. Composés de formule **(I)**, 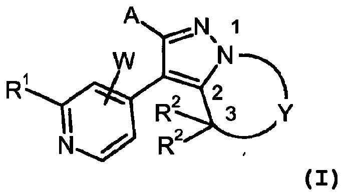 dans laquelle les symboles ont les significations suivantes :
Y forme conjointement avec l'atome d'azote « 1 » adjacent et les deux atomes de carbone « 2 » et « 3 » un cycle hétérocyclique non aromatique à 5 à 7 chaînons, dont les autres chaînons formant le cycle sont choisis dans le groupe constitué par C(R²)₂, O, S, NR³, C(R²)=C(R²), C(R²)=N, N=N, C(=O), C(=S), C(=NR⁴), S(=O)ₚ(N=R⁴)_{q} et SiR^{5a}R^{5b};
R² représente chaque fois indépendamment H, un atome d'halogène, un groupe cyano, hydroxy, -CHO, -NHCHO, -N₃, -N=C=O, -N=C=S, -SH, -C(=O)NH₂, -C(=O)NHCN, -C(=O)OR⁶, -C (=O) NHOR^{6a}, alkyle en C₁-C₅, alcényle en C₂-C₅, alcynyle en C₂-C₅, cycloalkyle en C₃-C₆, halogénocycloalkyle(C₃-C₆), cycloalcényle en C₃-C₆, halogénoalkyle(C₁-C₅), alcoxy en C₁-C₅, halogénoalcoxy(C₁-C₅), cycloalcoxy en C₃-C₆, alcényloxy en C₂-C₅, halogénoalcényloxy(C₃-C₅), alcynyloxy en C₂-C₅, alkylcarbonyle en C₂-C₅, alkylcarbonyl(C₂-C₅)oxy, halogénoalkylcarbonyl(C₂-C₅)oxy, alcoxycarbonyl-alcoxy en C₃-C₅, alkyl(C₁-C₅)thio, halogéno-alkyl(C₁-C₅)thio, cycloalkyl(C₃-C₆)thio, alkyl-(thiocarbonyle) en C₂-C₅, alkylthio (thiocarbonyle) en C₂-C₅, alkyl(C₁-C₅)sulfinyle, halogéno-alkyl(C₁-C₅)sulfinyle, cycloalkyl(C₃-C₆)sulfinyle, alkyl(C₁-C₅)sulfonyle, halogénoalkyl(C₁-C₅)-sulfonyle, cycloalkyl(C₃-C₆)sulfonyle, trialkyl(C₃-C₅)silyle, halogénotrialkyl(C₃-C₅)-silyle, alkyl(C₁-C₅)amino, halogénoalkyl(C₂-C₅)-amino, cycloalkyl(C₃-C₆)amino, dialkyl(C₂-C₅)amino ou halogénodialkyl(C₃-C₅)amino ;
R³ représente H, -CN, -C(=O)NH₂, -C(=O)NHCN, -CHO, -NHCHO, -C(=O)OR⁶, -C(=O)NHOR^{6a}, hydroxy, alkyle en C₁-C₅, alcényle en C₂-C₅, alcynyle en C₂-C₅, cycloalkyle en C₃-C₆, halogénocycloalkyle(C₃-C₆), cycloalcényle en C₃-C₆, cycloalkylalkyle en C₄-C₁₀, alkylcycloalkyle en C₄-C₇, alkylcycloalkylalkyle en C₅-C₇, halogénoalkyle(C₂-C₅), alkyl(C₁-C₄)carbonyle, halogénoalkyl(C₁-C₄)carbonyle, cycloalkyl(C₃-C₇)-carbonyle, alcoxy(C₁-C₆)carbonyle, halogéno-alcoxy(C₁-C₄)carbonyle, cycloalcoxy(C₃-C₆)-carbonyle, alcoxyalkyl(C₂-C₆)carbonyle, alcoxy-alcoxy(C₂-C₆) carbonyle, (alkyl(C₁-C₆)thio)-carbonyle, alcoxy(C₁-C₆)(thiocarbonyle), alkyl (C₁-C₆) (thiocarbonyle), alkyl (C₁-C₆) thio (thiocarbonyle), alkyl(C₁-C₆)aminocarbonyle, cycloalkyl(C₃-C₆)aminocarbonyle, dialkyl(C₂-C₆)-aminocarbonyle, alkyl(C₁-C₆)amino(thiocarbonyle), dialkyl(C₂-C₆)amino(thiocarbonyle), alcoxy-(alkyl)(C₂-C₆)aminocarbonyle, alcoxy en C₁-C₅, halogénoalcoxy(C₁-C₅), alkyl(C₁-C₅)thio, halogéno-alkyl(C₁-C₅)thio, cycloalkyl(C₃-C₆)thio, alkyl(C₁-C₅)aminosulfonyle, trialkyl(C₃-C₅)silyle ou halogénotrialkyl(C₃-C₅)silyle ;
R⁴ représente chaque fois H, un groupe cyano, amino, hydroxy, alkyle en C₁-C₆, cycloalkyle en C₃-C₆, alkyl(C₁-C₄)carbonyle, halogénoalkyl(C₁-C₄)-carbonyle, alcoxy en C₁-C₄, phényle ou benzoyle ;
R^{5a} R^{5b} représentent indépendamment l'un de l'autre un groupe alkyle en C₁-C₄, alcényle en C₂-C₅, alcynyle en C₂-C₅, cycloalkyle en C₃-C₅, halogénocycloalkyle(C₃-C₆), cycloalkylalkyle en C₄-C₇, alkylcycloalkyle en C₄-C₇, alkylcycloalkylalkyle en C₅-C₇, halogénoalkyle(C₁-C₅), alcoxy en C₁-C₅ ou halogénoalcoxy(C₁-C₅) ;
R⁶ représente chaque fois H, un groupe alkyle en C₁-C₆, halogénoalkyle(C₁-C₆), cycloalkyle en C₃-C₆, cycloalkylalkyle en C₄-C₇, alkylcycloalkyle en C₄-C₇ ou benzyle ;
R^{6a} représente chaque fois un groupe alkyle en C₁-C₆, halogénoalkyle(C₁-C₆), cycloalkyle en C₃-C₆, cycloalkylalkyle en C₄-C₇ ou alkylcycloalkyle en C₄-C₇ ;
A représente un cycle phényle, qui peut éventuellement être une plusieurs fois substitué par R⁷ ou représente un cycle thiophényle qui peut éventuellement être une plusieurs fois substitué par R⁸ ;
R⁷ représente chaque fois indépendamment un atome d'halogène, un groupe alkyle en C₁-C₆, alcényle en C₂-C₆, alcynyle en C₂-C₆, cycloalkyle en C₃-C₆, halogénocycloalkyle(C₃-C₆), alkylcycloalkyle en C₄-C₇, halogénoalkyle (C₁-C₆), halogéno-alcényle(C₂-C₆), cyano, nitro, alcoxy en C₁-C₆, halogénoalcoxy(C₁-C₆), alkyl(C₁-C₆)thio, alkyl(C₁-C₆)sulfinyle, alkyl (C₁-C₆) sulfonyle, halogénoalkyl(C₁-C₆)thio, halogénoalkyl(C₁-C₆)-sulfinyle, halogénoalkyl(C₁-C₆)sulfonyle, alkyl (C₁-C₆) amino, dialkyl(C₂-C₆)amino, alkyl(C₁-C₆)carbonyle, alcoxy(C₁-C₆)carbonyle, alkyl(C₁-C₆) aminocarbonyle, dialkyl(C₂-C₆)aminocarbonyle ou trialkyl(C₃-C₆)silyle ;
R⁸ représente chaque fois indépendamment un atome d'halogène, un groupe cyano, alkyle en C₁-C₃ ; alcoxy en C₁-C₃, halogénoalkyle(C₁-C₃), halogénoalcoxy(C₁-C₃) ;
R¹ représente H, un atome d'halogène, un groupe cyano, hydroxy, alkyle en C₁-C₄, CONR^{9a}R^{9b}, COOH, COOR¹², -NR^{9a}R^{9b}, -N (R^{9b}) COR^{9a}, -N(R^{9b}) CSR^{9a}, -N (R^{9b}) COOR¹², -N (R^{9b}) SO₂R¹², -NR¹⁰-NR^{11a}R^{11b}, -S(O)ₘR¹², -OR¹², -N=CR^{13a}R^{13b} ou -NR¹⁰N=CR^{14a}R^{14b} ;
R^{9a} et R^{11a} représentent chacun indépendamment l'un de l'autre H, un groupe alkyle en C₁-C₆, alcényle en C₂-C₆, alcynyle en C₂-C₆, halogénoalkyle(C₁-C₆), halogénoalcényle(C₂-C₆), halogénoalcynyle(C₂-C₆), alcoxyalkyle en C₂-C₆, alcoxyalcoxyalkyle en C₃-C₆, alcoxyalcényle en C₃-C₆, alcoxyalcynyle en C₃-C₆, dialcoxyalkyle en C₃-C₆, trialcoxyalkyle en C₄-C₁₀, halogénoalcoxyalkyle en C₂-C₆, alcoxyhalogénoalkyle en C₂-C₆, halogénoalcoxyhalogénoalkyle en C₂-C₆, hydroxyalkyle(C₁-C₆), cyanoalkyle(C₁-C₁₀), alkylthioalkyle en C₂-C₆, alkylsulfinylalkyle en C₂-C₆, alkylaminoalkyle en C₃-C₆, halogénoalkylaminoalkyle en C₃-C₆, cycloalkylaminoalkyle en C₅-C_{10,} dialkylaminoalkyle en C₄-C₁₀, halogénodialkylaminoalkyle en C₄-C₁₀, cycloalkyl(alkyl)aminoalkyle en C₅-C₁₀ ou -(CR^{15a}R^{15b})ₘR¹⁶ ;
R^{9b} et R^{11b} représentent chacun indépendamment l'un de l'autre H, un groupe alkyle en C₁-C₆, alcényle en C₂-C₆, alcynyle en C₂-C₆, halogénoalkyle(C₁-C₆), halogénoalcényle(C₂-C₆), halogénoalcynyle(C₂-C₆), alcoxyalkyle en C₂-C₆, alcoxyalcoxyalkyle en C₃-C₆, alcoxyalcényle en C₃-C₆, alcoxyalcynyle en C₃-C₆, dialcoxyalkyle en C₃-C₆, trialcoxyalkyle en C₄-C₁₀, halogénoalcoxyalkyle en C₂-C₆, alcoxyhalogénoalkyle en C₂-C₆, halogénoalcoxyhalogénoalkyle en C₂-C₆, hydroxyalkyle(C₁-C₆), cyanoalkyle(C₁-C₁₀), alkylthioalkyle en C₂-C₆, alkylsulfinylalkyle en C₂-C₆, alkylaminoalkyle en C₃-C₆, halogénoalkylaminoalkyle en C₃-C₆, cycloalkylaminoalkyle en C₅-C₁₀, dialkylaminoalkyle en C₄-C₁₀, halogénodialkylaminoalkyle en C₄-C₁₀, cycloalkyl(alkyl)aminoalkyle en C₅-C₁₀ ou -(CR^{15a}R^{15b})ₘR¹⁶ ;
ou
R^{9a} et R^{9b} ou respectivement R^{11a} et R^{11b}
forment chaque fois avec l'atome d'azote ou l'unité (NCO) ou l'unité (NCS), auquel/à laquelle ils sont liés, un cycle à 3 à 6 chaînons, qui peut éventuellement contenir additionnellement des chaînons formant le cycle, choisis dans le groupe constitué par O, NR³, C(=O), C(=S), C(=NR⁴), SiR^{5a}R^{5b} et S(=O)ₚ(=NR⁴)_{q}, et qui peut éventuellement être substitué sur les atomes de carbone formant le cycle par 1 à 4 substituants choisis dans le groupe constitué par halogéno, -CN, alkyle en C₁-C₂ et alcoxy en C₁-C₂ ;
R¹² représente chaque fois un groupe alkyle en C₁-C_{10,} alcényle en C₂-C₁₀, alcynyle en C₂-C₁₀, halogénoalkyle(C₁-C₁₀), halogénoalcényle(C₂-C₁₀), halogénoalcynyle (C₂-C₁₀), alcoxyalkyle en C₂-C₁₀, alcoxyalcoxyalkyle en C₃-C₁₀, alcoxyalcényle en C₃-C₁₀, alcoxyalcynyle en C₃-C₁₀, dialcoxyalkyle en C₃-C₁₀, trialcoxyalkyle en C₄-C₁₀, halogénoalcoxyalkyle en C₂-C₁₀, alcoxyhalogénoalkyle en C₂-C₁₀, halogénoalcoxyhalogénoalkyle en C₂-C₁₀, hydroxy-alkyle(C₂-C₁₀), cyanoalkyle(C₂-C₁₀), alkylthioalkyle en C₂-C₁₀, alkylsulfinylalkyle en C₂-C₁₀, alkylaminoalkyle en C₃-C₁₀, halogénoalkylaminoalkyle en C₃-C₁₀, cycloalkylaminoalkyle en C₅-C₁₀, dialkylaminoalkyle en C₄-C₁₀, halogénodialkylaminoalkyle en C₄-C₁₀, cycloalkyl(alkyl)aminoalkyle en C₆-C₁₀, ou -(CR^{15a}R^{15b})ₘR¹⁶ ;
R^{15a}, R^{15b} représentent indépendamment l'un de l'autre H, un atome d'halogène, un groupe alkyle en C₁-C₅, halogénoalkyle en C₁-C₅ ou alcoxy en C₁-C₅ ;
ou
une paire géminée de R^{15a} et R^{15b} forme conjointement avec l'atome de carbone auquel ils sont liés, C(=O) ou un cycle cycloalkyle en C₃-C₆ ou un cycle halogénocycloalkyle en C₃-C₆ ;
R¹⁶ représente un groupe phényle, cycloalkyle en C₃-C₈, cycloalcényle en C₃-C₈, un cycle hétéroaromatique à 5 ou 6 chaînons ou naphtalényle ou un système cyclique bicyclique hétéroaromatique à 8, 9 ou 10 chaînons ; ou un cycle hétérocyclique non aromatique à 5 ou 6 chaînons, qui contient éventuellement des chaînons formant le cycle, choisis dans le groupe constitué par C(=O), C(=S), C(=NR⁴), SiR^{5a}R^{5b} et S(=O)ₚ(=NR⁴)_{q} ; chaque cycle ou chaque système cyclique peut éventuellement être substitué sur les atomes de carbone formant le cycle par jusqu'à 5 substituants choisis indépendamment les uns des autres dans R¹⁷ ;
R¹⁷ représente chaque fois indépendamment un atome d'halogène un groupe alkyle en C₁-C₆, alcényle en C₂-C₆, alcynyle en C₂-C₆, cycloalkyle en C₃-C₆, alkylcycloalkyle en C₄-C₁₀, halogénocycloalkyle en C₃-C₆, halogénoalkyle en C₁-C₆, halogénoalcényle en C₂-C₆, cyano, nitro, alcoxy en C₁-C₆, cycloalcoxy en C₃-C₈, halogénocycloalcoxy(C₃-C₈), halogéno-alcoxy(C₁-C₆), alkyl (C₁-C₆) thio, alkyl (C₁-C₆)-sulfinyle, alkyl(C₁-C₆)sulfonyle, halogéno-alkyl(C₁-C₆)thio, halogénoalkyl(C₁-C₆)sulfinyle, halogénoalkyl(C₁-C₆)sulfonyle, alkyl(C₁-C₆)amino, dialkyl(C₁-C₆) amino, alkyl(C₁-C₆)carbonyle, alcoxy(C₁-C₆)carbonyle, alyl(C₁-C₆)aminocarbonyle, dialkylaminocarbonyle en C₃-C₆, trialkyl(C₃-C₆)-silyle, phényle, naphtalényle ou un cycle hétéroaromatique à 5 ou 6 chaînons ;
m représente 0, 1 ou 2 ;
R¹⁰ représente chaque fois H, un groupe alkyle en C₁-C₅, alcényle en C₂-C₅, alcynyle en C₂-C₅, cycloalkyle en C₃-C₆, halogénocycloalkyle en C₃-C₆, halogénoalkyle en C₁-C₅, halogénoalcényle en C₂-C₅, halogénoalcynyle en C₂-C₅, alcoxyalkyle en C₂-C₅, alkylcarbonyle en C₂-C₅ ou alcoxy en C₁-C₅ ;
R^{13a}, R^{13b} représentent indépendamment l'un de l'autre H, -CN, -C(=O)OR¹⁸, un groupe alkyle en C₁-C₆, alcényle en C₂-C₆, alcynyle en C₂-C₆, cycloalkyle en C₃-C₈, halogénocycloalkyle(C₃-C₈), cycloalcényle en C₃-C₈, cycloalkylalkyle en C₄-C₁₀, alkylcycloalkyle en C₄-C₁₀, alkylcycloalkylalkyle en C₅-C₁₀, alkyl(C₁-C₆)amino, dialkyl(C₂-C₆)amino, alkylaminoalkyle en C₂-C₆, halogénoalkylaminoalkyle en C₂-C₆, cycloalkylaminoalkyle en C₄-C₆, dialkylaminoalkyle en C₃-C₆, halogénodialkylaminoalkyle en C₃-C₆, cycloalkyl(alkyl)aminoalkyle en C₅-C₁₀, alcoxy en C₁-C₆, halogénoalcoxy(C₁-C₆), cycloalcoxy(C₃-C₁₀), alkyl (C₁-C₁₀) thio, halogéno-alkyl(C₁-C₁₀)thio, cycloalkyl (C₃-C₁₀)thio, trialkyl(C₃-C₁₀)silyle ou halogénotrialkyl(C₃-C₁₀)-silyle ; ou phényle ou un cycle hétéroaromatique à 5 ou 6 chaînons, un système cyclique bicyclique hétéroaromatique à 8, 9 ou 10 chaînons, ou un cycle hétérocyclique non aromatique à 5 ou 6 chaînons, qui contient éventuellement des chaînons formant le cycle, choisis dans le groupe constitué par NR³, C(=O), C(=S), C(=NR⁴), SiR^{5a}R^{5b} et S(=O)ₚ(=NR⁴)_{q} ; chaque cycle ou chaque système cyclique peut éventuellement être substitué sur les atomes de carbone formant le cycle par 1 à 5 substituants choisis dans le groupe constitué par alkyle en C₁-C₃, halogéno, -CN et alcoxy en C₁-C₃ ;
ou
R^{13a} et R^{13b} forment ensemble avec l'atome de carbone, auquel ils sont liés, un cycle à 3 à 6 chaînons, ledit cycle peut éventuellement contenir des chaînons formant le cycle, choisis dans le groupe constitué par NR³, C(=O), C(=S), C(=NR⁴), SiR^{5a}R^{5b} ou S(=O)ₚ(=NR⁴)_{q} et peut éventuellement être substitué sur les atomes de carbone formant le cycle par 1 à 4 substituants choisis dans le groupe constitué par alkyle en C₁-C₂, halogéno, -CN et alcoxy en C₁-C₂ ;
R^{14a}, R^{14b} représentent indépendamment l'un de l'autre H, -CN, -C(=O)OR¹⁸, un groupe alkyle en C₁-C₆, alcényle en C₂-C₆, alcynyle en C₂-C₆, cycloalkyle en C₃-C₈, halogénocycloalkyle(C₃-C₈), cycloalcényle en C₃-C₈, cycloalkylalkyle en C₄-C₁₀, alkylcycloalkyle en C₄-C₁₀, alkylcycloalkylalkyle en C₅-C₁₀, alkyl(C₁-C₆)amino, dialkyl (C₂-C₆) amino, alkylaminoalkyle en C₂-C₆, halogénoalkylaminoalkyle en C₂-C₆, cycloalkylaminoalkyle en C₄-C₆, dialkylaminoalkyle en C₃-C₆, halogénodialkylaminoalkyle en C₃-C₆, cycloalkyl(alkyl)aminoalkyle en C₅-C₁₀, alcoxy en C₁-C₆, halogénoalcoxy(C₁-C₆), cycloalcoxy(C₃-C₁₀) alkyl(C₁-C₁₀)thio, halogéno-alkyl(C₁-C₁₀)thio, cycloalkyl(C₃-C₁₀)thio, trialkyl(C₃-C₁₀)silyle ou halogénotrialkyl(C₃-C₁₀)-silyle ; ou phényle ou un cycle hétéroaromatique à 5 ou 6 chaînons, un système cyclique bicyclique hétéroaromatique à 8, 9 ou 10 chaînons, ou un cycle hétérocyclique non aromatique à 5 ou 6 chaînons, qui contient éventuellement des chaînons formant le cycle, choisis dans le groupe constitué par NR³, C(=O), C(=S), C(=NR⁴), SiR^{5a}R^{5b} et S(=O)ₚ(=NR⁴)_{q} ; chaque cycle ou chaque système cyclique peut éventuellement être substitué sur les atomes de carbone formant le cycle par 1 à 5 substituants choisis dans le groupe constitué par alkyle en C₁-C₃, halogéno, -CN et alcoxy en C₁-C₃ ;
ou
R^{14a}, R^{14b} forment ensemble avec l'atome de carbone, auquel ils sont liés, un cycle à 3 à 6 chaînons, ledit cycle peut éventuellement contenir des chaînons formant le cycle, choisis dans le groupe constitué par NR³, C(=O), C(=S), C(=NR⁴), SiR^{5a}R^{5b} ou S(=O)ₚ(=NR⁴)_{q} et peut éventuellement être substitué sur les atomes de carbone formant le cycle par 1 à 4 substituants choisis dans le groupe constitué par alkyle en C₁-C₂, halogéno, -CN et alcoxy en C₁-C₂ ;
p, q représentent indépendamment l'un de l'autre 0, 1 ou 2, étant entendu que la somme de p et q est égale à 1 ou 2 ;
R¹⁸ représente un groupe alkyle en C₁-C₆, halogénoalkyle en C₁-C₆, cycloalkyle en C₃-C₆, cycloalkylalkyle en C₄-C₇ ou alkylcycloalkyle en C₄-C₇,
W représente H
ou
W représente un atome d'halogène, CN, un groupe halogénoalkyle(C₁-C₄), alkyle en C₁-C₄, alcoxy en C₁-C₄,
ainsi que leurs sels à efficacité agrochimique.

2. Composés de formule **(I)** selon la revendication 1,
formule dans laquelle les symboles ont les significations suivantes :
Y forme conjointement avec l'atome d'azote « 1 » adjacent et les deux atomes de carbone « 2 » et « 3 » un cycle hétérocyclique non aromatique à 5 à 7 chaînons, dont les autres chaînons formant le cycle sont choisis dans le groupe constitué par C(R²)₂, O, S, SO₂, NR³, -C(R²)=C(R²)-, C(=O) et C (=S) ;
R² représente chaque fois indépendamment H, un atome d'halogène, un groupe cyano, hydroxy, -CHO, -C(=O)OR⁶, -C(=O)NHOR^{6a}, alkyle en C₁-C₅, alcényle en C₂-C₅, alcynyle en C₂-C₅, cycloalkyle en C₃-C₆, halogénocycloalkyle(C₃-C₆), cycloalcényle en C₃-C₆, halogénoalkyle(C₁-C₅), alcoxy en C₁-C₅, halogénoalcoxy(C₁-C₅), cycloalcoxy en C₃-C₆, alcényloxy en C₂-C₅, halogénoalcényloxy(C₃-C₅), alcynyloxy en C₂-C₅, alkylcarbonyle en C₂-C₅, alkyl(C₁-C₅)thio, halogénoalkyl(C₁-C₅)thio ou cycloalkyl(C₃-C₆)thio ;
R³ représente H, -CN, -C(=O)NH₂, -C(=O)NHCN, -CHO, - C(=O)OR⁶, -C(=O) NHOR^{6a}, alkyle en C₁-C₅, halogénoalkyle (C₂-C₅), alkyl(C₁-C₄)carbonyle, halogénoalkyl(C₁-C₄)carbonyle, cycloalkyl(C₃-C₆)-carbonyle, alcoxy(C₁-C₄)carbonyle, halogéno-alcoxy(C₁-C₄)carbonyle, cycloalcoxy(C₃-C₆)-carbonyle, alcoxyalkyl(C₃-C₆)carbonyle, alcoxy-alcoxy(C₃-C₆)carbonyle, (alkyl(C₁-C₄)thio) - carbonyle, alcoxy(C₁-C₄)(thiocarbonyle), alkyl (C₁-C₄) (thiocarbonyle), alkyl (C₁-C₄) thio (thiocarbonyle), alkyl(C₁-C₄)aminocarbonyle, cycloalkyl(C₃-C₆)aminocarbonyle, dialkyl(C₂-C₆)-aminocarbonyle, alkyl(C₂-C₆)amino(thiocarbonyle), dialkyl(C₂-C₆)amino(thiocarbonyle) ou alcoxy-(alkyl)(C₃-C₆)aminocarbonyle ;
R⁶ représente chaque fois H ou un groupe alkyle en C₁-C₄ ;
R^{6a} représente chaque fois un groupe alkyle en C₁-C₄ ;
A représente un cycle phényle qui peut éventuellement être une plusieurs fois substitué par R⁷ ou représente un cycle thiophényle qui peut éventuellement être une plusieurs fois substitué par R⁸ ;
R⁷ représente chaque fois indépendamment un atome d'halogène, un groupe alkyle en C₁-C₄, halogénoalkyle(C₁-C₄), cyano, alcoxy en C₁-C₄, halogénoalcoxy(C₁-C₄), alkyl(C₁-C₄)thio, alkyl(C₁-C₄)sulfinyle, alkyl(C₁-C₄)sulfonyle ou halogénoalkyl(C₁-C₄)thio, ;
R⁸ représente chaque fois indépendamment un atome d'halogène, un groupe cyano, alkyle en C₁-C₃ ; alcoxy en C₁-C₃, halogénoalkyle(C₁-C₃) ;
R¹ représente H, un atome d'halogène, un groupe alkyle en C₁-C₃, cyano, -NR^{9a}R^{9b}, -N(R^{9b}) COR^{9a}, -N(R^{9b})CSR^{9a}, -N(R^{9b})COOR¹², -OR¹², -S(O)ₘR^{6a}, COOR¹² ou -CONR^{9a}R^{9b} ;
R^{9a} représente chaque fois H, un groupe alkyle en C₁-C₆, alcényle en C₂-C₆, alcynyle en C₂-C₆, halogénoalkyle(C₁-C₆), halogénoalcényle(C₂-C₆), halogénoalcynyle(C₂-C₆), alcoxyalkyle en C₂-C₆, alcoxyalcoxyalkyle en C₃-C₆, alcoxyalcényle en C₃-C₆, alcoxyalcynyle en C₃-C₆, dialcoxyalkyle en C₃-C₆, halogénoalcoxyalkyle en C₂-C₆, alcoxyhalogénoalkyle en C₂-C₆, halogénoalcoxyhalogénoalkyle en C₂-C₆, hydroxyalkyle(C₁-C₆), cyanoalkyle(C₁-C₆), alkylthioalkyle en C₂-C₆, alkylsulfinylalkyle en C₂-C₆, alkylaminoalkyle en C₃-C₆, halogénoalkylaminoalkyle en C₃-C₆, cycloalkylaminoalkyle en C₅-C₁₀, dialkylaminoalkyle en C₄-C₁₀, halogénodialkylaminoalkyle en (C₄-C₁₀) ou -(CR^{15a}R^{15b})ₘR¹⁶ ;
R^{9b} représente chaque fois H, un groupe alkyle en C₁-C₆, alcényle en C₂-C₆, alcynyle en C₂-C₆, halogénoalkyle (C₁-C₆) ou - (CR^{15a}R^{15b})ₘR¹⁶ ;
ou
R^{9a}, R^{9b} forment chaque fois ensemble avec l'atome d'azote ou l'unité (NCO) ou l'unité (NCS), auquel/à laquelle ils sont liés, un cycle à 3 à 6 chaînons, qui peut éventuellement contenir en outre également des chaînons formant le cycle, choisis dans le groupe constitué par NR³, C(=O), C(=S), O et est éventuellement substitué sur les atomes de carbone formant le cycle par 1 à 4 substituants choisis dans le groupe constitué par halogéno, -CN, alkyle en C₁-C₂ et alcoxy en C₁-C₂ ;
R¹² représente chaque fois un groupe alkyle en C₁-C₆, alcényle en C₂-C₆, alcynyle en C₂-C₆, halogénoalkyle(C₁-C₆), halogénoalcényle (C₂-C₆) ou -(CR^{15a}R^{15b})ₘR¹⁶ ;
R^{15a}, R^{15b} représentent indépendamment l'un de l'autre H, un atome d'halogène ou un groupe alkyle en C₁-C₄ ;
R¹⁶ représente un groupe phényle, cycloalkyle en C₃-C₈, cycloalcényle en C₃-C₈, un cycle hétéroaromatique à 5 ou 6 chaînons ou naphtalényle ou un système cyclique bicyclique hétéroaromatique à 8, 9 ou 10 chaînons ; ou un cycle hétérocyclique non aromatique à 5 ou 6 chaînons, qui contient éventuellement des chaînons formant le cycle, choisis dans le groupe constitué par C(=O), C(=S), C(=NR⁴) ; chaque cycle ou chaque système cyclique peut éventuellement être substitué sur les atomes de carbone formant le cycle par jusqu'à 3 substituants choisis chacun indépendamment dans R¹⁷ ;
R¹⁷ représente chaque fois indépendamment un atome d'halogène un groupe cyano, alkyle en C₁-C₆, alcényle en C₂-C₆, alcynyle en C₂-C₆, cycloalkyle en C₃-C₆, halogénoalkyle en C₁-C₆, alcoxy en C₁-C₆ ;
m représente 0, 1 ou 2 ;
W représente H
ou
W représente un atome de fluor, de chlore, CN, CF₃, le groupe méthyle, éthyle, méthoxy,
ainsi que leurs sels à efficacité agrochimique.

3. Composés de formule **(I)** selon une ou plusieurs des revendications 1 et 2,
formule dans laquelle les symboles ont les significations suivantes :
Y forme conjointement avec l'atome d'azote « 1 » adjacent et les deux atomes de carbone « 2 » et « 3 » un cycle hétérocyclique non aromatique à 5 à 7 chaînons, choisi dans le groupe constitué par : H-1, H-2, H-3, H-4, H-5, H-6, H-7, H-8, H-9 et H-10 représentés dans le schéma 1, s étant un nombre compris entre 0 et 4 ; 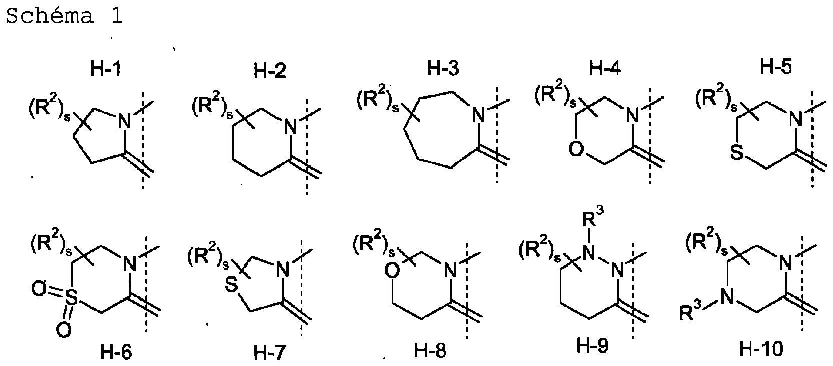
R² représente chaque fois indépendamment H, F, Cl, Br, I, un groupe cyano, hydroxy, -CHO, -C(=O)OR⁶, méthyle, éthyle, isopropyle, n-propyle, trifluorométhyle, difluorométhyle, dichlorométhyle, pentafluoroéthyle, méthoxy, éthoxy, n-propoxy, isopropoxy, trifluorométhoxy, difluoro-méthoxy, ou acétyle, propionyle, isobutyryle, 2,2-diméthylpropanoyle ;
R³ représente H, -CHO, méthyle, éthyle, isopropyle, n-propyle, acétyle, propionyle, isobutyryle, 2,2-diméthylpropanoyle, trifluoroacétyle, difluoroacétyle, CH₃OC(O), CH₃CH₂C(O), (CH₃)₂CHC(O) ou CF₃OC(O), CF₂HOC(O) ;
R⁶ représente chaque fois H, le groupe méthyle, éthyle, isopropyle, n-propyle ;
A représente un cycle phényle ou thiophène qui est éventuellement substitué par des radicaux choisis parmi F, Cl, Br, I, cyano, méthyle, éthyle, isopropyle, n-propyle, trifluorométhyle, difluorométhyle, dichlorométhyle, pentafluoroéthyle, ou méthoxy, éthoxy, n-propoxy, isopropoxy ;
R¹ représente H, F, Cl, Br, I, CH₃, S(O)ₘMe, -NR^{9a}R^{9b}, N (R^{9b}) COR^{9a}, N(R^{9b})COOR¹² ;
R^{9a} représente chaque fois H, un groupe méthyle, éthyle, isopropyle, n-propyle, n-butyle, sec-butyle, tert-butyle, isobutyle, n-pentyle, n-hexyle, -CH=CH₂, -CH₂CH=CH₂, -CH=CHCH₃, -CH₂C≡CH, -C≡CH, trifluorométhyle, difluorométhyle, dichlorométhyle, pentafluoroéthyle, méthoxyméthyle, éthoxyméthyle, méthoxyéthyle, tert-butoxyméthyle, hydroxyméthyle, 1-hydroxyéthyle, 2-hydroxypropane-2-yle ou -(CH₂)ₘR¹⁶ ;
R^{9b} représente chaque fois H, le groupe méthyle, éthyle, isopropyle, n-propyle, n-butyle, sec-butyle, tert-butyle, isobutyle, n-pentyle, n-hexyle, -CH₂CH=CH₂, -CH=CHCH₃, -CH₂C≡CH, -C≡CH ;
R^{9a}, R^{9b} forment chaque fois ensemble avec l'atome d'azote, auquel ils sont liés, un cycle à 5 à 6 chaînons, qui est éventuellement substitué sur les atomes de carbone formant le cycle par 1 ou 2 substituants choisis dans le groupe constitué par F, Cl, Br, I, -CN et méthyle, éthyle ;
R¹² représente un groupe méthyle, éthyl, isopropyle, n-propyle, n-butyle, sec-butyle, tert-butyle, isobutyle, -CH₂CH=CH₂, -CH=CHCH₃, CH₂C≡CH, -C≡CH, trifluorométhyle, difluorométhyle, dichlorométhyle, ou -(CH₂)ₘR¹⁶ ;
R¹⁶ représente un groupe cyclopropyle, cyclobutyle, cyclopentyle, cyclohexyle, cyclopentényle, cyclohexényle, phényle ou thiényle, dont chacun peut éventuellement être substitué par jusqu'à 2 radicaux choisis dans le groupe R¹⁷;
R¹⁷ représente chaque fois indépendamment F, Cl, Br, I, le groupe méthyle, éthyle, isopropyle, n-propyle, n-butyle, sec-butyle, tert-butyle, isobutyle, trifluorométhyle, difluorométhyle, dichlorométhyle, méthoxy, éthoxy ou cyano ;
m représente 0, 1 ou 2 ;
W représente H
ou
W représente un atome de fluor, de chlore, CN, CF₃, le groupe méthyle, éthyle,
ainsi que leurs sels à efficacité agrochimique, en tant que fongicides.

4. Composés de formule **(I)** selon une ou plusieurs des revendications 1 à 3,
formule dans laquelle les symboles ont les significations suivantes :
Y forme conjointement avec l'atome d'azote « 1 » adjacent et les deux atomes de carbone « 2 » et « 3 » un cycle hétérocyclique non aromatique à 5 à 7 chaînons, choisi dans le groupe constitué par : H-1, H-2, H-3, H-4, H-5 et H-8 représentés dans le schéma 2, s étant un nombre compris entre 0 et 4 ; 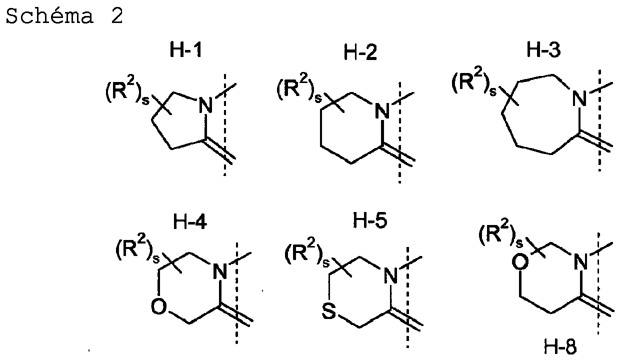
R² représente chaque fois indépendamment H, un atome de fluor, de clore, le groupe cyano, CF₃, méthyle ou méthoxy ;
A représente un cycle phényle ou thiophène, qui est éventuellement substitué par des radicaux choisis parmi F, Cl, cyano, CH₃, CF₃ ;
R¹ représente H, un atome de fluor, de chlore, S(O)ₘMe, NR^{9a}R^{9b}, N(R^{9b})COR^{9a}, N(R^{9b})COOR¹² ;
R^{9a} représente chaque fois H, un groupe méthyle, éthyle, n-propyle, isopropyle, n-butyle, isobutyle, sec-butyle, tert-butyle, n-pentyle, n-hexyle, méthoxyméthyle, éthoxyméthyle, éthoxyéthyle, méthoxyéthyle, méthoxypropyle, hydroxyméthyle, 1-hydroxyéthyle, 2-hydroxyéthyle, hydroxypropyle ou -(CH₂)ₘR¹⁶ ;
R^{9b} représente chaque fois H, le groupe méthyle, éthyle, n-propyle, isopropyle, n-butyle, allyle ou propargyle ;
R¹² représente un groupe méthyle, éthyle, n-propyle, isopropyle, tert-butyle, allyle, propargyle ou -(CH₂)ₘR¹⁶ ;
R¹⁶ représente un groupe cyclopropyle, cyclopentyle, cyclobutyle, cyclohexyle, thiényle ou phényle, dont chacun peut être éventuellement substitué par un radical choisi dans le groupe R¹⁷ ;
m représente 0, 1 ou 2 ;
R¹⁷ représente le groupe méthyle, éthyle, un atome de fluor, de chlore, CF₃, OMe, cyano ;
W représente H,
ou
W représente un atome de fluor, de chlore, le groupe cyano ;
ainsi que leurs sels à efficacité agrochimique, en tant que fongicides.

5. Procédé non thérapeutique pour la lutte contre des champignons phytopathogènes et producteurs de mycotoxines, **caractérisé en ce qu'**on applique sur les champignons et/ou leur habitat des pyridinylpyrazoles bicycliques de formule **(I)** selon une ou plusieurs des revendications 1 à 4.

6. Composition destinée à la lutte contre des champignons phytopathogènes et producteurs de mycotoxines, **caractérisée par** une teneur en au moins un pyridinylpyrazole bicyclique de formule **(I)** selon une ou plusieurs des revendications 1 à 4, en plus d'excipients et/ou de substances tensioactives.

7. Utilisation de pyridinylpyrazoles bicycliques de formule **(I)** selon une ou plusieurs des revendications 1 à 4, pour la lutte contre des champignons nuisibles phytopathogènes.

8. Procédé pour la préparation de compositions destinées à la lutte contre des champignons nuisibles phytopathogènes, **caractérisé en ce qu'**on mélange des pyridinylpyrazoles bicycliques de formule **(I)** selon une ou plusieurs des revendications 1 à 4 avec des excipients et/ou des substances tensioactives.

9. Utilisation de composés de formule **(I)** selon une ou plusieurs des revendications 1 à 4, pour le traitement de plantes transgéniques.

10. Utilisation de composés de formule **(I)** selon une ou plusieurs des revendications 1 à 4, pour le traitement de semences ainsi que de semences de plantes transgéniques.
